# EUROPÄISCHE PATENTSCHRIFT

(11) **EP 2 234 985 B1**
(45) Veröffentlichungstag und Bekanntmachung des Hinweises auf die Patenterteilung: **07.03.2012**
(21) Anmeldenummer: 08864824.1
(22) Anmeldetag: 09.12.2008
(51) Int. Cl.: C07D 239/22, A61K 31/505, A61P 11/00

(54) **4- (4-CYANO-2-THIOARYL) -DIHYDROPYRIMIDINONE UND IHRE VERWENDUNG**
4-(4-CYANO-2-THIOARYL)DIHYDROPYRIMIDINONES AND USE THEREOF
4-(4-CYANO-2-THIOARYLE)-DIHYDROPYRIMIDINONE ET SON UTILISATION

(30) Priorität: 20.12.2007 DE 102007061766; 07.05.2008 DE 102008022521; 17.10.2008 DE 102008052013
(43) Veröffentlichungstag der Anmeldung: 06.10.2010
(73) Patentinhaber: Bayer Pharma Aktiengesellschaft, 13353 Berlin (DE)
(72) Erfinder: VON NUSSBAUM, Franz, 40219 Düsseldorf (DE); KARTHAUS, Dagmar, 42697 Solingen (DE); ANLAUF, Sonja, 42115 Wuppertal (DE); DELBECK, Martina, 42579 Heiligenhaus (DE); LI, Volkhart Min-Jian, 42553 Velbert (DE); MEIBOM, Daniel, 51373 Leverkusen (DE); LUSTIG, Klemens, 42113 Wuppertal (DE); SCHAMBERGER, Jens, 42115 Wuppertal (DE)
(86) Internationale Anmeldenummer: PCT/EP2008/010411
(87) Internationale Veröffentlichungsnummer: WO 2009/080199

(56) Entgegenhaltungen:
- WO-A-2004/024700
- WO-A-2005/082863
- WO-A-2005/082864

## Beschreibung

Die vorliegende Anmeldung betrifft neue 4-(4-Cyano-2-thioaryl)-dihydropyrimidin-2-on-Derivate, Verfahren zu ihrer Herstellung, ihre Verwendung allein oder in Kombinationen zur Behandlung und/oder Prävention von Krankheiten sowie ihre Verwendung zur Herstellung von Arzneimittel zur Behandlung und/oder Prävention von Krankheiten, insbesondere zur Behandlung und/oder Prävention von Erkrankungen der Lunge und des Herz-Kreislauf-Systems.

Die Humane Leukozyten-Elastase (HLE, EC 3.4.21.37), auch Humane Neutrophile Elastase (HNE, hNE) genannt, gehört zur Familie der Serinproteasen. Das proteolytische Enzym findet sich in den azurophilen Granula der polymorphkemigen Leukozyten (engl. *polymorphonuclear leukocytes, PMN leukocytes*). Die intrazelluläre Elastase nimmt eine wichtige Funktion in der Pathogenabwehr wahr, indem über Phagozytose aufgenommene Fremdpartikel abgebaut werden. Aktivierte neutrophile Zellen setzen die HNE aus den Granula in den Extrazellulärraum frei (extrazelluläre HNE), wobei ein Teil der freigesetzten HNE an der Außenseite der neutrophilen Zellmembran verbleibt (membranständige HNE). Das hochaktive Enzym ist in der Lage, eine Vielzahl von Bindegewebsproteinen abzubauen, z.B. die Proteine Elastin, Kollagen und Fibronektin. Elastin kommt in hohen Konzentrationen in allen Gewebetypen vor, die eine hohe Elastizität zeigen, z.B. in der Lunge und in Arterien. Bei einer Vielzahl von pathologischen Prozessen (z.B. Gewebeverletzungen) spielt die HNE eine Rolle beim Gewebeab- und -umbau (engl. *tissue remodeling*). Darüber hinaus ist die HNE ein wichtiger Modulator bei entzündlichen Prozessen. HNE induziert beispielsweise eine erhöhte Genexpression von Interleukin-8 (IL-8).

Es wird daher angenommen, dass die HNE bei vielen Erkrankungen, Verletzungen und pathologischen Veränderungen, deren Entstehung und/oder Progression mit einem entzündlichen Geschehen und/oder einem proliferativen und hypertrophen Gewebe- und Gefäßumbau in Zusammenhang steht, eine wichtige Rolle spielt. Dies können insbesondere Erkrankungen und/oder Schädigungen der Lunge oder des Herz-Kreislauf-Systems sein, oder es kann sich hierbei um eine Sepsis, um Krebs-Erkrankungen oder um andere entzündliche Erkrankungen handeln.

In diesem Zusammenhang zu nennende Erkrankungen und Schädigungen der Lunge sind insbesondere die chronisch-obstruktive Lungenerkrankung (engl. *chronic obstructive pulmonary disease,* COPD), das akute Atemwegssyndrom (engl. *acute respiratory distress syndrome,* ARDS), die zystische Fibrose (engl. *cystic fibrosis,* CF; auch Mukoviszidose genannt), das Lungenemphysem (engl. *lung emphysema*) und die akute Lungenschädigung (engl. *acute lung injury,* ALI). Erkrankungen und Schädigungen des Herz-Kreislauf-Systems, in denen die HNE involviert ist, sind zum Beispiel Gewebeveränderungen bei einer Herzinsuffizienz und Reperfusionsschäden nach einem Myokardinfarkt (engl. *acute myocardial infarct,* AMI), der kardiogene Schock, das akute Koronarsyndrom (engl. *acute coronary syndrome,* ACS) sowie Aneurysmen. Erkrankungen in Zusammenhang mit einer Sepsis sind beispielsweise eine systemische entzündliche Reaktion (engl. *systemic inflammatory response syndrome,* SIRS), die schwere Sepsis, der septische Schock und das multiple Organversagen (engl. *multi-organ failure,* MOF; *multi-organ dysfunction,* MODS) sowie die intravaskuläre Gerinnung (engl. *disseminated intravascular coagulation,* DIC). Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Andere entzündliche Krankheiten, bei denen die HNE eine Rolle spielt, sind rheumatoide Erkrankungen, zum Beispiel die rheumatoide Arthritis, chronische Darmentzündungen (engl. *inflammatory bowel disease,* IBD; Morbus Crohn, engl. *Crohn's disease,* CD; Colitis ulcerosa, engl. *ulcerative colitis,* UC) und die Arteriosklerose.

Im Allgemeinen geht man davon aus, dass Elastase-vermittelten pathologischen Prozessen ein verschobenes Gleichgewicht zugrunde liegt zwischen der freien Elastase und dem körpereigenen Elastase-Inhibitorprotein (hauptsächlich das alpha-1-Antitrypsin, AAT) [Neutrophils and protease/antiprotease imbalance, Stockley, Am. J. Respir. Crit. Care Med. 160, 49-52 (1999)]. AAT liegt im Plasma im hohen Überschuss vor und neutralisiert so sehr schnell freie HNE. In verschiedenen pathologischen Prozessen ist die Konzentration an freier Elastase erhöht, so dass lokal die Balance zwischen Protease und Protease-Inhibitor zu Gunsten der Protease verschoben ist. Zudem ist die membranständige Elastase der aktivierten PMN-Zellen vor einer Inhibition durch AAT weitestgehend geschützt. Gleiches gilt für die freie Elastase, die sich in einem erschwert zugänglichen Mikrokompartiment zwischen der neutrophilen Zelle und der angrenzenden Gewebezelle (z.B. Endothelzelle) befindet. Zusätzlich herrschen stark oxidierende Bedingungen im Umfeld von aktivierten Leukozyten (engl. *oxidative burst*), wodurch AAT oxidiert wird und in der inhibitorischen Wirkung mehrere Größenordnungen verliert.

Neue Elastase-inhibierende Wirkstoffe (exogen verabreichte Inhibitoren der HNE) sollten demnach ein niedriges Molekulargewicht aufweisen, um in der Lage zu sein, auch die membranständige HNE und die im geschützten Mikrokompartiment befindliche HNE (s.o.) zu erreichen und zu inhibieren. Hierzu ist auch eine gute Stabilität der Substanzen *in vivo* notwendig (geringe *in vivo*-Clearance). Außerdem sollten diese Verbindungen stabil sein unter oxidativen Bedingungen, um im Krankheitsgeschehen nicht an inhibitorischer Potenz zu verlieren.

Die Pulmonale Arterielle Hypertonie (PAH) ist eine progrediente Lungenerkrankung, die unbehandelt durchschnittlich innerhalb von 2.8 Jahren nach Diagnosestellung zum Tode führt. Eine zunehmende Verengung der Lungenstrombahn führt zu einer Mehrbelastung des rechten Herzens, die bis zum Rechtsherzversagen gehen kann. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonal-arteriellen Hypertonie ist gekennzeichnet durch Vasokonstriktion und Remodeling der Pulmonalgefäße. Bei der chronischen PAH kommt es zu einer Neomuskularisierung primär nicht muskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet (M. Humbert et al., J. Am. Coll. Cardiol. 2004, 43, 13S-24S). Mit einer Prävalenz von 1-2 pro einer Million handelt es sich bei PAH um eine äußerst seltene Erkrankung. Das mittlere Alter der Patienten wurde auf 36 Jahre geschätzt, nur 10% der Patenten waren über 60 Jahre alt. Deutlich mehr Frauen als Männer sind betroffen (G.E. D'Alonzo et al., Ann. Intern. Med. 1991, 115, 343-349).

Trotz aller Fortschritte in der Therapie der pulmonal-arteriellen Hypertonie gibt es bisher keine Aussicht auf Heilung dieser schwerwiegenden Erkrankung. Auf dem Markt befindliche Standardtherapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um primär hämodynamische Therapieprinzipien, die den Gefäßtonus beeinflussen, jedoch keinen direkten Einfluss auf die pathogenen Remodeling-Prozesse haben. Darüber hinaus ist die Anwendbarkeit dieser Medikamente durch die z.T. gravierenden Nebenwirkungen und/oder aufwendigen Applikationsformen eingeschränkt. Der Zeitraum, über den unter einer spezifischen Monotherapie die klinische Situation der Patienten verbessert oder stabilisiert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen.

Neue Kombinationstherapien sind eine der aussichtsreichsten zukünftigen Therapieoptionen zur Behandlung der pulmonalen arteriellen Hypertonie. In diesem Zusammenhang ist die Erkundung neuer pharmakologischer Mechanismen zur Behandlung der PAH von besonderem Interesse (Ghofrani et al., Herz 2005, 30, 296-302; E.B. Rosenzweig, Expert Opin. Emerging Drugs 2006, 11, 609-619; T. Ito et al., Curr. Med. Chem. 2007, 14, 719-733). Vor allem solche Therapieoptionen, die direkt in das Remodeling-Geschehen eingreifen (anti-Remodeling-Mechanismen, reverse-Remodeling-Mechanismen), könnten Grundlage einer mehr ursächlichen Behandlung sein und somit einen großen Vorteil für die Patienten bringen. Neue Therapien sollten hierbei mit den bekannten kombinierbar sein. Um in einer solchen Kombinationstherapie das Risiko für störende Wechselwirkungen zwischen den Medikamenten zu minimieren, sollten diese neuen Wirkstoffe metabolisierende P450 CYP-Enzyme nicht oder in nur sehr geringem Maße hemmen.

Heute geht man davon aus, dass die Elastase beim pathologischen Remodeling eine zentrale Rolle spielt. In Tiermodellen und in Patienten mit einem erhöhten arteriellen Lungenblutdruck (pulmonale arterielle Hypertension) konnte eine Fragmentierung des Bindegewebes (interne elastische Lamina) festgestellt werden [Rabinovitch et al., Lab. Invest. 55, 632-653 (1986)], und in Tiermodellen für die pulmonale arterielle Hypertension (hypoxisches Ratten- und Mausmodell, Monocrotalin-Rattenmodell) konnte eine erhöhte Elastase-Aktivität gezeigt werden, die mit der Fragmentierung des Bindegewebes einherging [Todorovich-Hunter et al., Am. Rev. Respir. Dis. 146, 213-223 (1992)]. Man vermutet, dass der zu beobachtende Gewebeumbau im Verlauf des Krankheitsgeschehens der pulmonalen arteriellen Hypertonie durch ein Elastase-vermitteltes Freisetzen von bindegewebsständigen Wachstumsfaktoren induziert wird, z.B. des basischen Fibroblasten-Wachstumsfaktors (engl. *basic fibroblast growth factor,* bFGF) [Rabinovitch, Am. J. Physiol. 277, L5-L12 (1999)]. Im hypoxischen Mausmodell für die pulmonale arterielle Hypertension konnte ein positiver Effekt mit einem überexprimierten Elastase-Inhibitorprotein gezeigt werden [Zaidi et al., Circulation 105, 516-521 (2002)]. Im Monocrotalin-Rattenmodell für die pulmonale arterielle Hypertension konnte eine positive Wirkung mit synthetischen, niedermolekularen Elastase-Inhibitoren gezeigt werden; hierbei war auch ein günstiger Effekt beim Gewebeumbau zu verzeichnen [Cowan et al., Nature Med. 6, 698-702 (2000)]. Alle bisher bekannten niedermolekularen Elastase-Inhibitoren sind jedoch wenig selektiv, chemisch reaktiv und/oder nur begrenzt oral verfügbar, was eine klinische Entwicklung eines oralen Elastase-Inhibitors in diesen Indikationen bisher vereitelte.

Der Begriff "pulmonale arterielle Hypertonie" umfasst bestimmte Formen der pulmonalen Hypertonie, wie sie z.B. von der Weltgesundheitsorganisation (WHO) festgelegt worden sind (Clinical Classification of Pulmonary Hypertension, Venedig 2003; G. Simonneau et al., J. Am. Coll. Cardiol. 2004, 43, 5S-12S).

Die pulmonale arterielle Hypertonie beinhaltet nach dieser Einteilung die Idiopathische Pulmonale Arterielle Hypertonie (IPAH, früher auch als primäre pulmonale Hypertonie, PPH, bezeichnet), die Familiär bedingte Pulmonale Arterielle Hypertonie (FPAH), die persistierende pulmonale Hypertonie der Neugeborenen sowie die Assoziierte Pulmonal-Arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditäre Teleangiektasie, Hämoglobinopathien, myeloproliferative Erkrankungen und Splenektomie.

Andere Formen der pulmonalen Hypertonie umfassen beispielsweise die mit Linksberzerkrankungen assoziierte pulmonale Hypertonie, z.B. bei ventrikulären oder valvulären Erkrankungen, die mit Erkrankungen der Atemwege und/oder der Lunge assoziierte pulmonale Hypertonie, z.B. bei chronisch-obstruktiver Lungenerkrankung, interstitieller Lungenkrankheit oder Lungenfibrose, die auf chronische thrombotische und/oder embolische Erkrankungen zurückzuführende pulmonale Hypertonie, z.B. bei thromboembolischer Obstruktion von Lungenarterien, sowie die durch allgemein entzündliche Krankheitsprozesse oder durch spezielle Ursachen hervorgerufene pulmonale Hypertonie (z.B. bei Schistosomiasis, Sarkoidose und Tumorerkrankungen).

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen.

Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Pharmakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P.J. Barnes, N. Engl. J. Med 343, 269-280 (2000)]. Die chronische Entzündung der Lunge, hervorgerufen durch Zigarettenrauch oder andere Reizstoffe, ist die treibende Kraft der Krankheitsentwicklung. Der zugrunde liegende Mechanismus beinhaltet Immunzellen, die im Zuge der inflammatorischen Reaktion der Lunge verschiedene Chemokine ausschütten. Hierdurch werden neutrophile Zellen und im weiteren Verlauf alveolare Makrophagen zum Lungenbindegewebe und Lumen gelockt. Neutrophile Zellen sezernieren einen Protease-Cocktail, der hauptsächlich HNE und Proteinase 3 enthält. Hierdurch wird lokal die Protease/Antiprotease-Balance zu Gunsten der Proteasen verschoben, was u.a. zu einer unkontrollierten Elasase-Aktivität und in Folge hiervon zu einem überschießenden Abbau des Elastins der Alveolaren führt [J.E. Gadek et al., J. Clin. Invest. 68, 889-898 (1981); Z. Werb et al., J. Invest. Dermatol. 79, 154-159 (1982); A. Janoff, Am. Rev. Respir. Dis. 132, 417-433 (1985); P.J. Bames, N. Engl. J. Med. 343, 269-280 (2000)]. Dieser Gewebeabbau verursacht einen Kollaps der Bronchien. Dies geht einher mit einer verminderten Elastizität der Lunge, was zu einer Behinderung der Atemströmung und beeinträchtigter Atmung führt. Darüber hinaus kann eine häufige und andauernde Entzündung der Lunge zu einem Remodeling der Bronchien und in der Folge zu einer Ausbildung von Läsionen führen. Solche Läsionen tragen zum Auftreten des chronischen Hustens bei, der eine chronische Bronchitis kennzeichnet.

Alpha-1-Antitrypsin (AAT) ist ein kleines körpereigenes Protein und stellt, wie oben erwähnt, den wichtigsten endogenen Elastase-Hemmer dar. Bei Patienten mit einer genetisch bedingten Defizienz dieses Proteins (AATD) ist die Protease/Antiprotease-Balance verschoben. Der Wirkradius und die Wirkdauer der HNE ist in AATD-Patienten entsprechend um einen Faktor 2.5 bzw. 6.5 erhöht [T.G. Liou und E.J. Campbell, Biochemistry 1995, 16171-16177]. AATD-Patienten haben ein erhöhtes Risiko, ein Lungenemphysem oder COPD zu entwickeln, und bei vielen AATD-Patienten ist eine Lungentransplantation indiziert.

Die akute Lungenschädigung (ALI) sowie die ausgeprägtere Form hiervon, das akute Lungenversagen (ARDS), sind schwerwiegende Erkrankungen, die mit einer Mortalität von 50-60% einhergehen. Nach der Definition der North American-European Consensus Conference (NAECC) von 1994 sind ALI und ARDS definiert durch eine akute auslösende Erkrankung, bilaterale radiologisch sichtbare Infiltrate, einen PaO₂/FiO₂-Index von ≤ 300 mmHg (ALI) bzw. ≤ 200 mmHg (ARDS), einen pulmonal-kapillären Verschlussdruck von < 18 mmHg bzw. fehlende klinische Hinweise auf linksatriale Hypertension.

Der Entstehung einer akuten Lungenschädigung können sowohl pulmonale als auch extrapulmonale Erkrankungen vorausgehen. Als lungenspezifische prädisponierende Faktoren gelten Aspiration von Mageninhalt, Pneumonien, Rauchgasvergiftung, Lungenkontusion sowie ein Beinahe-Ertrinken. Vor allem Magensaftaspiration und Pneumonien werden häufig als Ausgangserkrankung für ALI/ARDS pulmonalen Ursprungs gesehen. Als indirekte Ereignisse kommen vor allem Polytrauma, Sepsis, mehrfache Bluttransfusionen, akute Pankreatitis und Verbrennungen vor. Die Inzidenz liegt bei 17.9 Fällen für ALI bzw. 13.5 Fällen für ARDS pro 100 000 Einwohner und Jahr [Luhr et al., Am. J. Respir. Crit. Care Med. 159, 1849-1861 (1999)].

Eine zentrale Rolle für die Entstehung dieser Erkrankungen stellen die massiven entzündlichen Veränderungen in der Lunge dar, die durch ein weitverzweigtes System von Mediatoren ausgelöst werden. Eine wichtige Rolle bei der Entwicklung der Lungenschädigung spielen auch die neutrophilen Granulozyten, deren Anzahl sich mit dem Andauern des entzündlichen Prozesses ständig erhöht [Chollet-Martin et al., Am. J. Respir. Crit. Care Med. 154, 594-601 (1996)]. Die Wirkung der Mediatoren bedingt eine Schädigung der alveolokapillären Membranen, hieraus resultiert eine Permeabilitätserhöhung der alveolär-kapillären Barriere. Durch die Permeabilitätserhöhung kann proteinreiche Flüssigkeit in die Alveolen und auch in das Interstitium eindringen; es bildet sich ein pulmonales Niederdrucködem aus. Charakteristisch für ALI/ARDS ist, dass es sich hierbei um ein nicht-kardiogen induziertes Ödem handelt. Die Ödemflüssigkeit enthält vor allem Fibrin, Erythrozyten, Leukozyten, hyaline Membranen und andere Proteine. Das proteinreiche Exsudat führt zusammen mit den Produkten von aktivierten Neutrophilen zu einer Dysfunktion des Surfactants. Die inflammatorischen Prozesse führen zur Schädigung und zum Verlust von Pneumozyten des Typs II, die Surfactant bilden, so dass ein Herabsinken der Surfactant-Produktion resultiert. Durch den Surfactant-Mangel erhöht sich die Oberflächenspannung in den Alveolen; Alveolen kollabieren, es bilden sich Atelektasen aus. Bei weiter bestehender Perfusion entsteht somit eine Ventilations-Perfusions-Störung, die in einer Erhöhung des pulmonalen Rechts-Links-Shunts mündet. Des Weiteren sinkt die Compliance herab, der alveoläre Totraum hingegen nimmt zu, denn es gibt auch Areale, die zwar belüftet, aber aufgrund einer pulmonalen Hypertension nicht mehr ausreichend perfundiert werden.

In der bronchoalveolaren Waschflüssigkeit von ARDS-Patienten (BALF) konnte eine erhöhte Elastase-Aktivität gemessen werden, die mit dem Schweregrad der Lungenschädigung einhergeht. In Tiermodellen, in denen die Lunge geschädigt wird (z.B. durch Gabe von LPS), kann dieser Effekt nachgestellt werden. Eine Behandlung mit Elastase-Hemmern (z.B. Sivelestat oder Elafin, s.u.) vermindert hier deutlich die Elastase-Aktivität in der BALF und verbessert die Lungenfunktion.

Zur Behandlung einer akuten Lungenschädigung, die mit SIRS assoziiert ist, ist ein Elastase-Hemmer in Japan und Südkorea zugelassen (Sivelestat, Elaspol^{®}). Die reversible, aber reaktive Verbindung besitzt nur eine relativ schwache Wirksamkeit gegenüber der HNE (Kᵢ 200 nM) und wirkt ebenfalls gegenüber der Elastase des Pankreas (IC₅₀ 5.6 µM). Der Wirkstoff wird intravenös verabreicht, eine orale Applikation ist nicht möglich.

Auch Elafin und strukturelle Analoga werden als therapeutisch nutzbare Elastase-Inhibitoren untersucht. Elafin ist ein körpereigenes kleines Protein, welches sowohl Elastase als auch Proteinase 3 hemmt. Aufgrund des proteinergen Charakters ist jedoch eine orale Verabreichung von Elafin nicht möglich.

In WO 2004/024700, WO 2004/024701, WO 2005/082863 und WO 2005/082864 werden verschiedene 1,4-Diaryl-dihydropyrimidin-2-on-Derivate als HNE-Inhibitoren zur Behandlung von chronisch-obstruktiven Lungenerkrankungen, akutem Koronarsyndrom, Myokardinfarkt und Herzinsuffizienz offenbart. Di- und Multimere solcher Verbindungen zur Behandlung von Atemwegserkrankungen werden in WO 2006/082412, WO 2006/136857 und WO 2007/042815 beansprucht. 4-Aryl-dihydropyrimidin-2-on-Derivate als Inhibitoren der Calciumkanal-Funktion zur Behandlung von Hypertonie werden in WO 2005/009392 beschrieben. In WO 2007/129060 werden Tetrahydropyrrolopyrimidindione und Multimere hiervon als HNE-Inhibitoren offenbart. Zwischenzeitlich ist in WO 2008/003412 die Verwendung von 1,4-Diaryl-dihydropyrimidin-2-on-Derivaten zur Behandlung der pulmonalen arteriellen Hypertonie beschrieben worden.

Es wurde nun gefunden, dass sich bestimmte 1,4-Diaryl-dihydropyrimidin-2-on-Derivate in besonderem Maße für die Behandlung und/oder Prävention von Erkrankungen eignen. Diese im Folgenden beschriebenen Verbindungen sind niedermolekulare, nicht-reaktive und selektive Inhibitoren der humanen neutrophilen Elastase (HNE), die überraschenderweise eine deutlich stärkere Inhibition dieser Protease im Vergleich zu den aus dem Stand der Technik bekannten Verbindungen zeigen. Darüber hinaus weisen die erfindungsgemäßen Verbindungen eine unerwartet niedrige *in vitro*-Clearance gegenüber Hepatozyten auf und verfügen damit über eine verbesserte metabolische Stabilität. Diese Substanzen stellen somit vielversprechende Ausgangspunkte für neue Arzneimittel zur Behandlung und/oder Prävention insbesondere von Erkrankungen der Lunge und des Herz-Kreislauf-Systems dar.

Die 1,4-Diaryl-dihydropyrimidin-2-on-Derivate der vorliegenden Erfindung zeichnen sich strukturell im Vergleich zu den Verbindungen aus dem Stand der Technik durch einen *ortho*-Sulfanyl-, *ortho*-Sulfinyl- oder *ortho*-Sulfonyl-Substituenten in der 4-Aryl-Kopfgruppe des Dihydropyrimidinons aus, welcher überraschenderweise zu den oben beschriebenen verbesserten Eigenschaften der Verbindungen führt.

Im Einzelnen betrifft die vorliegende Erfindung Verbindungen der allgemeinen Formel (I) 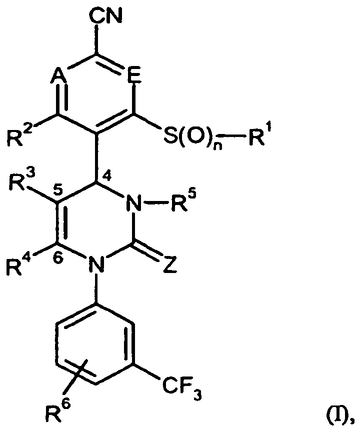 in welcher
- A und E: beide für C-R⁷ stehen oder eines der beiden Ringglieder A und E für N und das andere für C-R⁷ steht, worin R⁷ jeweils Wasserstoff, Fluor oder Chlor bedeutet,
- Z: für O oder S steht,
- n: für die Zahl 0, 1 oder 2 steht,
- R¹: für (C₁-C₆)-Alkyl, das mit Hydroxy, (C₁-C₄)-Alkoxy, Amino, Mono- oder Di-(C₁-C₄)-alkyl-amino, Hydroxycarbonyl, Aminocarbonyl, (C₃-C₆)-Cycloalkyl, Phenyl oder 5- oder 6-gliedrigem Heteroaryl oder bis zu fünffach mit Fluor substituiert sein kann, oder für (C₂-C₆)-Alkenyl, (C₃-C₆)-Cycloalkyl, Phenyl oder 5- oder 6-gliedriges Heteroaryl steht, wobei die genannten (C₃-C₆)-Cycloalkyl-Gruppen bis zu zweifach, gleich oder verschieden, mit (C₁-C₄)-Alkyl, Hydroxy und/oder (C₁-C₄)-Alkoxy substituiert sein können und die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und/oder Trifluormethoxy substituiert sein können,
- R²: für Wasserstoff, Fluor oder Chlor steht,
- R³: für Cyano oder eine Gruppe der Formel -C(=O)-R⁸, -C(=O)-O-R⁸, -C(=O)-NH₂ oder -C(=O)-NH-R⁸ steht, worin R⁸ (C₁-C₆)-Alkyl, (C₃-C₆)-Alkenyl oder (C₃-C₆)-Cycloalkyl bedeutet, wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und/oder Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
- R⁴: für Methyl oder Ethyl steht
- oder R³ und R⁴: miteinander verbunden sind und zusammen eine anellierte Gruppe der Formel 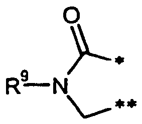 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₆)-Alkyl oder (C₃-C₆)-Cycloalkyl bedeutet, wobei (C₁-C₆)-Alkyl mit Hydroxy, (C₁-C₄)-Alkoxy, Aminocarbonyl, Aminocarbonylamino, (C₁-C₄)-Acylamino oder (C₃-C₆)-Cycloalkyl substituiert sein kann,
- R⁵: für Wasserstoff oder (C₁-C₆)-Alkyl, das mit Cyano, Hydroxy, (C₁-C₄)-Alkoxy, Amino, Mono- oder Di-(C₁-C₄)-alkylamino oder (C₃-C₆)-Cycloalkyl oder bis zu dreifach mit Fluor substituiert sein kann, oder für Phenyl, Pyridyl oder Pyrimidinyl steht, wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
- oder R⁵: für eine Gruppe der Formel -C(=O)-O-R¹⁰, -L¹-C(=O)-O-R¹¹, -L²-C(=O)-NR¹²R¹³, -L²-SO₂NR¹²R¹³, -L²-C(=O)-NR¹⁴-NR¹²R¹³ oder -L²-SO₂-R¹⁵ steht, worin

- L¹: (C₁-C₆)-Alkandiyl bedeutet,
- L²: eine Bindung oder (C₁-C₆)-Alkandiyl bedeutet,
- R¹⁰: (C₁-C₆)-Alkyl, das mit (C₃-C₆)-Cycloalkyl oder Phenyl substituiert sein kann, bedeutet,

- R¹¹: Wasserstoff oder (C₁-C₆)-Alkyl, das mit (C₃-C₆)-Cycloalkyl oder Phenyl substituiert sein kann, bedeutet,
- R¹² und R¹³: gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten, wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und/oder Aminocarbonyl substituiert sein können und in (C₁-C₆)-Alkyl eine CH₂-Gruppe, soweit in einer chemisch stabilen Verbindung resultierend, gegen ein O-Atom ausgetauscht sein kann und (C₃-C₆)-Cycloalkyl und 4- bis 6-gliedriges Heterocyclyl darüber hinaus bis zu zweifach, gleich oder verschieden, mit (C₁-C₄)-Alkyl substituiert sein können, welches seinerseits mit Hydroxy, (C₁-C₄)-Alkoxy oder Hydroxycarbonyl substituiert sein kann,
- oder R¹² und R¹³: zusammen mit dem Stickstoffatom, an das sie gebunden sind, einen 4- bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- oder Di-(C₁-C₄)-alkyl-amino, Hydroxycarbonyl, Aminocarbonyl, (C₃-C₆)-Cycloakyl, 4- bis 6-gliedrigem Heterocyclyl und/oder 5- oder 6-gliedrigem Heteroaryl substituiert sein kann, wobei (C₁-C₄)-Alkyl seinerseits mit Hydroxy, (C₁-C₄)-Alkoxy oder Hydroxycarbonyl substituiert sein kann,
- R¹⁴: Wasserstoff oder (C₁-C₄)-Alkyl bedeutet
- und R¹⁵: (C₁-C₆)-Alkyl, (C₃-C₆)-Cycloalkyl, Phenyl oder 5- oder 6-gliedriges Heteroaryl bedeutet, wobei (C₁-C₆)-Alkyl mit Chlor, Hydroxy, (C₁-C₄)-Alkoxy, Mono- oder Di-(C₁-C₄)-alkylamino oder (C₃-C₆)-Cycloalkyl oder bis zu dreifach mit Fluor substituiert sein kann und Phenyl und 5- oder 6-gliedriges Heteroaryl ihrerseits bis zu zweifach, gleich oder verschieden, mit Fluor, Chlor, Cyano, (C₁-C₄)-Alkyl, Difluormethyl, Trifluormethyl, (C₁-C₄)-Alkoxy und/oder Trifluormethoxy substituiert sein können,
- und R⁶: für Wasserstoff, Fluor oder Chlor steht,
sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachstehend aufgeführten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomeren und Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
(C₁-C₆)-Alkyl und (C₁-C₄)-Alkyl stehen im Rahmen der Erfindung für einen geradkettigen oder verzweigten Alkylrest mit 1 bis 6 bzw. 1 bis 4 Kohlenstoffatomen. Bevorzugt ist ein geradkettiger oder verzweigter Alkylrest mit 1 bis 4 Kohlenstoffatomen. Beispielhaft und vorzugsweise seien genannt: Methyl, Ethyl, *n*-Propyl, Isopropyl, *n*-Butyl, *iso*-Butyl, *sec*.-Butyl, *tert*.-Butyl, 1-Ethylpropyl, *n*-Pentyl, Neopentyl und *n*-Hexyl.
(C₁-C₆)-Alkandiyl steht im Rahmen der Erfindung für einen geradkettigen oder verzweigten divalenten Alkylrest mit 1 bis 6 Kohlenstoffatomen. Bevorzugt ist ein geradkettiger oder verzweigter Alkandiylrest mit 1 bis 4 Kohlenstoffatomen. Beispielhaft und vorzugsweise seien genannt: Methylen, Ethan-1,2-diyl (1,2-Ethylen), Ethan-1,1-diyl, Propan-1,3-diyl (1,3-Propylen), Propan-1,1-diyl, Propan-1,2-diyl, Propan-2,2-diyl, Butan-1,4-diyl (1,4-Butylen), Butan-1,2-diyl, Butan-1,3-diyl, Butan-2,3-diyl, Pentan-1,5-diyl (1,5-Pentylen), Pentan-2,4-diyl, 3-Methylpentan-2,4-diyl und Hexan-1,6-diyl (1,6-Hexylen).
(C₂-C₆)-Alkenyl und (C₃-C₆)-Alkenyl stehen im Rahmen der Erfindung für einen geradkettigen oder verzweigten Alkenylrest mit 2 bis 6 bzw. 3 bis 6 Kohlenstoffatomen und einer oder zwei Doppelbindungen. Bevorzugt ist ein geradkettiger oder verzweigter Alkenylrest mit 3 bis 6 Kohlenstoffatomen und einer Doppelbindung. Beispielhaft und vorzugsweise seien genannt: Allyl, Isopropenyl, *n*-But-2-en-1-yl, *n*-But-3-en-1-yl, *n*-Pent-2-en-1-yl, *n*-Pent-3-en-1-yl, *n*-Pent-4-en-1-yl, 3-Methylbut-2-en-1-yl und 4-Methylpent-3-en-1-yl.
(C₁-C₄)-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, *n*-Propoxy, Isopropoxy, *n*-Butoxy und *tert*.-Butoxy.
(C₁-C₄)-Alkoxycarbonyl steht im Rahmen der Erfindung für einen geradkettigen oder verzweigten Alkoxyrest mit 1 bis 4 Kohlenstoffatomen, der über eine Carbonylgruppe verknüpft ist. Beispielhaft und vorzugsweise seien genannt: Methoxycarbonyl, Ethoxycarbonyl, *n*-Propoxycarbonyl, Isopropoxycarbonyl, *n*-Butoxycarbonyl und *tert*.-Butoxycarbonyl.
Mono-(C₁-C₄)-alkylamino steht im Rahmen der Erfindung für eine Amino-Gruppe mit einem geradkettigen oder verzweigten Alkylsubstituenten, der 1 bis 4 Kohlenstoffatome aufweist. Beispielhaft und vorzugsweise seien genannt: Methylamino, Ethylamino, *n*-Propylamino, Isopropylamino, *n*-Butylamino und *tert*.-Butylamino.
Di-(C₁-C₄)-alkylamino steht im Rahmen der Erfindung für eine Amino-Gruppe mit zwei gleichen oder verschiedenen geradkettigen oder verzweigten Alkylsubstituenten, die jeweils 1 bis 4 Kohlenstoffatome aufweisen. Beispielhaft und vorzugsweise seien genannt: *N,N*-Dimethylamino, *N,N*-Di-ethylamino, *N*-Ethyl-*N*-methylamino, *N*-Methyl-*N*-*n*-propylamino, *N*-Isopropyl-*N*-methylamino, *N-*Isopropyl-*N-n*-propylamino, *N,N*-Diisopropylamino, *N*-*n*-Butyl-*N*-methylamino und *N*-*tert*.-Butyl-*N*-methylamino.
(C₁-C₄)-Acyl [(C₁-C₄)-Alkanoyl] steht im Rahmen der Erfindung für einen geradkettigen oder verzweigten Alkylrest mit 1 bis 4 Kohlenstoffatomen, der in der 1-Position ein doppelt gebundenes Sauerstoffatom trägt und über die 1-Position verknüpft ist. Beispielhaft und vorzugsweise seien genannt: Formyl, Acetyl, Propionyl, *n*-Butyryl und *iso*-Butyryl.
(C₁-C₄)-Acylamino steht im Rahmen der Erfindung für eine Amino-Gruppe mit einem geradkettigen oder verzweigten Acyl-Substituenten, der 1 bis 4 Kohlenstoffatome aufweist und über die Carbonylgruppe mit dem *N*-Atom verknüpft ist. Beispielhaft und vorzugsweise seien genannt: Formylamino, Acetylamino, Propionylamino, *n*-Butyrylamino und *iso*-Butyrylamino.
(C₃-C₆)-Cycloalkyl steht im Rahmen der Erfindung für eine monocyclische, gesättigte Cycloalkylgruppe mit 3 bis 6 Ring-Kohlenstoffatomen. Beispielhaft und vorzugsweise seien genannt: Cyclopropyl, Cyclobutyl, Cyclopentyl und Cyclohexyl.
4- bis 6-gliedriges Heterocyclyl steht im Rahmen der Erfindung für einen monocyclischen, gesättigten Heterocyclus mit insgesamt 4 bis 6 Ringatomen, der ein oder zwei Ring-Heteroatome aus der Reihe N, O, S, SO und/oder SO₂ enthält und über ein Ring-Kohlenstoffatom oder gegebenenfalls ein Ring-Stickstoffatom verknüpft ist. Bevorzugt ist ein 5- oder 6-gliedriger Heterocyclus mit ein oder zwei Ring-Heteroatomen aus der Reihe N, O und/oder S. Beispielhaft seien genannt: Azetidinyl, Oxetanyl, Pyrrolidinyl, Pyrazolidinyl, Tetrahydrofuranyl, Thiolanyl, Piperidinyl, Piperazinyl, Tetrahydropyranyl, Tetrahydrothiopyranyl, Morpholinyl und Thiomorpholinyl. Bevorzugt sind Pyrrolidinyl, Tetrahydrofuranyl, Piperidinyl, Piperazinyl, Tetrahydropyranyl und Morpholinyl.
5- oder 6-gliedriges Heteroaryl steht im Rahmen der Erfindung für einen aromatischen Heterocyclus (Heteroaromaten) mit insgesamt 5 oder 6 Ringatomen, der ein oder zwei Ring-Heteroatome aus der Reihe N, O und/oder S enthält und über ein Ring-Kohlenstoffatom oder gegebenenfalls ein Ring-Stickstoffatom verknüpft ist. Beispielhaft seien genannt: Furyl, Pyrrolyl, Thienyl, Pyrazolyl, Imidazolyl, Oxazolyl, Thiazolyl, Isoxazolyl, Isothiazolyl, Pyridyl, Pyrimidinyl, Pyridazinyl und Pyrazinyl. Bevorzugt sind Thienyl, Thiazolyl, Pyridyl, Pyrimidinyl, Pyridazinyl und Pyrazinyl.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit einem oder zwei gleichen oder verschiedenen Substituenten ist bevorzugt. Ganz besonders bevorzugt ist die Substitution mit einem Substituenten.

In einer besonderen Ausführungsform umfasst die vorliegende Erfindung Verbindungen der Formel (I), in welcher
- A und E: beide für C-R⁷ stehen oder eines der beiden Ringglieder A und E für N und das andere für C-R⁷ steht, worin

- R¹: jeweils Wasserstoff, Fluor oder Chlor bedeutet,

- Z: für O oder S steht,
- n: für die Zahl 0, 1 oder 2 steht,
- R¹: für (C₁-C₆)-Alkyl, das mit Hydroxy, (C₁-C₄)-Alkoxy, Amino, Mono- oder Di-(C₁-C₄)-alkyl-amino, Hydroxycarbonyl, Aminocarbonyl, (C₃-C₆)-Cycloalkyl oder Phenyl substituiert sein kann, oder für (C₂-C₆)-Alkenyl, (C₃-C₆)-Cycloalkyl oder Phenyl steht, wobei die genannten (C₃-C₆)-Cycloalkyl-Gruppen bis zu zweifach, gleich oder verschieden, mit (C₁-C₄)-Alkyl, Hydroxy und/oder (C₁-C₄)-Alkoxy substituiert sein können und die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und/oder Trifluormethoxy substituiert sein können,
- R²: für Wasserstoff, Fluor oder Chlor steht,
- R³: für Cyano oder eine Gruppe der Formel -C(=O)-R⁸, -C(=O)-O-R⁸, -C(=O)-NH₂ oder -C(=O)-NH-R⁸ steht, worin R⁸ (C₁-C₆)-Alkyl, (C₃-C₆)-Alkenyl oder (C₃-C₆)-Cycloalkyl bedeutet, wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und/oder Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
- R⁴: für Methyl oder Ethyl steht
- oder R³ und R⁴: miteinander verbunden sind und zusammen eine anellierte Gruppe der Formel 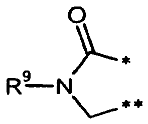 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₆)-Alkyl oder (C₃-C₆)-Cycloalkyl bedeutet, wobei (C₁-C₆)-Alkyl mit Hydroxy, (C₁-C₄)-Alkoxy, Aminocarbonyl, (C₁-C₄)-Acylamino oder (C₃-C₆)-Cycloalkyl substituiert sein kann,

- R⁵: für Wasserstoff oder (C₁-C₆)-Alkyl, das bis zu dreifach mit Fluor substituiert sein kann, oder für Phenyl, Pyridyl oder Pyrimidinyl steht, wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
- oder R⁵: für eine Gruppe der Formel -C(=O)-O-R¹⁰, -L¹-C(=O)-O-R¹¹, -L²-C(=O)-NR¹²R¹³, -L²-SO₂-NR¹²R¹³, -L²-C(=O)-NR¹⁴-NR¹²R¹³ oder -L²-SO₂-R¹⁵ steht, worin

- L¹: (C₁-C₆)-Alkandiyl bedeutet,
- L²: eine Bindung oder (C₁-C₆)-Alkandiyl bedeutet,
- R¹⁰: (C₁-C₆)-Alkyl bedeutet,
- R¹¹: Wasserstoff oder (C₁-C₆)-Alkyl bedeutet,
- R¹² und R¹³: gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten, wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und/oder Aminocarbonyl substituiert sein können und in (C₁-C₆)-Alkyl eine CH₂-Gruppe, soweit in einer chemisch stabilen Verbindung resultierend, gegen ein O-Atom ausgetauscht sein kann, oder R¹² und R¹³ zusammen mit dem Stickstoffatom, an das sie gebunden sind, einen 4- bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- und/oder Di-(C₁-C₄)-alkylamino substituiert sein kann, wobei (C₁-C₄)-Alkyl seinerseits mit Hydroxy oder (C₁-C₄)-Alkoxy substituiert sein kann, R¹⁴ Wasserstoff oder (C₁-C₄)-Alkyl bedeutet und R¹⁵ (C₁-C₆)-Alkyl, (C₃-C₆)-Cycloalkyl, Phenyl oder 5- oder 6-gliedriges Heteroaryl bedeutet, wobei (C₁-C₆)-Alkyl mit Fluor, Chlor, Hydroxy, (C₁-C₄)-Alkoxy, Mono- oder Di-(C₁-C₄)-alkylamino substituiert sein kann und Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
- und R⁶: für Wasserstoff, Fluor oder Chlor steht,
sowie ihre Salze, Solvate und Solvate der Salze.

Bevorzugt im Rahmen der vorliegenden Erfindung sind Verbindungen der Formel (I), in welcher
- A und E: beide für CH stehen
- und R²: für Wasserstoff steht,
sowie ihre Salze, Solvate und Solvate der Salze.

Gleichfalls bevorzugt sind Verbindungen der Formel (I), in welcher
- Z: für O steht,
sowie ihre Salze, Solvate und Solvate der Salze.

Besonders bevorzugt im Rahmen der vorliegenden Erfindung sind Verbindungen der Formel (I), in welcher
- A und E: beide für CH stehen,
- Z: für O steht,
- n: für die Zahl 0 oder 2 steht,
- R¹: für (C₁-C₄)-Alkyl, das mit Hydroxy, (C₁-C₄)-Alkoxy, Hydroxycarbonyl, Aminocarbonyl, (C₃-C₆)-Cycloalkyl, Phenyl oder 5-gliedrigem Heteroaryl oder bis zu dreifach mit Fluor substituiert sein kann, oder für (C₃-C₆)-Cycloalkyl, Phenyl oder 5-gliedriges Heteroaryl steht, wobei die genannten Phenyl- und Heteroaryl-Gruppen bis zu zweifach, gleich oder verschieden, mit Fluor, Chlor, Cyano, Methyl, Trifluormethyl, Methoxy und/oder Trifluormethoxy substituiert sein können,
- R²: für Wasserstoff steht,
- R³: für Cyano, Acetyl oder (2-Hydroxyethoxy)carbonyl steht,
- R⁴: für Methyl steht
- oder R³ und R⁴: miteinander verbunden sind und zusammen eine anellierte Gruppe der Formel bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₄)-Alkyl oder (C₃-C₆)-Cycloalkyl bedeutet, wobei (C₁-C₄)-Alkyl mit Hydroxy oder (C₁-C₄)-Alkoxy substituiert sein kann,
- R⁵: für Wasserstoff oder (C₁-C₄)-Alkyl, das mit Cyano oder Di-(C₁-C₄)-alkylamino substituiert sein kann, oder für eine Gruppe der Formel -L²-C(=O)-NR¹²R¹³, -L²-C(=O)-NH-NR¹²R¹³ oder -L²-SO₂-R¹⁵ steht, worin L² eine Bindung, -CH₂-, -CH₂CH₂- oder -CH(CH₃)- bedeutet, R¹² Wasserstoff oder (C₁-C₄)-Alkyl, das mit Hydroxy oder (C₁-C₄)-Alkoxy substituiert sein kann, bedeutet, R¹³ Wasserstoff, (C₁-C₆)-Alkyl oder (C₃-C₆)-Cycloalkyl bedeutet, wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und/oder Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann, oder R¹² und R¹³ zusammen mit dem Stickstoffatom, an das sie gebunden sind, einen 5- oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy, Oxo, Hydroxycarbonyl, Aminocarbonyl, 4- bis 6-gliedrigem Heterocyclyl oder 5- oder 6-gliedrigem Heteroaryl substituiert sein kann, wobei (C₁-C₄)-Alkyl seinerseits mit Hydroxy, (C₁-C₄)-Alkoxy oder Hydroxycarbonyl substituiert sein kann, und R¹⁵ (C₁-C₄)-Alkyl, (C₃-C₆)-Cycloalkyl oder Phenyl bedeutet, wobei (C₁-C₄)-Alkyl mit (C₃-C₆)-Cycloalkyl substituiert sein kann und Phenyl bis zu zweifach, gleich oder verschieden, mit Fluor, Chlor, Cyano, Methyl, Trifluormethyl, Methoxy und/oder Trifluormethoxy substituiert sein kann,
- und R⁶: für Wasserstoff oder Fluor steht,
sowie ihre Salze, Solvate und Solvate der Salze.

In einer weiteren besonders bevorzugten Ausführungsform umfasst die vorliegende Erfindung Verbindungen der Formel (I), in welcher
- A und E: beide für CH stehen,
- Z: für O steht,
- n: für die Zahl 0 oder 2 steht,
- R¹: für (C₁-C₄)-Akyl, das mit Hydroxy, (C₁-C₄)-Alkoxy, Hydroxycarbonyl, Aminocarbonyl, (C₃-C₆)-Cycloalkyl oder Phenyl substituiert sein kann, oder für (C₃-C₆)-Cycloalkyl oder Phenyl steht, wobei die genannten Phenyl-Gruppen bis zu zweifach, gleich oder verschieden, mit Fluor, Chlor, Cyano, Methyl, Trifluormethyl, Methoxy und/oder Trifluormethoxy substituiert sein können,
- R²: für Wasserstoff steht,
- R³: für Cyano oder Acetyl steht,
- R⁴: für Methyl steht
- oder R³ und R⁴: miteinander verbunden sind und zusammen eine anellierte Gruppe der Formel 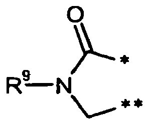 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₄)-Alkyl oder (C₃-C₆)-Cycloalkyl bedeutet, wobei (C₁-C₄)-Alkyl mit Hydroxy oder (C₁-C₄)-Alkoxy substituiert sein kann,
- R⁵: für Wasserstoff, (C₁-C₄)-Alkyl oder eine Gruppe der Formel -L²-C(=O)-NR¹²R¹³, -L²-C(=O)-NH-NR¹²R¹³ oder -L²-SO₂-R¹⁵ steht, worin L² eine Bindung, -CH₂-, -CH₂CH₂- oder -CH(CH₃)- bedeutet, R¹² Wasserstoff oder (C₁-C₄)-Alkyl, das mit Hydroxy oder (C₁-C₄)-Alkoxy substituiert sein kann, bedeutet, R¹³ Wasserstoff, (C₁-C₆)-Alkyl oder (C₃-C₆)-Cycloalkyl bedeutet, wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und/oder Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann, oder R¹² und R¹³ zusammen mit dem Stickstoffatom, an das sie gebunden sind, einen 5- oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy oder Oxo substituiert sein kann, wobei (C₁-C₄)-Alkyl seinerseits mit Hydroxy oder (C₁-C₄)-Alkoxy substituiert sein kann, und R¹⁵ (C₁-C₄)-Alkyl, (C₃-C₆)-Cycloalkyl oder Phenyl bedeutet, wobei Phenyl bis zu zweifach, gleich oder verschieden, mit Fluor, Chlor, Cyano, Methyl, Trifluorethyl, Methoxy und/oder Trifluormethoxy substituiert sein kann,
- und R⁶: für Wasserstoff oder Fluor steht,
sowie ihre Salze, Solvate und Solvate der Salze.

Insbesondere bevorzugt im Rahmen der vorliegenden Erfindung sind Verbindungen der Formel (I), in welcher
- A und E: beide für CH stehen,
- Z: für O steht,
- n: für die Zahl 2 steht,
- R¹: für (C₁-C₄)-Alkyl, das mit Hydroxy, (C₁-C₄)-Alkoxy, Cyclopropyl, Cyclobutyl oder Phenyl oder bis dreifach mit Fluor substituiert sein kann, steht,
- R²: für Wasserstoff steht,
- R³: für Cyano oder (2-Hydroxyethoxy)carbonyl steht,
- R⁴: für Methyl steht,
- R⁵: für Wasserstoff, (C₁-C₄)-Alkyl oder eine Gruppe der Formel -L²-C(=O)-NH-R¹³ oder -SO₂-R¹⁵ steht, worin L² eine Bindung oder -CH₂- bedeutet, R¹³ Wasserstoff oder (C₁-C₄)-Alkyl, das mit Hydroxy oder (C₁-C₄)-Alkoxy substituiert sein kann, oder (C₃-C₆)-Cycloalkyl bedeutet und R¹⁵ (C₁-C₄)-Alkyl oder (C₃-C₆)-Cycloalkyl bedeutet,
- und R⁶: für Wasserstoff steht,
sowie ihre Salze, Solvate und Solvate der Salze.

Ganz besonders bevorzugt im Rahmen der vorliegenden Erfindung sind Verbindungen der Formel (I), in welcher
- A und E: beide für CH stehen,
- Z: für O steht,
- n: für die Zahl 2 steht,
- R¹: für (C₁-C₄)-Alkyl, das mit Hydroxy oder (C₁-C₄)-Alkoxy substituiert sein kann, steht,
- R²: für Wasserstoff steht,
- R³: für Cyano steht,
- R⁴: für Methyl steht,
- R⁵: für Wasserstoff, (C₁-C₄)-Alkyl oder eine Gruppe der Formel -CH₂-C(=O)-NH-R¹³ oder -SO₂-R¹⁵ steht, worin R¹³ Wasserstoff oder (C₁-C₄)-Alkyl, das mit Hydroxy oder (C₁-C₄)-Alkoxy substituiert sein kann, bedeutet und R¹⁵ (C₁-C₄)-Alkyl oder (C₃-C₆)-Cycloalkyl bedeutet,
- und R⁶: für Wasserstoff steht,
sowie ihre Salze, Solvate und Solvate der Salze.

Von besonderer Bedeutung sind Verbindungen gemäß Formel (I) mit der in Formel *(I-ent)* wiedergegebenen Konfiguration an der 4-Position des Dihydropyrimidin-Rings 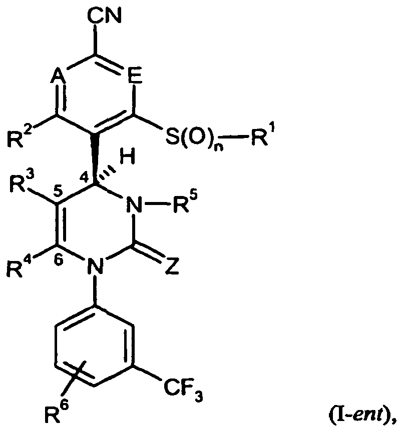 worin A, E, Z, n, R¹, R², R³, R⁴, R⁵ und R⁶ jeweils die oben angegebenen Bedeutungen haben, und ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I), dadurch gekennzeichnet, dass man eine Verbindung der Formel (II) 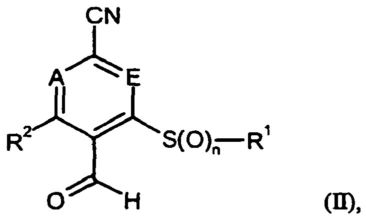 in welcher A, E, n, R¹ und R² jeweils die oben angegebenen Bedeutungen haben,
in Gegenwart einer Säure oder eines Säureanhydrids in einer 3-Komponenten-Eintopf-Reaktion oder sequentiell mit einer Verbindung der Formel (III) 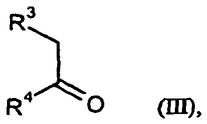 in welcher R³ und R⁴ die oben angegebenen Bedeutungen haben,
und einer Verbindung der Formel (IV) 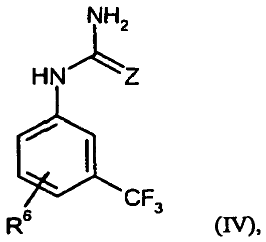 in welcher Z und R⁶ die oben angegebenen Bedeutungen haben,
zu einer Verbindung der Formel (I-A) 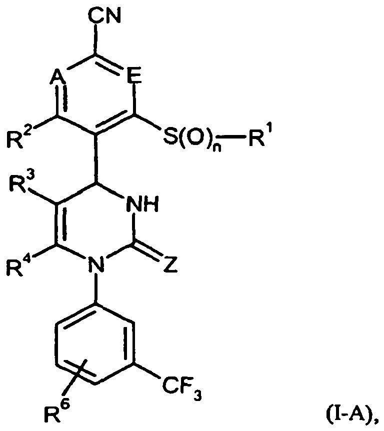 in welcher A, E, Z, n, R¹, R², R³, R⁴ und R⁶ jeweils die oben angegebenen Bedeutungen haben, umsetzt und diese im Falle, dass R⁵ in Formel (I) nicht für Wasserstoff steht, in Gegenwart einer Base mit einer Verbindung der Formel (V)

R^{5A}-X , (V)

in welcher
- R^{5A}: die oben angegebene Bedeutung von R⁵ hat, jedoch nicht für Wasserstoff steht, und
- X: für eine Abgangsgruppe wie beispielsweise Halogen, Mesylat, Tosylat oder Triflat steht, zu einer Verbindung der Formel (I-B) 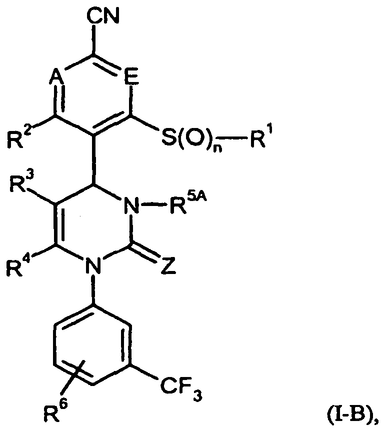
in welcher A, E, Z, n, R¹, R², R³, R⁴, R^{5A} und R⁶ jeweils die oben angegebenen Bedeutungen aufweisen,

reagiert
und gegebenenfalls die so erhaltenen Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

Für den Verfahrensschritt (II) + (III) + (IV) → (I-A) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-tert.-butylether, 1,2-Dimethoxyethan, Dioxan oder Tetrahydrofuran, Alkohole wie Methanol, Ethanol, n-Propanol, Isopropanol, n-Butanol oder tert.-Butanol, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Acetonitril, Dimethylsulfoxid oder N,N-Dimethylformamid. Ebenso ist es möglich, Gemische der genannten Lösungsmittel einzusetzen. Bevorzugt wird Methyl-tert.-butylether, Tetrahydrofuran oder Dioxan verwendet.

Als Säure für den Verfahrensschritt (II) + (III) + (IV) → (I-A) eignen sich übliche anorganische oder organische Säuren oder Säureanhydride. Hierzu gehören bevorzugt Carbonsäuren wie beispielsweise Essigsäure oder Trifluoressigsäure, Sulfonsäuren wie Methansulfonsäure, Trifluormethansulfonsäure oder p-Toluolsulfonsäure, Salzsäure, Schwefelsäure, Phosphorsäure, Phosphonsäuren, oder Phosphorsäure- oder Phosphonsäureanhydride oder -ester wie Polyphosphorsäure, Phosphorsäuretriethylester, Polyphosphorsäureethylester, Phosphorpentoxid oder Propanphosphonsäureanhydrid. Bevorzugt wird Phosphorsäuretriethylester in Kombination mit Phosphorpentoxid verwendet. Die Säure wird im Allgemeinen in einer Menge von 0.25 Mol bis 100 Mol, bezogen auf 1 Mol der Verbindung (III), eingesetzt.

Der Verfahrensschritt (II) + (III) + (IV) → (I-A) wird im Allgemeinen in einem Temperaturbereich von +20°C bis +150°C, bevorzugt bei +50°C bis +100°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Für den Verfahrensschritt (I-A) + (V) → (I-B) geeignete Lösungsmittel sind übliche organische Lösungsmittel, die sich unter den Reaktionsbedingungen nicht verändern. Dazu zählen beispielsweise Ether wie Diethylether, Diisopropylether, Methyl-tert.-butylether, 1,2-Dimethoxyethan, Dioxan oder Tetrahydrofuran, Kohlenwasserstoffe wie Pentan, Hexan, Cyclohexan, Benzol, Toluol oder Xylol, Halogenkohlenwasserstoffe wie Dichlormethan, 1,2-Dichlorethan, Trichlormethan oder Chlorbenzol, oder andere Lösungsmittel wie Ethylacetat, Aceton, Methylethylketon, Methyl-tert.-butylketon, Acetonitril, Dimethylsulfoxid, *N*,*N*-Dimethylformamid, N,N=Dimethylpropylenharnstoff (DMPU) oder N-Methylpyrrolidon (NMP). Ebenso ist es möglich, Gemische der genannten Lösungsmittel einzusetzen. Bevorzugt wird Tetrahydrofuran, Acetonitril oder Dimethylformamid verwendet.

Als Base für den Verfahrensschritt (I-A) + (V) → (I-B) eignen sich übliche anorganische oder organische Basen. Hierzu gehören insbesondere Alkali- oder Erdalkalicarbonate wie Lithium-, Natrium-, Kalium-, Calcium- oder Cäsiumcarbonat, Alkali-Alkoholate wie Natrium- oder Kalium-tert.-butylat, Alkalihydride wie Natrium- oder Kaliumhydrid, Amide wie Lithium- oder Kalium-bis(trimethylsilyl)amid oder Lithiumdiisopropylamid (LDA), organische Amine wie Triethylamin, *N*-Methylmorpholin, *N*-Methylpiperidin, *N*,*N*-Diisopropylethylamin, 1,5-Diazabicyclo[4.3.0]non-5-en (DBN), 1,8-Diazabicyclo[5.4.0]undec-7-en (DBU), Pyridin oder 4-*N*,*N*-Dimethylaminopyridin, oder Phosphazen-Basen (so genannte "Schwesinger-Basen") wie beispielsweise P1-t-Bu, P2-t-Bu oder P4-t-Bu. Bevorzugt wird Kaliumcarbonat, Cäsiumcarbonat, Natriumhydrid, Triethylamin, *N*,*N*-Diisopropylethylamin oder Lithium-bis(trimethylsilyl)amid verwendet; besonders bevorzugt sind Natriumhydrid und Lithium-bis(trimethylsilyl)amid. Die Base wird im Allgemeinen in einer Menge von 0.1 Mol bis 10 Mol, bevorzugt von 1 Mol bis 3 Mol, bezogen auf 1 Mol der Verbindung (I-A), eingesetzt.

Der Verfahrensschritt (I-A) + (V) → (I-B) wird im Allgemeinen in einem Temperaturbereich von -78°C bis +100°C, bevorzugt bei -78°C bis +80°C, besonders bevorzugt bei -78°C bis +25°C durchgeführt. Die Umsetzung kann bei normalem, erhöhtem oder erniedrigtem Druck erfolgen (z.B. von 0.5 bis 5 bar). Im Allgemeinen arbeitet man bei Normaldruck.

Erfindungsgemäße Verbindungen der Formel (I), in welcher R³ und R⁴ miteinander verbunden sind und zusammen eine anellierte Gruppe der Formel 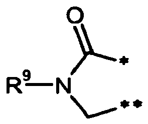 bilden, worin * und ** die oben beschriebenen Verknüpfungspunkte darstellen und R⁹ die oben angegebene Bedeutung hat,
können auch dadurch hergestellt werden, dass man eine Verbindung der Formel (I-C) 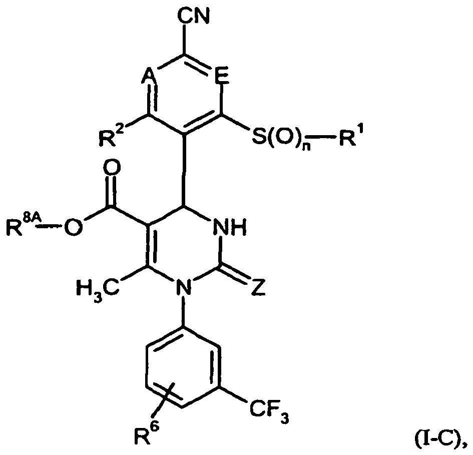 in welcher A, E, Z, n, R¹, R² und R⁶ jeweils die oben angegebenen Bedeutungen haben und
- R^{8A}: für (C₁-C₆)-Alkyl, (C₃-C₆)-Alkenyl oder (C₃-C₆)-Cycloalkyl steht,
in einem inerten Lösungsmittel zu einer Verbindung der Formel (VI) 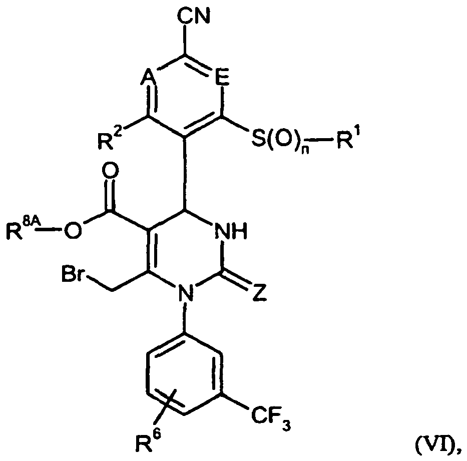 in welcher A, E, Z, n, R¹, R², R⁶ und R^{8A} jeweils die oben angegebenen Bedeutungen haben, bromiert und dann mit einer Verbindung der Formel (VII)

R⁹-NH₂ , (VII)

in welcher R⁹ die oben angegebene Bedeutung hat,
zu einer Verbindung der Formel (I-D) 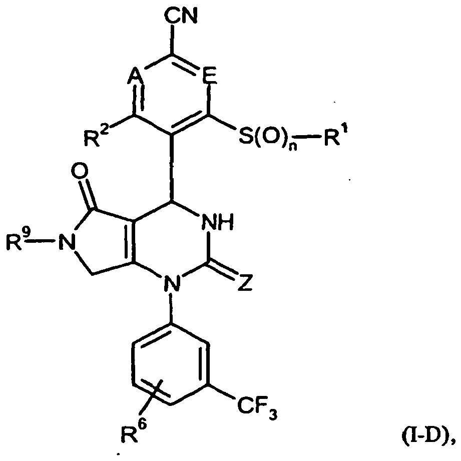 in welcher A, E, Z, n, R¹, R², R⁶ und R⁹ jeweils die oben angegebenen Bedeutungen haben, cyclisierend umsetzt und gegebenenfalls diese anschließend mit einer Verbindung der Formel (V), wie oben beschrieben, in eine Verbindung der Formel (I-E) 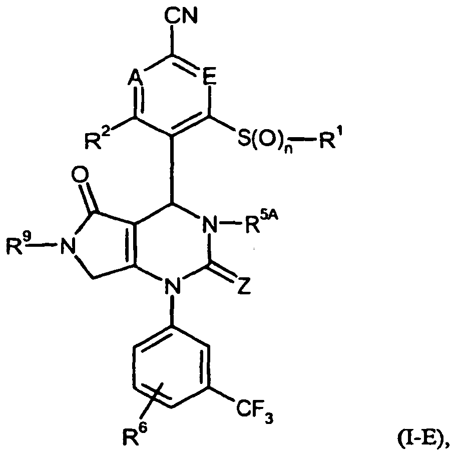 in welcher A, E, Z, n, R¹, R², R^{5A}, R⁶ und R⁹ jeweils die oben angegebenen Bedeutungen aufweisen,
überführt.

Die Bromierung im Verfahrensschritt (I-C) → (VI) wird bevorzugt mit Hilfe von elementarem Brom in einem üblichen inerten Lösungsmittel wie Chloroform bei einer Temperatur von -20°C bis +40°C durchgeführt. Eine im Rest R^{8A} gegebenenfalls vorhandene CC-Doppelbindung [R^{8A} = (C₃-C₆)-Alkenyl] kann unter diesen Reaktionsbedingungen gleichfalls bromiert werden, dies hat jedoch keine störende Einwirkung bei der nachfolgenden Ringschluss-Reaktion mit der Verbindung (VII).

Die Lactam-Bildung im Verfahrensschritt (VI) + (VII) → (I-D) wird vorzugsweise in einem Ether wie Tetrahydrofuran oder Dioxan als inertem Lösungsmittel bei einer Temperatur von -20°C bis +60°C durchgeführt. Gegebenenfalls kann die Verwendung eines tertiären Amins, wie Triethylamin, *N*-Methylmorpholin, *N*-Methylpiperidin oder *N*,*N*-Diisopropylethylamin, als Hilfsbase von Vorteil sein.

Die Verbindung der Formel (I-C) ihrerseits ist entsprechend der zuvor beschriebenen Umsetzung (II) + (III) + (IV) → (I-A) zugänglich.

Weitere erfindungsgemäße Verbindungen der Formel (I) können, falls zweckmäßig, auch durch Umwandlungen von funktionellen Gruppen einzelner Substituenten, insbesondere den unter R¹, R³ und R⁵ aufgeführten, ausgehend von anderen, nach obigem Verfahren erhaltenen Verbindungen der Formel (I) hergestellt werden. Diese Umwandlungen werden nach üblichen, dem Fachmann bekannten Methoden durchgeführt und umfassen beispielsweise Reaktionen wie nukleophile oder elektrophile Substitutionsreaktionen, Übergangsmetall-vermittelte Kupplungsreaktionen (z.B. Suzuki- oder Heck-Reaktion), Oxidation, Reduktion, Hydrierung, Alkylierung, Acylierung, Aminierung, Hydroxylierung, Veretherung, Veresterung, Esterspaltung und -hydrolyse, Bildung von Nitrilen, Carbonamiden, Sulfonamiden, Carbamaten und Harnstoffen, sowie die Einführung und Entfernung temporärer Schutzgruppen [vgl. auch nachfolgende Reaktionsschemata 2-5 sowie die Ausführungsbeispiele].

Eine Trennung der erfindungsgemäßen Verbindungen in die entsprechenden Enantiomere und/oder Diastereomere kann, je nach Zweckmäßigkeit, auf der Stufe der Verbindungen (I-B) bzw. (I-E) oder auch auf der Stufe der Verbindungen (I-A), (I-C) oder (I-D) erfolgen, wobei letztere dann in separierter Form entsprechend den zuvor beschriebenen Verfahrensschritten weiter umgesetzt werden. Eine solche Auftrennung der Stereoisomeren läßt sich nach üblichen, dem Fachmann bekannten Methoden durchführen; vorzugsweise werden chromatographische Verfahren, insbesondere die HPLC-Chromatographie an chiraler Phase, verwendet.

Die Verbindungen der Formeln (III), (IV), (V) und (VII) sind kommerziell erhältlich oder als solche literaturbekannt oder sie können nach gängigen, in der Literatur beschriebenen Methoden hergestellt werden.

Die Verbindungen der Formel (II) sind zum Teil literaturbekannt oder sie können in Analogie zu in der Literatur beschriebenen Verfahren hergestellt werden [vgl. auch nachfolgende Reaktionsschemata 6-9 und dort angegebene Literatur].

Bei dem zuvor beschriebenen Verfahren kann es gegebenenfalls synthetisch zweckmäßig sein, statt der Verbindung der Formel (II) zunächst eine Verbindung der Formel (II-A) 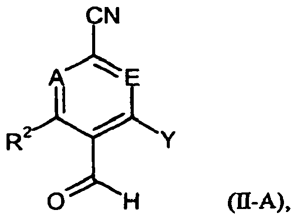 in welcher A, E und R² die oben angegebenen Bedeutungen haben
und
- Y: für eine austauschbare Gruppe wie beispielsweise Fluor, Chlor, Brom, Iod, Nitro oder Amino steht,

in der geschilderten Reaktionssequenz einzusetzen und den ortho-Thio-Substituenten R¹-S(O)ₙ- der Aryl-Kopfgruppe dann auf der Stufe des - der Verbindung (I-A) oder (I-B) entsprechenden - Dihydropyrimidinons im Austausch gegen den Rest Y einzuführen [vgl. nachfolgendes Reaktionsschema 10]. Die Verbindungen der Formel (II-A) sind gleichfalls zum Teil literaturbekannt oder können analog literaturbekannter Methoden hergestellt werden.

Die oben beschriebenen Verfahren können durch die folgenden Reaktionsschemata veranschaulicht werden: 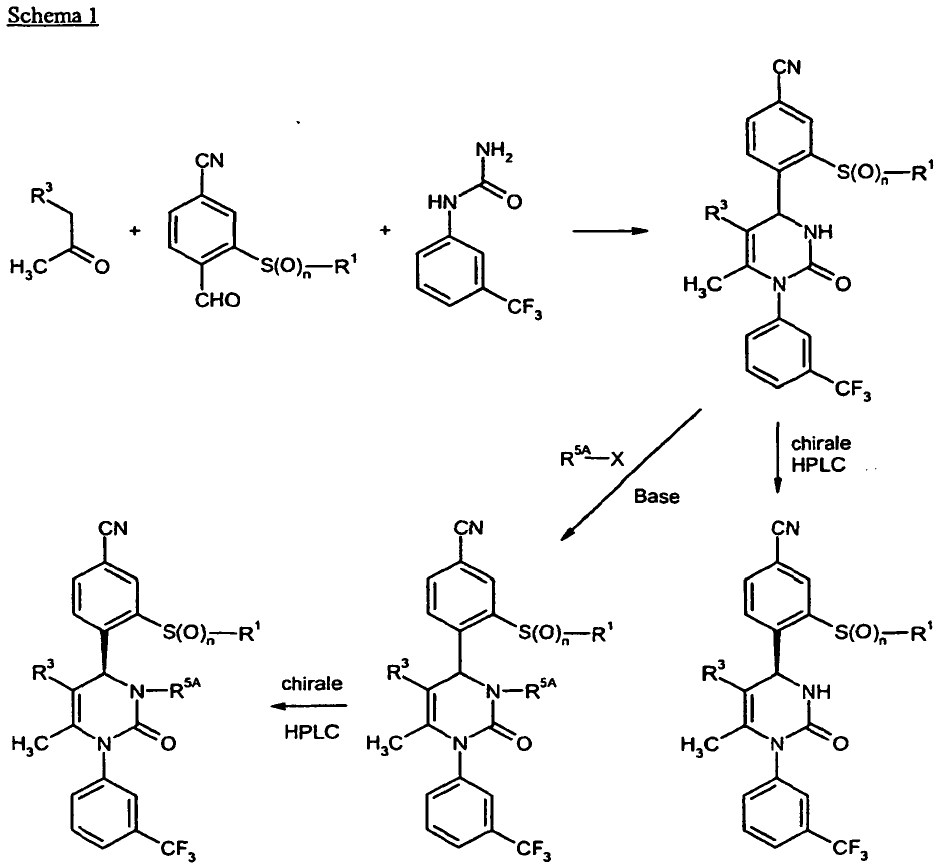 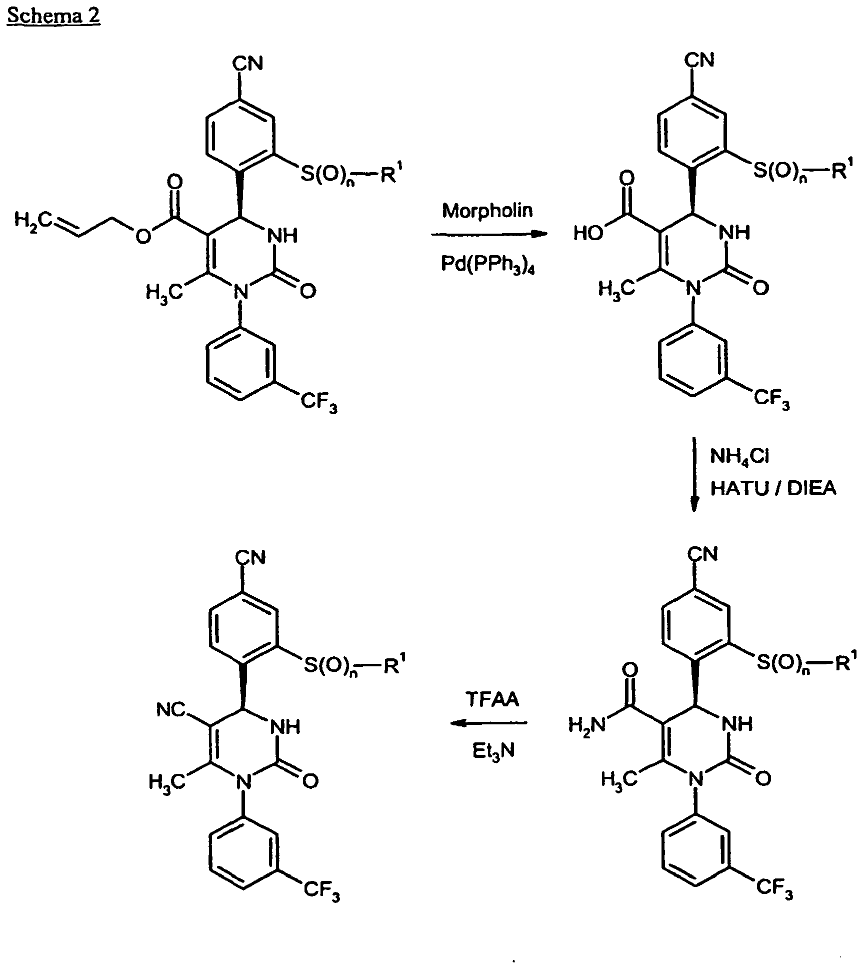 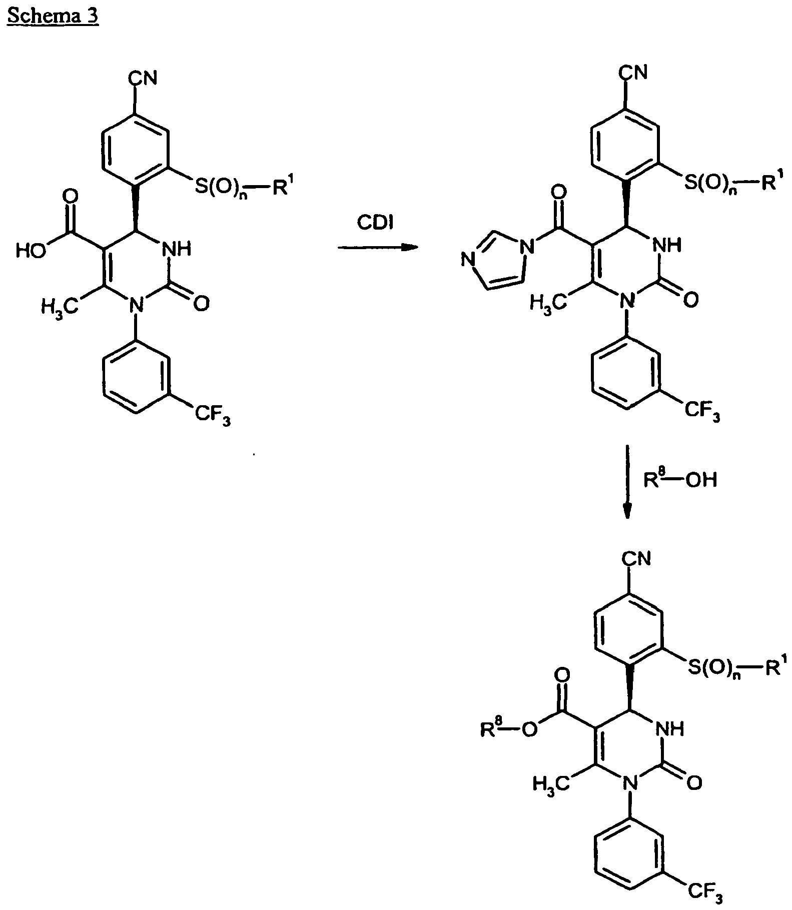 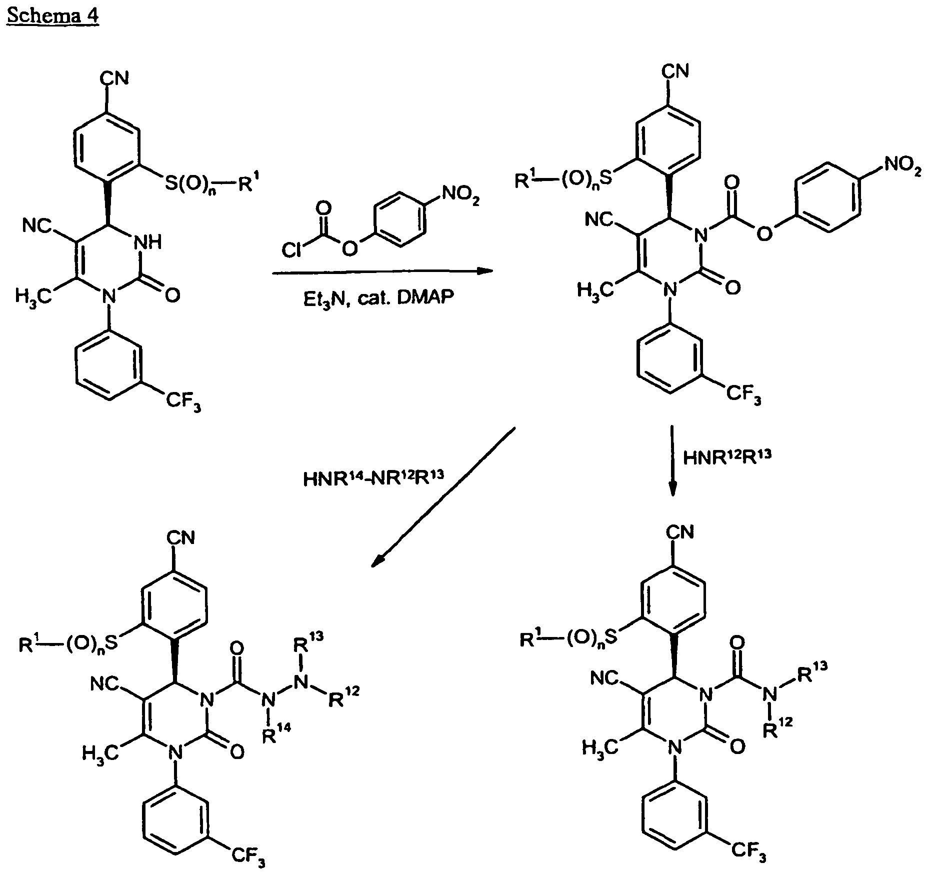 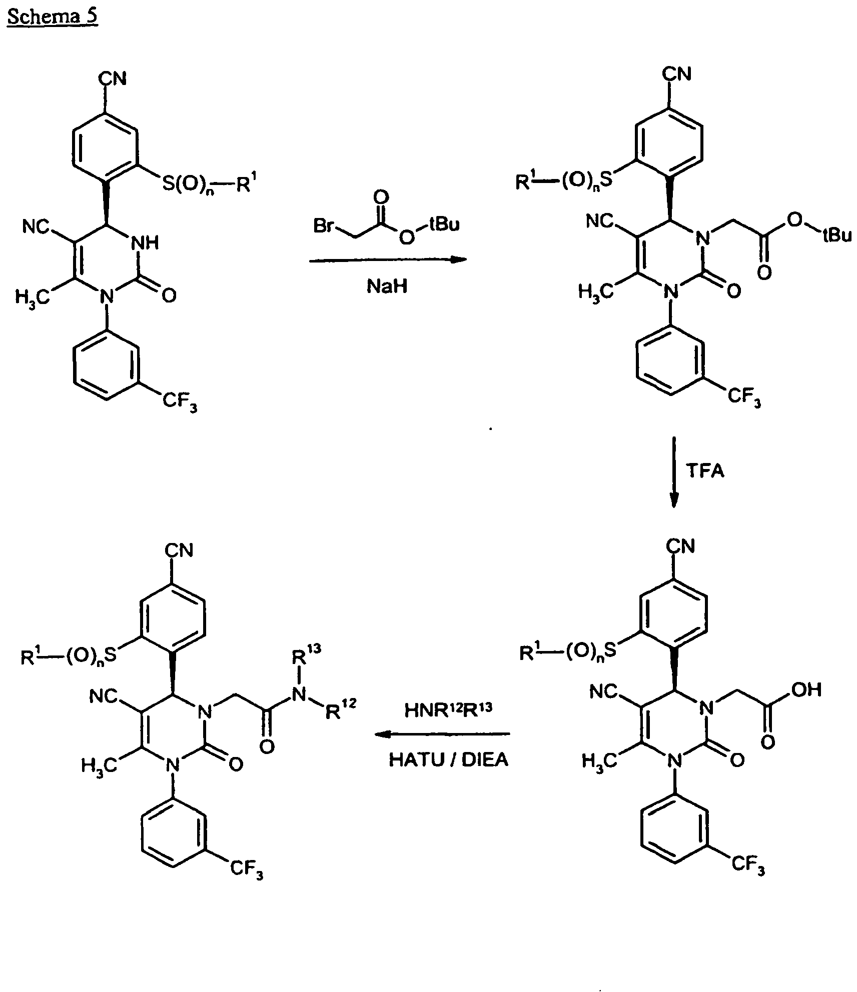 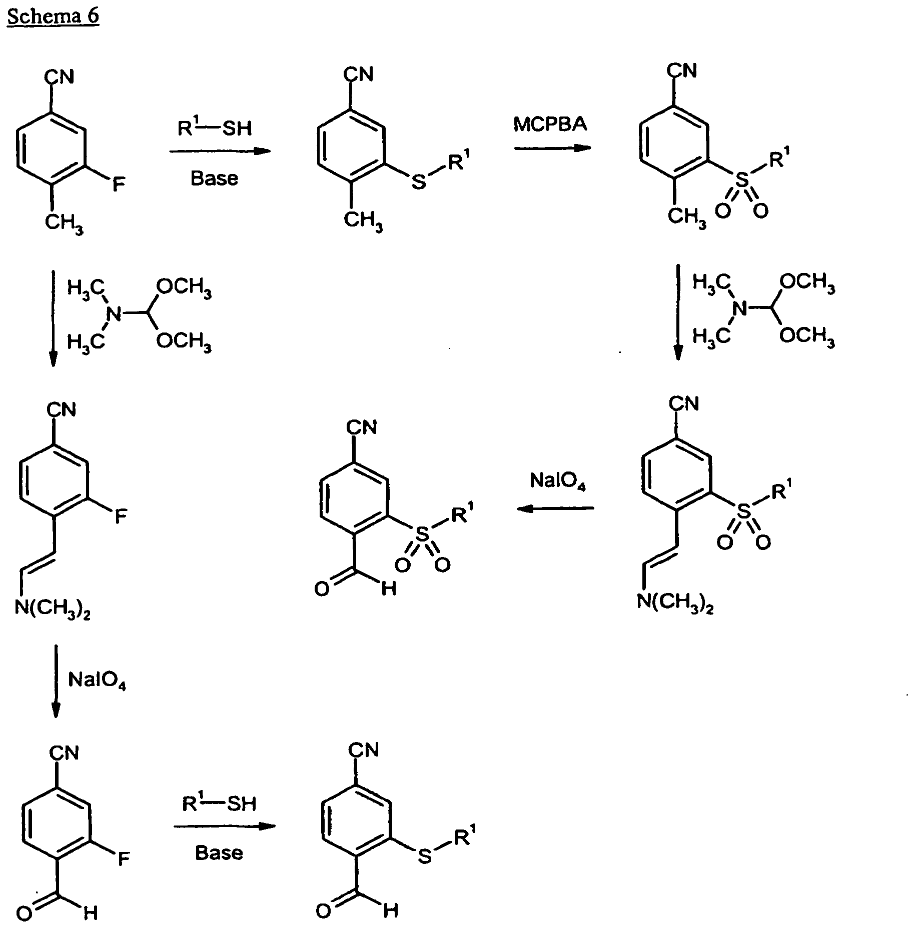 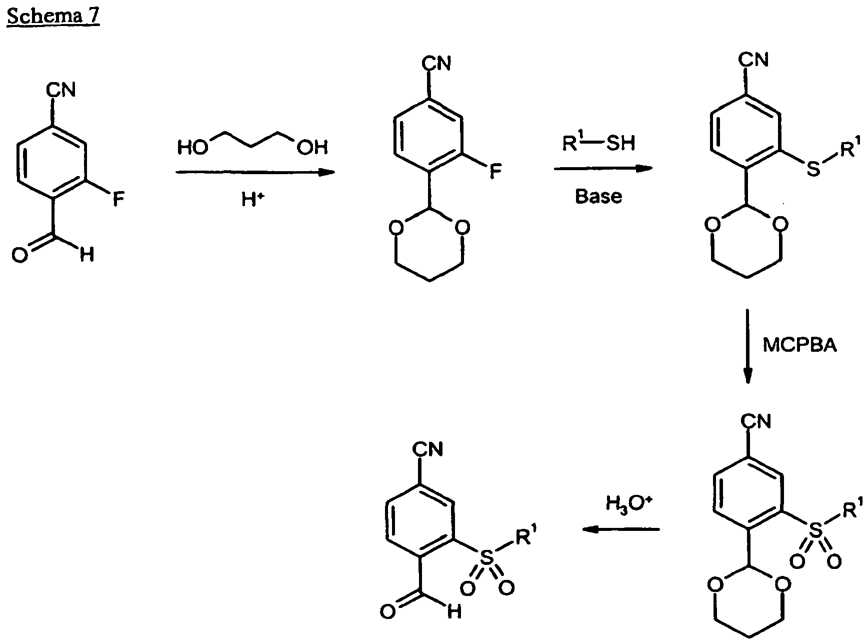 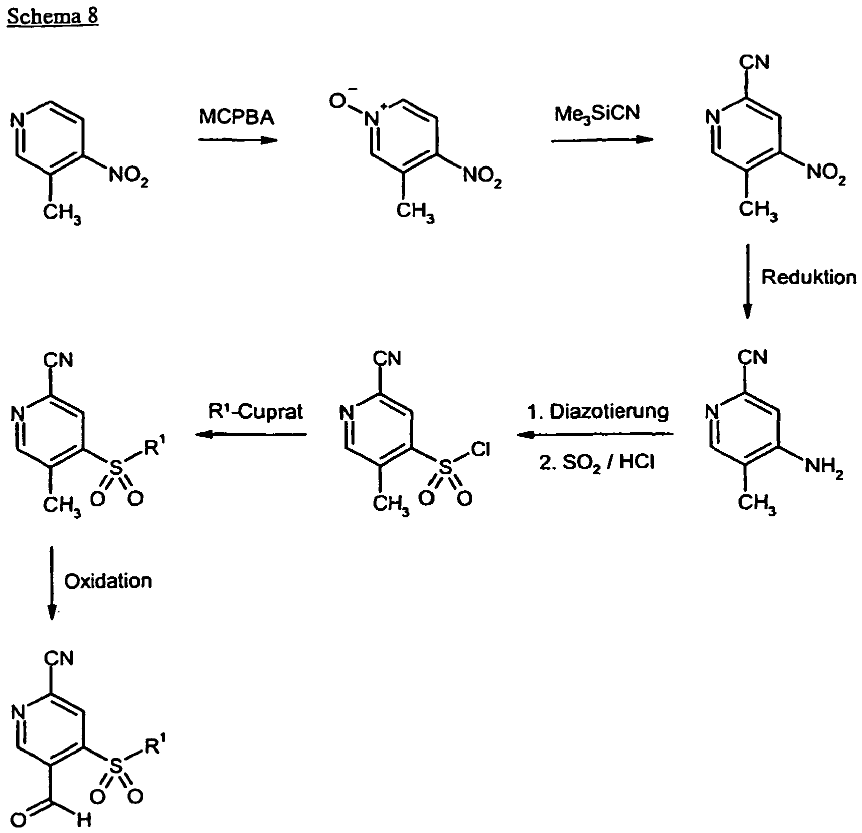 [vgl. z.B. W.K. Fife, J. Org. Chem. 48, 1375 (1983); H. Vorbrüggen und K. Krolikiewicz, Synthesis, 316 (1983); R.T. Shuman et al., J. Org. Chem. 55, 738 (1990); C.S. Burgey et al., J. Med. Chem. 46 (4), 461 (2003); V.M. Naidan et al., J. Gen. Chem. USSR (Engl. Transl.) 55 (2), 346 (1985)]. 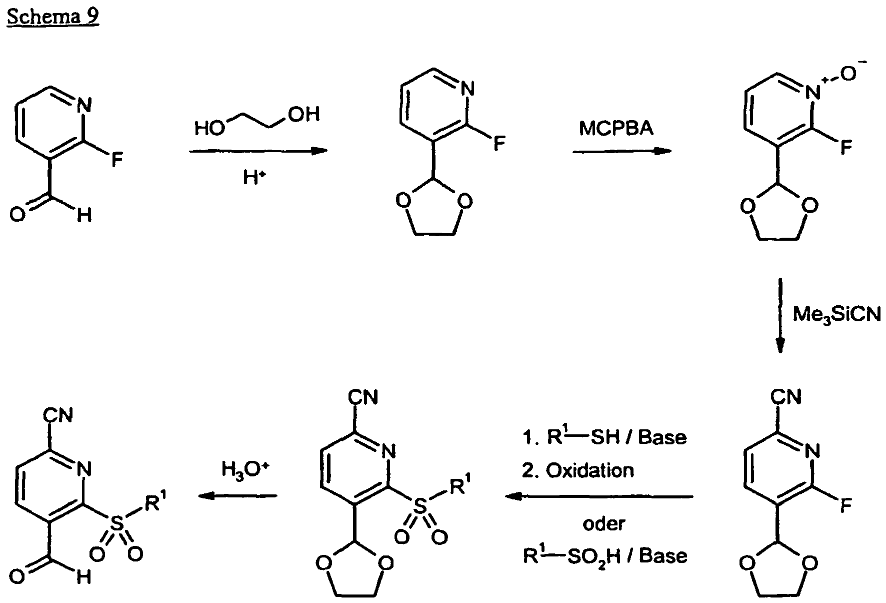 [vgl. z.B. W.K. Fife, J. Org. Chem. 48, 1375 (1983); H. Vorbrüggen und K. Krolikiewicz, Synthesis, 316 (1983); R.T. Shuman et al., J. Org. Chem. 55, 738 (1990); C.S. Burgey et al., J. Med. Chem. 46 (4), 461 (2003); J.J. Li et al., J. Med. Chem. 39, 1846 (1996); K.N. Dack et al., Bioorg. Med. Chem. Lett. 8 (16), 2061 (1998)].

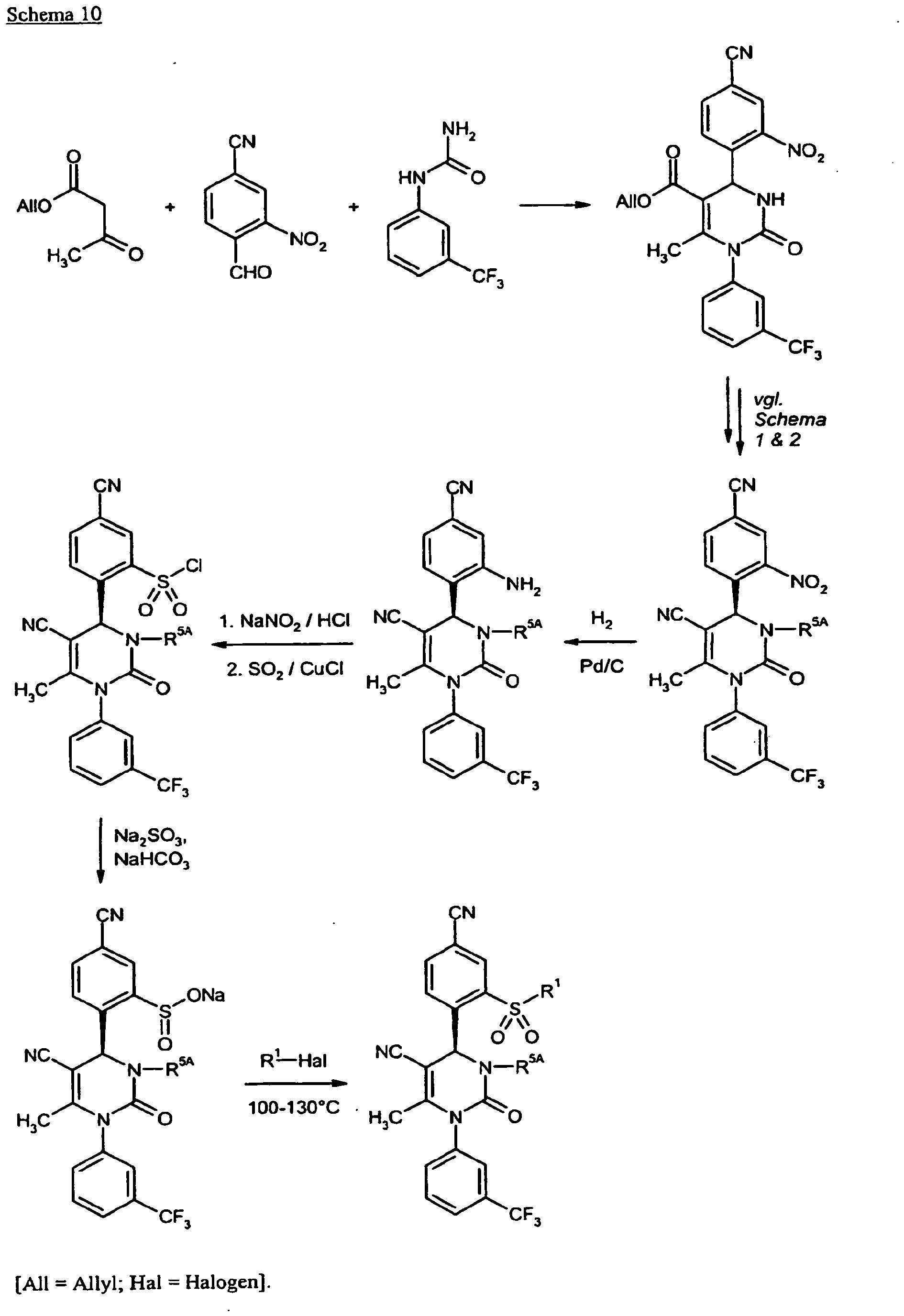

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen sind niedermolekulare, nicht-reaktive und selektive Inhibitoren der humanen neutrophilen Elastase, die überraschenderweise eine deutlich stärkere Inhibition dieser Protease im Vergleich zu den aus dem Stand der Technik bekannten Verbindungen zeigen. Darüber hinaus weisen die erfindungsgemäßen Verbindungen unerwartet eine niedrigere *in vitro-*Clearance gegenüber Hepatozyten auf und verfügen damit über eine verbesserte metabolische Stabilität.

Die erfindungsgemäßen Verbindungen eignen sich daher in besonderem Maße zur Behandlung und/oder Prävention von Erkrankungen und pathologischen Prozessen, insbesondere solchen, bei denen im Zuge eines Entzündungsgeschehens und/oder eines Gewebe- oder Gefäßumbaus die neutrophile Elastase (HNE) involviert ist.

Dazu zählen im Sinne der vorliegenden Erfindung insbesondere Erkrankungen wie die pulmonale arterielle Hypertonie (PAH) und andere Formen der pulmonalen Hypertonie (PIT), die chronisch-obstruktive Lungenerkrankung (COPD), das akute Atemwegssyndrom (ARDS), akute Lungenschädigung (ALI), alpha-1-Antitrypsin-Defizienz (AATD), Lungenfibrose, Lungenemphysem, zystische Fibrose (CF), akutes Koronarsyndrom (ACS), Herzmuskelentzündungen (Myokarditis) und andere autoimmune Herzerkrankungen (Perikarditis, Endokarditis, Valvolitis, Aortitis, Kardiomyopathien), Myokardinfarkt, kardiogener Schock, Herzinsuffizienz, Aneurysmen, Sepsis (SIRS), multiples Organversagen (MODS, MOF), Arteriosklerose, entzündliche Erkrankungen der Niere, chronische Darmentzündungen (IBD, CD, UC), Pankreatitis, Peritonitis, rheumatoide Erkrankungen, entzündliche Hauterkrankungen sowie entzündliche Augenerkrankungen.

Die erfindungsgemäßen Verbindungen können weiterhin verwendet werden zur Behandlung und/oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiolitis obliterans, Bronchiektasie, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Darüber hinaus können die erfindungsgemäßen Verbindungen auch eingesetzt werden zur Behandlung und/oder Prävention von mikro- und makrovaskulären Schädigungen (Vasculitis), Reperfusionsschäden, arteriellen sowie venösen Thrombosen, diabetischer und nicht-diabetischer Nephropathie, der Glomerulonephritis, der Glomerulosklerose, des nephrotischen Syndroms, der hypertensiven Nephrosklerose, der Mikroalbuminurie, der akuten und chronischen Niereninsuffizienz, des akuten und chronischen Nierenversagens, Cystitis, Urethritis, Prostatitis, Epidymititis, Oophoritis, Salpingitis, Vulvovaginitis, erektiler Dysfunktion, Hunner-Geschwür, Peyronie-Krankheit, arterieller Hypertonie, Schock, atrialen und ventrikulären Arrhythmien, transitorischen und ischämischen Attacken, Herzmuskelschwäche, Hirnschlag, endothelialer Dysfunktion, peripheren und kardialen Gefäßerkrankungen, peripheren Durchblutungsstörungen, Ödembildung wie zum Beispiel pulmonales Ödem, Hirnödern, renales Ödem und Herzinsuffizienz-bedingtes Ödem, Restenosen wie nach Thrombolysetherapien, percutan-transluminalen Angioplastien (PTA), transluminalen Koronarangioplastien (PTCA), Herztransplantationen und Bypass-Operationen, bei erhöhtem Spiegel von Fibrinogen und von LDL geringer Dichte sowie bei erhöhten Konzentrationen von Plasminogenaktivator-Inhibitor 1 (PAI-1), von Dyslipidämien (Hypercholesterolämie, Hypertriglyceridämie, erhöhte Konzentrationen der postprandialen Plasma-Triglyceride, Hypoalphalipoproteinämie, kombinierte Hyperlipidämien) sowie metabolischen Erkrankungen (Metabolisches Syndrom, Hyperglykämie, Insulin-abhängiger Diabetes, Nicht-Insulin-abhängiger Diabetes, Gestationsdiabetes, Hyperinsulinämie, Insulinresistenz, Glukose-Intoleranz, Fettsucht (Adipositas) und diabetische Spätfolgen wie Retinopathie, Nephropathie und Neuropathie), Krebserkrankungen (Hautkrebs, Hirntumore, Brustkrebs, Knochenmarktumore, Leukämien, Liposarcome, Karzinome des Magen-Darm-Traktes, der Leber, Bauchspeicheldrüse, Lunge, Niere, Harnleiter, Prostata und des Genitaltraktes sowie bösartige Tumore des lymphoproliferativen Systems wie z.B. Hodgkin's und Non-Hodgkin's Lymphom), von Erkrankungen des Gastrointestinaltraktes und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von pulmonaler arterieller Hypertonie (PAH) und anderen Formen der pulmonalen Hypertonie (PH), chronisch-obstruktiven Lungenerkrankungen (COPD), akuter Lungenschädigung (ALI), akutem Atemwegssyndrom (ARDS), Lungenemphysem, alpha-1-Antitrypsin-Defizienz (AATD), zystischer Fibrose (CF), Sepsis und systemisch-inflammatorischem Response-Syndrom (SIRS), multiplem Organversagen (MOF, MODS), entzündlichen Darmerkrankungen (IBD, Morbus Crohn, Colitis), chronischer Bronchitis, Bronchiolitis, Asthma, Rhinitis, rheumatoider Arthritis, entzündlichen Haut- und Augenkrankheiten, Arteriosklerose und Krebserkrankungen.

Weiterer Gegenstand der vorliegenden Erfindung ist die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung ist ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als geeignete Kombinationswirkstoffe seien beispielhaft und vorzugsweise genannt:
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- NO-unabhängige, jedoch Häm-abhängige Stimulatoren der löslichen Guanylatcyclase, wie insbesondere die in WO 00/06568, WO 00/06569, WO 02/42301 und WO 03/095451 beschriebenen Verbindungen;
- NO- und Häm-unabhängige Aktivatoren der löslichen Guanylatcyclase, wie insbesondere die in WO 01/19355, WO 01/19776, WO 01/19778, WO 01/19780, WO 02/070462 und WO 02/070510 beschriebenen Verbindungen;
- Prostacyclin-Analoga, wie beispielhaft und vorzugsweise Iloprost, Beraprost, Treprostinil oder Epoprostenol;
- Verbindungen, die die lösliche Epoxidhydrolase (sEH) inhibieren, wie beispielsweise *N,N*'-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazine oder Trimetazidine;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Inhibitoren, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika;
- bronchodilatorisch wirkende Mittel, beispielhaft und vorzugsweise aus der Gruppe der betaadrenergen Rezeptor-Agonisten, wie insbesondere Albuterol, Isoproterenol, Metaproterenol, Terbutalin, Formoterol oder Salmeterol, oder aus der Gruppe der Anticholinergika, wie insbesondere Ipratropiumbromid;
- anti-inflammatorisch wirkende Mittel, beispielhaft und vorzugsweise aus der Gruppe der Glucocorticoide, wie insbesondere Prednison, Prednisolon, Methylprednisolon, Triamcinolon, Dexamethason, Beclomethason, Betamethason, Flunisolid, Budesonid oder Fluticason; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Bei einer bevorzugten Ausführungsform der Erfindung werden die erfindungsgemäßen Verbindungen in Kombination mit einem Kinase-Inhibitor, wie beispielhaft und vorzugsweise Bortezomib, Canertinib, Erlotinib, Gefitinib, Imatinib, Lapatinib, Lestaurtinib, Lonafamib, Pegaptinib, Pelitinib, Semaxanib, Sorafenib, Sunitinib, Tandutinib, Tipifarnib, Vatalanib, Fasudil, Lonidamin, Leflunomid, BMS-3354825 oder Y-27632, eingesetzt.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, DU-176b, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten, Rho-Kinase-Inhibitoren sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazern, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Inhibitor, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Rho-Kinase-Inhibitor, wie beispielhaft und vorzugsweise Fasudil, Y-27632, SLx-2119, BF-66851, BF-66852, BF-66853, KI-23095, SB-772077, GSK-269962A oder BA-1049, verabreicht.

Bei einer bevorzugten Ausführungsform der Erfindung werden die erfindungsgemäßen Verbindungen in Kombination mit einem Diuretikum, wie beispielhaft und vorzugsweise Furosemid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin, Cerivastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nicht-toxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Aerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale, die intravenöse und die inhalative Applikation.

Die erfindungsgemäßen Verbindungen können in die angeführten Applikationsformen überführt werden. Dies kann in an sich bekannter Weise durch Mischen mit inerten, nicht-toxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich, sofern nicht anders angegeben, jeweils auf das Volumen.

### A. Beispiele

### Abkürzungen:

- aq.: wässrig, wässrige Lösung
- c: Konzentration
- cat.: katalytisch
- CDI: *N,N*'-Carbonyldiimidazol
- DC: Dünnschichtchromatographie
- DCI: direkte chemische Ionisation (bei MS)
- dest.: destilliert
- DIEA: *N*,*N*-Diisopropylethylamin
- DMAP: 4-*N*,*N*-Dimethylaminopyridin
- DMF: Dimethylformamid
- DMSO: Dimethylsulfoxid
- d. Th.: der Theorie (bei Ausbeute)
- ee: Enantiomerenüberschuss
- ent: enantiomerenrein, Enantiomer
- eq.: Äquivalent(e)
- ESI: Elektrospray-Ionisation (bei MS)
- Et: Ethyl
- GC-MS: Gaschromatographie-gekoppelte Massenspektrometrie
- h: Stunde(n)
- HATU: O-(7-Azabenzotriazol-1-yl)-*N,N,N;N'*-tetramethyluronium-Hexafluorophosphat
- HPLC: Hochdruck-, Hochleistungsflüssigchromatographie
- konz.: konzentriert
- LC-MS: Flüssigchromatographie-gekoppelte Massenspektrometrie
- MCPBA: meta-Chlorperbenzoesäure
- Me: Methyl
- min: Minute(n)
- MPLC: Mitteldruckflüssigchromatographie
- MS: Massenspektrometrie
- MTBE: Methyl-tert.-butylether
- NMR: Kernresonanzspektrometrie
- Ph: Phenyl
- PyBOP: Benzotriazol-1-yloxy-tris(pyrrolidino)phosphonium-hexafluorophosphat
- quant.: quantitativ (bei Ausbeute)
- rac: racemisch, Racemat
- RT: Raumtemperatur
- Rₜ: Retentionszeit (bei HPLC)
- Schmp.: Schmelzpunkt
- tBu: *tert*.-Butyl
- TFA: Trifluoressigsäure
- TFAA: Trifluoressigsäureanhydrid
- THF: Tetrahydrofuran
- UV: Ultraviolett-Spektrometrie
- v/v: Volumen zu Volumen-Verhältnis (einer Lösung)

### HPLC-, GC-MS- und LC-MS-Methoden:

### Methode 1 (GC-MS):

Instrument: Micromass GCT, GC 6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

### Methode 2 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 9.0 min 90% B → 9.2 min 2% B → 10 min 2% B; Fluss: 0.75 ml/min; Säulentemperatur: 30°C; UV-Detektion: 210 nm.

### Methode 3 (analytische HPLC):

Instrument: HP 1100 mit DAD-Detektion; Säule: Kromasil 100 RP-18, 60 mm x 2.1 mm, 3.5 µm; Eluent A: 5 ml HClO₄ (70%-ig) / Liter Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

### Methode 4 (LC-MS):

Instrument: Micromass QuattroPremier mit Waters UPLC Acquity; Säule: Thermo Hypersil GOLD 1.9µ 50 mm x 1 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 1.5 min 10% A → 2.2 min 10% A; Fluss: 0.33 ml/min; Ofen: 50°C; UV-Detektion: 210 nm.

### Methode 5 (LC-MS):

Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 0.1 min 90% A → 3.0 min 5% A → 4.0 min 5% A → 4.01 min 90% A; Fluss: 2 ml/min; Ofen: 50°C; UV-Detektion: 210 nm.

### Methode 6 (LC-MS):

Gerätetyp MS: Micromass ZQ; Gerätetyp HPLC: HP 1100 Series; UV DAD; Säule: Phenomenex Gemini 3µ 30 mm x 3.0 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2.5 min 30% A → 3.0 min 5% A → 4.5 min 5% A; Fluss: 0.0 min 1 ml/min → 2.5 min/3.0 min/4.5 min 2 ml/min; Ofen: 50°C; UV-Detektion: 210 nm.

### Methode 7 (präparative HPLC/MS):

Instrument MS: Waters ZQ 2000; Instrument HPLC: Agilent 1100, 2-Säulen-Schaltung; Autosampler: HTC PAL; Säule: YMC-ODS-AQ, 50 mm x 4.6 mm, 3.0 µm; Eluent A: Wasser +0.1% Ameisensäure, Eluent B: Acetonitril + 0.1 % Ameisensäure; Gradient: 0.0 min 100% A → 0.2 min 95% A → 1.8 min 25% A → 1.9 min 10% A → 2.0 min 5% A → 3.2 min 5% A → 3.21 min 100% A → 3.35 min 100% A; Ofen: 40°C; Fluss: 3.0 ml/min; UV-Detektion: 210 nm.

### Methode 8 (LC-MS):

Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%-ige Ameisensäure, Eluent B: 1 1 Acetonitril + 0.5 ml 50%-ige Ameisensäure; Gradient: 0.0 min 90% A → 2 min 65% A → 4.5 min 5% A → 6 min 5% A; Fluss: 2 ml/min; Ofen: 40°C; UV-Detektion: 210 nm.

### Methode 9 (LC-MS):

Instrument: Waters Acquity SQD UPLC System; Säule: Waters Acquity UPLC HSS T3 1.8µ, 50 mm x 1 mm; Eluent A: 1 1 Wasser + 0.25 ml 99%-ige Ameisensäure, Eluent B: 1 1 Acetonitril + 0.25 ml 99%-ige Ameisensäure; Gradient: 0.0 min 90% A → 1.2 min 5% A → 2.0 min 5% A; Fluss: 0.40 ml/min; Ofen: 50°C; UV-Detektion: 210-400 nm.

### Methode 10 (LC-MS):

Instrument: Micromass Quattro Micro MS mit HPLC Agilent Serie 1100; Säule: Thermo Hypersil GOLD 3µ 20 mm x 4 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 3.0 min 10% A → 4.0 min 10% A → 4.01 min 100% A (Fluss 2.5 ml/min) → 5.00 min 100% A; Ofen: 50°C; Fluss: 2 ml/min; UV-Detektion: 210 nm.

### Ausgangsverbindungen und Intermediate:

### Beispiel 1A

4-Methyl-3-(methylsulfanyl)benzonitril

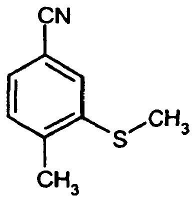

### Methode A:

Die Reaktion wurde unter Argon durchgeführt. 3-Fluor-4-methylbenzonitril (3000 mg, 22.2 mmol) und Natriummethanthiolat (1572 mg, 20.2 mmol) wurden in DMF (30 ml) vorgelegt, mit Kaliumcarbonat (6973 mg, 50.5 mmol) versetzt und über Nacht unter Rückfluss gerührt. Der Ansatz wurde danach eingeengt, der Rückstand mit Methylenchlorid/Methanol (10:1) aufgeschlämmt und das darin unlösliche Kaliumcarbonat abfiltriert. Das Filtrat wurde wieder eingeengt und der Rückstand an Kieselgel chromatographiert (Laufmittel: Cyclohexan/Essigsäureethylester 10:1). Man erhielt 2.51 g (64% d. Th.) der gewünschten Verbindung.

### Methode B:

Die Reaktion wurde unter Zuhilfenahme eines Chlorlaugenwäschers durchgeführt. 3-Fluor-4-methylbenzonitril (200 g, 1479.9 mmol) wurde in DMF (1.5 Liter) vorgelegt, auf 40°C erwärmt und portionsweise (je ca. 25 g) mit Natriummethanthiolat (insgesamt 126.8 g, 1627.9 mmol) versetzt. Während der Zugabe stieg die Temperatur auf 100°C an. Das Reaktionsgemisch wurde zunächst 1.5 h bei 175°C Badtemperatur und dann über Nacht bei Raumtemperatur gerührt. Der Ansatz wurde danach auf Wasser (7.5 Liter) gegossen und zweimal mit Essigsäureethylester (jeweils 1875 ml) extrahiert. Die vereinigten organischen Phasen wurden mit gesättigter Natriumchlorid-Lösung (1875 ml) gewaschen, am Rotationsverdampfer eingeengt und der Rückstand an Kieselgel chromatographiert (Laufmittel: Petrolether/Essigsäureethylester 95:5, ca. 30 Liter). Nach Abziehen des Lösungsmittels am Rotationsverdampfer und Trocknen im Hochvakuum erhielt man 172 g (71% d. Th.) der gewünschten Verbindung.
GC-MS (Methode 1): Rₜ = 5.25 min; MS (ESIpos): *m*/*z* (%) = 163.0 (100) [M]⁺
¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 2A

4-Methyl-3-(methylsulfonyl)benzonitril

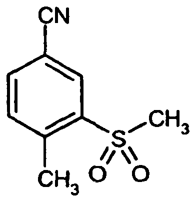

### Methode A:

4-Methyl-3-(methylsulfanyl)benzonitril (14050 mg, 80.1 mmol; Beispiel 1A) wurde in Dichlormethan (700 ml) gelöst, auf 0°C gekühlt und langsam mit 3-Chlorperbenzoesäure (50923 mg, 206.6 mmol) versetzt. Anschließend wurde zunächst 40 min bei 0°C und dann über Nacht bei Raumtemperatur gerührt. Die ausgefallene 3-Chlorbenzoesäure wurde abfiltriert, das Filtrat mit 1 N Natronlauge gewaschen, die organische Phase über Natriumsulfat getrocknet und eingeengt. Der Rückstand wurde mittels Kieselgel-Chromatographie gereinigt (Laufmittel: Cyclohexan/Essigsäureethylester 1:1, 1:2). Man erhielt 13.65 g (81% d. Th.) der gewünschten Verbindung.

### Methode B:

3-Chlorperbenzoesäure (2501 g, 10144.4 mmol) wurde in 27.2 Liter Dichlormethan gelöst, auf 10°C gekühlt und portionsweise mit 4-Methyl-3-(methylsulfanyl)benzonitril (552 g, 3381.5 mmol; Beispiel 1A) versetzt. Nach vollständiger Zugabe wurde 5 h bei RT gerührt. Die ausgefallene 3-Chlorbenzoesäure wurde abgesaugt und der Feststoff mit Dichlormethan (3 Liter) nachgewaschen. Die vereinigten Filtrate wurden mit 1 N Natronlauge (15 Liter) gerührt, das Gemisch filtriert und die organische Phase abgetrennt. Diese wurde nochmals mit 1 N Natronlauge (15 Liter) gerührt, von der Natronlauge abgetrennt, getrocknet und am Rotationsverdampfer eingeengt. Der Rückstand wurde in Diethylether (4 Liter) aufgeschlämmt, 10 min gerührt und dann filtriert. Der Feststoff wurde mit etwas Diethylether nachgewaschen und im Hochvakuum getrocknet. Man erhielt 613 g (93% d. Th.) der gewünschten Verbindung.
GC-MS (Methode 1): Rₜ = 6.59 min; MS (ESIpos): *m*/*z* (%) = 195.0 (100) [M]⁺
¹H-NMR (400 MHz, DMSO-d₆): δ = 2.30 (s, 3H), 2.54 (s, 3H), 7.38 (d, 1H), 7.52 (dd, 1H), 7.58 (br. s, 1H).

### Beispiel 3A

4-[2-(Dimethylamino)ethenyl]-3-(methylsulfonyl)benzonitril

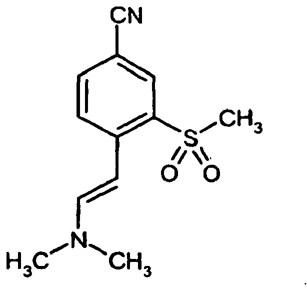

### Methode A:

Die Reaktion wurde unter Argon durchgeführt. 4-Methyl-3-(methylsulfonyl)benzonitril (13000 mg, 66.6 mmol; Beispiel 2A) und 1,1-Dimethoxy-*N,N*-dimethylmethanamin (10315 mg, 86.6 mmol) wurden 14 h bei 140°C in DMF (200 ml) gerührt. Zur Vervollständigung der Reaktion wurde danach erneut 1,1-Dimethoxy-*N,N*-dimethylmethanamin (3967 mg, 33.3 mmol) zugegeben und weitere 24 h bei 140°C gerührt. Das DMF wurde dann am Rotationsverdampfer abgezogen und der Rückstand ohne weitere Aufreinigung in der Folgestufe umgesetzt.

### Methode B:

Die Reaktion wurde unter Argon durchgeführt. 4-Methyl-3-(methylsulfonyl)benzonitril (612 g, 3134.6 mmol; Beispiel 2A) wurde in DMF (6.12 Liter) vorgelegt, mit 1,1-Dimethoxy-*N*,*N*-di-methylmethanamin (859 g, 7209.5 mmol) versetzt und 7 h bei 140°C gerührt. Die Reaktionsmischung wurde dann auf 35 Liter 10%=ige Natriumchlorid-Lösung gegossen und zweimal mit jeweils 10 Liter Essigsäureethylester extrahiert. Die vereinigten organischen Phasen wurden mit gesättigter Natriumchlorid-Lösung (5 Liter) gewaschen, getrocknet, am Rotationsverdampfer eingeengt und der Rückstand über Nacht im Hochvakuum getrocknet. Auf diese Weise erhielt man 1098 g (98% d. Th.) der gewünschten Verbindung.
GC-MS (Methode 1): Rₜ = 8.95 min; MS (ESIpos): *m*/*z* (%) = 250.0 (10) [M]⁺.

### Beispiel 4A

4-Formyl-3-(methylsulfonyl)benzonitril

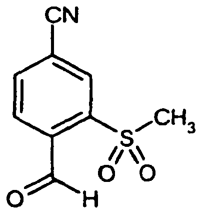

### Methode A:

4-[2-(Dimethylamino)ethenyl]-3-(methylsulfonyl)benzonitril (16666 mg, 66.6 mmol; Beispiel 3A) wurde in Wasser/THF (1:1, 500 ml) vorgelegt, mit Natriumperiodat (42722 mg, 199.7 mmol) versetzt und über Nacht bei Raumtemperatur gerührt. Der ausgefallene Feststoff wurde abfiltriert und mit Essigsäureethylester nachgewaschen. Die vereinigten organischen Phasen wurden mit gesättigter Natriumhydrogencarbonat- und gesättigter Natriumchlorid-Lösung gewaschen, über Natriumsulfat getrocknet, filtriert und eingeengt. Der Rückstand wurde per Kieselgel-Chromatographie gereinigt (Laufmittel: Cyclohexan/Essigsäureethylester 1:1). Man erhielt 4.6 g (33% d. Th.) der gewünschten Verbindung.

### Methode B:

4-[2-(Dimethylamino)ethenyl]-3-(methylsulfonyl)benzonitril (1098 g, 3070.5 mmol; Beispiel 3A) wurde in THF/Wasser (1:1, 13.8 Liter) vorgelegt, mit Natriumperiodat (1970 g, 9211.4 mmol) versetzt und 1 h bei Raumtemperatur gerührt. Der ausgefallene Feststoff wurde abgesaugt und mit Essigsäureethylester (17 Liter) nachgewaschen. Die vereinigten Filtrate wurden mit Wasser (17 Liter) versetzt, und nach erfolgter Extraktion wurde die wässrige Phase abgetrennt. Die organische Phase wurde mit gesättigter Natriumhydrogencarbonat-Lösung (8.5 Liter) und gesättigter Natriumchlorid-Lösung (8.5 Liter) gewaschen, dann getrocknet und am Rotationsverdampfer eingeengt. Die Reinigung des Rückstands erfolgte mittels Kieselgel-Chromatographie (Laufmittel: Dichlormethan/Essigsäureethylester 9:1, 60 Liter). Die Produktfraktionen wurden eingeengt, der Rückstand in Petrolether aufgeschlämmt, dann abgesaugt und der Feststoff im Hochvakuum über Nacht getrocknet. Auf diese Weise erhielt man 436 g (65% d. Th.) der gewünschten Verbindung.
GC-MS (Methode 1): Rₜ = 6.89 min; MS (ESIpos): *m*/*z* (%) = 191.1 (15) [M-18]⁺, 161.0 (100).
¹H-NMR (400 MHz, DMSO-d₆: δ = 3.57 (s, 3H), 8.10 (d, 1H), 8.39 (dd, 1H), 8.45 (d, 1H), 10.63 (s, 1H).

### Beispiel 5A

(4*S*)-4-(4-Cyano-2-(methylsulfonyl)phenyl]-6-methyl-2-oxo-1-[3-(trifluormethyl)phenyl]-1,2,3,4-tetrahydropyrimidin-5-carbonsäure 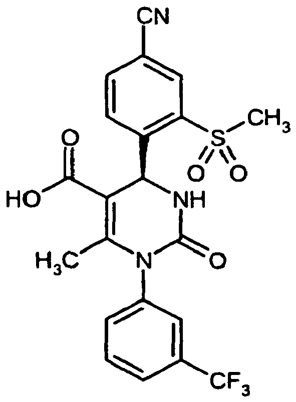 Die Reaktion wurde unter Argon durchgeführt. (4S)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (800 mg, 1.54 mmol; Beispiel 4) und Morpholin (1.5 eq., 201 mg, 2.31 mmol) wurden in trockenem THF (25 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 89 mg, 0.077 mmol) zugegeben und das Reaktionsgemisch 90 min bei RT gerührt (IPLC-Monitoring). Das Reaktionsgemisch wurde dann eingeengt und der Rückstand in Essigsäureethylester (500 ml) aufgenommen. Die organische Phase wurde mit gesättigter Ammoniumchlorid-Lösung (50 ml), mit Wasser (50 ml) und mit konz. Kochsalz-Lösung (50 ml) gewaschen, über Natriumsulfat getrocknet, filtriert und im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90:10). Es wurde ein farbloser, amorpher Feststoff erhalten (696 mg, 86% Reinheit, 81% d. Th.).
LC-MS (Methode 6): Rₜ = 2.1 min; MS (ESIpos): m/z (%) = 480.1 (100) [M+H]⁺; MS (ESIneg): *m*/*z* (%) = 478.1 (100) [M-H]-
¹H-NMR (400 MHz, DMSO-d₆): δ = 2.14 (s, 3H), 3.45 (s, 3H), 6.35 (d, 1H), 7.14 (d, 1H), 7.72 (m, 2H), 7.80 (m, 1H), 7.86 (s, 1H), 8.11 (d, 1H), 8.27 (d, 1H), 8.36 (s, 1H), 12.64 (br. s, 1H).

### Beispiel 6A

4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat 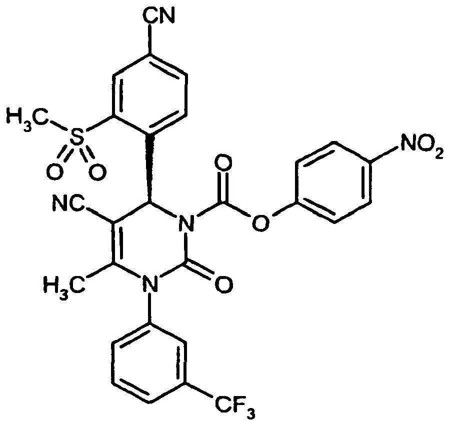 (4*S*)-4-[4-Cyano-2(methylsulfonyl)phenyl]-6-methyl-2-oxo-1-[3-(trifluormethyl)phenyl]-1,2,3,4-tetrahydropyrimidin-5-carbonitril (1.00 g, 2.17 mmol; Beispiel 6) und Triethylamin (659 mg, 6.52 mmol) wurden zusammen mit einer Spatelspitze 4-*N,N*-Dimethylaminopyridin in Dichlormethan (8.3 ml) suspendiert. Anschließend wurde Chlorameisensäure-4-nitrophenylester (875 mg, 4.34 mmol) hinzugefügt. Die entstandene Lösung wurde 5 min bei Raumtemperatur nachgerührt und dann mit Wasser (1 ml) versetzt. Weiterhin wurde Toluol (7 ml) zum Kolbeninhalt gegeben, und der Ansatz wurde im Vakuum eingeengt. Der Rückstand wurde über Kieselgel flash-chromatographiert (Laufmittel: Dichlormethan). Die eingeengten Produktfraktionen wurden in Ethanol (20 ml) verrührt und der resultierende Feststoff abgesaugt. Man erhielt so die Zielverbindung (559 mg, 39% d. Th.).
HPLC (Methode 3): Rₜ = 4.92 min (94%); MS (ESIpos): *m*/*z* (%) = 626.3 (18) [M+H]⁺
¹H-NMR (400 MHz, DMSO-d₆): δ = 1.88 (s, 3H), 3.39 (s, 3H), 7.31 (s, 1H), 7.37 (d, 2H), 7.78-8.22 (m, 5H), 8.24 (d, 2H), 8.41 (d, 1H), 8.51 (s, 1H).

### Beispiel 7A

(*rac*)-2,3-Dibrompropyl-6-(brommethyl)-4-[4-cyano-2-(methylsulfonyl)phenyl]-2-oxo-1-[3-(trifluormethyl)phenyl]-1,2,3,4-tetrahydropyrimidin-5-carboxylat

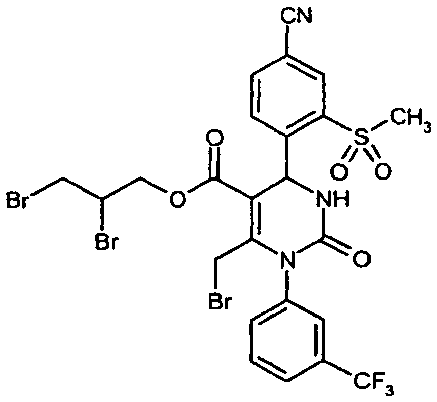

Bei 0°C wurde zu einer Lösung von (*rac*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (Beispiel 3; 519 mg, 1.0 mmol) in Chloroform (10 ml) eine Lösung von Brom (335.6 mg, 2.1 mmol, 2.1 eq.) in Chloroform (2 ml) getropft. Das Reaktionsgemisch wurde bei RT über Nacht gerührt. Danach zeigte die HPLC-Kontrolle vollständigen Umsatz. Das Gemisch wurde mit Dichlormethan (50 ml) verdünnt und anschließend mit 10%-iger wässriger Natriumsulfit-Lösung (2 x 50 ml) sowie gesättigter wässriger Kochsalz-Lösung (30 ml) gewaschen. Die organische Phase wurde über Natriumsulfat getrocknet, filtriert, im Vakuum eingeengt und der Rückstand im Hochvakuum getrocknet. Man erhielt einen Feststoff als Rohprodukt (880 mg, quant., Reinheit ca. 94% laut LC-MS), der ohne weitere Aufreinigung weiter umgesetzt wurde.

LC-MS (Methode 6): Rₜ = 2.79 min; MS (ESIpos): *m*/*z* (%) = 759.8 (100) [M+H]⁺; MS (ESIneg): *m*/*z* (%) = 714.9 (90), 758.0 (100) [M-H]⁻.

### Beispiel 8A

3-Fluor-4-formylbenzonitril

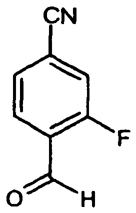

Die Reaktion wurde unter Argon durchgeführt. 3-Fluor-4-methylbenzonitril (121 g, 895 mmol) und *N,N*-Dimethylformamid-dimethylacetal (245 g, 2.06 mol) wurden in DMF (1.8 Liter) gelöst und über Nacht unter Rückfluss gerührt. Der Kolbeninhalt wurde danach auf Wasser (2 Liter) gegossen, und es wurde zweimal mit Essigsäureethylester extrahiert und die vereinigten organischen Phasen mit gesättigter Kochsalz-Lösung gewaschen. Die organische Phase wurde eingeengt und der Rückstand in THF/Wasser (1:1, 2.7 Liter) erneut gelöst. Es wurde mit Natriumperiodat (503 g, 2.35 mol) versetzt und eine Stunde bei Raumtemperatur gerührt. Dann wurde vom ausgefallenen Niederschlag abgetrennt, das Filtrat gewonnen und mehrfach mit Essigsäureethylester extrahiert. Die vereinigten organischen Phasen wurden einmal mit gesättigter Natriumhydrogencarbonat-Lösung und einmal mit gesättigter Natriumchlorid-Lösung gewaschen, getrocknet und zu einem Öl eingeengt. Dieses wurde per Säulenchromatographie an Kieselgel gereinigt (Laufmittel: Petrolether/Dichlormethan 6:4, dann 4:6, zuletzt reines Dichlormethan). Die Produktfraktionen wurden eingeengt. Man erhielt 28.0 g (20% d. Th.) der Zielverbindung als weißen kristallinen Feststoff.
GC-MS (Methode 1): Rₜ = 3.63 min; MS (ESIpos): *m*/*z* (%) = 149.0 (48) [M]⁺, 150.0 (5) [M+H]⁺
¹H-NMR (400 MHz, DMSO-d₆): δ = 7.89 (d, 1H), 8.00 (t, 1H), 8.11 (d, 1H), 10.24 (s, 1H).

### Beispiel 9A

4-Formyl-3-(methylsulfanyl)benzonitril 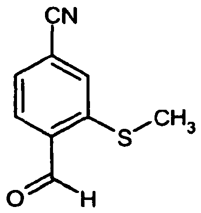 3-Fluor-4-formylbenzonitril (2.00 g, 13.4 mmol; Beispiel 8A) wurde in DMSO (27 ml) gelöst und unter Eisbad-Kühlung mit Natriummethanthiolat (1.50 g, 21.5 mmol) versetzt. Es wurde 45 min nachgerührt und dann mit Wasser (100 ml) verdünnt. Das dabei ausfallende Produkt wurde abgesaugt, mit Wasser gewaschen und im Vakuum getrocknet. Man erhielt 1.36 g (51% d. Th.) der Zielverbindung als gelben kristallinen Feststoff.
GC-MS (Methode 1): Rₜ = 5.90 min; MS (ESIpos): *m*/*z* (%) = 177.0 (100) [M]⁺, 178.0 (11) [M+H]⁺.

### Beispiel 10A

(*rac*)-4-[4-Cyano-2-(methylsulfanyl)phenyl]-6-methyl-2-oxo-1-[3gtrifluomethyl)phenyl]-1,2,3,4-tetrahydropyrimidin-5-carbonsäure 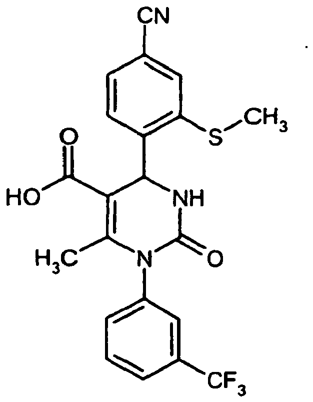 (*rac*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure-altytester (750 mg, 1.54 mmol; Beispiel 39) wurde in THF (10 ml) gelöst und mit Morpholin (201 mg, 2.308 mmol) versetzt. Die Reaktionslösung wurde mit Argon gesättigt (30 min Durchleiten von Argon). Anschließend wurde Tetrakis(triphenylphosphin)palladium(0) (7.47 mg, 0.006 mmol) zugegeben und die Mischung über Nacht bei RT gerührt. Da nach HPLC-Kontrolle nur wenig Umsatz zu beobachten war, wurde erneut Tetrakis-(triphenylphosphin)palladium(0) (7.47 mg, 0.006 mmol) zugesetzt und weitere 3 h bei RT gerührt. Der Kolbeninhalt wurde dann über Kieselgur filtriert und der Rückstand mit THF nachgewaschen. Das Filtrat wurde im Vakuum eingeengt und der Rückstand aus Diethylether (15 ml) umkristallisiert. Die Kristalle wurden abgesaugt und im Hochvakuum getrocknet. Es wurden 663 mg (96% d. Th.) der Zielverbindung erhalten.
LC-MS (Methode 4): Rₜ = 1.10 min; MS (ESIpos): *m*/*z* (%) = 448.0 (100) [M+H]⁺; MS (ESIneg): *m*/*z* (%) = 446.3 (100) [M-H]⁻.

### Beispiel 11A

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure 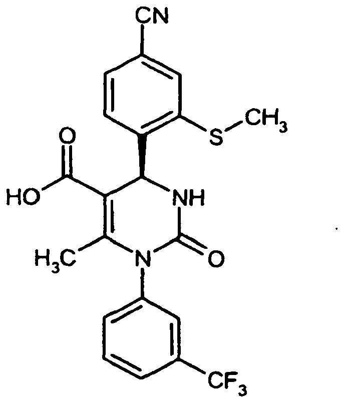 (*rac*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure (663 mg, 1.48 mmol; Beispiel 10A) wurde durch präparative HPLC-Chromatographie an chiraler Phase in die Enantiomere aufgetrennt [Säule: chirale Kieselgel-Phase basierend auf dem Selektor Poly(*N*-methacryloyl-D-leucin-dicyclopropylmethylamid); Säulendimension: 670 mm x 40 mm; Probenvorbereitung: Probe wurde in 20 ml Methanol/Ethylacetat 1:3 gelöst; Injektionsvolumen: 15 ml; Gradienten-Elution: Ethylacetat (100%) → Methanol (100%); Fluss: 80 ml/min; Temperatur: 25°C; Detektion: 260 nm]. Es wurden 279 mg (84% d. Th., 96% ee) des 4S-Enantiomeren als farbloser, amorpher Feststoff erhalten.
HPLC (Methode 2): Rₜ = 4.15 min.
MS (DCI / NH₃): *m*/*z* = 448.1 [M+H]⁺
¹H-NMR (400 MHz, DMSO-d₆): δ = 2.07 (s, 3H), 2.57 (s, 3H), 5.80 (d, 1H), 7.62-7.83 (m, 7H), 8.02 (d, 1H).
Drehwert: [α]²⁰_{Na} = +14.0° (*c* = 0.210 in DMF).

### Beispiel 12A

*tert*.-Butyl-[(3*R*)-1-{[(6*S*)-5-cyano-6-[4-yano-2-(methylsulfonyl)phenyl]-4-methyl-2-oxo-3-[3-(trifluormethyl)phenyl]-3,6-dihydropyrimidin-1(2*H*)-yl]carbonyl}piperidin-3-yl]carbamat 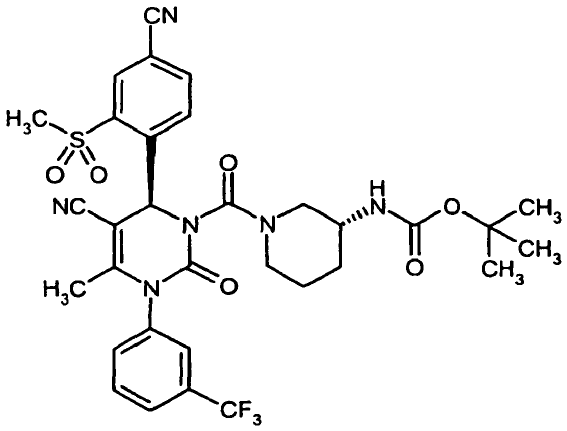 4-Nitrophenyl-(6*S*)-5-yano-6-(4-cyano-2-(methylsulfonyl)phenyl]-4-methyl-2-oxo-3-[3-(trifluormethyl)phenyl]-3,6-dihydropyrimidin-1(2*H*)-carboxylat (Beispiel 6A; 100 mg, 0.16 mmol) wurde in Acetonitril (1.25 ml) gelöst und unter Rühren mit *tert*.-Butyl-(3R)-piperidin-3-ylcarbamat (96.1 mg, 0.48 mmol) versetzt. Es wurde über Nacht bei RT gerührt. Das Reaktionsgemisch wurde dann direkt mittels präparativer HPLC gereinigt (Säule: Kromasil C18, 125 mm x 20 mm, 5 µm; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Die Produktfraktionen wurden vereinigt und im Vakuum eingeengt. Es wurden 81 mg (74% d. Th.) der Zielverbindung erhalten.
HPLC (Methode 2): Rₜ = 4.90 min.
MS (ESIpos): *m*/*z* (%) = 709.1 (35) [M+Na]⁺.

### Beispiel 13A

(*rac*)-Ethyl6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat 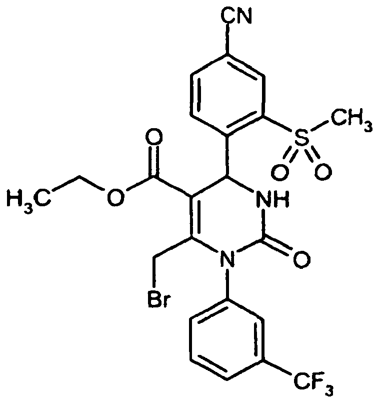 (*rac*)-Ethyl 4-[4-cyano-2-(methylsulfonyl)phenyl]-6-methyl-2-oxo-1-[3-(trifluormethyl)phenyl]-1,2,3,4-tetrahydropyrimidin-5-carboxylat (Beispiel 115; 3.00 g, 5.62 mmol) wurde in Chloroform (49.9 ml) gelöst und die Lösung im Eisbad auf 0°C abgekühlt. Dann wurde Brom (987 mg, 6.18 mmol) zugetropft. Das Eisbad wurde danach entfernt und das Reaktionsgemisch 1 h bei RT gerührt. Anschließend wurde mit Natriumthiosulfat-Lösung (50 ml) versetzt, die organische Phase abgetrennt und diese über Natriumsulfat getrocknet und im Vakuum eingeengt. Der Rückstand wurde in Diethylether verrührt, der Feststoff abgesaugt und mit Diethylether gewaschen. Es wurden 2.96 g (90% d. Th.) der Zielverbindung als gelber, kristalliner Feststoff erhalten.
HPLC (Methode 2): Rₜ = 4.73 min.
MS (ESIpos): *m*/*z* (%) = 586 (57), 588 (62) [M+H]⁺
¹H-NMR (400 MHz, DMSO-d₆): δ = 0.97 (t, 3H), 3.50 (s, 3H), 3.94-4.09 (q, 2H), 4.18 (d, 1H), 4.70 (d, 1H), 6.48 (s, 1H), 7.47 (s, 1H), 7.70-7.95 (m, 4H), 8.07 (br. s, 1H), 8.31 (d, 1H), 8.42 (s, 1H).

### Beispiel 14A

2,3-Dibrompropyl (4*S*)-6-(bronunethyl)-4-[4-cyano-2-(methylsulfonyl)phenyl]-3-methyl-2-oxo-l-[3-(trifluormethyl)phenyl]-1,2,3,4-tetrahydropyrimidin-5-carboxylat

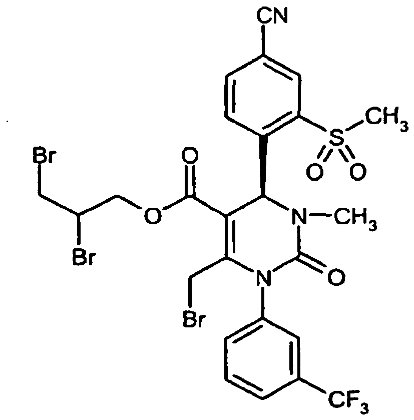

Bei 0°C wurde zu einer Lösung von (4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure-allylester (510 mg, 0.96 mmol) in Chloroform (15 ml) eine Lösung von Brom (320.8 mg, 2.0 mmol, 2.1 eq.) in Chloroform (5 ml) getropft. Das Reaktionsgemisch wurde 2 h bei RT gerührt. Danach zeigte die HPLC-Kontrolle vollständigen Umsatz. Das Gemisch wurde mit Dichlormethan (100 ml) verdünnt und anschließend mit 10%-iger wässriger Natriumsulfit-Lösung (2 x 50 ml) und gesättigter wässriger Kochsalz-Lösung (30 ml) gewaschen. Die organische Phase wurde über Natriumsulfat getrocknet, filtriert, im Vakuum eingeengt und der Rückstand im Hochvakuum getrocknet. Man erhielt einen Feststoff als Rohprodukt (803 mg, quant.), der ohne weitere Aufreinigung weiter umgesetzt wurde.
LC-MS (Methode 4): Rₜ = 1.50 min; MS (ESIpos): *m*/*z* (%) = 773.7 (100) [M+H]⁺.

### Beispiel 15A

4-(1,3-Dioxan-2-yl)-3-fluorbenzonitril 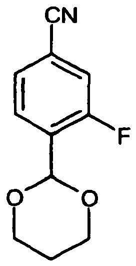 4-Cyano-2-fluorbenzaldehyd (65 g, 436 mmol) und 1,3-Propandiol (36.5 g, 479 mmol; 1.1 eq.) wurden zusammen mit 4-Toluolsulfonsäure-Monohydrat (1.66 g, 8.72 mmol; 0.02 eq.) in Toluol (1000ml) vorgelegt und 6 h am Wasserabscheider in der Siedehitze gerührt. Das Reaktionsgemisch wurde dann mit gesättigter Natriumhydrogencarbonat-Lösung (3 x 300 ml) und gesättigter Kochsalz-Lösung (3 x 300 ml) gewaschen. Die organische Phase wurde über Natriumsulfat getrocknet und eingeengt. Die Titelverbindung wurde als Feststoff erhalten (92 g, quant.).
GC-MS (Methode 1): Rₜ = 5.49 min; MS (ESIpos): *m*/*z* (%) = 206.1 (100) [M-H]⁺
¹H-NMR (400 MHz, DMSO-d₆): δ = 1.46 (d, 1H), 2.00 (m, 1H), 4.00 (t, 2H), 4.15 (dd, 2H), 5.80 (s,1H), 7.70 (m, 2H), 7.90 (m, 1H).

### Beispiel 16A

4-(1,3-Dioxan-2-yl)-3-(ethylsulfanyl)benzonitril

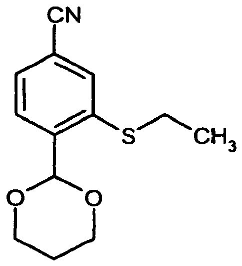

Die Reaktion wurde unter Argon durchgeführt. 4-(1,3-Dioxan-2-yl)-3-fluorbenzonitril (4.14 g, 20 mmol) und Natriumethanthiolat (1.68 g, 20 mmol; 1 eq.) wurden in DMF (100 ml) vorgelegt. Bei RT wurde Kaliumcarbonat (6.91 g, 50 mmol; 2.5 eq.) zugegeben und die Mischung 5 h bei 100°C gerührt. Die DC-Kontrolle zeigte dann vollständige Umsetzung. Es wurde eingeengt und der Rückstand über Kieselgel flash-chromatographiert (Laufmittel: Cyclohexan → Cyclohexan/Essigsäureethylester 4:1). Die Titelverbindung wurde als Feststoff erhalten (4.8 g, 97% d. Th.).
MS (DCI / NH₃): *m*/*z* = 266.1 [M+NH3]⁺
¹H-NMR (400 MHz, DMSO-d₆): δ = 1.25 (t, 3H), 1.45 (d, 1H, 2.00 (m, 1H), 3.10 (q, 2H), 4.00 (m, 2H), 4.1 (m, 2H), 5.70 (s, 1H), 7.65 (m, 2H), 7.85 (m, 1H).

### Beispiele 17A

4-(1,3-Dioxan-2-yl)-3-(ethylsulfonyl)benzonitril 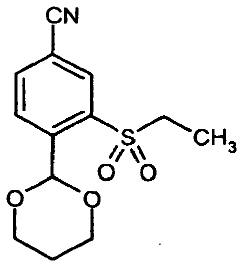 4-(1,3-Dioxan-2-yl)-3-(ethylsulfanyl)benzonitril (1060 mg, 4.25 mmol) wurde in Dichlormethan (45 ml) vorgelegt. Bei 0°C wurde MCPBA (2641 mg, 15.3 mmol; 3.6 eq.) portionsweise zugegeben. Es wurde langsam auf RT erwärmt und nachfolgend 16 h gerührt. Das Reaktionsgemisch wurde dann mit Dichlormethan (200 ml) verdünnt, anschließend mit 5%-iger wässriger Natriumcarbonat-Lösung (6 x 50 ml) und gesättigter Kochsalz-Lösung (50 ml) gewaschen, über festem Natriumsulfat getrocknet, filtriert und eingeengt. Das erhaltene Produkt (1230 mg, quant., 100% Reinheit laut LC-MS) wurde als solches weiter umgesetzt.
LC-MS (Methode 4): Rₜ = 0.97 min; MS (ESIpos): m/z (%) = 208.0 (100), 282.1 (20) [M+H]⁺
MS (DCI / NH₃): *m*/*z* (%) = 282.0 (15) [M+H]⁺, 299.1 (100) [M+NH]⁺
¹H-NMR (400 MHz, DMSO-d₆): δ = 1.13 (t, 3H), 1.50 (d, 1H), 2.05 (m, 1H), 3.40 (q, 2H), 4.00 (m, 2H), 4.20 (m, 2H), 6.30 (s, 1H), 8.00 (m, 2H), 8.25 (m, 1H).

### Beispiel 18A

3-(Ethylsulfonyl)-4-formylbenzonitril 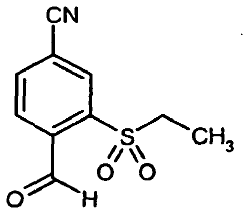 4-(1,3-Dioxan-2-yl)-3-(ethylsulfonyl)benzonitril (1230 mg, 4.4 mmol) und Pyridinium-4-toluolsulfonat (814 mg, 3.3 mmol; 0.75 eq.) wurden in Aceton/Wasser (1:1, 20 ml) in einer Emrys-Mikrowelle unter Rühren für 7 min auf 165°C erhitzt. Das Reaktionsgemisch wurde dann auf Wasser (150 ml) gegeben und mit Essigsäureethylester extrahiert (6 x 50 ml). Die vereinten organischen Phasen wurden anschließend mit gesättigter Kochsalz-Lösung (30 ml) gewaschen, über Natriumsulfat getrocknet, filtriert und im Vakuum eingeengt. Das Rohprodukt wurde an Kieselgel flash-chromatographiert (Laufmittel: Cyclohexan → Cyclohexan/Essigsäureethylester 3:2). Die Titelverbindung wurde als Feststoff isoliert (0.89 g, 90% d. Th.).
¹H-NMR (400 MHz, DMSO-d₆): δ = 1.22 (t, 3H), 3.65 (q, 2H), 8.10 (m, 2H), 8.40 (m, 1H), 10.60 (s, land.

### Beispiel 19A

(*rac*)-4-[4-Cyano-2-(ethylsulfonyl)phenyl]-6-methyl-2-oxo-1-[3-(trifluorrnethyl)phenyl]-1,2,3,4-tetrahydropyrimidin-5-carbonsäure

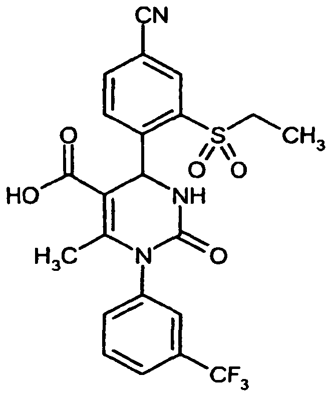

Die Reaktion wurde unter Argon durchgeführt. (*rac*)-4-[4-Cyano-2-(ethylsulfonyl)phenyl]-6-methyl-2-oxo-1-[3-(trifluormethyl)phenyl]-1,2,3,4-tetrahydropyrimidin-5-carbonsäure-allylester (1300 mg, 2.44 mmol) und Morpholin (1.5 eq., 318 mg, 3.66 mmol) wurden in trockenem THF (65 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 141 mg, 0.122 mmol) zugegeben und das Reaktionsgemisch 16 h bei RT gerührt (HPLC-Kontrolle). Das Gemisch wurde dann eingeengt und der Rückstand in Essigsäureethylester (100 ml) aufgenommen. Die organische Phase wurde mit 0.1 N Salzsäure (2 x 40 ml) und mit gesättigter Kochsalz-Lösung (3 x 30 ml) gewaschen, über Natriumsulfat getrocknet, filtriert und im Vakuum eingeengt. Der Rückstand wurde mit Methanol (10 ml) versetzt. Das ausgefallene Produkt wurde abgesaugt und mit Methanol (2 x 5 ml) gewaschen. Die Titelverbindung wurde als Feststoff isoliert (1120 mg, 93% d. Th.).

LC-MS (Methode 5): Rₜ 1.76 min; MS (ES1pos): *m*/*z* (%) = 494.1 (100) [M+H]⁺; MS (ESIneg): *m*/*z* (%) = 492.1 1 (100) [M-H]^{- 1}H-NMR (400 MHz, DMSO-d₆): δ = 1.70 (t, 3H), 2.13 (s, 3H), 3.60 (m, 2H), 6.21 (br. d, 1H), 7.00 (d, 1H), 7.70-7.85 (m, 4H), 8.10 (br. d, 1H), 8.30-8.35 (m, 2H), 12.65 (br. s, 1H).

### Beispiel 20A

4-(1,3-Dioxan-2-yl)-3-[(2-hydroxyethyl)sulfanyl]benzonitril 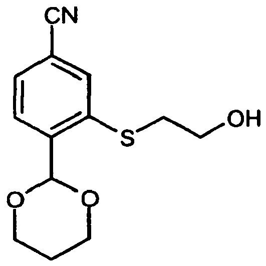 Die Reaktion wurde unter Argon durchgeführt. 4-(1,3-Dioxan-2-yl)-3-fluorbenzonitril (2.07 g, 10 mmol; 1.1 eq.) und 2-Mercaptoethanol (0.71 g, 9.1 mmol; 1 eq.) wurden in DMF (50 ml) vorgelegt. Bei RT wurde Kaliumcarbonat (3.14 g, 22.7 mmol; 2.5 eq.) zugegeben und die Mischung 1 h in der Siedehitze gerührt. Die DC-Kontrolle zeigte dann vollständigen Umsatz. Es wurde eingeengt und der Rückstand über Kieselgel flash-chromatographiert (Laufmittel: Cyclohexan → Cyclohexan/Essigsäureethylester 1:2). Die Titelverbindung wurde als Feststoff erhalten (2.33 g, 88% d. Th.).

¹H-NMR (400 MHz, DMSO-d₆): δ = 1.45 (d, 1H), 2.00 (m, 1H), 3.14 (t, 2H), 3.55 (q, 2H), 4.00 (m, 2H), 4.15 (m, 2H), 5.00 (t, 1H), 5.70 (s, 1H), 7.65 (m, 2H), 7.90 (m, 1H).

### Beispiel 21A

4-(1,3-Dioxan-2-yl)-3-[(2-hydroxyethyl)sulfonyl]benzonitril 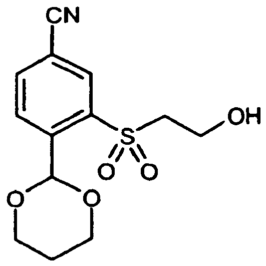 4-(1,3-Dioxan-2-yl)-3-[(2-hydroxyethyl)sulfanyl]benzonitril (2000 mg, 7.55 mmol) wurde in Dichlormethan (60 ml) vorgelegt. Bei 0°C wurde MCPBA (4460 mg, 18.1 mmol; 2.4 eq.) portionsweise zugegeben. Es wurde langsam auf RT erwärmt und nachfolgend 16 h gerührt. Das Reaktionsgemisch wurde dann mit Dichlormethan (100 ml) verdünnt, anschließend mit 5%-iger wässriger Natriumhydrogencarbonat-Lösung (4 x 50 ml) sowie gesättigter Kochsalz-Lösung (50 ml) gewaschen, über festem Natriumsulfat getrocknet, filtriert und eingeengt. Das erhaltene Produkt (2.25 g, quant., 100% Reinheit laut LC-MS) wurde als solches weiter umgesetzt.
LC-MS (Methode 5): Rₜ = 1.17 min; MS (ESIpos): *m*/*z* = 298 [M+H]⁺
¹H-NMR (400 MHz, DMSO-d₆): δ = 1.50 (d, 1H), 2.05 (m, 1H), 3.60 (t, 2H), 3.70 (q, 2H), 4.00 (m, 2H), 4.20 (m, 2H), 4.90 (t, 1H), 6.30 (s, 1H), 8.00 (d, 1H), 8.25 (dd, 1H), 8.30 (d, 1H).

### Beispiel 22A

3-[(2-Hydroxyethyl)sulfonyl]-4-formylbenzonitril 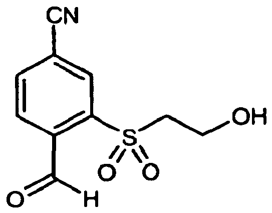 4-(1,3-Dioxan-2-yl)-3-[(2-hydroxyethyl)sulfonyl]benzonitril (1970 mg, 6.6 mmol) und Pyridinium-4-toluolsulfonat (1249 mg, 4.97 mmol; 0.75 eq.) wurden in Aceton/Wasser (1:1, 30 ml) in einer Ernrys-Mikrowelle unter Rühren für 5 min auf 160°C erhitzt. Das Reaktionsgemisch wurde dann auf Wasser (200 ml) gegeben und mit Essigsäureethylester extrahiert (8 x 50 ml). Die vereinten organischen Phasen wurden anschließend mit gesättigter Kochsalz-Lösung (2 x 50 ml) gewaschen, über Natriumsulfat getrocknet, filtriert und im Vakuum eingeengt. Zur Ausbeute-Erhöhung wurde die Kochsalz-Waschlösung mit Essigsäureethylester re-extrahiert. Die Titelverbindung wurde als Feststoff erhalten (1.57 g, 99% d. Th.).
MS (DCI / NH₃): *m*/*z* = 257.1 [M+NI-14]⁺.

### Beispiel 23A

(*rac*)-4-[4-Cyano-2-((2-hydroxyethyl)sulfonyl)phenyl]-6-methyl-2-oxo-1-[3-(trifluormethyl)-phenyl]-1,2,3,4-tetrahydropyrimidin-5-carbonsäure

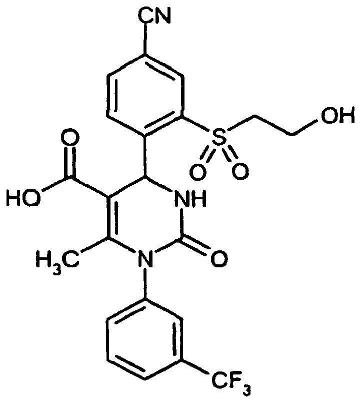

Die Reaktion wurde unter Argon durchgeführt. (*rac*)-4-[4-Cyano-2-((2-hydroxyethyl)sulfonyl)-phenyl]-6-methyl-2-oxo-1-[3(trifluormethyl)phenyl]-1,2,3,4-tetrahydropyrimidin-5-carbonsäure-allylester (150 mg, 0.273 mmol) und Morpholin (1.5 eq., 35.6 mg, 0.409 mmol) wurden in trockenem THF (4 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 15.8 mg, 0.014 mmol) zugegeben und das Reaktionsgemisch 1 h bei RT gerührt (HPLC-Kontrolle). Das Gemisch wurde dann eingeengt und der Rückstand in Essigsäureethylester (60 ml) aufgenommen. Die organische Phase wurde nacheinander mit gesättigter Ammoniumchlorid-Lösung (3 x 20 ml), Wasser (20 ml) und gesättigter Kochsalz-Lösung (20 ml) gewaschen, über Natriumsulfat getrocknet, filtriert und im Vakuum eingeengt. Das Rohprodukt wurde mittels präparativer HPLC aufgereinigt (Säule: Gromsil C-18; Eluent: Acetonitril/Wasser + 0.1% TFA 10:90 → 80:20). Die Titelverbindung wurde als Feststoff erhalten (60 mg, 43% d. Th.).

LC-MS (Methode 6): Rₜ = 2.04 min; MS (ESIpos): *m*/*z* (%) = 510.1 (100) [M+H]⁺; MS (ESIneg): *m*/*z* (%) = 508.1 (100) [M-H]⁻.

### Beispiel 24A

(rac)-4-{2-[(2-{[tert.-Butyl(dimethyl)silyl]oxy}ethyl)sulfonyl]-4-cyanophenyl}-6-methyl-2-oxo-1-[3-(trifluormethyl)phenyl]-1,2,3,4-tetrahydropyrimidin-5-carboxamid 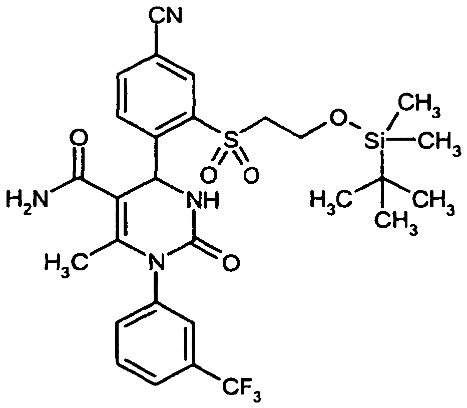 Der Ansatz wurde unter Argon durchgeführt. (*rac*)-4-[4-Cyano-2-((2-hydroxyethylsulfonyl)-phenyl]-6-methyl-2-oxo-1-[3-(trifluormethyl)phenyl]-1,2,3,4-tetrahydropyrimidin-5-carboxamid (600 mg, 1.18 mmol) wurde in DMF (40 ml) vorgelegt. Bei 0°C wurde Imidazol (803 mg, 11.8 mmol; 10 eq.) zugegeben, gefolgt von *tert*.-Butyl(dimethyl)silylchlorid (1245 mg, 8.26 mmol; 7 eq.). Es wurde langsam auf RT erwärmt und nachfolgend 16 h gerührt. Das Reaktionsgemisch wurde dann im Hochvakuum eingeengt, der Rückstand in Essigsäureethylester (200 ml) aufgenommen und die organische Phase mit Ammoniumchlorid-Lösung (3 x 50 ml) und gesättigter Kochsalz-Lösung (50 ml) gewaschen. Die organische Phase wurde anschließend über Natriumsulfat getrocknet, filtriert und eingeengt. Es wurde ein Feststoff als Rohprodukt erhalten (740 mg, quant.), welches ohne weitere Aufreinigung umgesetzt wurde.

LC-MS (Methode 6): Rₜ = 2.69 min; MS (ESIpos): *m*/*z* (%) = 623.2 (100) [M+H]⁺.

### Beispiel 25A

(*rac*)-4- {2-[(2-{[*tert*.-Butyl(dimethyl)silyl]oxy} ethyl)sulfonyl]-4-cyanophenyl}-6-methyl-2-oxo-1-[3-(trifluormethyl)phenyl]-1,2,3,4-tertrahydropyrimidin-5-carbonitril 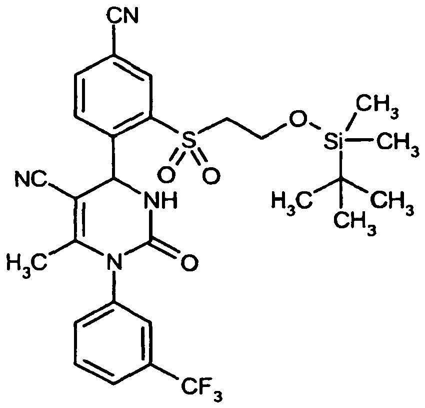 Die Reaktion wurde unter Argon durchgeführt. (*rac*)-4-{2-[(2-{[*tert*.-Butyl(dimethyl)silyl]oxy}-ethyl)sulfonyl]-4-cyanophenyl)-6-methyl-2-oxo-1-[3-(trifluormethyl)phenyl]-1,2,3,4-tetrahydro-pyrmidin-5-carboxamid (740 mg, 1.18 mmol) wurde in trockenem THF (30 ml) vorgelegt, mit Methoxycarbonylsulfamoyl-triethylammoniumhydroxid (Burgess-Reagens; 1.699 g, 7.13 mmol; 6 eq.) versetzt und die Mischung bei RT gerührt. Die HPLC-Kontrolle zeigte nach 75 min vollständigen Umsatz. Das Reaktionsgemisch wurde dann in Essigsäureethylester (150 ml) aufgenommen. Die organische Phase wurde mit gesättigter Kochsalz-Lösung (3 x 50 ml) gewaschen, über festem Natriumsulfat getrocknet, filtriert und eingeengt. Der Rückstand wurde an Kieselgel flash-chromatographiert (Laufmittel: Cyclohexan/Essigsäureethylester 1:1). Die Titelverbindung wurde als farbloser Feststoff erhalten (550 mg, 77% d. Th.).

LC-MS (Methode 6): Rₜ = 2.96 min; MS (ES1pos): *m*/*z* (%) = 605.2 (100) [M+H]⁺; MS (ESIneg): *m*/*z* (%) = 560.2 (100), 603.3 (50) [M-H]⁻.

### Beispiel 26A

4-(1,3-Dioxan-2-yl)-3-(propan-2-ylsulfanyl)benzonitril 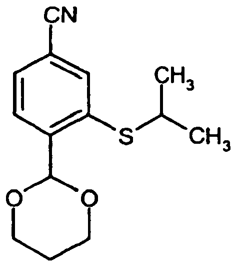 Die Reaktion wurde unter Argon durchgeführt. 4-(1,3-Dioxan-2-yl)-3-fluorbenzonitril (5.51 g, 27 mmol) und Natrium-2-propanthiolat (2.90 g, 26.6 mmol, 90% Reinheit; 1 eq.) wurden in DMF (125 ml) vorgelegt. Bei RT wurde Kaliumcarbonat (9.19 g, 66.5 mmol; 2.5 eq.) zugegeben und die Mischung 4 h bei 100°C gerührt. Die DC-Kontrolle zeigte dann vollständige Umsetzung. Das Reaktionsgemisch wurde eingeengt und der Rückstand über Kieselgel flash-chromatographiert (Laufmittel: Cyclohexan → Cyclohexan/Essigsäureethylester 4:1). Die Titelverbindung wurde als Feststoff isoliert (6.72 g, 96% d. Th.).

¹H-NMR (400 MHz, DMSO-d₆): δ = 1.25 (d, 6H), 1.45 (d, 1H), 2.00 (m, 1H), 3.70 (m, 1H), 4.00 (m, 2H), 4.15 (m, 2H), 5.75 (s, 1H), 7.70 (m, 2H), 7.95 (m, 1H).

### Beispiel 27A

4-(1,3-Dioxan-2-yl)-3-(propan-2-ylsulfonyl)benzonitril 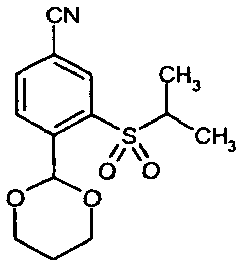 4-(1,3-Dioxan-2-yl)-3-(propan-2-ylsulfanyl)benzonitril (1090 mg, 4.14 mmol) wurde in Dichlormethan (40 ml) vorgelegt. Bei 0°C wurde MCPBA (2449 mg, 9.9 mmol; 2.4 eq.) portionsweise zugegeben. Es wurde langsam auf RT erwärmt und nachfolgend 16 h gerührt. Das Reaktionsgemisch wurde dann mit Dichlormethan (150 ml) verdünnt, anschließend mit gesättigter Natriumhydrogencarbonat-Lösung (50 ml), 5%-iger wässriger Natriumcarbonat-Lösung (5 x 50 ml) sowie gesättigter Kochsalz-Lösung (50 ml) gewaschen, über festem Natriumsulfat getrocknet, filtriert und eingeengt. Das so erhaltene Produkt (1.17 g, 96%, 89% Reinheit laut LC-MS) wurde ohne weitere Aufreinigung umgesetzt.
LC-MS (Methode 5): Rₜ = 1.63 min; MS (ESIpos): *m*/*z* (%) = 240.3 (100), 296.3 (40) [M+H]⁺
MS (DCI / NH₃): *m*/*z* (%) = 296.1 (25) [M+H]⁺, 313.1 (100) [M+NH₄]⁺.

### Beispiel 28A

4-Formyl-3-[(1-methylethyl)sulfonyl]benzonitril 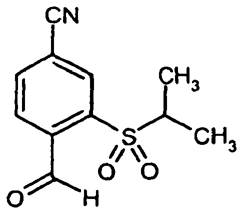 4-(1,3-Dioxan-2-yl)-3-(propan-2-ylsulfonyl)benzonitril (1090 mg, 3.7 mmol) und Pyridinium-4-toluolsulfonat (696 mg, 2.8 mmol; 0.75 eq.) wurden in Aceton/Wasser (1:1, 15 ml) in einer Emrys-Mikrowelle unter Rühren für 7 min auf 165°C erhitzt. Das Reaktionsgemisch wurde dann auf Wasser (150 ml) gegeben und mit Dichlormethan extrahiert (9 x 30 ml). Die vereinten organischen Phasen wurden anschließend mit gesättigter Kochsalz-Lösung (30 ml) gewaschen, über Natriumsulfat getrocknet, filtriert und im Vakuum eingeengt. Das Rohprodukt wurde an Kieselgel flash-chromatographiert (Laufmittel: Cyclohexan → Cyclohexan/Essigsäureethylester 3:2). Die Titelverbindung wurde als Feststoff isoliert (0.764 g, 87% d. Th.).
¹H-NMR (400 MHz, DMSO-d₆): δ = 1.25 (d, 6H), 3.80 (m, 1H), 8.10 (m, 1H), 8.40 (m, 2H), 10.60 (s, 1H).

### Beispiel 29A

(*rac*)-4- {4-Cyano-2-[(1-methylethyl)sulfonyl]phenyl}-6-methyl-2-oxo-1-[3-(trifluormethyl)-phenyl]-1,2,3,4-tetrahydropyrimidin-5-carbonsäure

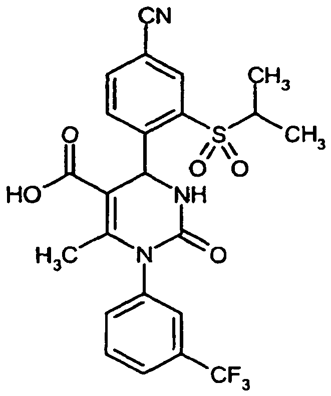

Die Reaktion wurde unter Argon durchgeführt. 4-{4-Cyano-2-[(1-methylethyl)sulfonyl]phenyl}-6-methyl-2-oxo-1-[3-(trifluormethyl)phenyl]-1,2,3,4-tetrahydropyrimidin-5-carbonsäure-allylester (1200 mg, 2.19 mmol) und Morpholin (1.5 eq., 286 mg, 3.30 mmol) wurden in trockenem THF (60 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 127 mg, 0.110 mmol) zugegeben und das Reaktionsgemisch 0.5 h bei RT gerührt (HPLC-Kontrolle). Das Gemisch wurde dann eingeengt und der Rückstand in Essigsäureethylester (100 ml) aufgenommen. Die organische Phase wurde mit 0.1 N Salzsäure (40 ml) und mit gesättigter Kochsalz-Lösung (3 x 30 ml) gewaschen, über Natriumsulfat getrocknet, filtriert und im Vakuum eingeengt. Der Rückstand wurde mit Methanol (10 ml) versetzt. Das ausgefallene Produkt wurde abgesaugt und mit wenig Methanol (2 x 5 ml) gewaschen. Die Titelverbindung wurde als Feststoff isoliert (1060 mg, 95% d. Th.).

LC-MS (Methode 5): Rₜ = 1.81 min; MS (ESIpos): *m*/*z* (%) = 508.1 (100) [M+H]⁺; MS (ESIneg): *m*/*z* (%) = 506.1 (100) [M-H]⁻.

### Beispiel 30A

4-(1,3-Dioxan-2-yl)-3-(phenylsulfanyl)benzonitril

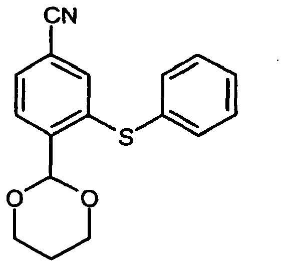

Die Reaktion wurde unter Argon durchgeführt. 4-(1,3-Dioxan-2-yl)-3-fluorbenzonitril (207 mg, 1 mmol) und Thiophenol (100 mg, 0.9 mmol; 1.1 eq.) wurden in DMF (3 ml) vorgelegt. Bei RT wurde Kaliumcarbonat (314 mg, 2.27 mmol; 2.5 eq.) zugegeben und die Mischung 4 h bei 100°C gerührt. Eine LC/MS-Kontrolle zeigte dann vollständige Umsetzung. Die Reaktionsmischung wurde direkt mittels präparativer HPLC aufgereinigt (Säule: Gromsil C-18; Eluent: Acetonitril/ Wasser + 0.1% TFA 10:90 → 90:10). Die Titelverbindung wurde als Feststoff erhalten (238 mg, 80% d. Th.).
¹H-NMR (400 MHz, DMSO-d₆): δ = 1.45 (d, 1H), 2.00 (m, 1H), 4.00 (m, 2H), 4.15 (dd, 2H), 5.85 (s, 1H), 7.35 (s, 1H), 7.45 (m, 5H), 7.75 (m, 2H).

### Beispiel 31A

4-(1,3-Dioxan-2-yl)-3-(phenylsulfonyl)benzonitril 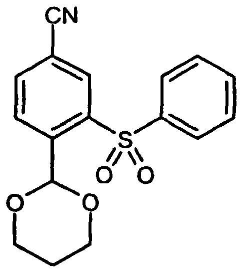 4-(1,3-Dioxan-2-yl)-3-(phenylsulfanyl)benzonitril (230 mg, 0.773 mmol) wurde in Dichlormethan (7 ml) vorgelegt. Bei 0°C wurde MCPBA (458 mg, 1.85 mmol, 70% Gehalt; 2.4 eq.) portionsweise zugegeben. Es wurde langsam auf RT erwärmt und zunächst 3 h gerührt, dann weitere 12 h bei 5°C stehen gelassen. Das Reaktionsgemisch wurde anschließend mit Wasser (0.5 ml) versetzt und im Vakuum eingeengt. Das Rohprodukt wurde mittels präparativer HPLC aufgereinigt (Säule: Gromsil C-18; Eluent: Acetonitril/Wasser + 0.1% TFA 10:90 → 75:25). Die Titelverbindung wurde als Feststoff erhalten (260 mg, quant.).
LC-MS (Methode 5): Rₜ = 1.84 min; MS (ESIpos): m/z (%) = 330.2 (90) [M+H]⁺

### Beispiel 32A

4-Formyl-3-(phenylsulfonyl)benzonitril 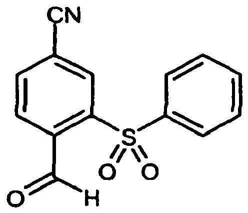 4-(1,3-Dioxan-2-yl)-3-(phenylsulfonyl)benzonitril (260 mg, 0.789 mmol) und Pyridinium-4-toluolsulfonat (149 mg, 0.592 mmol; 0.75 eq.) wurden in Aceton/Wasser (1:1, 3 ml) in einer Emrys-Mikrowelle unter Rühren für 6 min auf 160°C erhitzt. Das Reaktionsgemisch wurde dann auf Wasser (50 ml) gegeben und mit Essigsäureethylester extrahiert (8 x 20 ml). Die vereinten organischen Phasen wurden anschließend mit gesättigter Kochsalz-Lösung (2 x 30 ml) gewaschen, über Natriumsulfat getrocknet, filtriert und im Vakuum eingeengt. Die Titelverbindung wurde als Feststoff erhalten (0.21 g, quant.).
LC-MS (Methode 6): Rₜ = 2.11 min; MS (ESIpos): *m*/*z* (%) = 272.2 (100) [M+H]^{+ 1}H-NMR (400 MHz, DMSO-d₆): δ = 7.70 (m, 2H), 7.80 (m, 1H), 8.00 (d, 1H), 8.15 (m, 2H), 8.35 (dd, 1H), 8.70 (d, 1H), 10.65 (s, 1H).

### Beispiel 33A

(*rac*)-4-{4-Cyano-2-[phenylsulfonyl]phenyl}-6-methyl-2-oxo-1-[3-(trifluormethyl)phenyl]-1,2,3,4-tetrahydropyrimidin-5-carbonsäure

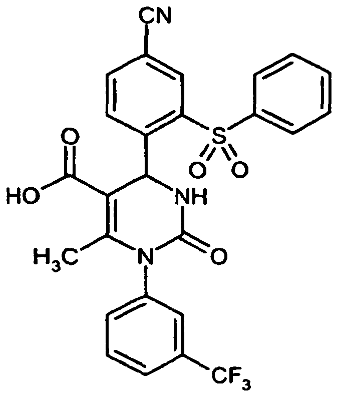

Die Reaktion wurde unter Argon durchgeführt. 4-[4-Cyano-2-(phenylsulfonyl)phenyl]-6-methyl-2-oxo-1-[3-(trifluormethyl)phenyl]-1,2,3,4-tetrahydropyrimidin-5-carbonsäure-allylester (220 mg, 0.378 mmol) und Morpholin (1.5 eq., 43 mg, 0.567 mmol) wurden in trockenem THF (6 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 22 mg, 0.019 mmol) zugegeben und das Reaktionsgemisch 16 h bei RT gerührt (HPLC-Kontrolle). Das Gemisch wurde dann eingeengt und der Rückstand in Essigsäureethylester (100 ml) aufgenommen. Die organische Phase wurde nacheinander mit 0.1 N Salzsäure (6 ml), gesättigter Ammoniumchlorid-Lösung (3 x 50 ml), Wasser (30 ml) und gesättigter Kochsalz-Lösung (30 ml) gewaschen, über Natriumsulfat getrocknet, filtriert und im Vakuum eingeengt. Das Rohprodukt wurde mittels präparativer HPLC aufgereinigt (Säule: Gromsil C-18; Eluent: Acetonitril/Wasser + 0.1% TFA 10:90 → 80:20). Das Zielprodukt wurde als Feststoff erhalten (104 mg, 51 % d. Th.).

LC-MS (Methode 6): Rₜ = 2.35 min; MS (ESIpos): *m*/*z* (%) = 542.1 (100) [M+H]⁺; MS (ESIneg): *m*/*z* (%) = 540.2 (80) [M-H]⁻.

### Beispiel 34A

(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonsäure

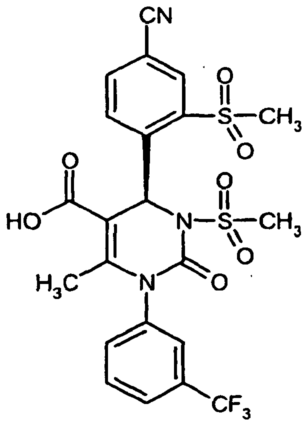

Die Reaktion wurde unter Argon durchgeführt. (4S)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonsäure-allylester (288 mg, 0.482 mmol) und Morpholin (1.5 eq., 63 mg, 0.723 mmol) wurden in trockenem THF (5 ml) bei RT vorgelegt. Das Reaktionsgemisch wurde mehrfach entgast (Evakuierung gefolgt von Belüftung mit Argon). Unter Schutzgas wurde Tetrakis(triphenylpbosphin)-palladium(0) (0.05 eq., 28 mg, 0.024 mmol) zugegeben und das Reaktionsgemisch 1.5 h bei RT gerührt (HPLC-Kontrolle). Das Gemisch wurde dann im Vakuum eingeengt und der Rückstand mittels präparativer HPLC aufgereinigt (Säule: Gromsil C-18; Eluent: Acetonitril/Wasser + 0.1% TFA 10:90 → 75:25). Man erhielt die Titelverbindung als einen Feststoff (187 mg, 70% d. Th.).

LC-MS (Methode 5): Rₜ = 1.81 min; MS (ESIpos): *m*/*z* (%) = 540.1 (100), 558.1 (40) [M+H]⁺; MS (ESIneg): *m*/*z* (%) = 556.2 (10) [M-H]⁻.

### Beispiel 35A

(4*S*)-4-[4-cyano-2-(methylsulfonyl)phenyl]-3,6-dimethyl-2-oxo-1-[3-(trifluormethyl)phenyl]-1,2,3,4-tetrahydropyrimidin-5-carbonsäure

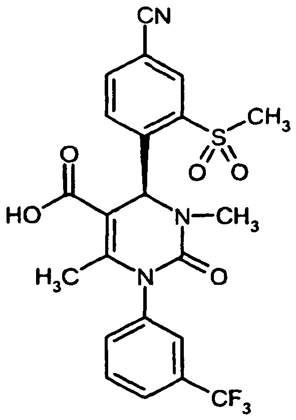

Die Reaktion wurde unter Argon durchgeführt. (4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure-allylester (157 mg, 0.294 mmol) und Morpholin (1.5 eq., 38 mg, 0.441 mmol) wurden in trockenem THF (5 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 17 mg, 0.015 mmol) zugegeben und das Reaktionsgemisch 16 h bei RT gerührt (HPLC-Kontrolle). Das Gemisch wurde dann im Vakuum eingeengt und der Rückstand mittels präparativer HPLC aufgereinigt (Säule: Gromsil C-18; Eluent: Acetonitril/Wasser + 0.1% TFA 10:90 → 90:10). Man erhielt die Titelverbindung als einen Feststoff (120 mg, 83% d. Th.).
LC-MS (Methode 5): Rₜ = 1.84 min; MS (ESIpos): m/z (%) = 494.1 (100) [M+H]⁺; MS (ESIneg): *m*/*z* (%) = 492.1 (100) [M-H]⁻
¹H-NMR (400 MHz, DMSO-d₆): δ = 2.05 (s, 3H), 2.75 (s, 3H), 3.45 (s, 3H), 6.70 (s, 1H), 7.75-7.80 (m, 3H), 7.90 (br. s, 1H), 8.15 (br. d, 1H), 8.30 (dd, 1H), 8.45 (d, 1H), 12.75 (br. s, 1H).

### Beispiel 36A

(*rac*)-Allyl 4-(4-cyano-2-nitrophenyl)-6-methyl-2-oxo-1-[3-(trifluormethyl)phenyl]-1,2,3,4-tetrahydropyrimidin-5-carboxylat

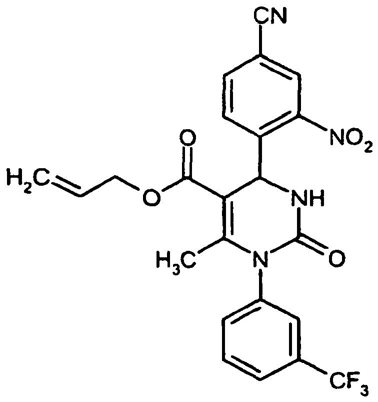

Die Reaktion wurde unter Argon durchgeführt. Allylacetoacetat (5.94 g, 41.5 mmol; 1.0 eq.) wurde in THF (117 ml) bei RT vorgelegt. Anschließend wurden 4-Cyano-2-nitrobenzaldehyd (10.45 g, 41.5 mmol, 70% Reinheit; 1.0 eq.), 1-[3-(Trifluormethyl)phenyl]harnstoff (8.48 g, 41.5 mmol) und Phosphorsäuretriethylester (17.7 g) zugegeben. Das Gemisch wurde 16 h unter Rückfluss gerührt. Zur Aufarbeitung wurde zunächst mit Eiswasser versetzt und dann in Essigsäureethylester (400 ml) aufgenommen. Die organische Phase wurde über festem Natriumsulfat getrocknet, filtriert und im Vakuum eingeengt. Das Rohprodukt wurde aus heißem Wasser/Isopropanol (2:1, ∼400 ml) umkristallisiert. Der erhaltene Feststoff wurde in Diethylether (60 ml) verrührt, erneut abgesaugt, mit wenig Diethylether nachgewaschen und im Hochvakuum getrocknet. Die Titelverbindung wurde als Feststoff erhalten (16.63 g, 82% d. Th.).
LC-MS (Methode 8): Rₜ = 3.70 min; MS (ESIpos): *m*/*z* (%) = 487.1 (100) [M+H]⁺
¹H-NMR (400 MHz, DMSO-d₆): δ = 2.10 (s, 3H), 4.40 (m, 2H), 4.95 (d, 1H), 5.05 (d, 1H), 5.70 (m, 1H), 6.15 (d, 1H), 6.05 (d, 1H), 7.70-7.90 (m, 4H), 8.10 (br. d, 1H), 8.25 (dd, 1H), 8.45 (d, 1H), 8.55 (d, 1H).

### Beispiel 37A

(*rac*)-4-(4-Cyano-2-nitrophenyl)-6-methyl-2-oxo-1-[3-(trifluormethyl)phenyl]-1,2,3,4-tetrahydropyrimidin-5-carbonsäure

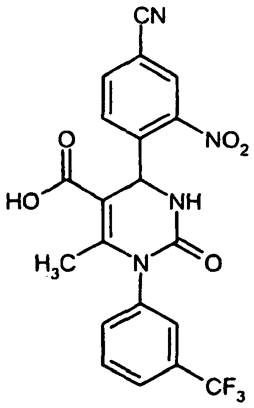

Die Reaktion wurde unter Argon durchgeführt. (*rac*)-Allyl 4-(4-cyano-2-nitrophenyl)-6-methyl-2-oxo-1-[3-(trifluormethyl)phenyl]-1,2,3,4-tetrahydropyrimidin-5-carboxylat (15.0 g, 30.8 mmol) und Morpholin (1.5 eq., 4.03 g, 46.3 mmol) wurden in trockenem THF (300 ml) bei RT vorgelegt. Das Reaktionsgemisch wurde mehrfach entgast (Evakuierung gefolgt von Belüftung mit Argon). Unter Schutzgas wurde Tetrakis(triphenylphosphin)palladium(0) (0.05 eq., 1.78 g, 1.54 mmol) zugegeben und das Reaktionsgemisch 2 h bei RT gerührt (HPLC-Kontrolle). Das Gemisch wurde dann eingeengt und der Rückstand in Essigsäureethylester (700 ml) aufgenommen. Die organische Phase wurde mit 0.5 N Salzsäure (500 ml) und mit gesättigter Kochsalz-Lösung (300 ml) gewaschen, über Natriumsulfat getrocknet, filtriert und im Vakuum eingeengt. Das Rohprodukt wurde aus Essigsäureethylester umkristallisiert und im Hochvakuum getrocknet. Die Titelverbindung wurde als Feststoff erhalten (12.87 g, 93% d. Th.).
¹H-NMR (400 MHz, DMSO-d₆): δ = 2.05 (s, 3H), 6.00 (d, 1H), 7.65-7.90 (m, 4H), 8.10 (d, 1H), 8.25 (dd, 1H), 8.40 (d, 1H), 8.50 (d, 1H), 12.5 (br. s, 1H).

### Beispiel 38A

(4*R*)-4-(4-Cyano-2-nitrophenyl)-6-methyl-2-oxo-1-[3-(trifluormethyl)phenyl]-1,2,3,4-tetrahydropyrimidin-5-carbonsäure 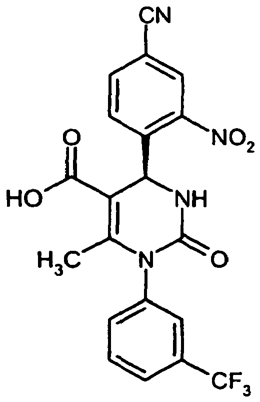 (*rac*)-4-(4-Cyano-2-nitrophenyl)-6-methyl-2-oxo-1-[3-(trifluormethyl)phenyl]-1,2,3,4-tetrahydropyrimidin-5-carbonsäure (590 g) wurde durch präparative HPLC-Chromatographie an chiraler Phase in die Enantiomere aufgetrennt [Säule: chirale Kieselgel-Phase basierend auf dem Selektor Poly(*N* methacryloyl-L-leucin-*tert*.-butylamid); Säulendimension: 670 mm x 40 mm; Probenvorbereitung: 100 g Probe gelöst in 2000 ml TT3F; Injektionsvolumen: 70 ml; Eluent: Essigsäureethylester/Methanol 100:1 → 1:100; Fluss: 80 ml/min; Temperatur: 24°C; Detektion: 260 nm]. Es wurden 280 g (95% d. Th.; 99.6% ee) des 4*R*-Enantiomeren erhalten.

Der Enantiomerenüberschuss (ee-Wert) wurde chromatographisch bestimmt [Säule: chirale Kieselgel-Phase basierend auf dem Selektor Poly(*N*-methacryloyl-L-leucin-*tert*.-butylamid); Säulendimension: 250 mm x 4.6 mm; Eluent: EssigsäureethylesterIMethanol 10:1; Fluss: 2 ml/min; Detektion: 265 nm; Rₜ = 1.38 min].

### Beispiel 39A

(4*R*)-4-(4-Cyano-2-nitrophenyl)-6-methyl-2-oxo-1-[3-(trifluormethyl)phenyl]-1,2,3,4-tetrahydropyrimidin-5-carboxamid

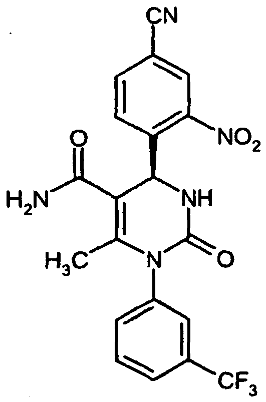

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carbonsäure (6.0 g, 11.4 mmol, 85% Reinheit), DMAP (140 mg, 1.143 mmol; 0.1 eq.), DIEA (1.77 g, 13.7 mmol; 1.2 eq.) und PyBOP (7.14 g, 13.71 mmol; 1.2 eq.) wurden in trockenem THF (34 ml) bei RT vorgelegt, nach kurzem Rühren (15 min) mit 0.5 M Ammoniak-Lösung in THF (5 eq., 57.1 mmol) versetzt und anschlie-βend 1 h bei RT gerührt. Das Reaktionsgemisch wurde dann mit Essigsäureethylester (250 ml) versetzt. Die organische Phase wurde nacheinander mit gesättigter Natriumhydrogencarbonat-Lösung, Wasser und gesättigter Kochsalz-Lösung gewaschen, über Natriumsulfat getrocknet, filtriert und im Vakuum eingeengt. Das Rohprodukt wurde an Kieselgel flash-chromatographiert (Laufmittel: Dichlormethan/Methanol 20:1). Die Titelverbindung wurde als farbloser Feststoff erhalten (5.0 g, 98% d. Th.).

MS (ESIpos): *m*/*z* (%) = 446.2 (100) [M+H]⁺.

### Beispiel 40A

(4*R*)-4-(4-Cyano-2-nitrophenyl)-6-methyl-2-oxo-1-[3-(trifluormethyl)phenyl]-1,2,3,4-tetrahydropyrimidin-5-carbonitril

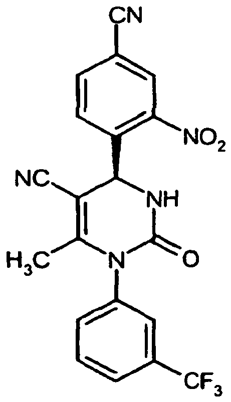

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carboxamid (5.0 g, 10.1 mmol; 90% Reinheit) wurde in trockenem THF (135 ml) vorgelegt, mit Methoxycarbonylsulfamoyl-triethylammoniumhydroxid (Burgess-Reagens; 3.85 g, 16.17 mmol; 1.6 eq.) versetzt und anschließend 2 h bei RT gerührt. Das Reaktionsgemisch wurde dann mit Essigsäureethylester (300 ml) versetzt. Die organische Phase wurde zweimal mit Wasser und einmal mit gesättigter Kochsalz-Lösung gewaschen, über Natriumsulfat getrocknet, filtriert und im Vakuum eingeengt. Das Rohprodukt wurde aus Cyclohexan/Essigsäureethylester umkristallisiert. Die erhaltenen Kristalle wurden im Hochvakuum getrocknet. Die Titelverbindung wurde als Feststoff erhalten (2.8 g, 65% d. Th.).
¹H-NMR (400 MHz, DMSO-d₆): δ = 1.80 (s, 3H), 5.95 (s, 1H), 7.75-8.25 (m, 6H), 8.35 (dd, 1H), 8.65 (s, 1H).

### Beispiel 41A

(4*R*)-4-(4-Cyano-2-nitrophenyl)-3,6-dimethyl-2-oxo-1-[3-(trifluormethyl)phenyl]-1,2,3,4-tetrahydropyrimidin-5-carbonitril

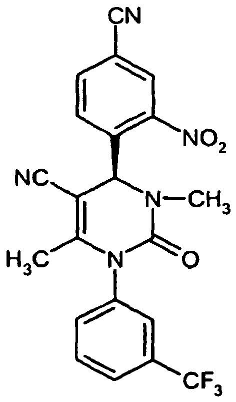

Die Reaktion wurde unter Argon durchgeführt. (4*R*)-4-(4-Cyano-2-nitrophenyl)-6-methyl-2-oxo-1-[3-(trifluormethyl)phenyl]-1,2,3,4-tetrahydropyrimidin-5-carbonitril (5.0 g, 11.7 mmol) wurde in absolutem THF (500 ml) vorgelegt und bei -78°C mit einer 1 M Lösung von Lithiumhexamethyldisilazid (LiHMDS) in THF (13.5 ml, 13.5 mmol; 1.15 eq.) versetzt. Nach 30 min Rühren wurde Iodmethan (8.30 g, 58.5 mmol; 5 eq.) in THF zugegeben und die Mischung für 16 h unter allmählicher Erwärmung von -78°C auf RT gerührt. Das Reaktionsgemisch wurde anschließend im Vakuum eingeengt und zunächst mit 1 N Salzsäure (14.0 ml), dann mit MTBE (500 ml) versetzt. Die organische Phase wurde nacheinander mit Wasser (2 x), gesättigter Natriumhydrogencarbonat-Lösung, gesättigter Ammoniumchlorid-Lösung und gesättigter Kochsalz-Lösung gewaschen, über Natriumsulfat getrocknet, filtriert und im Vakuum eingeengt. Die Titelverbindung wurde als Feststoff erhalten (4.3 g, 83% d. Th.).
LC-MS (Methode 4): Rₜ = 1.28 min; MS (ESIpos): m/z (%) = 442.2 (100) [M+H]⁺ MS (ESIneg): *m*/*z* (%) = 440.2 (50) [M-H]⁻
¹H-NMR (400 MHz, DMSO-d₆): δ = 1.80 (s, 3H), 2.70 (s, 3H), 5.95 (s, 1H), 7.75-8.25 (m, 5H), 8.35 (dd, 1H), 8.65 (s, 1H).

### Beispiel 42A

(4*R*)-4-(2-Amino-4-cyanophenyl)-3,6-dimethyl-2-oxo-1-[3-(trifluormethyl)phenyl]-1,2,3,4-tetrahydropyrimidin-5-carbonitril 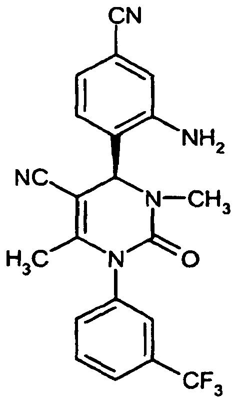 (4*R*)-4-(4-Cyano-2-nitrophenyl)-3,6-dimethyl-2-oxo-1-[3-(trifluormethyl)phenyl]-1,2,3,4-tetrahydropyrimidin-5-carbonitril (6.0 g, 11.3 mmol) wurde unter Argon in Methanol (420 ml) gelöst. Anschließend wurde 10% Palladium auf Aktivkohle (5.5 g) zugegeben und für 5.5 h bei RT und Normaldruck hydriert (genaue HPLC-Kontrolle). Das Reaktionsgemisch wurde dann über Kieselgur abfiltriert und der Filter-Rückstand mit Methanol (1000 ml) nachgewaschen. Das Filtrat wurde eingeengt und das Rohprodukt an Kieselgel flash-chromatographiert (Laufmittel: Essigsäureethylester/Cyclohexan 2:1). Die Titelverbindung wurde als Feststoff erhalten (2.28 g, 40% d. Th.).

LC-MS (Methode 9): Rₜ = 1.06 min; MS (ESIpos): *m*/*z* (%) = 412.3 (80) [M+H]⁺; MS (ESIneg): *m*/*z* (%) = 410.3 (100) [M-H]⁻.

### Beispiel 43A

5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid 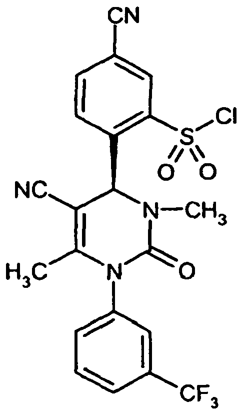 Unter Argon wurde (4*R*)-4-(2-Amino-4-cyanophenyl)-3,6-dimethyl-2-oxo-1-[3-(trifluormethyl)-phenyl]-1,2,3,4-tetrahydropyrimidin-5-carbonitril (2.1 g, 5.1 mmol) in einem 2:1:1-Gemisch aus Essigsäure/konz. Salzsäure/Wasser (insgesamt 50 ml) bei -10°C vorgelegt. Dazu wurde langsam eine Lösung von Natriumnitrit (371 mg, 5.38 mmol) in Wasser (2 ml) getropft und die Mischung für 40 min bei -10°C bis -5°C gerührt. Diese Lösung wurde dann zu 45 ml einer auf -10°C vorgekühlten, mit Schwefeldioxid gesättigten Suspension von Kupfer(I)chlorid (101.4 mg, 1.0 mmol) in Eisessig (44 ml) gegeben. Der Ansatz wurde ca. 30 min bei 0°C und dann 1 h bei +15°C gerührt (Reaktionskontrolle per HPLC und LC-MS). Die Reaktionsmischung wurde danach auf 0°C gekühlt und anschließend pipettenweise in ca. 300 ml eiskaltes Wasser gegeben. Der Niederschlag wurde abfiltriert und in Essigsäureethylester (150 ml) aufgenommen. Die Lösung wurde zweimal mit gesättigter Kochsalz-Lösung gewaschen, über Natriumsulfat getrocknet, filtriert und im Vakuum eingeengt. Die Titelverbindung wurde als ein Feststoff erhalten (2.13 g, 77% d. Th., 92% Reinheit), welcher ohne weitere Aufreinigung in der Folgereaktion eingesetzt wurde.
LC-MS (Methode 4): Rₜ = 1.37 min; MS (ESIpos): *m*/*z* (%) = 495.1 (100) [M+H]⁺
¹H-NMR (400 MHz, DMSO-d₆): δ = 1.80 (s, 3H), 2.70 (s, 3H), 6.55 (s, 1H), 7.75-8.00 (m, 6H), 8.10 (s, 1H).

### Beispiel 44A

Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat 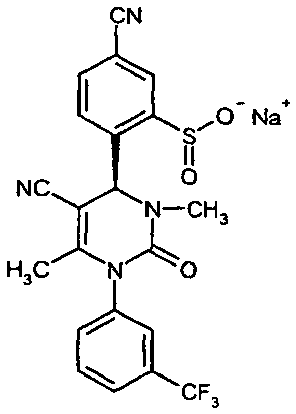 5-Cyano-2- {(4*S*)-5-cyano-3,6-dmethyl-2-oxo-1-[3-(trifluormethyl)phenyl]-1,2,3,4-tetrahydropyrimidin-4-yl}benzolsulfonylchlorid (600 mg) wurde in THF (4.25 ml) gelöst. Anschließend wurden Natriumsulfit (195 mg, 1.55 mmol; 1.5 eq.) und Natriumhydrogencarbonat (303 mg, 3.61 mmol; 3.5 eq.), in Wasser (1.7 ml) gelöst, zugegeben. Es wurde 1 h bei RT gerührt. Die Reaktionslösung wurde dann direkt lyophilisiert. Man erhielt einen Feststoff als Produkt (962 mg, 58% d. Th., 32% Reinheit), welcher ohne weitere Aufreinigung in den Folgereaktionen eingesetzt wurde.
LC-MS (Methode 9): Rₜ = 0.80 min; MS (ESIpos): *m*/*z* (%) = 461.2 (80) [M+H]⁺, MS (ESIneg): *m*/*z* (%) = 459.2 (100) [M-H]-.

### Beispiel 45A

(4*S*)-4-(4-Cyano-2-sulfanylphenyl)-3,6-dimethyl-2-oxo-1-[3-(trifluormethyl)phenyl]-1,2,3,4-tetrahydropyrimidin-5-carbonitril 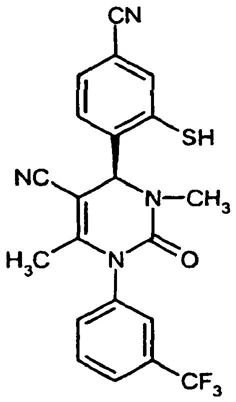 5-Cyano-2- {(4*S*)-5-cyano-3,6-dimethyl-2-oxo-1-[3-(trifluormethyl)phenyl]-1,2,3,4-tetrahydropyrimidin-4-yl}benzolsulfonylchlorid (52 mg, 105 µmol) wurde unter Argon in Dichlormethan (10 ml) gelöst. Es wurde Triphenylphosphin-Polystyrol-Harz (Kapazität 1 mmol/g, 315 mg, 315 µmol, 3 eq.) zugegeben und die Mischung für 15 h geschüttelt. Danach wurde nochmals Triphenylphosphin-Polystyrol-Harz (1 mmol/g, 105 mg, 105 µmol, 1 eq.) nachgegeben und die Mischung weitere 5 h geschüttelt. Die Reaktionsmischung wurde dann filtriert, das Filtrat im Vakuum eingeengt und der Rückstand im Hochvakuum getrocknet. Die Titelverbindung wurde als Feststoff erhalten (90 mg, 50% Reinheit, quant.), welcher ohne weitere Aufreinigung in Folgereaktionen eingesetzt wurde.
LC-MS (Methode 4): Rₜ = 1.25 min; MS (ESIpos): *m*/*z* (%) = 429.1 (100) [M+H]⁺; MS (ESIneg): *m*/*z* (%) = 427.0 (40) [M-H]⁻
¹H-NMR (400 MHz, DMSO-d₆): δ = 1.80 (s, 3H), 2.65 (s, 3H), 3.15 (s, 1H), 5.65 (s, 1H), 7.60-7.90 (m, 7H), 8.00 (s, 1H).

### Ausführungsbeispiele:

### Beispiel 1

(*rac*)-4-{5-Acetyl-6-methyl-2-oxo-1-[3-(trifluormethyl)phenyl]-1,2,3,4-tetrahydropyrimidin-4-yl} - 3-(methylsulfonyl)benzonitril

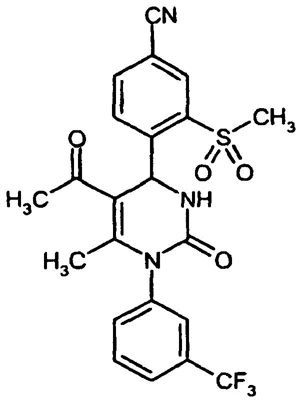

Die Reaktion wurde unter Argon durchgeführt. Phosphorsäuretriethylester (251 mg, 1.38 mmol) und Diphosphorpentoxid (130 mg, 0.918 mmol) wurden über Nacht bei 50°C gerührt. Dann wurde mit MTBE (5 ml) verdünnt, und 4-Formyl-3-(methylsulfonyl)benzonitril (240 mg, 1.15 mmol; Beispiel 4A), 1-[3-(Trifluormethyl)phenyl]harnstoff (234 mg, 1.15 mmol) sowie 2,4-Pentandion (173 mg, 1.72 mmol) wurden hinzugefügt. Das Gemisch wurde über Nacht unter Rückfluss gerührt. Zur Aufarbeitung wurde das Lösungsmittel im Vakuum entfernt und der Rückstand mit Diethylether aufgeschlämmt und dann abgesaugt. Man erhielt 404 mg (73% d. Th.) der Zielverbindung.
MS (DCI / NH₃): *m*/*z* (%) = 478.2 (100) [M+H]⁺, 495.2 (28) [M+NH₄]⁺
HPLC (Methode 2): Rₜ = 4.38 min.
¹H-NMR (400 MHz, DMSO-d₆): δ = 2.10 (s, 3H), 2.29 (s, 3H), 3.57 (s, 3H), 6.32 (s, 1H), 7.16 (s, 1H), 7.68-7.86 (m, 4H), 8.02 (d, 1H), 8.23 (d, 1H), 8.37 (s, 1H).

### Beispiel 2

4-{(4*S*)-5-Acetyl-6-methyl-2-oxo-1-[3-(trifluormethyl)phenyl]-1,2,3,4-tetrahydropyrimidin-4-yl}-3-(methylsulfonyl)benzonitril 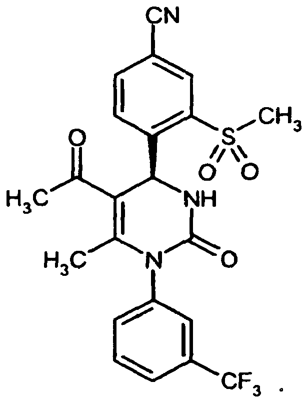 (*rac*)-4-{5-Acetyl-6-methyl-2-oxo-1-[3-(trifluormethyl)phenyl]-1,2,3,4-tetrahydropyrimidin-4-yl}-3-(methylsulfonyl)benzonitril (Beispiel 1, 290 mg) wurde durch präparative HPLC-Chromatographie an chiraler Phase in die Enantiomere aufgetrennt [Säule: Daicel Chiralpak IA, 250 mm x 20 mm; Probenvorbereitung: Probe wurde in 30 ml MTBE/Methanol/Acetonitril 1:1:1 gelöst; Injektionsvolumen: 1.0 ml; Eluent: MTBE/Methanol 1:1; Fluss: 15 ml/min; Temperatur: 30°C; Detektion: 220 nm]. Auf diese Weise wurden 121 mg (83% d. Th.) des 4S-Enantiomeren als farbloser, amorpher Feststoff erhalten.
HPLC (Methode 2): Rₜ = 4.38 min.
¹H-NMR (400 MHz, DMSO-d₆): δ = 2.10 (s, 3H), 2.28 (s, 3H), 3.57 (s, 3H), 6.33 (d, 1H), 7.20 (d, 1H), 7.68-7.88 (m, 4H), 8.02 (d, 1H), 8.23 (d, 1H), 8.37 (s, 1H).
Drehwert: [α]²⁰_{Na} = +18.7°. (c = 0.56 in DMF).

### Beispiel 3

(*rac*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester

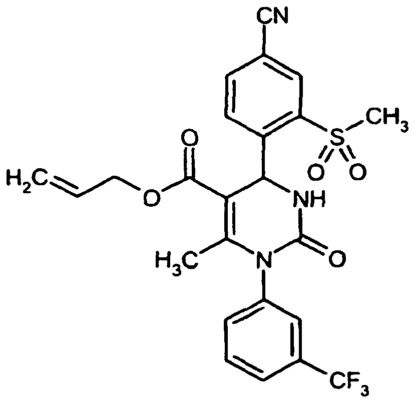

Die Reaktion wurde unter Argon durchgeführt. Phosphorsäuretriethylester (22.98 g, 126 mmol) und Diphosphorpentoxid (11.94 g, 84.1 mmol) wurden über Nacht bei 50°C gerührt. Dann wurde mit MTBE (450 ml) verdünnt, und 4-Formyl-3-(methylsulfonyl)benzonitril (22.00 g, 105 mmol; Beispiel 4A), 1-[3-(Trifluormethyl)phenyl]harnstoff (21.47 g, 105 mmol) sowie Allylacetoacetat (22.42 g, 158 mmol) wurden hinzugefügt. Das Gemisch wurde über Nacht unter Rückfluss gerührt. Da die Umsetzung nicht vollständig war, wurde die Reaktionsmischung aufkonzentriert, indem ca. 350 ml MTBE abdestilliert wurden. Danach wurde für weitere 4 h unter Rückfluss erhitzt. Zur Aufarbeitung wurde das Lösungsmittel im Vakuum entfernt und der Rückstand mit Diethylether aufgeschlämmt und dann abgesaugt. Der Feststoff wurde mit dest. Wasser (350 ml) und anschließend mit Diethylether (50 ml) gewaschen. Man erhielt 34.74 g (64% d. Th.) der Titelverbindung.
LC-MS (Methode 4): Rₜ = 1.28 min; MS (ESIpos): *m*/*z* (%) = 520.2 (100) [M+H]⁺
¹H-NMR (400 MHz, DMSO-d₆): δ = 2.15 (s, 3H), 3.45 (s, 3H), 4.45 (m, 2H), 4.95 (d, 1H), 5.05 (d, 1H), 5.65 (m, 1H), 6.40 (d, 1H), 7.20 (d, 1H), 7.70 (m, 2H), 7.80 (m, 1H), 7.85 (br. s, 1H), 8.10 (br. d, 1H), 8.25 (d, 1H), 8.35 (s, 1H).

### Beispiel 4

(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester 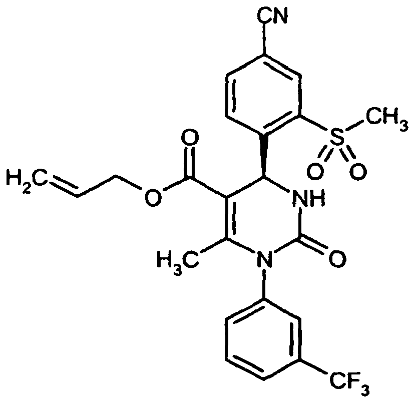 (*rac*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (Beispiel 3, 2.33 g) wurde durch präparative HPLC-Chromatographie an chiraler Phase in die Enantiomere aufgetrennt [Säule: chirale Kieselgel-Phase basierend auf dem Selektor Poly(*N*-methacryloyl-D-leucin-dicyclopropylmethylamid); Probenvorbereitung: jeweils 1 g Probe gelöst in 70 ml THF/Essigsäureethylester/Isohexan 20:25:25; Injektionsvolumen: 8 ml; Eluent: Isohexan/Isopropanol 1:1; Fluss: 60 ml/min; Temperatur: 24°C; Detektion: 260 nm]. Auf diese Weise wurden 0.8 g (69% d. Th., >99.5% ee) des 4S-Enantiomeren erhalten. Der Enantiomerenüberschuss (ee-Wert) wurde chromatographisch bestimmt [Säule: chirale Kieselgel-Phase basierend auf dem Selektor Poly(*N*-methacryloyl-D-leucin-dicyclopropylmethylamid), 250 mm x 4.6 mm; Eluent: Isohexan/Essigsäureethylester 1:1; Fluss: 2 ml/min; Detektion: 260 nm; Rₜ = 1.45 min].
LC-MS (Methode 5): Rₜ = 2.11 min; MS (ESIpos): *m*/*z* (%) = 520.1 (100) [M+H]⁺; MS (ESIneg): *m*/*z* (%) = 475.2 (100), 518.2 (100) [M-H]-
¹H-NMR (400 MHz, DMSO-d₆): δ = 2.10 (s, 3H), 3.45 (s, 3H), 4.45 (m, 2H), 4.95 (d, 1H), 5.05 (d, 1H), 5.65 (m, 1H), 6.40 (d, in), 7.20 (d, 1H), 7.70 (m, 2H), 7.80 (m, 1H), 7.85 (br. s, 1H), 8.10 (br. d, 1H), 8.25 (d, 1H), 8.35 (s, 1H).

### Beispiel 5

(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carboxamid 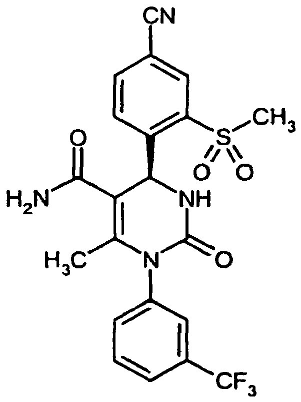 Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (696 mg, 1.45 mmol; Beispiel 5A) und HATU (2 eq., 1104 mg, 2.9 mmol) wurden in trockenem DMF (35 ml) bei 0°C vorgelegt und nach kurzem Rühren (20 min) mit Ammoniumchlorid (5 eq., 388 mg, 7.26 mmol) sowie DIEA (7 eq., 1314 mg, 10.16 mmol) versetzt. Das Gemisch wurde 4 h bei RT gerührt (HPLC-Monitoring). Danach wurde eingeengt und der Rückstand mittels präparativer HPLC gereinigt (Säule: Gromsil C-18 10 µm; Eluent: Acetonitril/Wasser + 0.1% TFA 10:90 → 90:10). Es wurde ein farbloser, amorpher Feststoff erhalten (612 mg, 88% d. Th.).
LC-MS (Methode 6): Rₜ = 1.94 min; MS (ESIpos): *m*/*z* (%) = 479.1 (100) [M+H]⁺; MS (ESIneg): *m*/*z* (%) = 434.1 (100), 477 (40) [M-H]⁻
¹H-NMR (400 MHz, DMSO-d₆): δ = 1.80 (s, 3H), 3.40 (s, 3H), 6.35 (s, 1H), 7.20 (s, 1H), 7.25 (br. s, 1H), 7.45 (br. s, 1H), 7.65-7.80 (m, 4H), 8.10 (d, 1H), 8.30 (s, 1H), 8.35 (d, 1H).

### Beispiel 6

(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril

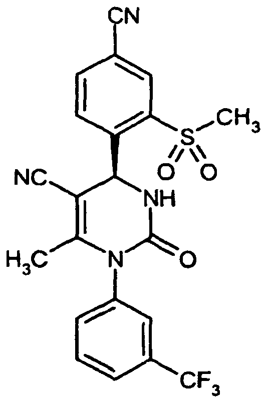

### Methode A:

Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carboxamid (560 mg, 1.17 mmol; Beispiel 5) wurde in trockenem THF (35 ml) vorgelegt, mit Methoxycarbonylsulfamoyl-triethylammoniumhydroxid (Burgess-Reagens; 1115 mg, 4.68 mmol, 4 eq.) versetzt und bei RT gerührt. Die HPLC-Kontrolle zeigte nach 90 min vollständigen Umsatz. Das Reaktionsgemisch wurde eingeengt und der Rückstand über präparative HPLC gereinigt (Säule: Gromsil C-18 10 µm; Eluent: Acetonitril/Wasser + 0.1% TFA 10:90 → 90:10). Die Titelverbindung wurde als farbloser, amorpher Feststoff erhalten (470 mg, 87% d. Th.).

### Methode B:

(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carboxamid (10.4 g, 21.7 mmol; Beispiel 5) wurde zusammen mit Triethylamin (5.63 g, 55.6 mmol) in trockenem THF (50 ml) gelöst Unter schwacher Wärmetönung (bis 35°C) wurde Trifluoressigsäureanhydrid (11.69 g, 55.6 mmol) zugetropft. Nach 15 min Rühren war die Umsetzung vollständig (HPLC-Monitoring). Es wurde gesättigte Natriumhydrogencarbonat-Lösung (250 ml) zum Ansatz getropft und dann zweimal mit Essigsäureethylester extrahiert. Die vereinigten organischen Phasen wurden mit gesättigter Kochsalz-Lösung gewaschen, mit Magnesiumsulfat getrocknet, mit 30 g Kieselgel versetzt und am Rotationsverdampfer eingeengt. Das so auf Kieselgel aufgezogene Rohprodukt wurde an weiteren 500 g Kieselgel chromatographisch gereinigt (Laufmittel: Dichlormethan/Essigsäureethylester 2:1). Es wurden 6.46 g (65% d. Th.) der Titelverbindung erhalten.
Schmp.: 258-259°C
Drehwert: [a]²⁰_{Na} = -222.0° (c = 0.48 in DMF)
LC-MS (Methode 6): Rₜ = 2.28 min; MS (ESIpos): m/z (%) = 461.1 (100) [M+H]⁺; MS (ESIneg): m/z (%) = 352.3 (70), 416.1 (100), 459.2 (70) [M-H]⁻
¹H-NMR (400 MHz, DMSO-d₆): δ = 1.80 (s, 3H), 3.40 (s, 3H), 6.45 (s, 1H), 7.70-7.85 (m, 3H), 7.95 (br. s, 1H), 8.30-8.40 (m, 4H).

### Beispiel 7

*tert*.-Butyl-[(6*R*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-yl]acetat

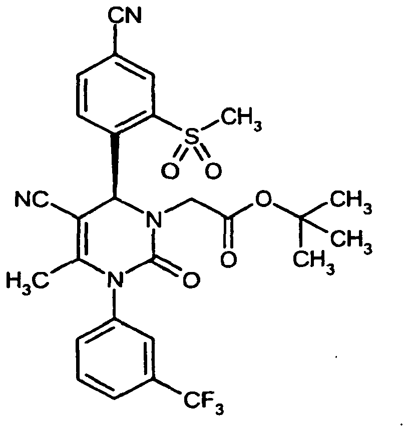

Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (290 mg, 630 µmol; Beispiel 6) und Natriumhydrid (60% in Mineralöl, 30.2 mg, 756 µmol, 1.2 eq.) wurden bei 0°C in THF (12 ml) vorgelegt und nach kurzem Rühren (15 min) mit Bromessigsäure-tert.-butylester (175 mg, 882 µmol, 1.4 eq.) versetzt. Man ließ das Reaktionsgemisch auf RT kommen. Nach 150 min beobachtete man vollständigen Umsatz. Das Reaktionsgemisch wurde dann auf Essigsäureethylester (100 ml) gegeben. Die organische Phase wurde mit gesättigter Kochsalz-Lösung (2 x 20 ml) gewaschen, über Natriumsulfat getrocknet, filtriert und im Vakuum eingeengt. Das Rohprodukt wurde mittels präparativer HPLC gereinigt (Säule: Gromsil C-18 10 µm; Eluent: Acetonitril/Wasser + 0.1% TFA 10:90 → 90:10). Man erhielt die Zielverbindung als Feststoff (309 mg, 85% d. Th.).

LC-MS (Methode 4): Rₜ = 1.38 min; MS (ESIpos): *m*/*z* (%) = 519.1 (100); MS (ESIneg): *mlz* (%) = 573.4 (100) [M-H]⁻.

### Allgemeine Vorschrift 1: Synthese von N-Aminocarbonyl-dihydronyrimidinon-Derivaten (Verfahren A)

4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (Beispiel 6A, 1 eq.) wurde in Acetonitril gelöst und unter Rühren mit dem entsprechenden Amin (3 eq.) versetzt. Nach erfolgter Umsetzung (HPLC-Monitoring) wurde das Reaktionsgemisch direkt mittels präparativer HPLC gereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Die Produktfraktionen wurden vereinigt und im Vakuum eingeengt.

### Beispiel 8

(6*S*)-5-Cyano-6-[4-cyano-2-(methylsulfonyl)phenyl]-*N*-[2-hydroxy-1-(hydroxymethyl)ethyl]-4-methyl-2-oxo-3-[3-(trifluormethyl)phenyl]-3,6-dihydropyrimidin-1(2*H*)-carboxamid 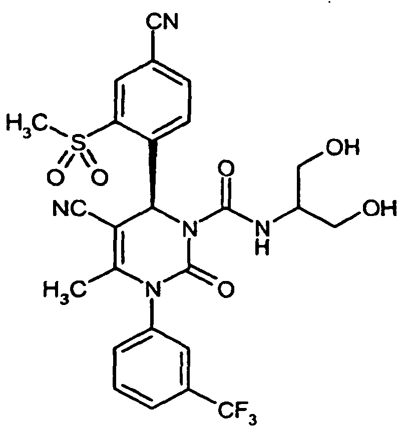 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit 2-Amino-1,3-propandiol (34.1 mg, 0.374 mmol) in Acetonitril (1 ml) zur Zielverbindung (50 mg, 69% d. Th.) umgesetzt.
HPLC (Methode 3): Rₜ = 4.27 min.
MS (ESIpos): *m*/*z* (%) = 578.3 (100) [M+H]^{+ 1}H-NMR (400 MHz, DMSO-d₆): δ = 1.78 (s, 3H), 3.20-3.57 (m, 5H), 3.50 (s, 3H), 4.70 (t, 1H), 4.76 (t, 1H), 7.27 (s, 1H), 7.74-8.23 (m, 5H)_{.} 8.29 (d, 1H), 8.44 (s, 1H), 8.90-9.02 (br. s, 1H).

### Beispiel 9

(6*S*)-*N*-(2-Amino-2-oxoethyl)-5-cyano-6-[4-cyano-2-(methylsulfonyl)phenyl]-4-methyl-2-oxo-3-[3-(trifluormethyl)phenyl]-3,6-dihydropyrimidin-1 (2*H*)-carboxamid

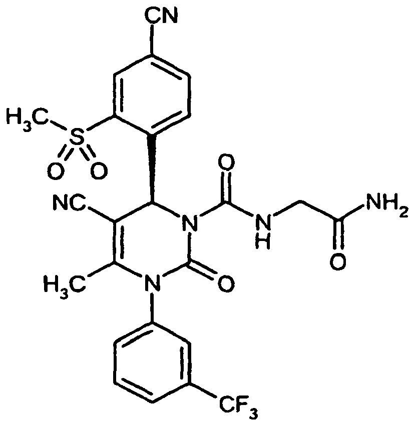

Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (197 mg, 0.315 mmol; Beispiel 6A) mit Glycinamid-Hydrochlorid (104 mg, 0.945 mmol) und *N,N-*Diisopropylethylamin (122 mg, 0.945 mmol) in Acetonitril (2.5 ml) zur Zielverbindung (72 mg, 39% d. Th.) umgesetzt.
HPLC (Methode 2): Rₜ = 4.24 min.
MS (ESIpos): *m*/*z* (%) = 561.3 (100) [M+H]⁺, 583.2 (50) [M+Na]⁺
¹H-NMR (400 MHz, DMSO-d₆): δ = 1.80 (s, 3H), 3.49 (s, 3H), 3.67 (dd, 2H), 7.12 (s, 1H), 7.26 (s, 1H), 7.36 (s, 1H), 7.72-8.23 (m, 5H), 8.29 (d, 1H), 8.43 (s, 1H), 9.14 (br. s, 1H).

### Beispiel 10

(6*S*)-5-Cyano-6-[4-cyano-2-(methylsulfonyl)phenyl]-*N*-(2-hydroxyethyl)-4-methyl-2-oxo-3-[3-(trifluormethyl)phenyl]-3,6-dihydropyrimidin-1(2*H*)-carboxamid

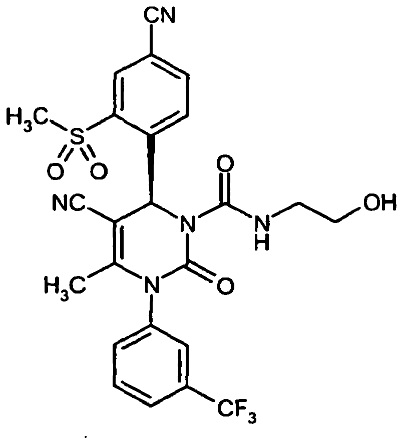

Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (80.0 mg, 0.128 mmol; Beispiel 6A) mit 2-Aminoethanol (23.4 mg, 0.384 mmol) in Acetonitril (1 ml) zur Zielverbindung (27 mg, 39% d. Th.) umgesetzt.
HPLC (Methode 2): Rₜ = 4.39 min.
MS (ESIpos): *m*/*z* (%) = 548.2 (100) [M+H]⁺
¹H-NMR (400 MHz, DMSO-d₆): δ = 1.78 (s, 3H), 3.03-3.45 (m, 4H), 3.50 (s, 3H), 4.74 (m, 1H), 7.26 (s, 1H), 7.74-8.22 (m, 5H), 8.29 (d, 1H), 8.43 (s, 1H), 8.94 (br. s, 1H).

### Beispiel 11

(6*S*)-5-Cyano-6-[4-cyano-2-(methylsulfonyl)phenyl]-4-methyl-*N*-morpholin-4-yl-2-oxo-3-[3-(trifluormethyl)phenyl]-3,6-dihydropyrimidin-1(2*H*)-carboxamid 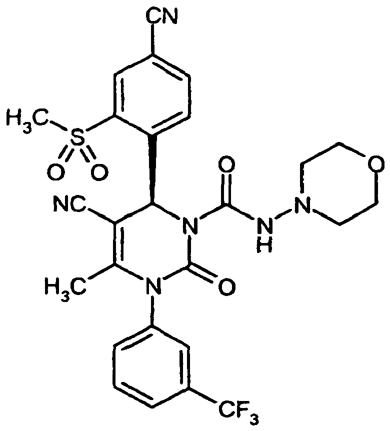 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit *N*-Aminomorpholin (38.2 mg, 0.374 mmol) in Acetonitril (1 ml) zur Zielverbindung (46 mg, 61% d. Th.) umgesetzt.
HPLC (Methode 3): Rₜ = 4.49 min.
MS (ESIpos): *m*/*z* (%) = 589.3 (100) [M+H]⁺
¹H-NMR (400 MHz, DMSO-d₆): δ = 1.80 (s, 3H), 2.53-2.72 (m, 4H), 3.47-3.56 (m, 4H), 3.50 (s, 3H), 7.11 (s, 1H), 7.74-8.21 (m, 5H), 8.32 (d, 1H), 8.43 (s, 1H), 9.63 (s, 1H).

### Beispiel 12

(6*S*)-5-Cyano-6-[4-cyano-2-(methylsulfonyl)phenyl]-*N*'*,N*'-bis(2-hydroxyethyl)-4-methyl-2-oxo-3-[3-(trifluormethyl)phenyl]-3,6-dihydropyrimidin-1(2*H*)-carbohydrazid 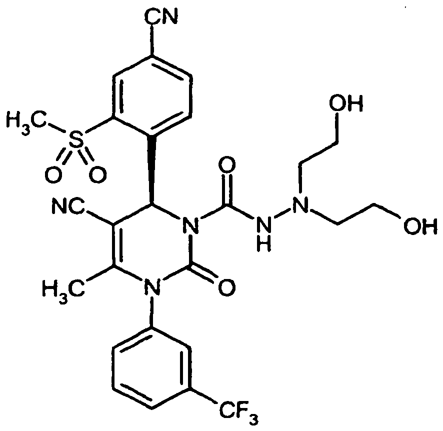 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit 2,2'-Hydrazin-1,1-diyldiethanol (45.0 mg, 0.374 mmol) in Acetonitril (1 ml) zur Zielverbindung (40 mg, 51% d. Th.) umgesetzt.
HPLC (Methode 3): Rₜ = 4.23 min.
MS (ESIpos): *m*/*z* (%) = 607.3 (100) [M+H]⁺
¹H-NMR (400 MHz, DMSO-d₆): δ = 1.80 (s, 3H), 2.70 (t, 4H), 3.20 (m, 4H), 3.46 (s, 3H), 4.15 (t, 2H), 7.11 (s, 1H), 7.74-8.37 (m, 6H), 8.44 (s, 1H), 9.60 (s, 1H).

### Beispiel 13

(6*S*)-5-Cyano-6-[4-cyano-2-(methylsulfonyl)phenyl]-*N*-(2-hydroxy-1,1-dimethylethyl)-4-methyl-2-oxo-3-[3-(trifluormethyl)phenyl]-3,6-dihydropyrimidin-1(2*H*)-carboxamid 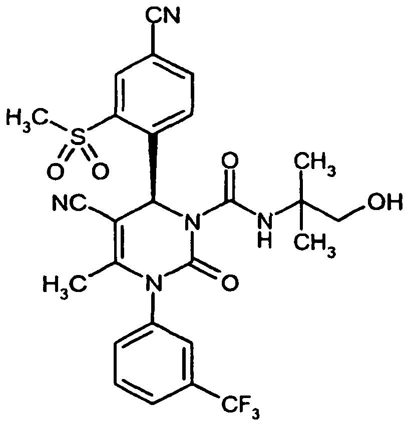 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (75.0 mg, 0.120 mmol; Beispiel 6A) mit 2-Amino-2-methylpropanol (32.1 mg, 0.360 mmol) in Acetonitril (0.96 ml) zur Zielverbindung (51 mg, 74% d. Th.) umgesetzt.
HPLC (Methode 2): Rₜ = 4.65 min.
MS (ESIpos): *m*/*z* (%) = 576.2 (100) [M+H]⁺
¹H-NMR (400 MHz, DMSO-d₆): δ = 1.02 (s, 3H), 1.13 (s, 3H), 1.78 (s, 3H), 3.13-3.28 (m, 2H), 3.51 (s, 3H), 4.98 (t, 1H), 7.26 (s, 1H), 7.74-8.23 (m, 5H), 8.30 (d, 1H), 8.45 (s, 1H), 8.98 (br. s, 1H).

### Beispiel 14

(6*S*)-5-Cyano-6-[4-cyano-2-(methylsulfonyl)phenyl]-*N*-[2-(2-hydroxyethoxy)ethyl]-4-methyl-2-oxo-3-[3-(trifluormethyl)phenyl]-3,6-dihydropyrimidin-1(2*H*)-carboxamid 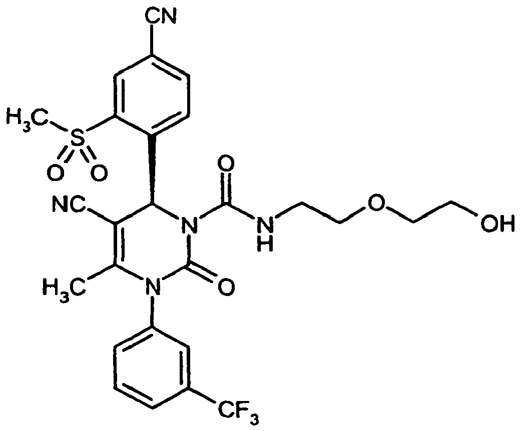 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1 (2*H*)-carboxylat (46.5 mg, 0.074 mmol; Beispiel 6A) mit 2-(2-Aminoethoxy)ethanol (23.4 mg, 0.223 mmol) in Acetonitril (1 ml) zur Zielverbindung (30 mg, 68% d. Th.) umgesetzt.
HPLC (Methode 2): Rₜ = 4.39 min.
MS (ESIpos): *m*/*z* (%) = 592.2 (100) [M+H]⁺
¹H-NMR (400 MHz, DMSO-d₆): δ = 1.78 (s, 3H), 3.16-3.25 (m, 2H), 3.26-3.43 (m, 6H), 3.50 (s, 3H), 4.52 (m, 1H), 7.25 (s, 1H), 7.74-8.23 (m, 5H), 8.29 (d, 1H), 8.44 (s, 1H), 8.90 (br. s, 1H).

### Beispiel 15

(6*S*)-5-Cyano-6-[4-cyano-2-(methylsulfonyl)phenyl]-*N*-cyclopropyl-4-methyl-2-oxo-3-[3-(trifluormethyl)phenyl]-3,6-dihydropyrimidin-1(2*H*)-carboxamid 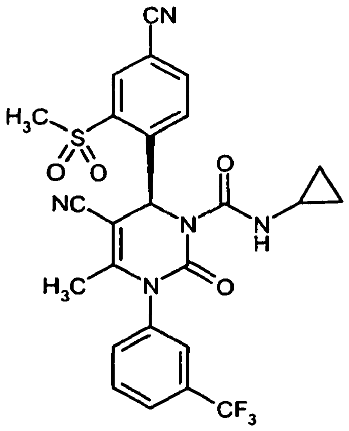 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (80.0 mg, 0.128 mmol; Beispiel 6A) mit Cyclopropylamin (21.9 mg, 0.384 mmol) in Acetonitril (1 ml) zur Zielverbindung (45 mg, 65% d. Th.) umgesetzt.
HPLC (Methode 3): Rₜ = 4.83 min.
MS (ESIpos): *m*/*z* (%) = 544.2 (100) [M+H]⁺
¹H-NMR (400 MHz, DMSO-d₆): δ = 0.28-0.65 (m, 4H), 1.78 (s, 3H), 2.51-2.61 (m, 1H), 3.52 (s, 3H), 7.21 (s, 1H), 7.74-8.19 (m, 5H), 8.30 (d, 1H), 8.44 (s, 1H), 8.72 (br. s, 1H).

### Beispiel 16

(6*S*)-5-Cyano-6-[4-cyano-2-(methylsulfonyl)phenyl]-*N*-(1,1-dioxidotetrahydrothiophen-3-yl)-4-methyl-2-oxo-3-[3-(trifluormethyl)phenyl]-3,6-dihydropyrimidin-1(2*H*)-carboxamid 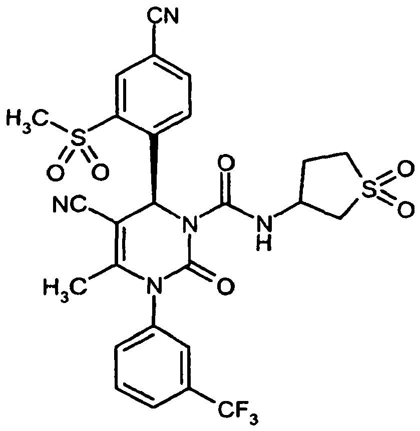 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit Tetrahydrothiophen-3-amin-1,1-dioxid (50.6 mg, 0.374 mmol) in Acetonitril (1 ml) zur Zielverbindung (52 mg, 66% d. Th.) umgesetzt.
HPLC (Methode 3): Rₜ = 4.55 min.
MS (ESIpos): *m*/*z* (%) = 622.3 (70) [M+H]⁺, 639.3 (38) [M+NH₄]⁺
¹H-NMR (400 MHz, DMSO-d₆): δ = 1.79 (s, 3H), 1.92-2.37 (m, 2H), 2.90-3.35 (m, 4H), 3.50 (s, 3H), 4.39 (m, 1H), 7.24 (s, 1H), 7.72-8.18 (m, 5H), 8.29 (d, 1H), 8.44 (s, 1H), 9.13 (br. s, 1H).

### Beispiel 17

(6*S*)-5-Cyano-6-[4-cyano-2-(methylsulfonyl)phenyl]-*N*-(2-methoxyethyl)-4-methyl-2-oxo-3-[3-(trifluormethyl)phenyl]-3,6-dihydropyrimidin-1(2*H*)-carboxamid 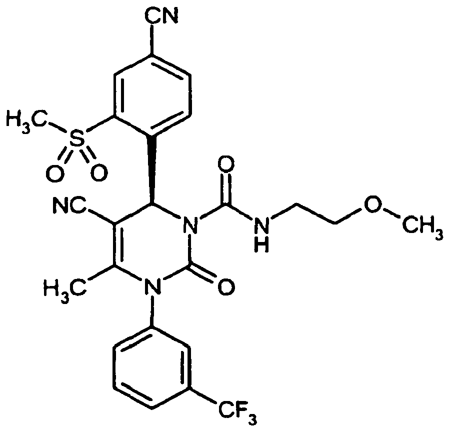 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit 2-Methoxyethylamin (28.1 mg, 0.374 mmol) in Acetonitril (1 ml) zur Zielverbindung (55 mg, 78% d. Th.) umgesetzt.
HPLC (Methode 3): Rₜ = 4.72 min.
MS (ESIpos): *m*/*z* (%) = 562.3 (100) [M+H]⁺
¹H-NMR (400 MHz, DMSO-d₆): δ = 1.78 (s, 3H), 3.15 (s, 3H), 3.17-3.32 (m, 4H), 3.50 (s, 3H), 7.25 (s, 1H), 7.74-8.23 (m, 5H), 8.29 (d, 1H), 8.43 (s, 1H), 8.90 (br. s, 1H).

### Beispiel 18

(4S)-4-[4-Cyano-2-(methylsulfonyl)phenyl]-3- {[(3*S*)-3-hydroxypyrrolidin-1-yl]carbonyl} -6-methyl-2-oxo-1-[3-(trifluormethyl)phenyl]-1,2,3,4-tetrahydropyrimidin-5-carbonitril 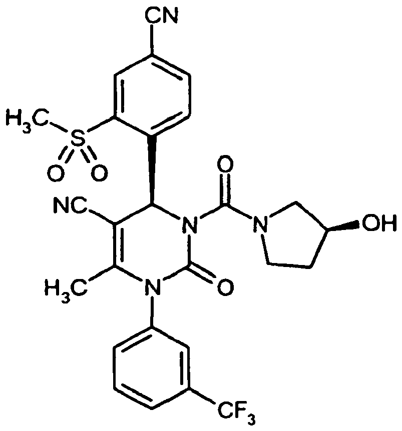 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (135 mg, 0.216 mmol; Beispiel 6A) mit (*S*)-(-)-3-Pyrrolidinol (56.4 mg, 0.647 mmol) in Acetonitril (1.7 ml) zur Zielverbindung (48 mg, 38% d. Th.) umgesetzt.
HPLC (Methode 2): Rₜ = 4.21 min.
MS (DCI / NH₃): *m*/*z* = 574 [M+H]⁺, 591.3 [M+NH₄]⁺
¹H-NMR (400 MHz, DMSO-d₆): δ = 1.59-1.95 (m, 2H), 1.83 (s, 3H), 2.86-3.36 (m, 2H), 3.44-3.72 (m, 2H), 3.48 (s, 3H), 4.19 (m, 1H), 4.87 und 5.08 (jeweils d, 1H), 6.82 (s, 1H), 7.73-8.43 (m, 7H).

### Beispiel 19

(4*S*)-4-[4-Cyano-2-(methylsulfonyl)phenyl]-3-{[(3*R*)-3-hydroxypyrrolidin-1-yl]carbonyl}-6-methyl-2-oxo-1-[3-(trifluormethyl)phenyl]-1,2,3,4-tetrahydropyrimidin-5-carbonitril 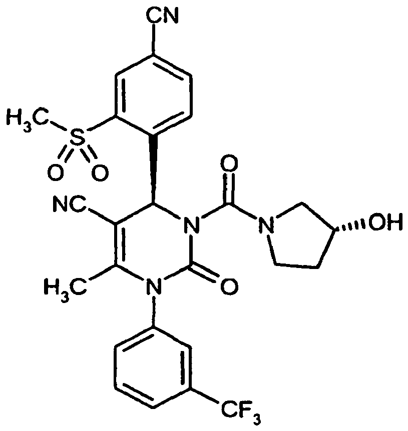 Nach der Allgemeinen Vorschrift 1 wurde 4-Nitrophenyl-(6*S*)-5-cyano-6-[4-cyano-2-(methylsulfonyl)phenyl]-methyl-2-oxo-3-[3-(trifluormethyl)phenyl]-3,6-dihydropyrimidin-1(2*H*)-carboxylat (135 mg, 0.216 mmol; Beispiel 6A) mit (*R*)-(+)-3-Pyrrolidinol (56.4 mg, 0.647 mmol) in Acetonitril (1.7 ml) zur Zielverbindung (92 mg, 74% d. Th.) umgesetzt.
HPLC (Methode 2): Rₜ = 4.21 min.
MS (ESI): *m*/*z* (%) = ESI⁺ 574.2 (10) [M+H]⁺, ESI⁻ 618.1 (100) [M-H+HCOOH]⁻
¹H-NMR (400 MHz, DMSO-d₆): δ = 1.63-1.88 (m, 2H), 1.82 (s, 3H), 2.95-3.23 (m, 2H), 3.34-3.84 (m, 2H), 3.49 (s, 3H), 4.17 und 4.26 (jeweils br. s, 1H), 4.86 und 4.91 (jeweils br. s, 1H), 6.84 (s, 1H), 7.73-8.25 (m, 6H), 8.34-8.41 (m, 1H).

### Beispiel 20

(4*S*)-4-[4-Cyano-2-(methylsulfonyl)phenyl]-3-{[4-(2-hydroxyethyl)piperazin-1-yl]carbonyl}-6-methyl-2-oxo-1-[3-(trifluormethyl)phenyl]-1,2,3,4-tetrahydropyrimidin-5-carbonitril 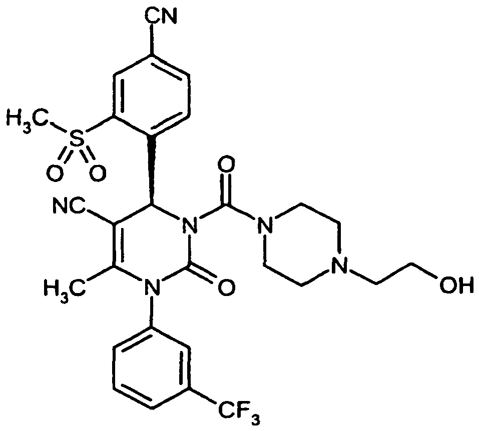 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78 mg, 0.125 mmol; Beispiel 6A) mit *N*-(2-Hydroxyethyl)piperazin (48.7 mg, 0.374 mmol) in Acetonitril (1.0 ml) zur Zielverbindung (68 mg, 85% d. Th.) umgesetzt.
HPLC (Methode 3): Rₜ = 4.18 min.
MS (ESIpos): *m*/*z* (%) = 617.3 (100) [M+H]⁺
¹H-NMR (400 MHz, DMSO-d₆): δ = 1.81 (s, 3H), 2.03-2.73 (m, 4H), 2.94-3.87 (m, 11H, darin t bei 3.47, s bei 3.51), 4.41 (br. s, 1H), 6.83 (s, 1H), 6.91-8.52 (m, 7H).

### Beispiel 21

2-[(6*S*)-5-Cyano-6-[4-cyano-2-(methylsulfonyl)phenyl]-4-methyl-2-oxo-3-[3-(trifluormethyl)-phenyl]-3,6-dihydropyrimidin-1(2*H*)-yl]essigsäure 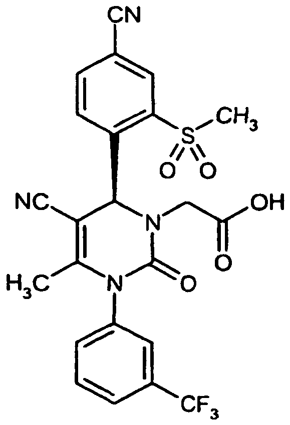 Die Reaktion wurde unter Argon durchgeführt. *tert*.-Butyl-[(6*R*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-yl]-acetat (350 mg, 609 µmol; Beispiel 7) wurde in Dichlormethan (60 ml) vorgelegt und mit Trifluoressigsäure (20 ml) versetzt. Der Ansatz wurde 90 min bei RT gerührt. Danach wurden die flüchtigen Bestandteile am Rotationsverdampfer abgezogen. Der Rückstand wurde in Toluol (50 ml) aufgenommen und erneut im Vakuum eingeengt. Diese Prozedur wurde noch einmal wiederholt. Das Rohprodukt wurde mittels präparativer HPLC gereinigt (Säule: Gromsil C-18 10 µm; Eluent: Acetonitril/Wasser + 0.1 % TFA 10:90 → 90:10). Die Titelverbindung wurde als farbloser Feststoff erhalten (290 mg, 92% d. Th.).

LC-MS (Methode 4): Rₜ = 1.11 min; MS (ESIpos): *m*/*z* (%) = 519.0 (100) [M+H]⁺; MS (ESIneg): *m*/*z* (%) = 352.1 (100), 415.9 (70), 517.0 (50) [M-H]⁻.

### Beispiel 22

2-[(6*S*)-5-Cyano-6-[4-cyano-2-(methylsulfonyl)phenyl]-4-methyl-2-oxo-3-[3-(trifluormethyl)- 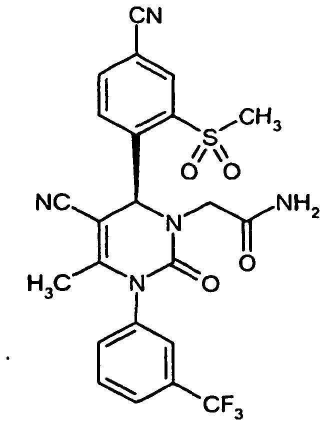 Die Reaktion wurde unter Argon durchgeführt. 2-[(6*S*)-5-Cyano-6-[4-cyano-2-(methylsulfonyl)-phenyl]-4-methyl-2-oxo-3-[3-(trifluormethyl)phenyl]-3,6-dihydropyrimidin-1(2*H*)-yl]essigsäure (90 mg, 174 µmol; Beispiel 21) wurde in DMF (3 ml) vorgelegt, bei 0°C mit HATU (330 mg, 868 µmol, 5 eq.) versetzt und für 20 min gerührt. Anschließend wurde Ammoniumchlorid (18.1 mg, 340 µmol, 5 eq.) sowie *N*,*N*-Diisopropylethylamin (224 mg, 1736 µmol, 10 eq.) zugesetzt und das Gemisch bei RT gerührt, bis vollständiger Umsatz erreicht war (nach einigen Stunden; HPLC-Kontrolle). Das Reaktionsgemisch wurde dann im Vakuum eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (85 mg, 94% d. Th.).
LC-MS (Methode 4): Rₜ = 1.07 min; MS (ESIpos): *m*/*z* (%) = 501.1 (70), 518.1 (100) [M+H]⁺; MS (ESIneg): *m*/*z* (%) = 516.7 (80) [M-H]⁻
¹H-NMR (400 MHz, DMSO-d₆): δ = 1.85 (s, 3H), 3.15 (br. d, 1H), 3.40 (s, 3H), 4.05 (d, 1H), 6.50 (s, 1H), 7.10 (s, 1H), 7.40 (s, 1H), 7.65-8.05 (m, 4H), 8.40 (m, 3H).

### Beispiel 23

2-[(6*S*)-5-Cyano-6-[4-cyano-2-(methylsulfonyl)phenyl]-4-methyl-2-oxo-3-[3-(trifluormethyl)-phenyl]-3,6-dihydropyrimidin-1(2*H*)-yl]-*N*-(2-hydroxyethyl)acetamid 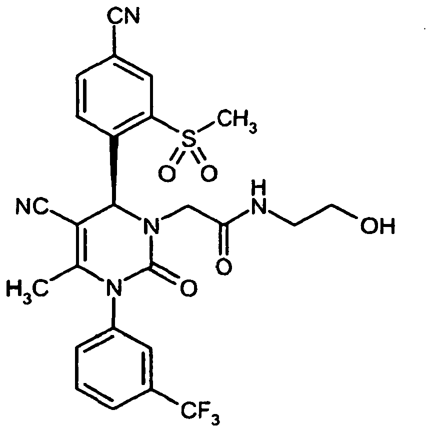 Die Reaktion wurde unter Argon durchgeführt. 2-[(6*S*)-5-Cyano-6-[4-cyano-2-(methylsulfonyl)-phenyl]-4-methyl-2-oxo-3-[3-(trifluormethyl)phenyl]-3,6-dihydropyrimidin-1(2*H*)-yl]essigsäure (45 mg, 87 µmol; Beispiel 21) wurde in DMF (1.5 ml) vorgelegt, bei 0°C mit HATU (165 mg, 434 µmol, 5 eq.) versetzt und für 20 min gerührt. Anschließend wurde 2-Aminoethanol (26.5 mg, 434 µmol, 5 eq.) sowie *N*,*N*-Diisopropylethylamin (56 mg, 434 µmol, 5 eq.) zugesetzt und das Gemisch 16 h bei RT gerührt, bis vollständiger Umsatz erreicht war (HPLC-Kontrolle). Das Reaktionsgemisch wurde dann im Vakuum eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (40 mg, 82% d. Th.).
LC-MS (Methode 6): Rₜ = 2.05 min; MS (ESIpos): *m*/*z* (%) = 562.1 (100) [M+H]⁺
¹H-NMR (400 MHz, DMSO-d₆): δ = 1.85 (s, 3H), 3.05 (m, 2H), 3.20 (d, 1H), 3.35 (m, 2H), 3.40 (s, 3H), 4.05 (d, 1H), 6.50 (s, 1H), 7.65-8.10 (m, 5H), 8.30-8.45 (m, 3H).

### Beispiel 24

(4*S*)-4-[4-Cyano-2-(methylsulfonyl)phenyl]-3-{2-[4-(2-hydroxyethyl)piperidin-1-yl]-2-oxoethyl}-6-methyl-2-oxo-1-[3-(trifluormethyl)phenyl]-1,2,3,4-tetrahydropyrimidin-5-carbonitril 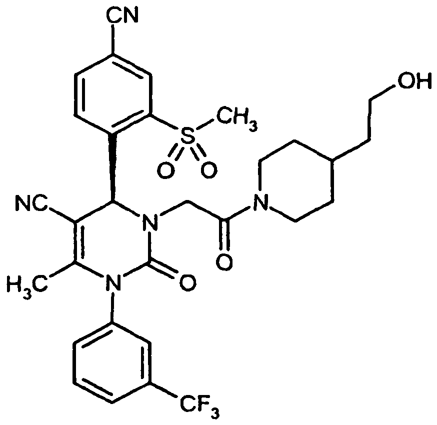 Die Reaktion wurde unter Argon durchgeführt. 2-[(6*S*)-5-Cyano-6-[4-cyano-2-(methylsulfonyl)-phenyl]-4-methyl-2-oxo-3-[3-(trifluormethyl)phenyl]-3,6-dihydropyrimidin-1(2*H*)-yl]essigsäure (45 mg, 87 µmol; Beispiel 21) wurde in DMF (1.5 ml) vorgelegt, bei 0°C mit HATU (165 mg, 434 µmol, 5 eq.) versetzt und für 20 min gerührt. Anschließend wurde 4-Piperidinethanol (33.6 mg, 260 µmol, 3 eq.) sowie *N*,*N*-Diisopropylethylamin (56 mg, 434 µmol, 5 eq.) zugesetzt und das Gemisch 16 h bei RT gerührt, bis vollständiger Umsatz erreicht war (HPLC-Kontrolle). Das Reaktionsgemisch wurde dann im Vakuum eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (26 mg, 48% d. Th., ca. 80% Reinheit).
LC-MS (Methode 6): Rₜ = 2.22 min; MS (ESIpos): *m*/*z* (%) = 630.2 (100) [M+H]⁺.

### Beispiel 25

(4*S*)-4-[4-Cyano-2-(methylsulfonyl)phenyl]-6-methyl-2-oxo-3-[2-oxo-2-(3-oxopiperazin-1-yl)-ethyl]-1-[3-(trifluormethyl)phenyl]-1,2,3,4-tetrahydropyrimidin-5-carbonitril 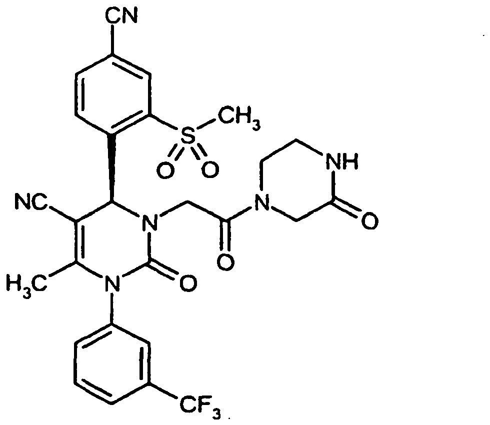 Die Reaktion wurde unter Argon durchgeführt. 2-[(6*S*)-5-Cyano-6-[4-cyano-2-(methylsulfonyl)-phenyl]-4-methyl-2-oxo-3-[3-(trifluormethyl)phenyl]-3,6-dihydropyrimidin-1(2*H*)-yl]essigsäure (50 mg, 96 µmol; Beispiel 21) wurde in DMF (2 ml) vorgelegt, bei 0°C mit HATU (183 mg, 482 µmol, 5 eq.) versetzt und für 20 min gerührt. Anschließend wurde 2-Oxopiperazin (48.3 mg, 482 µmol, 5 eq.) sowie *N*,*N*-Diisopropylethylamin (62 mg, 482 µmol, 5 eq.) zugesetzt und das Gemisch 30 min bei RT gerührt, bis vollständiger Umsatz erreicht war (HPLC-Kontrolle). Das Reaktionsgemisch wurde dann im Vakuum eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (32 mg, 55% d. Th.).
LC-MS (Methode 6): Rₜ = 2.05 min; MS (ESIpos): *m*/*z* (%) = 601.2 (100) [M+H]⁺
¹H-NMR (400 MHz, DMSO-d₆): δ = 1.85 (s, 3H), 3.05 (m, 2H), 3.20-3.65 (m, 3H), 3.40 (s, 3H), 3.80-4.00 (m, 2H), 4.45 (t, 1H), 6.35 (s, 1H), 7.65-8.10 (m, 5H), 8.30-8.45 (m, 3H).

### Beispiel 26

(4*S*)-4-[4-Cyano-2-(methylsulfonyl)phenyl]-3-{2-[4-hydroxypiperidin-1-yl]-2-oxoethyl}-6-methyl-2-oxo-1-[3-(trifluormethyl)phenyl]-1,2,3,4-tetrahydropyrimidin-5-carbonitril 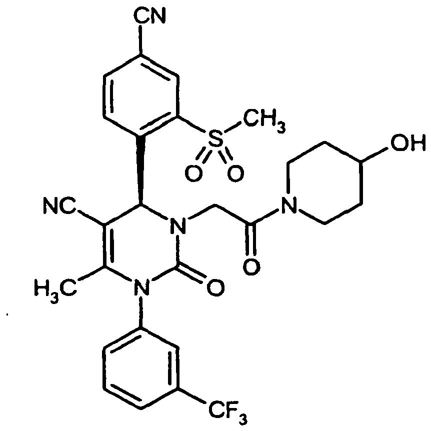 Die Reaktion wurde unter Argon durchgeführt. 2-[(6*S*)-5-Cyano-6-[4-[cyano-2-(methylsulfonyl)-phenyl]-4-methyl-2-oxo-3-[3-(trifluormethyl)phenyl]-3,6-dihydropyrimidin-1(2*H*)-yl]essigsäure (45 mg, 87 µmol; Beispiel 21) wurde in DMF (1.5 ml) vorgelegt, bei 0°C mit HATU (165 mg, 434 µmol, 5 eq.) versetzt und für 20 min gerührt. Anschließend wurde 4-Hydroxypiperidin (43.9 mg, 434 µmol, 5 eq.) sowie *N*,*N*-Diisopropylethylamin (56 mg, 434 µmol, 5 eq.) zugesetzt und das Gemisch 16 h bei RT gerührt, bis vollständiger Umsatz erreicht war (HPLC-Kontrolle). Das Reaktionsgemisch wurde dann im Vakuum eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (51 mg, 98% d. Th.).
LC-MS (Methode 6): Rₜ = 2.13 min; MS (ESIpos): *m*/*z* (%) = 602.1 (100) [M+H]⁺
¹H-NMR (400 MHz, DMSO-d₆): δ = 1.20 (m, 2H), 1.65 (m, 2H), 1.85 (s, 3H), 2.95 (m, 2H), 3.15 (m, 1H), 3.40 (s, 3H), 3.55-3.70 (m, 2H), 3.95 (m, 1H), 4.45 (m, 1H), 6.35 (s, 1H), 7.65-8.10 (m, 4H), 8.30-8.45 (m, 3H).

### Beispiel 27

(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonitril

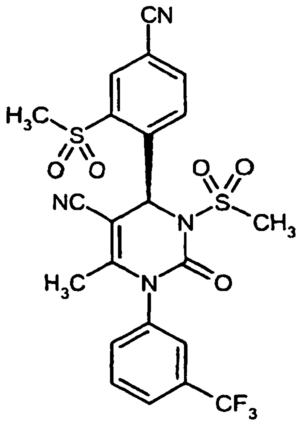

Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (69.1 mg, 150 µmol; Beispiel 6) wurde in THF (2 ml) vorgelegt und bei 0°C mit Natriumhydrid (60% in Mineralöl; 7.2 mg, 180 µmol) versetzt. Es wurde auf RT erwärmt und 20 min gerührt. Anschließend wurde eine Lösung von Methansulfonylchlorid (20.6 mg, 180 µmol, 1.2 eq.) in THF (1 ml) langsam zugetropft. Nach 16 h Reaktionszeit wurde Methansulfonylchlorid (6.7 mg, 60 µmol, 0.4 eq.) nachgegeben und erneut für 60 min bei RT gerührt. Das Reaktionsgemisch wurde dann eingeengt und das Rohprodukt mittels präparativer HPLC aufgereinigt (Säule: Gromsil C-18 10 µm; Eluent: Acetonitril/Wasser + 0.1% TFA 10:90 → 80:20). Man erhielt die Titelverbindung als einen farblosen Feststoff (47 mg, 58% d. Th.).
LC-MS (Methode 4): Rₜ = 1.22 min; MS (ESIpos): *m*/*z* (%) = 539.0 (30) [M+H]⁺; MS (ESIneg): *m*/*z* (%) = 353.3 (100), 415.9 (50), 457.2 (80), 535.6 (100) [M-H]⁻
¹H-NMR (400 MHz, DMSO-d₆): δ = 1.80 (s, 3H), ~3.40 (2s, 6H), 7.30 (s, 1H), 7.75-8.35 (m, 6H), 8.55 (s, 1H).

### Beispiel 28

(4*S*)-4-[4-Cyano-2-(methylsulfonyl)phenyl]-6-methyl-3-(isopropylsulfonyl)-2-oxo-1-[3-(trifluormethyl)phenyl]-1,2,3,4-tetrahydropyrimidin-5-carbonitril 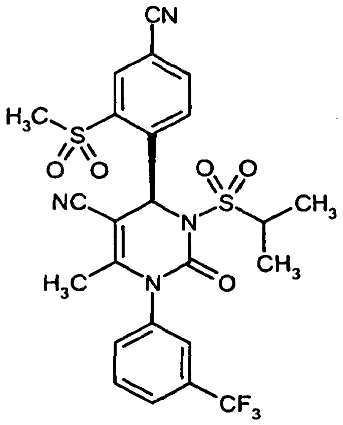 Analog zur Herstellung von Beispiel 27 wurden (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol; Beispiel 6), Natriumhydrid (60%, 11.2 mg, 282 µmol) und 2-Propansulfonylchlorid (40.3 mg, 282 µmol) für 16 h miteinander umgesetzt. Man erhielt die Titelverbindung als einen farblosen Feststoff (88 mg, 72% d. Th.).
LC-MS (Methode 5): Rₜ = 2.15 min; MS (ESIpos): m/z (%) = 567.2 (100) [M+H]⁺; MS (ESIneg): m/z (%) = 565.2 (100) [M-H]⁻
¹H-NMR (400 MHz, DMSO-d₆): δ = 1.10 (d, 3H), 1.35 (d, 3H), 1.80 (s, 3H), 3.40 (s, 3H), 4.05 (m, 1H), 7.25 (s, 1H), 7.75-8.25 (m, 6H), 8.55 (s, 1H).

### Beispiel 29

(4*S*)-4-[4-Cyano-2-(methylsulfonyl)phenyl]-6-methyl-3-{[2-(trifluormethoxy)phenyl]sulfonyl}-2-oxo-1-[3-(trifluormethyl)phenyl]-1,2,3,4-tetrahydropyrimidin-5-carbonitril

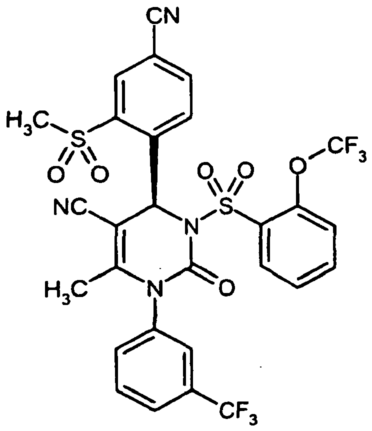

Analog zur Herstellung von Beispiel 27 wurden (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol; Beispiel 6), Natriumhydrid (60%, 11.2 mg, 282 µmol) und [2-(Trifluormethoxy)phenyl]-sulfonylchlorid (73.6 mg, 282 µmol) für 16 h miteinander umgesetzt. Man erhielt die Titelverbindung als einen farblosen Feststoff (115 mg, 77% d. Th.).
LC-MS (Methode 4): Rₜ = 1.39 min; MS (ESIpos): *m*/*z* (%) = 685.0 (40) [M+H]⁺; MS (ESIneg): *m*/*z* (%) = 457.5 (100), 683.6 (80) [M-H]⁻
¹H-NMR (400 MHz, DMSO-d₆): δ = 1.80 (s, 3H), 3.45 (s, 3H), 7.35-8.40 (m, 11H), 8.60 (s, 1H).

### Beispiel 30

(4S)-4-[4-Cyano-2-(methylsulfonyl)phenyl]-6-methyl-3-((chlormethyl)sulfonyl)-2-oxo-1-[3-(trifluormethyl)phenyl]-1,2,3,4-tetrahydropyrimidin-5-carbonitril

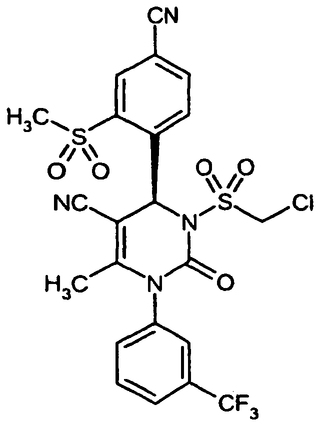

Analog zur Herstellung von Beispiel 27 wurden (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol; Beispiel 6), Natriumhydrid (60%, 11.3 mg, 282 µmol) und Chlormethansulfonsäurechlorid (42 mg, 282 µmol) für 3 h miteinander umgesetzt. Man erhielt die Titelverbindung als einen farblosen Feststoff (86 mg, 69% d. Th.).
LC-MS (Methode 5): Rₜ = 2.11 min; MS (ESIpos): *m*/*z* (%) = 573.1 (100) [M+H]⁺; MS (ESIneg): *m*/*z* (%) = 571.1 (100) [M-H]-
¹H-NMR (400 MHz, DMSO-d₆): δ = 1.85 (s, 3H), 3.45 (s, 3H), 5.45 (d, 1H), 5.55 (d, 1H), 7.35 (s, 1H), 7.80-8.35 (m, 6H), 8.60 (s, 1H).

### Beispiel 31

(4*S*)-4-[4-Cyano-2-(methylsulfonyl)phenyl]-6-methyl-3-(ethylsulfonyl)-2-oxo-1-[3-(trifluormethyl)phenyl]-1,2,3,4-tetrahydropyrimidin-5-carbonitril

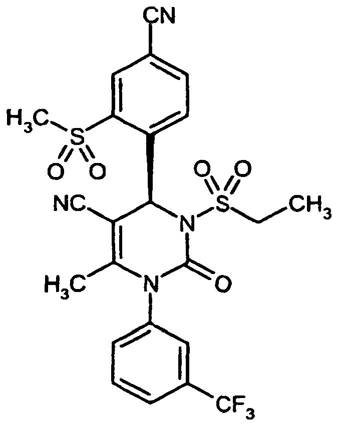

Analog zur Herstellung von Beispiel 27 wurden (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol; Beispiel 6), Natriumhydrid (60%, 11.2 mg, 282 µmol) und Ethansulfonsäurechlorid (36 mg, 282 µmol) für 3 h miteinander umgesetzt. Man erhielt die Titelverbindung als einen farblosen Feststoff (99 mg, 83% d. Th.).

LC-MS (Methode 5): Rₜ = 2.06 min; MS (ESIpos): *m*/*z* (%) = 553.1 (100) [M+H]⁺; MS (ESIneg): *m*/*z* (%) = 416.2 (50), 551.2 (90) [M-H]^{- 1}H-NMR (400 MHz, DMSO-d₆): δ = 1.15 (t, 3H), 1.80 (s, 3H), 3.40 (s, 3H), 3.55 (m, 1H), 3.75 (m, 1H), 7.30 (s, 1H), 7.70-8.35 (m, 6H), 8.55 (s, 1H).

### Beispiel 32

(4*S*)-4-[4-Cyano-2-(methylsulfonyl)phenyl]-6-methyl-3-(cyclopropylsulfonyl)-2-oxo-1-[3-(trifluormethyl)phenyl]-1,2,3,4-tetrahydropyrimidin-5-carbonitril

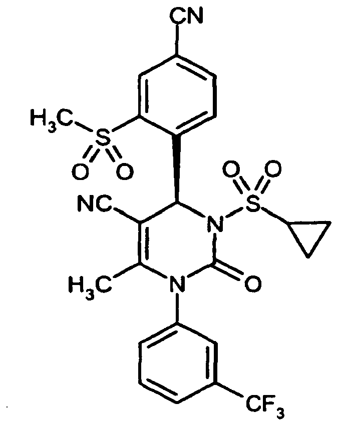

Analog zur Herstellung von Beispiel 27 wurden (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol; Beispiel 6), Natriumhydrid (60%, 11.2 mg, 282 µmol) und Cyclopropylsulfonylchlorid (39.7 mg, 282 µmol) für 16 h miteinander umgesetzt. Man erhielt die Titelverbindung als einen farblosen Feststoff (57 mg, 47% d. Th.).
LC-MS (Methode 5): Rₜ = 2.07 min; MS (ESIpos): *m*/*z* (%) = 565.1 (100) [M+H]⁺; MS (ESIneg): *m*/*z* (%) = 416.2 (80), 563.2 (80) [M-H]⁻
¹H-NMR (400 MHz, DMSO-d₆): δ = 0.30 (br. m, 1H), 0.85 (br. m, 1H), 1.15 (m, 2H), 1.80 (s, 3H), 3.10 (m, 1H), 3.40 (s, 3H), 7.30 (s, 1H), 7.75-8.35 (m, 6H), 8.55 (s, 1H).

### Beispiel 33

(4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonitril

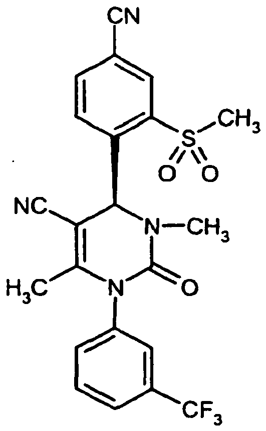

### Methode A:

Der Ansatz wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (75 mg, 163 µmol; Beispiel 6) wurde in THF (2 ml) vorgelegt und mit Natriumhydrid (60% in Mineralöl; 9.2 mg, 228 µmol) versetzt. Nach 20 min Rühren wurde Iodmethan (32.4 mg, 14.2 µl, 228 µmol) zugegeben und das Gemisch für weitere 120 min bei RT gerührt. Das Reaktionsgemisch wurde dann direkt mittels präparativer HPLC gereinigt (Säule: Kromasil-100A, C-18 5 µm; Eluent: Acetonitril/Wasser + 0.1% TFA 10:90 → 80:20). Man erhielt die Titelverbindung als einen farblosen Feststoff (18 mg, 23% d. Th.).

### Methode B:

Der Ansatz wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (460.4 mg, 1 mmol; Beispiel 6) wurde in absolutem THF (10 ml) vorgelegt und bei -78°C mit einer 1 M Lösung von Lithiumhexamethyldisilazid (LiHMDS) in THF (1 ml; 1 eq.) versetzt. Nach 20 min Rühren wurde Iodmethan (710 mg; 5 eq.) zugegeben und die Mischung für 60 h unter allmählicher Erwärmung von -78°C auf RT gerührt. Das Reaktionsgemisch wurde dann direkt mittels präparativer HPLC gereinigt (Säule: Gromsil, C-18 10 µm; Eluent: Acetonitril/Wasser + 0.1% TFA 10:90 → 90:10). Man erhielt die Titelverbindung als einen farblosen Feststoff (454 mg, 96% d. Th.).

### Methode C:

Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat (150 mg, 65 µmol; ca. 21% Reinheit) wurde unter Argon in einem druckfesten Glasrohr in DMF (1 ml) suspendiert. Molekularsieb (4 Å, 20 mg) und Methyliodid (82 µl, 1.3 mmol; 20 eq.) wurden zugegeben. Das versiegelte Rohr wurde für 15 h auf 115°C erhitzt. Die Reaktionsmischung wurde danach filtriert und das Filtrat im Vakuum eingeengt. Der Rückstand wurde mittels präparativer HPLC gereinigt (Gromsil C18-Säule; Eluent: Acetonitril/Wasser + 0.1% TFA). Nach Lyophilisation erhielt man die Titelverbindung als Feststoff (29.4 mg, 95% d. Th.).
LC-MS (Methode 4): Rₜ = 1.21 min; MS (ESIpos): m/z (%) = 475.0 (100) [M+H]⁺; MS (ESIneg): m/z (%) = 473.2 (100) [M-H]⁻
¹H-NMR (400 MHz, DMSO-d₆): δ = 1.80 (s, 3H), 2.65 (s, 3H), 3.40 (s, 3H), 6.45 (s, 1H), 7.65-8.40 (m, 6H), 8.45 (s, 1H).

### Beispiel 34

(*rac*)-4-{6-Methyl-2,5-dioxo-1-[3-(trifluormethyl)phenyl]-2,3,4,5,6,7-hexahydro-1H-pyrrolo-[3,4-d]pyrimidin-4-yl}-3-(methylsulfonyl)benzonitril 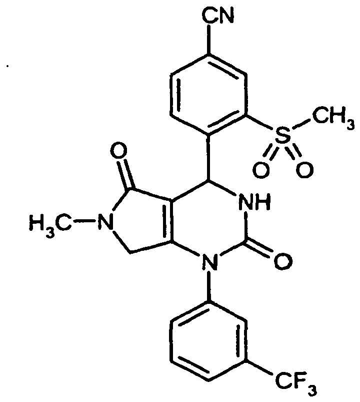 (*rac*)-*2*,3-Dibrompropyl-6-(brommethyl)-4-[4-cyano-2-(methylsulfonyl)phenyl]-2-oxo-1-[3-(trifluormethyl)phenyl]-1,2,3,4-tetrahydropyrimidin-5-carboxylat (758.2 mg, 1.0 mmol; Beispiel 7A) wurde unter Argon-Schutzgasatmosphäre mit einer 1 M Lösung von Methylamin in THF (20 ml, 20.0 mmol, 20 eq.) versetzt und 30 min bei RT gerührt. Das Reaktionsgemisch wurde dann im Vakuum eingeengt und das Rohprodukt mittels präparativer HPLC gereinigt (Säule: Gromsil C-18 10 µm; Eluent: Acetonitril/Wasser + 0.1% TFA 10:90 → 90:10). Man erhielt die Titelverbindung als einen farblosen Feststoff (328 mg, 67% d. Th.).

LC-MS (Methode 5): Rₜ = 1.61 min; MS (ESIpos): *m*/*z* (%) = 491.1 (100) [M+H]⁴; MS (ESIneg): *m*/*z* (%) = 489.1 (100) [M-H]^{- 1}H-NMR (400 MHz, DMSO-d₆): δ = 2.65 (s, 3H), 3.55 (s, 3H), 3.85 (m, 2H), 6.65 (s, 1H), 7.70-7.85 (m, 3H), 8.00 (s, 1H), 8.10 (s, 1H), 8.15 (d, 1H), 8.25 (dd, 1H), 8.30 (s, 1H).

### Beispiel 35

4-{(4*S*)-6-Methyl-2,5-dioxo-1-[3-(trifluormethyl)phenyl]-2,3,4,5,6,7-hexahydro-1*H*-pyrrolo-[3,4-d]pyrimidin-4-yl}-3-(methylsulfonyl)benzonitril 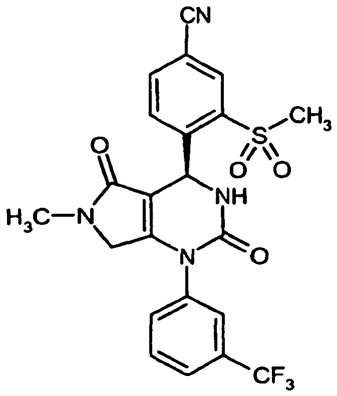 (*rac*)-4-{6-Methyl-2,5-dioxo-1-[3-(trifluormethyl)phenyl]-2,3,4,5,6,7-hexaxydro-1*H*-pyrrolo-[3,4-d]pyrimidin-4-yl}-3-(methylsulfonyl)benzonitril (Beispiel 34, 190 mg) wurde durch präparative HPLC-Chromatographie an chiraler Phase in die Enantiomere aufgetrennt [Säule: Daicel Chiralpak IC, 5 µm, 250 mm x 20 mm; Probenvorbereitung: Probe wurde in 70 ml Methanol/Acetonitril/MTBE 25:10:35 gelöst; Injektionsvolumen: 1 ml; Eluent: MTBE/Methanol 75:25 (0-7 min); Fluss: 15 ml/min; Temperatur: 30°C; Detektion: 220 nm]. Auf diese Weise wurden 97 mg (100% d. Th., >99.0% ee) des 4S-Enantiomeren erhalten. Der Enantiomerenüberschuss (ee-Wert) wurde chromatographisch bestimmt [Säule: Daicel Chiralpak IC, 5 µm, 250 mm x 4.6 mm; Eluent: MTBE/Methanol 75:25; Fluss: 1 ml/min; Temperatur: 25°C; Detektion: 220 nm; Rₜ = 6.62 min].

LC-MS (Methode 5): Rₜ = 1.59 min; MS (ESIpos): *m*/*z* (%) = 491.1 (100) [M+H]⁺; MS (ESIneg): *m*/*z* (%) = 446.2 (70), 489.2 (100) [M-H]⁻.

### Beispiel 36

4-{(4*S*)-6-Methyl-3-(methylsulfonyl)-2,5-dioxo-1-[3-(trifluormethyl)phenyl]-2,3,4,5,6,7-hexahydro-1*H*-pyrrolo[3,4-d]pyrimidin-4-yl}-3-(methylsulfonyl)benzonitril 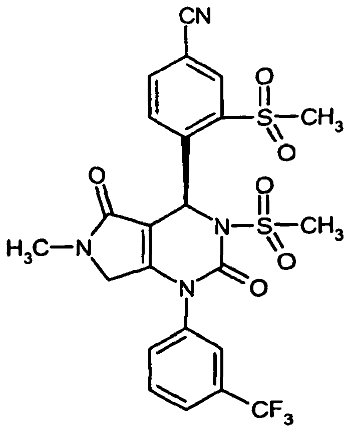 Analog zur Herstellung von Beispiel 27 wurden 4-{(4S)-6-Methyl-2,5-dioxo-1-[3-(trifluormethyl)-phenyl]-2,3,4,5,6,7-hexahydro-1*H*-pyrrolo[3,4-d]pyrimidin-4-yl}-3-(methylsulfonyl)benzonitril (20 mg, 41 µmol; Beispiel 35), Natriumhydrid (60%, 2.3 mg, 57 µmol) und Methansulfonylchlorid (6.5 mg, 57 µmol) für 2 h miteinander umgesetzt. Man erhielt die Titelverbindung als einen farblosen Feststoff (1.7 mg, 7% d. Th.). Daneben wurden 6.4 mg (32% d. Th.) des Ausgangsmaterials zurückgewonnen.

LC-MS (Methode 4): Rₜ = 1.10 min; MS (ESIpos): m/z (%) = 569.0 (100) [M+H]⁺; MS (ESIneg): m/z (%) = 568.4 (100) [M-H]⁻.

### Beispiel 37

(*rac*)-2-{4-[4-Cyano-2-(methylsulfonyl)phenyl]-6-methyl-2,5-dioxo-1-[3-(trifluormethyl)phenyl]-1,2,4,5,6,7-hexahydro-3*H*-pyrrolo[3,4-d]pyrimidin-3-yl}acetamid 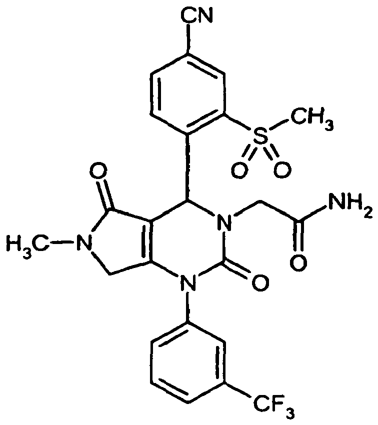 Die Titelverbindung wurde ausgehend von (*rac*)-4-{6-Methyl-2,5-dioxo-1-[3-(trifluormethyl)-phenyl]-2,3,4,5,6,7-hexahydro-1*H-*pyrrolo[3,4-d]pyrimidin-4-yl}-3-(methylsulfonyl)benzonitril (Beispiel 34) analog zur mehrstufigen Darstellung von 2-[(6*S*)-5-Cyano-6-[4-cyano-2-(methylsulfonyl)phenyl]-4-methyl-2-oxo-3-[3-(trifluormethyl)phenyl]-3,6-dihydropyrimidin-1(2*H*)-yl]-acetamid (Beispiel 22) aus (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (Beispiel 6) gewonnen.
LC-MS (Methode 5): Rₜ = 1.44 min; MS (ESIpos): *m*/*z* (%) = 548.1 (100) [M+H]⁺; MS (ESIneg): *m*/*z* (%) = 546.1 (100) [M-H]⁻
¹H-NMR (400 MHz, DMSO-d₆): δ = 2.70 (s, 3H), 3.30 (d, 1H), 3.90 (m, 2H), 3.95 (d, 1H), 6.55 (s, 1H), 6.95 (s, 1H), 7.30 (s, 1H), 7.75-7.85 (m, 3H), 8.00 (s, 1H), 8.20-8.30 (m, 2H), 8.35 (s, 1H) [eine Methylgruppe durch Lösungsmittel-Peak verborgen].

### Beispiel 38

(*rac*)-4-[4-Cyano-2-(methylsulfonyl)phenyl]-N-(2-hydroxyethyl)-6-methyl-2,5-dioxo-1-[3-(trifluormethyl)phenyl]-1,2,4,5,6,7-hexahydro-3*H*-pyrrolo[3,4-d]pyrimidin-3-carboxamid

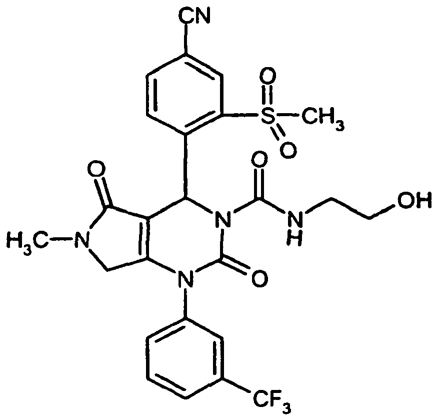

Die Titelverbindung wurde ausgehend von (*rac*)-4-{6-Methyl-2,5-dioxo-1-[3-(trifluormethyl)-phenyl]-2,3,4,5,6,7-hexahydro-1*H*-pyrrolo[3,4-d]pyrimidin-4-yl}-3-(methylsulfonyl)benzonitril (Beispiel 34) analog zur mehrstufigen Darstellung von (6*S*)-5-Cyano-6-[4-cyano-2-(methylsulfonyl)phenyl]-*N*-(2-hydroxyethyl)-4-methyl-2-oxo-3-[3-(trifluormethyl)phenyl]-3,6-dihydropyrimidin-1(2*H*)-carboxamid (Beispiel 10) aus (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (Beispiel 6) gewonnen. LC-MS (Methode 5): Rₜ = 1.56 min; MS (ESIpos): *m*/*z* (%) = 578.1 (100) [M+H]⁺; MS (ESIneg): *m*/*z* (%) = 489.2 (100), 576 (70) [M-H]⁻.

### Beispiel 39

(*rac*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure-allylester

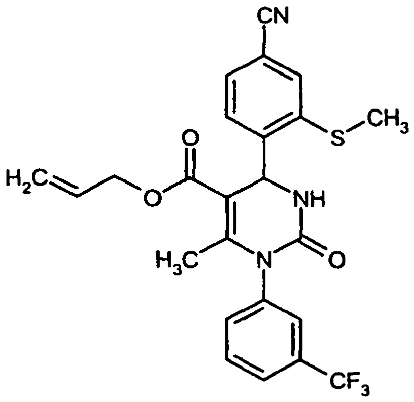

Die Reaktion wurde unter Argon durchgeführt. Phosphorsäuretriethylester (1.46 g, 8.04 mmol) und Diphosphorpentoxid (761 mg, 5.36 mmol) wurden über Nacht bei 50°C gerührt. Dann wurde mit MTBE (27 ml) verdünnt, und 4-Formyl-3-(methylsulfanyl)benzonitril (1.18 g, 6.70 mmol; Beispiel 9A), 1-[3-(Trifluormethyl)phenyl]harnstoff (1.37 g, 6.70 mmol) sowie Allylacetoacetat (1.43 g, 10.1 mmol) wurden hinzugefügt. Das Gemisch wurde über Nacht unter Rückfluss gerührt. Zur Aufarbeitung wurde das Lösungsmittel im Vakuum entfernt und der Rückstand mit Diethylether aufgeschlämmt und dann abgesaugt. Man erhielt 978 mg (19% d. Th.) der Titelverbindung.

LC-MS (Methode 4): Rₜ = 1.37 min; MS (ESIpos): *m*/*z* (%) = 488.3 (100) [M+H]⁺; MS (ESIneg): *m*/*z* (%) = 486.2 (65) [M-H]-.

### Beispiel 40

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carboxamid

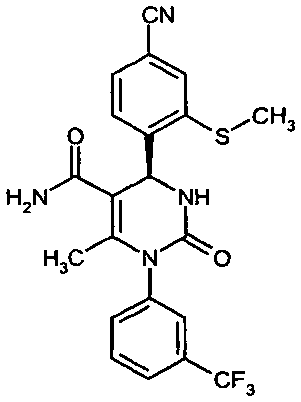

Die Reaktion wurde unter Argon durchgeführt. (4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonsäure (240 mg, 0.536 mmol; Beispiel 11A) wurde in THF (5 ml) gelöst und mit PyBOP (419 mg, 0.805 mmol) und Triethylamin (380 mg, 3.76 mmol) versetzt. Nach kurzem Rühren wurde auf 0°C abgekühlt und mit Ammoniumchlorid (143 mg, 2.68 mmol) versetzt. Die Reaktionsmischung wurde über Nacht bei RT gerührt und der Kolbeninhalt dann in 1 N Salzsäure gegeben. Es wurde zweimal mit Essigsäureethylester extrahiert, und die vereinigten organischen Phasen wurden mit 1 N Salzsäure und mit gesättigter Kochsalz-Lösung gewaschen, getrocknet und eingeengt. Der Rückstand wurde durch präparative HPLC gereinigt. Man erhielt 161 mg (67% d. Th.) der Titelverbindung.

LC-MS (Methode 4): Rₜ = 0.99 min; MS (ESIpos): *m*/*z* (%) = 447.1 (100) [M+H]⁺; MS (ESIneg): *m*/*z* (%) = 445.3 (100) [M-H]⁻.

### Beispiel 41

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril

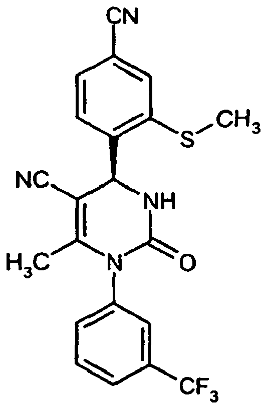

Die Reaktion wurde unter Argon durchgeführt. (4*S*)-4-[4-Cyano-2-(methylsulfanyl)phenyl]-6-methyl-2-oxo-1-[3-(trifluormethyl)phenyl]-1,2,3,4-tetrahydropyrimidin-5-carboxamid (95.0 mg, 0.213 mmol; Beispiel 40) wurde in THF (4 ml) gelöst und mit Methoxycarbonylsulfamoyl-triethylammoniumhydroxid (Burgess-Reagens; 101 mg, 0.426 mmol) versetzt. Nach 30 min Rühren bei Raumtemperatur zeigte die HPLC-Kontrolle vollständigen Umsatz an. Es wurde mit Essigsäureethylester (4 ml) verdünnt und mit Wasser (1 ml) versetzt. Die Mischung wurde dann über eine Merck Extrelut^{®} NT3-Säule gegeben und das Filtrat durch präparative HPLC gereinigt. Nach dem Einengen der Produktfraktionen erhielt man 96.0 mg (quantitativ) der Titelverbindung.
HPLC (Methode 3): Rₜ = 4.61 min.
MS (DCI / NH₃): *m*/*z* = 429.1 [M+H]⁺, 446.1 [M+NH₄]⁺
¹H-NMR (400 MHz, DMSO-d₆): δ = 1.80 (s, 3H), 2.61 (s, 3H), 5.76 (s, 1H), 7.67-7.89 (m, 7H), 8.28 (s, 1H).

### Beispiel 42

(4*S*)-4-[4-Cyano-2-(methylsulfinyl)phenyl]-6-methyl-2-oxo-1-[3-(trifluormethyl)phenyl]-1,2,3,4-tetrahydropyrimidin-5-carbonitril

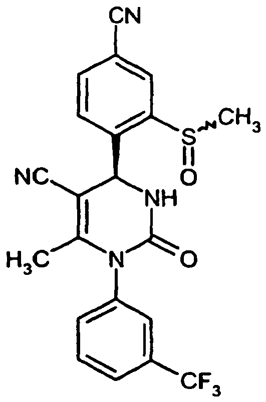

### Methode A:

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril (Beispiel 41; 55 mg, 0.13 mmol) wurde in Ethanol (5.5 ml) gelöst und mit Methyltrioxorhenium (3.20 mg, 0.013 mmol) sowie Wasserstoffperoxid (16.0 mg, 0.14 mmol) versetzt. Das Reaktionsgemisch wurde 60 min bei RT gerührt, dann im Vakuum eingeengt und der Rückstand mittels präparativer HPLC gereinigt. Es wurden 27 mg (47% d. Th.) der Zielverbindung als Diastereomerengemisch erhalten.

### Methode B:

(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril (3.00 g, 7.00 mmol; Beispiel 41) wurde in Methanol/Wasser (5:1, ~60 ml) vorgelegt, mit Natriumperiodat (2.85 g, 13.30 mmol; 1.9 eq.) versetzt und 16 h bei 30°C gerührt. Das Reaktionsgemisch wurde danach auf gesättigte wässrige Natriumhydrogencarbonat-Lösung (300 ml) gegeben und mit Essigsäureethylester (4 x 75 ml) extrahiert. Die vereinigten organischen Phasen wurden über Natriumsulfat getrocknet, filtriert und im Vakuum eingeengt. Das Rohprodukt wurde an Kieselgel flash-chromatographiert (Gradient Cyclohexan → Essigsäureethylester). Es wurde ein farbloser Feststoff erhalten (1.6 g, 51% d. Th.).
LC-MS (Methode 4): Rₜ = 1.05 min; MS (ESIpos): *mlz* (%) = 445.0 (100) [M+H]⁺.

### Beispiel 43

(4*S*)-4-{4-Cyano-2-[(*S*)-methylsulfinyl]phenyl}-6-methyl-2-oxo-1-[3-(trifluormethyl)phenyl]-1,2,3,4-tetrahydropyrimidin-5-carbonitril *und* (4*S*)-4-{4-Cyano-2-[(R)-methylsulfinyl]phenyl}-6-methyl-2-oxo-1-[3-(trifluormethyl)phenyl]-1,2,3,4-tetrahydropyrimidin-5-carbonitril (*Diastereomerentrennung*) 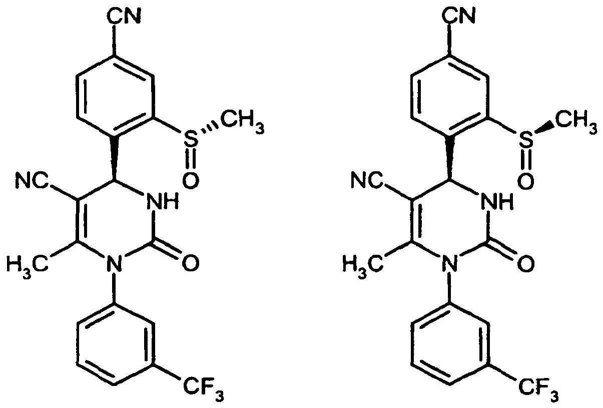 (4*S*)-4-[4-Cyano-2-(methylsulfinyl)phenyl]-6-methyl-2-oxo-1-[3-(trifluormethyl)phenyl]-1,2,3,4-tetrahydropyrimidin-5-carbonitril (Beispiel 42; 27 mg) wurde durch präparative HPLC-Chromatographie an chiraler Phase in die enantiomerenreinen Diastereomere aufgetrennt [Säule: Daicel Chiralpak IC, 5 µm, 250 mm x 20 mm; Probenvorbereitung: Probe wurde in 2 ml Methanol + 4 ml MTBE gelöst; Injektionsvolumen: 600 µl; Eluent: MTBE/Methanol 80:20; Fluss: 15 ml/min; Temperatur: 30°C; Detektion: 220 nm]. Der Enantiomerenüberschuss (ee-Wert) wurde chromatographisch bestimmt [Säule: Daicel Chiralpak IC, 5 µm, 250 mm x 4.6 mm; Eluent: MTBE/Methanol 75:25; Fluss: 1 ml/min; Temperatur: 25°C; Detektion: 230 nm].

### Diastereomer 1:

Ausbeute: 20 mg
Rₜ = 4.71 min, ee >99.0%
LC-MS (Methode 6): Rₜ = 2.02 min; MS (ESIpos): m/z (%) = 445.0 (100) [M+H]⁺; MS (ESIneg): m/z (%) = 442.9 (80) [M-H]⁻
¹H-NMR (400 MHz, CDCl₃): δ = 1.94 (s, 3H), 2.84 (s, 3H), 5.72 (s, 1H), 7.17 (s, 1H), 7.42-7.56 (m, 2H), 7.65 (t, 1H), 7.70-7.79 (m, 2H), 7.90 (d, 1H), 8.01 (s, 1H).

### Diastereomer 2:

Ausbeute: 7 mg
Rₜ = 6.04 min, ee >99.0%
LC-MS (Methode 6): Rₜ = 2.02 min; MS (ESIpos): *m*/*z* (%) = 445.0 (100) [M+H]⁺; MS (ESIneg): *m*/*z* (%) = 442.9 (100) [M-H]⁻
¹H-NMR (400 MHz, CDCl₃): δ = 1.97 (s, 3H), 2.77 (s, 3H), 5.97 (br. s, 1H), 7.06 (br. s, 1H), 7.31-7.50 (m, 2H), 7.63 (t, 1H), 7.68-7.78 (m, 2H), 7.89 (d, 1H), 8.11 (s, 1H).

### Beispiel 44

(*rac*)-4-{2,5-Dioxo-1-[3-(trifluormethyl)phenyl]-2,3,4,5,6,7-hexahydro-1H-pyrrolo[3,4-d]pyrimidin-4-yl}-3-(methylsulfonyl)benzonitril 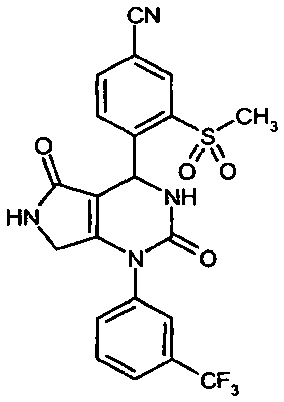 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (4 ml) gelöst, mit einer 7 N Lösung von Ammoniak in Methanol (5 ml) versetzt und 60 min bei RT gerührt. Das Reaktionsgemisch wurde danach im Vakuum eingeengt und der Rückstand in Acetonitril (3 ml) verrührt. Die entstandenen Kristalle wurden abgesaugt, mit Acetonitril und Wasser gewaschen und zur weiteren Aufreinigung mittels präparativer HPLC getrennt [Säule: Sunfire C-18, 5 µm, 19 mm x 150 mm; Probenvorbereitung: Probe wurde in 3 ml Acetonitril + 3 ml 1 %-ige wässrige TFA-Lösung + 2 ml THF gelöst; Injektionsvolumen: 1000 µl; Eluent: Acetonitril/Wasser/1% aq. TFA 25:60:15 (0-12 min); Fluss: 25 ml/min; Temperatur: 40°C; Detektion: 210 nm]. Es wurden 59 mg (36% d. Th.) der Zielverbindung erhalten.
HPLC (Methode 2): Rₜ = 3.89 min.
MS (ESIpos): *m*/*z* (%) = 476.9 (100) [M+H]⁺
¹H-NMR (400 MHz, DMSO-d₆): δ = 3.53 (s, 3H), 3.75 (dd, 2H), 6.61 (s, 1H), 7.70-7.86 (m, 4H), 8.01 (s, 1H), 8.08 (s, 1H), 8.16 (d, 1H), 8.27 (d, 1H), 8.34 (s, 1H).

### Beispiel 45

(*rac*)-4-{6-(2-Hydroxyethyl)-2,5-dioxo-1-[3-(trifluormethyl)phenyl]-2,3,4,5,6,7-hexahydro-1H-pyrrolo[3,4-d]pyrimidin-4-yl}-3-(methylsulfonyl)benzonitril 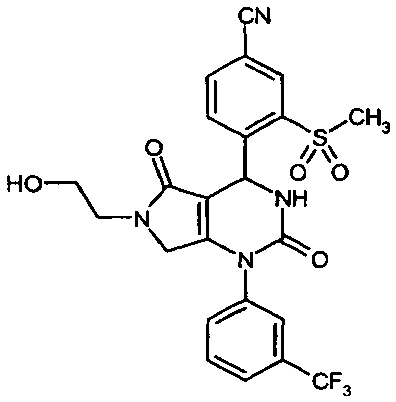 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (5 ml) gelöst, mit 2-Aminoethanol (62.5 mg, 1.02 mmol) versetzt und über Nacht bei RT gerührt. Das Reaktionsgemisch wurde danach direkt mittels präparativer HPLC gereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Es wurden 98 mg (54% d. Th.) der Titelverbindung erhalten.
HPLC (Methode 2): Rₜ = 3.79 min.
MS (ESIpos): *mlz* (%) = 521.1 (100) [M+H]⁺
¹H-NMR (400 MHz, DMSO-d₆): δ = 3.14-3.42 (m, 4H), 3.54 (s, 3H), 3.93 (d, 2H), 4.63 (t, 1H), 6.65 (s, 1H), 7.76 (t, 1H), 7.80-7.87 (m, 2H), 8.02 (s, 1H), 8.11 (s, 1H), 8.18 (d, 1H), 8.26 (d, 1H), 8.34 (s, 1H).

### Beispiel 46

(*rac*)-4-{6-Cyclopropyl-2,5-dioxo-1-[3-(trifluormethyl)phenyl]-2,3,4,5,6,7-hexahydro-1H-pyrrolo-[3,4-d]pyrimidin-4-yl}-3-(methylsulfonyl)benzonitril 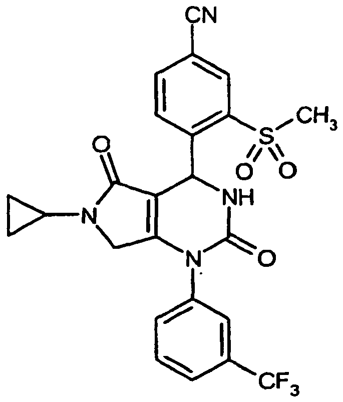 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (5 ml) gelöst, mit Cyclopropylamin (58.4 mg, 1.02 mmol) versetzt und über Nacht bei RT gerührt. Das Reaktionsgemisch wurde danach direkt mittels präparativer HPLC aufgereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Die produkthaltigen Fraktionen wurden vereinigt und einer erneuten HPLC-Trennung unterzogen [Säule: Sunfire C-18, 5 µm, 19 mm x 150 mm; Probenvorbereitung: Probe wurde in 2 ml Acetonitril + 2 ml 1%-ige wässrige TFA-Lösung + 1 ml THF gelöst; Injektionsvolumen: 1000 µl; Eluent: Acetonitril/Wasser/1% aq. TFA 35:52:13 (0-10 min); Fluss: 25 ml/min; Temperatur: 40°C; Detektion: 210 nm]. Es wurden so 38 mg (22% d. Th.) der Titelverbindung erhalten.
HPLC (Methode 2): Rₜ = 4.24 min.
MS (ESIpos): *m*/*z* (%) = 516.9 (100) [M+H]⁺
¹H-NMR (400 MHz, DMSO-d₆): δ = 0.48-0.60 (m, 4H), 2.43-2.48 (m, 1H), 3.54 (s, 3H), 3.81 (s, 2H), 6.62 (s, 1H), 7.74 (t, 1H), 7.80-7.85 (m, 2H), 7.98 (s, 1H), 8.10-8.17 (m, 2H), 8.26 (d, 1H), 8.34 (s, 1H),

### Beispiel 47

(*rac*)-4-{6-Ethyl-2,5-dioxo-1-[3-(trifluormethyl)phenyl]-2,3,4,5,6,7-hexahydro-1*H*-pyrrolo[3,4-d]-pyrimidin-4-yl}-3-(methylsulfonyl)benzonitril 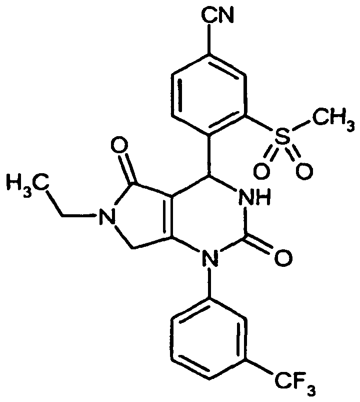 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (5 ml) gelöst, mit Ethylamin (46.1 mg, 1.02 mmol) versetzt und über Nacht bei RT gerührt. Das Reaktionsgemisch wurde danach direkt mittels präparativer HPLC gereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Es wurden 120 mg (70% d. Th.) der Titelverbindung erhalten.
HPLC (Methode 2): Rₜ = 4.20 min.
MS (ESIpos): *m*/*z* (%) = 505.0 (100) [M+H]⁺
¹H-NMR (400 MHz, DMSO-d₆): δ = 0.94 (t, 3H), 3.11-3.26 (m, 2H), 3.54 (s, 3H), 3.86 (s, 2H), 6.64 (s, 1H), 7.75 (t, 1H), 7.80-7.88 (m, 2H), 8.02 (s, 1H), 8.10 (s, 1H), 8.17 (d, 1H), 8.26 (d, 1H), 8.34 (s, 1H).

### Beispiel 48

4-{(4*S*)-6-Ethyl-2,5-dioxo-1-[3-(trifluormethyl)phenyl]-2,3,4,5,6,7-hexahydro-1*H*-pyrrolo[3,4-d]-pyrimidin-4-yl}-3-(methylsulfonyl)benzonitril 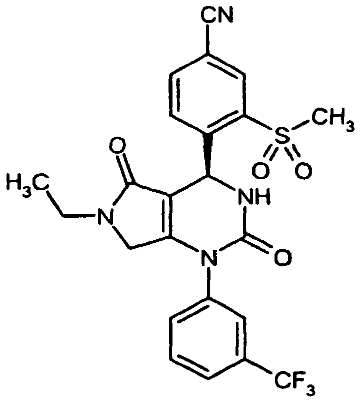 (*rac*)-4-{6-Ethyl-2,5-dioxo-1-[3-(trifluormethyl)phenyl]-2,3,4,5,6,7-hexahydro-1*H*-pyrrolo[3,4-d]-pyrimidin-4-yl}-3-(methylsulfonyl)benzonitril (Beispiel 47; 120 mg) wurde durch präparative HPLC-Chromatographie an chiraler Phase in die Enantiomere aufgetrennt [Säule: Daicel Chiralpak IC, 5 µm, 250 mm x 20 mm; Probenvorbereitung: Probe wurde in 1 ml Methanol + 1 ml Acetonitril + 3 ml MTBE gelöst; Injektionsvolumen: 700 µl; Eluent: MTBE/Methanol 80:20; Fluss: 15 ml/min; Temperatur: 30°C; Detektion: 220 nm]. Auf diese Weise wurden 42 mg (84% d. Th., >99.0% ee) des 4S-Enantiomeren erhalten. Der Enantiomerenüberschuss (ee-Wert) wurde chromatographisch bestimmt [Säule: Daicel Chiralpak IC, 5 µm, 250 mm x 4.6 mm; Eluent: MTBE/Methanol 80:20; Fluss: 1 ml/min; Temperatur: 25°C; Detektion: 230 nm; Rₜ = 6.83 min].
HPLC (Methode 2): Rₜ = 4.20 min.
MS (DCI / NH₃): *m*/*z* (%) = 505.1 [M+H]⁺
¹H-NMR (400 MHz, DMSO-d₆): δ = 0.94 (t, 3H), 3.10-3.27 (m, 2H), 3.54 (s, 3H), 3.86 (s, 2H), 6.64 (s, 1H), 7.75 (t, 1H), 7.80-7.88 (m, 2H), 8.02 (s, 1H), 8.10 (s, 1H), 8.17 (d, 1H), 8.26 (d, 1H), 8.34 (s, 1H).
Drehwert: [α]²⁰_{Na} = -107° (c = 0.230 in Aceton).

### Beispiel 49

(*rac*)*-N-*(2-{4-[4-Cyano-2-(methylsulfonyl)phenyl]-2,5-dioxo-1-[3-(trifluormethyl)phenyl]-1,2,3,4,5,7-hexahydro-6*H*-pyrrolo[3,4-d]pyrimidin-6-yl) ethyl)acetamid 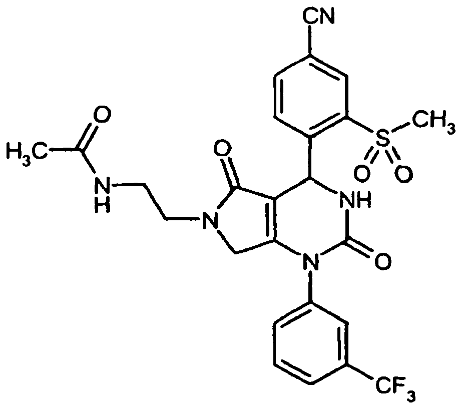 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (5 ml) gelöst, mit *N*-(2-Aminoethyl)acetamid (105 mg, 1.02 mmol) versetzt und über Nacht bei RT gerührt. Das Reaktionsgemisch wurde danach direkt mittels präparativer HPLC gereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Es wurden 107 mg (56% d. Th.) der Titelverbindung erhalten.
HPLC (Methode 2): Rₜ = 3.78 min.
MS (DCI/NH₃): *m*/*z* (%) = 562.1 (25) [M+H]⁺
¹H-NMR (400 MHz, DMSO-d₆): δ = 1.66 (s, 3H), 2.97-3.24 (m, 4H), 3.54 (s, 3H), 3.91 (s, 2H), 6.64 (s, 1H), 7.73-7.86 (m, 4H), 7.98 (s, 1H), 8.11-8.17 (m, 2H), 8.27 (d, 1H), 8.34 (s, 1H).

### Beispiel 50

(*rac*)-1-(2-{4-[4-Cyano-2-(methylsulfonyl)phenyl]-2,5-dioxo-1-[3-(trifluormethyl)phenyl]-1,2,3,4,5,7-hexahydro-6*H*-pyrrolo[3,4-d]pyrimidin-6-yl}ethyl)harnstoff 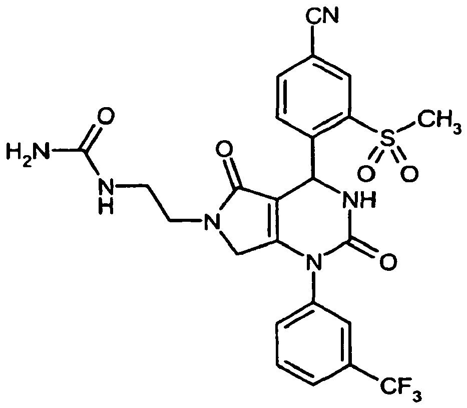 (*rac*)-Ethyl 6-(brommethyl)-4-[4-cyano-2-(methylsulfonyl)phenyl]-2-oxo-1-[3-(trifluormethyl)-phenyl]-1,2,3,4-tetrahydropyrimidin-5-carboxylat (200 mg, 0.34 mmol; Beispiel 13A) wurde in Acetonitril (5 ml) gelöst, mit 1-(2-Aminoethyl)harnstoff (106 mg, 1.02 mmol) versetzt und über Nacht bei RT gerührt. Das Reaktionsgemisch wurde danach direkt mittels präparativer HPLC gereinigt (Säule: Kromasil C18, 125 mm x 20 mm, 5 µm, 100 Å; Eluent A: Wasser mit 0.01% Ameisensäure, Eluent B: Acetonitril; Gradient: 0 min 10% B → 2 min 10% B → 9 min 90% B → 12 min 90% B → 12.1 min 10% B → 15 min 10% B; Fluss: 0.35 ml/min; UV-Detektion: 254 nm). Es wurden 69 mg (34% d. Th.) der Titelverbindung erhalten.
HPLC (Methode 2): Rₜ = 3.69 min.
MS (DCI/NH₃): *m*/*z* (%) = 563.1 (100) [M+H]⁺
¹H-NMR (400 MHz, DMSO-d₆): δ = 2.90-3.24 (m, 4H), 3.54 (s, 3H), 3.94 (m, 2H), 5.42 (s, 2H), 5.85 (t, 1H), 6.63 (s, 1H), 7.73-7.86 (m, 3H), 7.98 (s, 1H), 8.10-8.14 (m, 2H), 8.27 (d, 1H), 8.34 (s, 1H).

### Beispiel 51

(4*S*)-4-[4-Cyano-2-(methylsulfonyl)phenyl]-3-[(3-hydroxyazetidin-1-yl)carbonyl]-6-methyl-2-oxo-1-[3-(trifluormethyl)phenyl]- 1,2,3,4-tetrahydropyrimidin-5-carbonitril 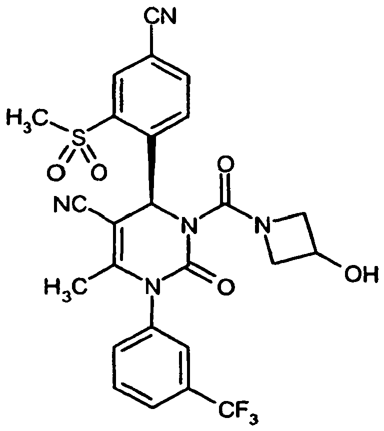 Nach der Allgemeinen Vorschrift 1 wurde 4-Nitrophenyl-(6S)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (80.0 mg, 0.128 mmol; Beispiel 6A) mit Azetidin-3-ol-Hydrochlorid (42.0 mg, 0.384 mmol) in Acetonitril (1 ml) zur Ziel verbindung (40 mg, 56% d. Th.) umgesetzt.
HPLC (Methode 2): Rₜ = 4.20 min.
MS (DCI / NH₃): *m*/*z* (%) = 560 [M+H]⁺, 577.2 [M+NH₄]⁺
¹H-NMR (400 MHz, DMSO-d₆): δ = 1.81 (s, 3H), 3.38-3.61 (m, 4H), 3.79-4.08 (m, 2H), 4.22-4.45 (m, 2H), 5.74 (t, 1H), 6.83 (s, 1H), 7.74-7.90 (m, 3H), 8.00 (s, 1H), 8.12 (br. s, 1H), 8.36-8.43 (m, 2H).

### Beispiel 52

(4*S*)-4-[4-Cyano-2-(methylsulfonyl)phenyl]-3-[(3-hydroxypyrrolidin-1-yl)carbonyl)-6-methyl-2-oxo-1-[3-(trifluormethyl)phenyl]-1,2,3,4-tetrahydropyrimidin-5-carbonitril 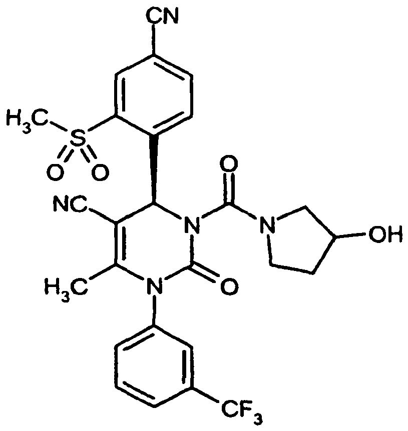 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (78.0 mg, 0.125 mmol; Beispiel 6A) mit Pyrrolidin-3-ol (32.6 mg, 0.374 mmol) in Acetonitril (1 ml) zur Zielverbindung (58 mg, 81% d. Th.) umgesetzt.
HPLC (Methode 2): Rₜ = 4.31 min.
MS (ESIpos): *m*/*z* (%) = 574.3 (100) [M+H]⁺
¹H-NMR (400 MHz, DMSO-d₆): δ = 1.61-1.93 (m, 5H), 2.87-3.26 (m, 2H), 3.42-3.84 (m, 5H), 4.13-4.29 (m, 1H), 4.83-5.10 (m, 1H), 6.84 (s, 1H), 7.73-7.90 (m, 3H), 7.98-8.42 (m, 4H).

### Beispiel 53

(4*S*)-4-[4-Cyano-2-(methylsulfonyl)phenyl]-3-[(3*R*)-3-methoxypiperidin-1-yl]carbonyl-6-methyl-2-oxo-1-[3-(trifluormethyl)phenyl]-1,2,3,4-tetrahydropyrimidin-5-carbonitril 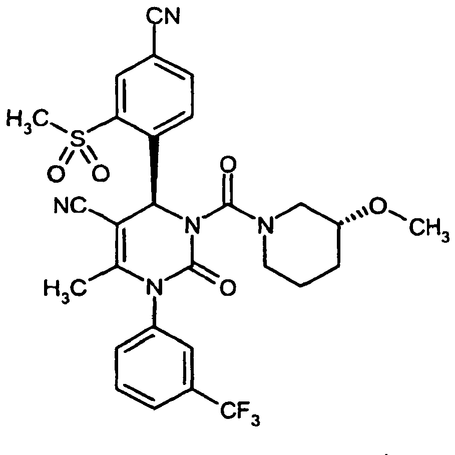 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (80.0 mg, 0.128 mmol; Beispiel 6A) mit (3*R*)-3-Methoxypiperidin (44.2 mg, 0.384 mmol) in Acetonitril (1 ml) zur Zielverbindung (45 mg, 57% d. Th.) umgesetzt.
HPLC (Methode 2): Rₜ = 4.70 min.
MS (ESIpos): *m*/*z* (%) = 602.0 (100) [M+H]⁺
¹H-NMR (400 MHz, DMSO-d₆): δ = 1.15-1.94 (m, 7H), 2.63-4.24 (m, 11H), 6.76-6.88 (m, 1H), 7.38-8.55 (m, 7H).

### Beispiel 54

(4*S*)-4-[4-Cyano-2-(methylsulfonyl)phenyl]-3-[(3-hydroxypiperidin-1-yl)carbonyl]-6-methyl-2-oxo-1-[3-(trifluormethyl)phenyl]-1,2,3,4-tetrahydropyrimidin-5-carbonitril 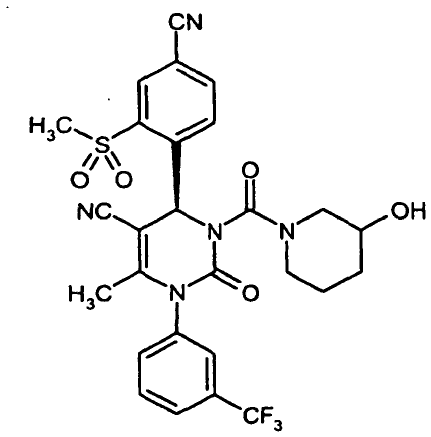 Nach der Allgemeinen Vorschrift 1 wurde 4-Nitrophenyl-(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (80.0 mg, 0.128 mmol; Beispiel 6A) mit Piperidin-3-ol (38.8 mg, 0.384 mmol) in Acetonitril (1 ml) zur Zielverbindung (58 mg, 77% d. Th.) umgesetzt.
HPLC (Methode 2): Rₜ = 4.31 min.
MS (DCI / NH₃): *mlz* (%) = 588 [M+H]⁺, 605.2 [M+NH₄]⁺
¹H-NMR (400 MHz, DMSO-d₆): δ = 0.78-1.92 (m, 7H), 2.24-5.00 (m, 9H), 6.75-6.87 (m, 1H), 7.72-8.07 (m, 4H), 8.18-8.52 (m, 3H).

### Beispiel 55

(4*S*)-3-[(*3R*)*-*3-Aminopiperidin-1-yl]carbonyl-4-[4-cyano-2-(methylsulfonyl)phenyl]-6-methyl-2-oxo-1-[3-(trifluormethyl)phenyl]-1,2,3,4-tetrahydropyrimidin-5-carbonitril 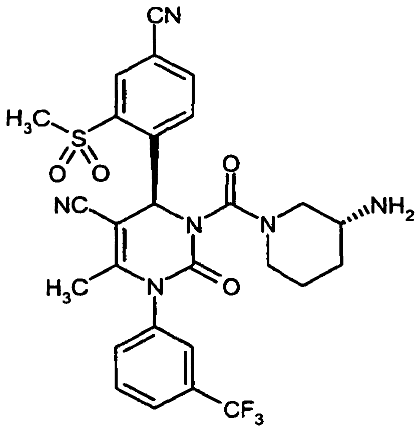 *tert*.-Butyl-[(3*R*)-1-{[(6*S*)-5-cyano-6-[4-cyano-2-(methylsulfonyl)phenyl]-4-methyl-2-oxo-3-[3-(trifluormethyl)phenyl]-3,6-dihydropyrimidin-1(2*H*)-yl]carbonyl)piperidin-3-yl]carbamat (Beispiel 12A; 56.0 mg, 0.082 mmol) wurde in einer 4 N Lösung von Chlorwasserstoff in Dioxan (2.15 ml) gelöst und 60 min bei RT gerührt. Danach wurde der Kolbeninhalt im Vakuum eingeengt und der Rückstand in Dichlormethan (15 ml) und 2 N Natronlauge (15 ml) aufgenommen. Nach Ausschütteln wurde die organische Phase abgetrennt, über Natriumsulfat getrocknet und im Vakuum eingeengt. Es wurden 47 mg (96% d. Th.) der Titelverbindung erhalten.
HPLC (Methode 2): Rₜ = 4.17 min.
MS (ESIpos): *m*/*z* (%) = 587.0 (100) [M+H]⁺
¹H-NMR (400 MHz, DMSO-d₆): δ = 1.40-1.84 (m, 7H), 2.03-2.35 (m, 2H), 2.74-2.90 (m, 1*H*), 3.52 (s, 3H), 3.74-4.05 (m, 1H), 6.67 (br. s, 1H), 6.80 (s, 1H), 7.27 (br. s, 1H), 7.73-8.06 (m, 4H), 8.21 (d, 1H), 8.37 (s, 1H), 8.48 (d, 1H).

### Allgemeine Vorschrift 2: Synthese von N-Aminocarbonyl-dihydropyrimidinon-Derivaten (Verfahren B)

0.1 mmol des entsprechenden Amins wurde in 0.2 ml Acetonitril gelöst und mit 25.8 mg (0.2 mmol) Diisopropylethylamin sowie 62.5 mg (0.1 mmol) 4-Nitrophenyl-(6S)-5-cyano-6-[4-cyano-2-(methylsulfonyl)phenyl]-4-methyl-2-oxo-3-[3-(trifluormethyl)phenyl]-3,6-dihydropyrimidin-1(2*H*)-carboxylat (Beispiel 6A), gelöst in 0.6 ml Acetonitril, versetzt. Das Reaktionsgemisch wurde über Nacht bei Raumtemperatur gerührt und dann das Acetonitril in einer Vakuumzentrifuge abgedampft. Das Rohprodukt wurde in 0.5 ml DMSO gelöst und mittels präparativer HPLC/MS (Methode 7) gereinigt. Nach der Allgemeinen Vorschrift 2 wurden die in der nachfolgenden Tabelle 1 wiedergegebenen Ausführungsbeispiele hergestellt:

**Tabelle 1**

| **Beispiel Nr.** | **Struktur** | **LC/MS-Daten (Methode 7)** |
|---|---|---|
| **56** | 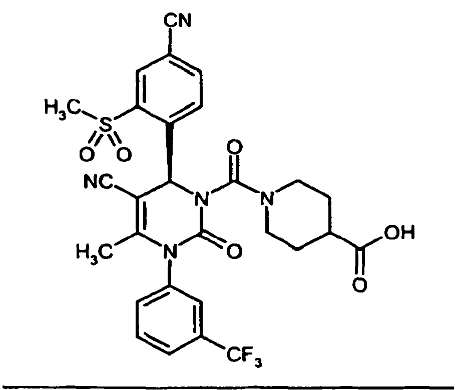 | MS (ESIpos): m/z = 616 (M+H)⁺; Rₜ = 2.01 min |
| **57** | 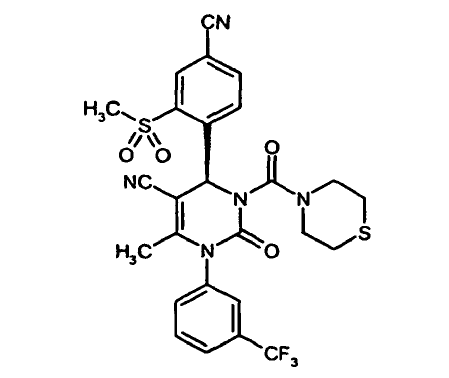 | MS (ESIpos): m/z = 590 (M+H)⁺; Rₜ = 2.21 min |
| **58** | 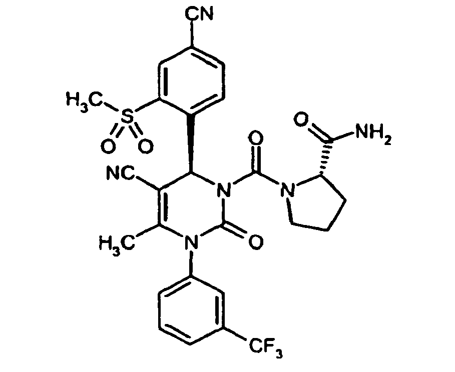 | MS (ESIpos): m/z = 601 (M+H)⁺; Rₜ = 1.97 min |
| **59** | 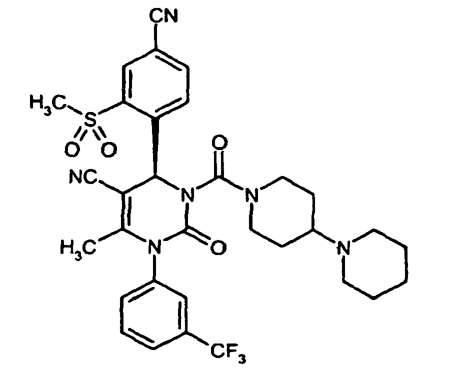 | MS (ESIpos): m/z = 655 (M+H)⁺; Rₜ = 1.57 min |
| **60** | 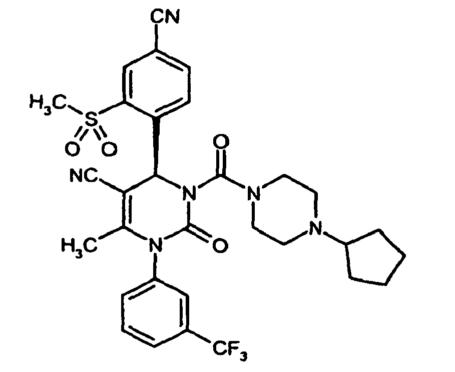 | MS (ESIpos): m/z = 641 (M+H)⁺; Rₜ = 1.61 min |
| **61** | 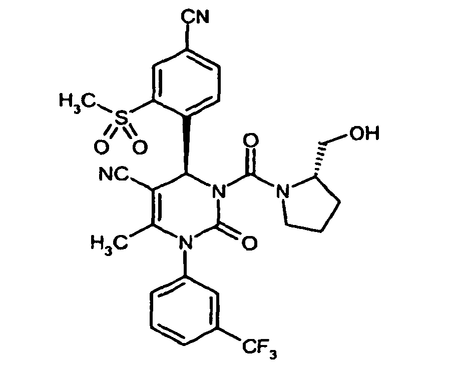 | MS (ESIpos): m/z = 588 (M+H)⁺; Rₜ = 2.04 min |
| **62** | 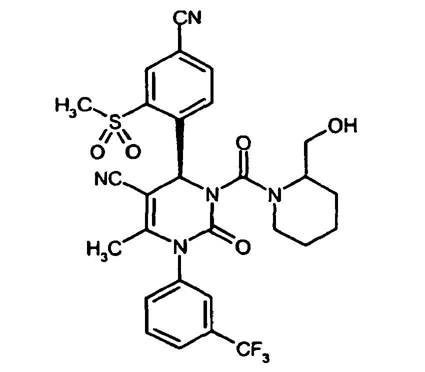 | MS (ESIpos): m/z = 602 (M+H)⁺; Rₜ = 2.09 min |
| **63** | 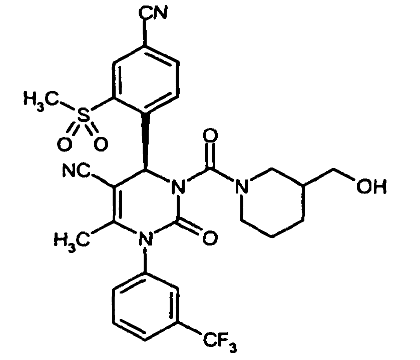 | MS (ESIpos): m/z = 602 (M+H)⁺; Rₜ = 2.03 min |
| **64** | 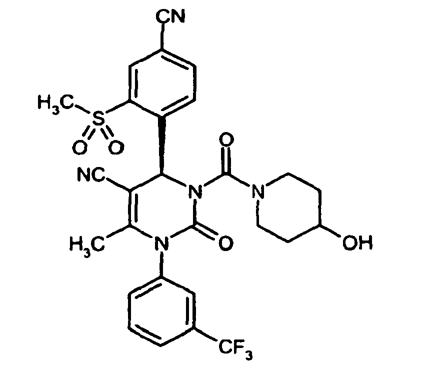 | MS (ESIpos): m/z = 588 (M+H)⁺; Rₜ = 1.96 min |
| **65** | 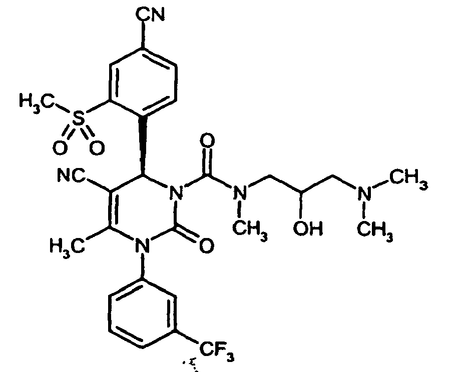 | MS (ESIpos): m/z = 619 (M+H)⁺; Rₜ = 1.51 min |
| **66** | 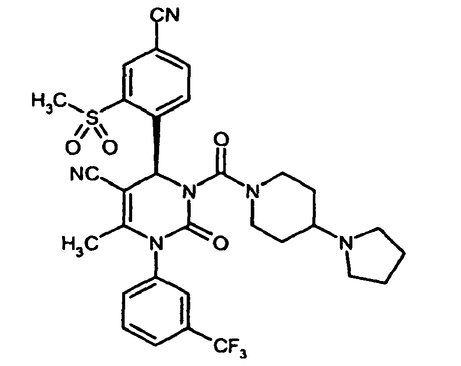 | MS (ESIpos): m/z = 641 (M+H)⁺; Rₜ = 1.54 min |
| **67** | 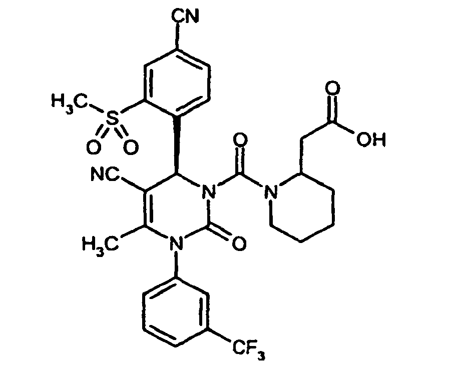 | MS (ESIpos): m/z = 630 (M+H)⁺; Rₜ = 2.07 min |
| **68** | 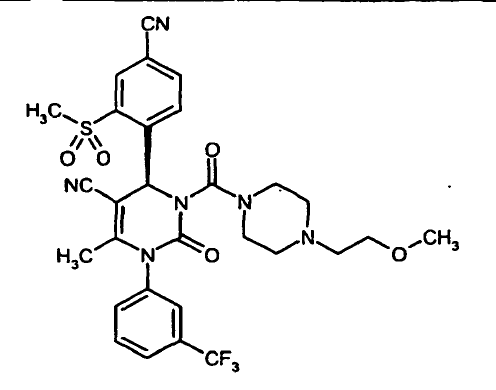 | MS (ESIpos): m/z = 631 (M+H)⁺; Rₜ = 1.55 min |
| **69** | 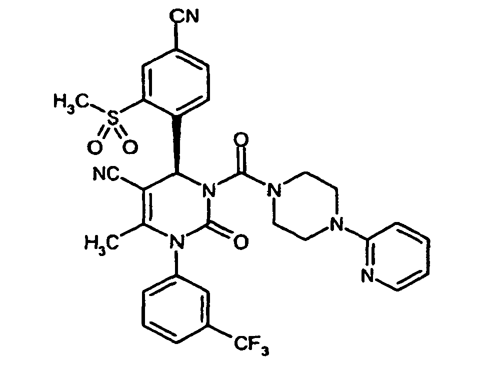 | MS (ESIpos): m/z = 650 (M+H)⁺; Rₜ = 1.74 min |
| **70** | 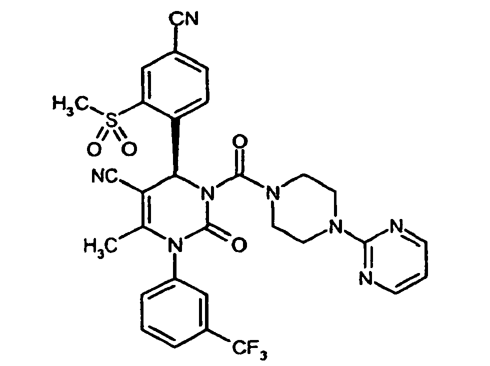 | MS (ESIpos): m/z = 651 (M+H)⁺; Rₜ = 2.18 min |
| **71** | 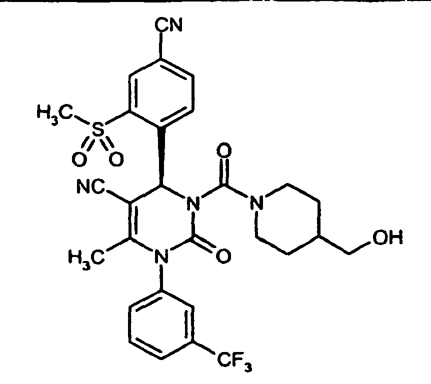 | MS (ESIpos): m/z = 602 (M+H)⁺; Rₜ = 1.97 min |
| **72** | 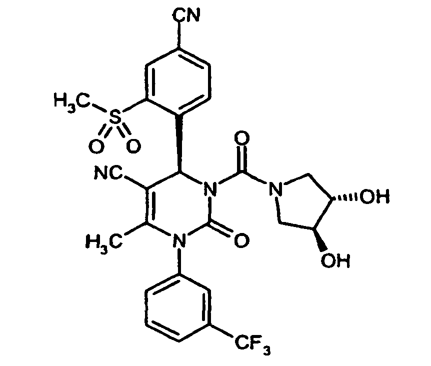 | MS (ESIpos): m/z = 590 (M+H)⁺; Rₜ = 1.84 min |
| **73** | 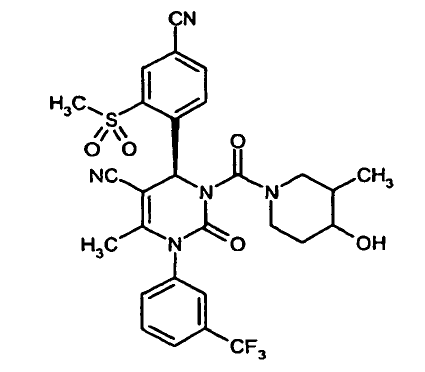 | MS (ESIpos): m/z = 602 (M+H)⁺; Rₜ = 2.02 min |
| **74** | 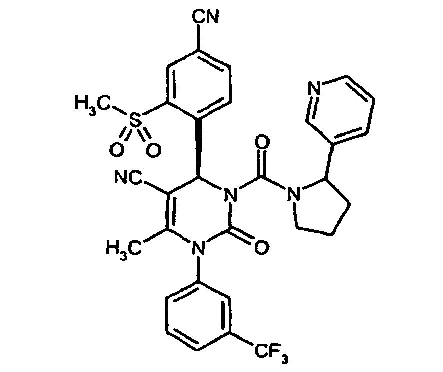 | MS (ESIpos): m/z = 635 (M+H)⁺; Rₜ = 1.80 min |
| **75** | 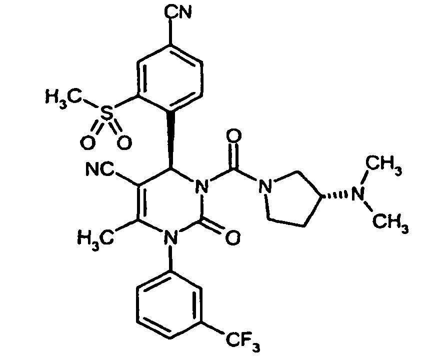 | MS (ESIpos): m/z = 601 (M+H)⁺; Rₜ = 1.53 min |
| **76** | 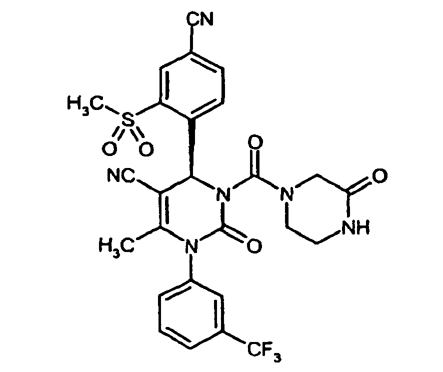 | MS (ESIpos): m/z = 587 (M+H)⁺; Rₜ = 1.89 min |
| **77** | 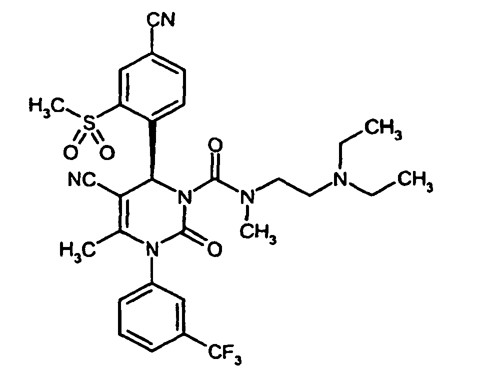 | MS (ESIpos): m/z = 617 (M+H)⁺; Rₜ = 1.56 min |
| **78** | 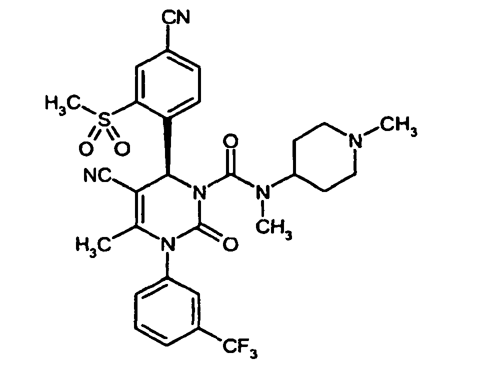 | MS (ESIpos): m/z = 615 (M+H)⁺; Rₜ = 1.54 min |
| **79** | 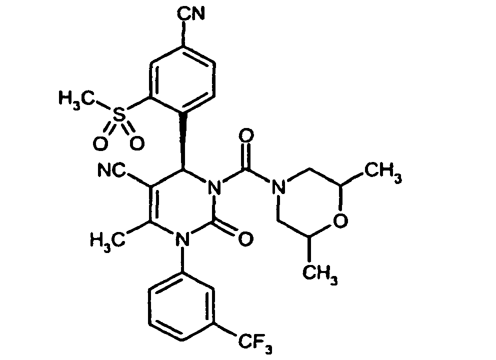 | MS (ESIpos): m/z = 602 (M+H)⁺; Rₜ = 2.17 min |
| **80** | 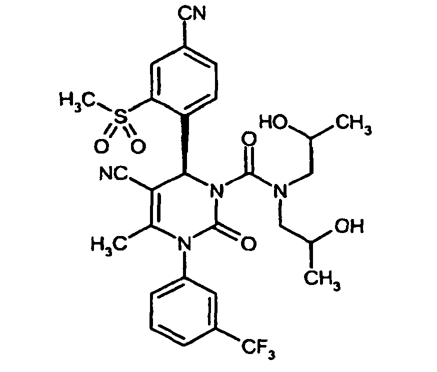 | MS (ESIpos): m/z = 620 (M+H)⁺; Rₜ = 2.00 min |
| **81** | 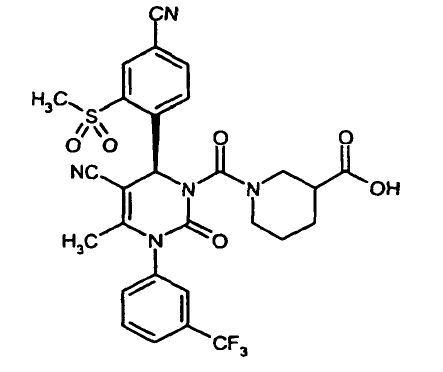 | MS (ESIpos): m/z = 616 (M+H)⁺; Rₜ = 2.03 min |
| **82** | 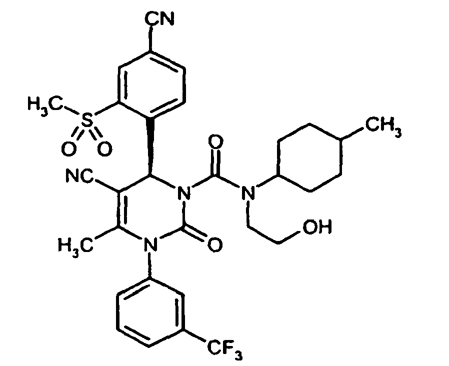 | MS (ESIpos): m/z = 644 (M+H)⁺; Rₜ = 2.25 min |
| **83** | 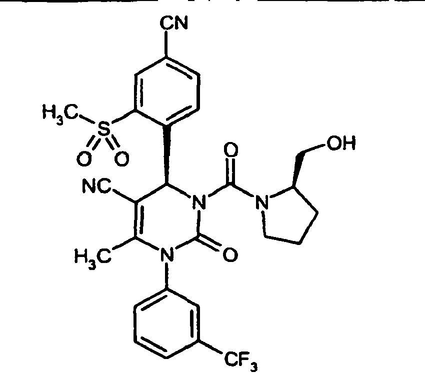 | MS (ESIpos): m/z = 588 (M+H)⁺; Rₜ = 2.03 min |
| **84** | 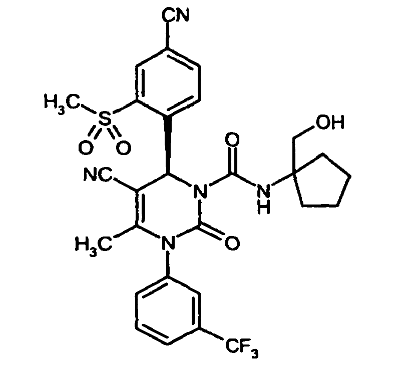 | MS (ESIpos): m/z = 602 (M+H)⁺; Rₜ = 2.19 min |
| **85** | 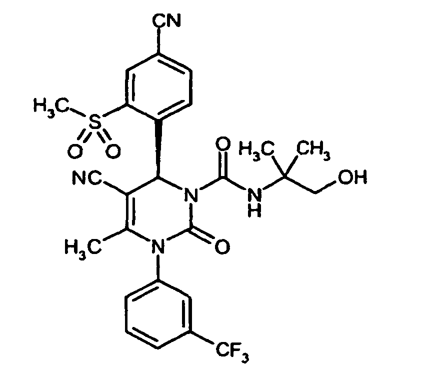 | MS (ESIpos): m/z = 576 (M+H)⁺; Rₜ = 2.12 min |
| **86** | 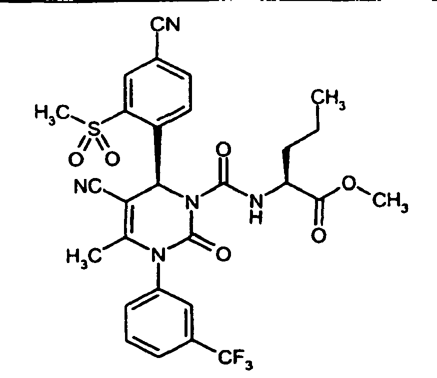 | MS (ESIpos): m/z = 618 (M+H)⁺; Rₜ = 2.31 min |
| **87** | 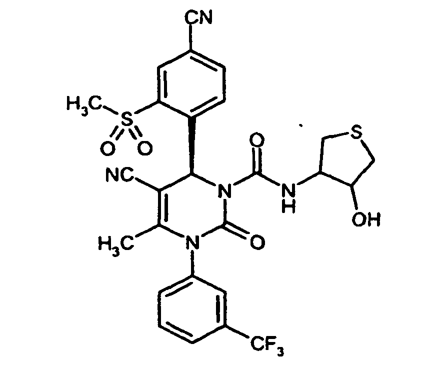 | MS (ESIpos): m/z = 606 (M+H)⁺; Rₜ = 2.13 min |
| **88** | 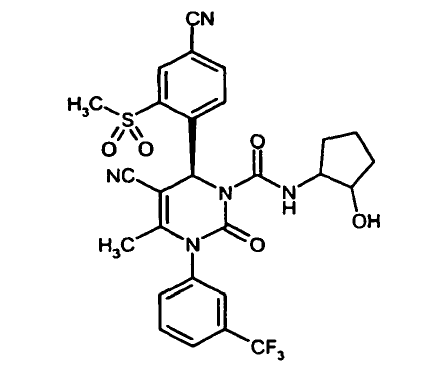 | MS (ESIpos): m/z = 588 (M+H)⁺; Rₜ = 2.14 min |
| **89** | 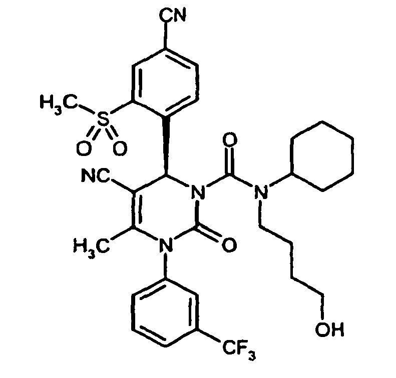 | MS (ESIpos): m/z = 658 (M+H)⁺; Rₜ = 2.21 min |

### Allgemeine Vorschrift 3: Synthese von N-Sulfonyl-dihydropyrimidinon-Derivaten

35.4 mg (0.077 mmol) (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (Beispiel 6) wurden unter Argon in 0.4 ml THF gelöst, bei 0°C mit 4.6 mg (0.192 mmol) Natriumhydrid versetzt und 20 min bei Raumtemperatur gerührt. Dann wurde eine Lösung von 0.0924 mmol des entsprechenden Sulfonsäurechlorids in 0.2 ml THF zugegeben und die Mischung weitere 30 min bei Raumtemperatur gerührt. Danach wurde mit 50 mg (0.93 mmol) Ammoniumchlorid versetzt und das THF in einer Vakuumzentrifuge abgedampft. Das Rohprodukt wurde in 0.5 ml DMSO aufgenommen, filtriert und das Filtrat mittels präparativer HPLC/MS (Methode 7) gereinigt.

Nach der Allgemeinen Vorschrift 3 wurden die in der nachfolgenden Tabelle 2 wiedergegebenen Ausführungsbeispiele hergestellt:

**Tabelle 2**

| **Beispiel Nr.** | **Struktur** | **LC/MS Daten (Methode 7)** |
|---|---|---|
| **90** | 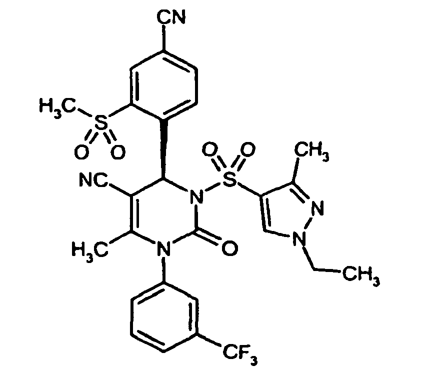 | MS (ESIpos): m/z = 632 (M+H)⁺; Rₜ = 2.17 min |
| **91** | 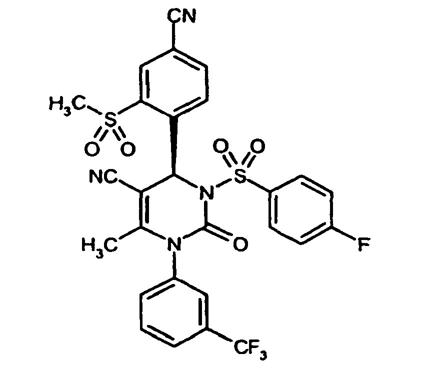 | MS (ESIpos): m/z = 619 (M+H)⁺; Rₜ = 2.26 min |
| **92** | 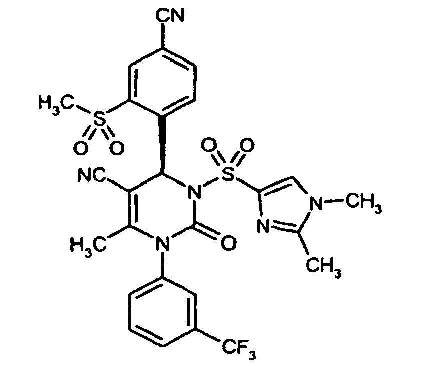 | MS (ESIpos): m/z = 619 (M+H)⁺; Rₜ = 1.88 min |
| **93** | 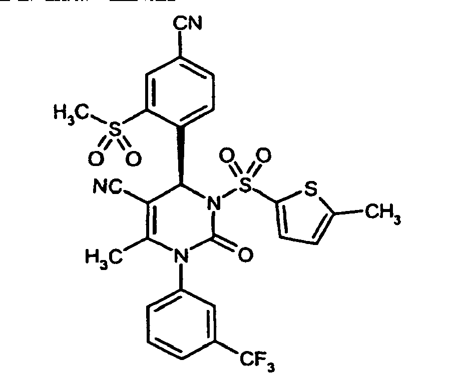 | MS (ESIpos): m/z = 621 (M+H)⁺; Rₜ = 2.24 min |
| **94** | 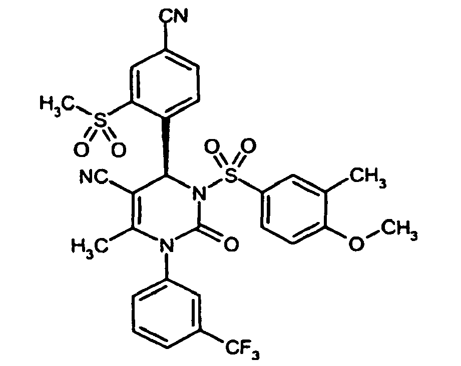 | MS (ESIpos): m/z = 645 (M+H)⁺; Rₜ = 2.28 min |
| **95** | 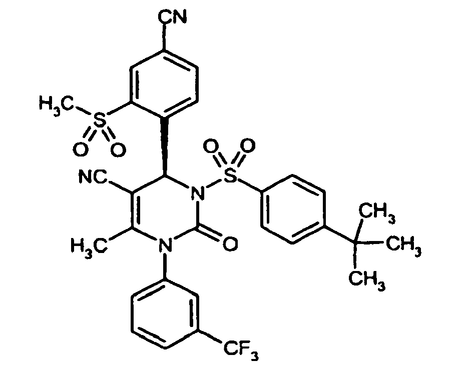 | MS (ESIpos): m/z = 657 (M+H)⁺; Rₜ = 2.37 min |
| **96** | 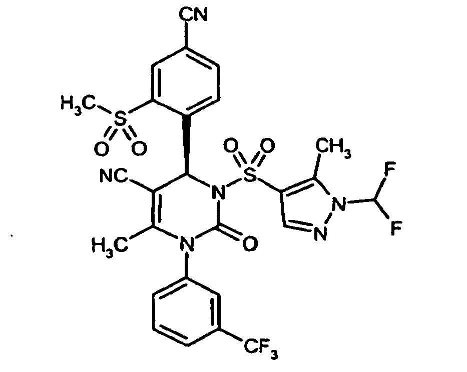 | MS (ESIpos): m/z = 655 (M+H)⁺; Rₜ = 2.21 min |
| **97** | 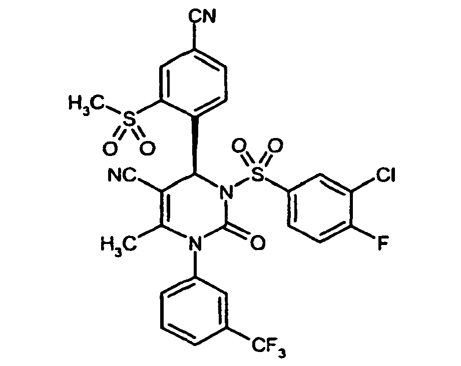 | MS (ESIpos): m/z = 654 (M+H)⁺; Rₜ = 2.30 min |
| **98** | 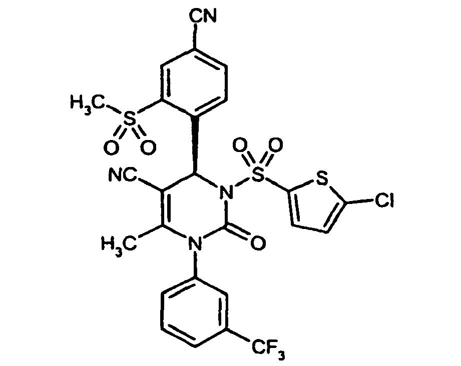 | MS (ESIpos): m/z = 642 (M+H)⁺; Rₜ = 2.29 min |
| **99** | 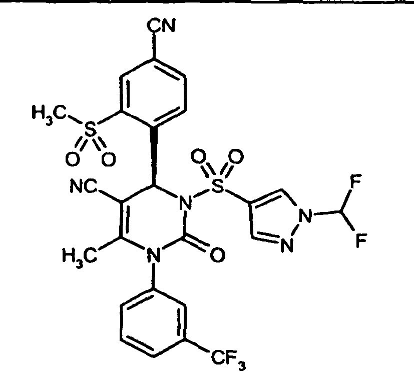 | MS (ESIpos): m/z = 641 (M+H)⁺; Rₜ = 2.18 min |
| **100** | 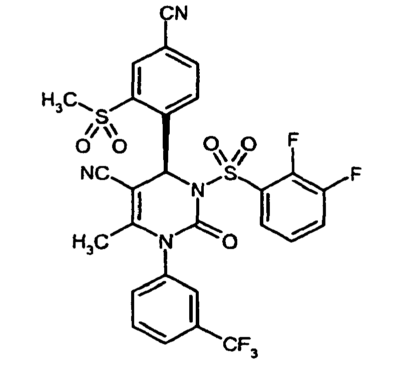 | MS (ESIpos): m/z = 637 (M+H)⁺; Rₜ = 2.23 min |
| **101** | 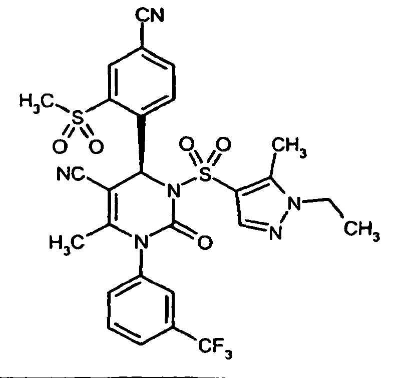 | MS (ESIpos): m/z = 633 (M+H)⁺; Rₜ = 2.16 min |
| **102** | 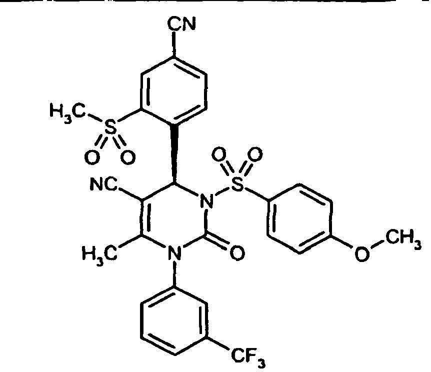 | MS (ESIpos): m/z = 631 (M+H)⁺; Rₜ = 2.23 min |
| **103** | 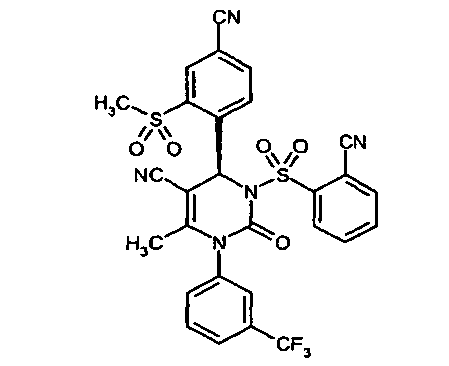 | MS (ESIpos): m/z = 626 (M+H)⁺; Rₜ = 2.17 min |
| **104** | 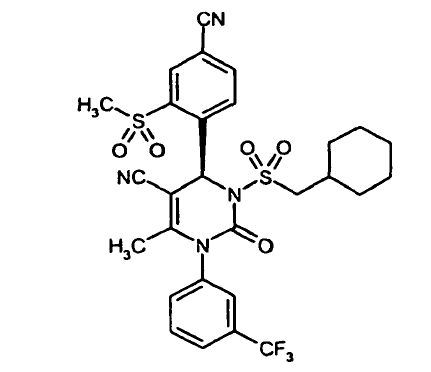 | MS (ESIpos): m/z = 621 (M+H)⁺; Rₜ = 2.33 min |
| **105** | 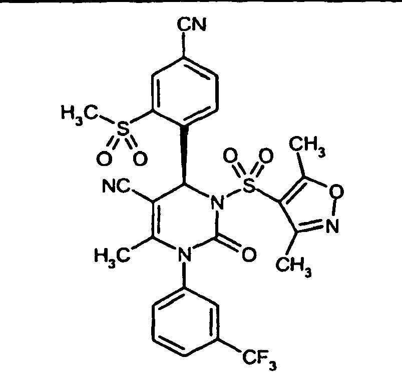 | MS (ESIpos): m/z = 620 (M+H)⁺; Rₜ = 2.23 min |
| **106** | 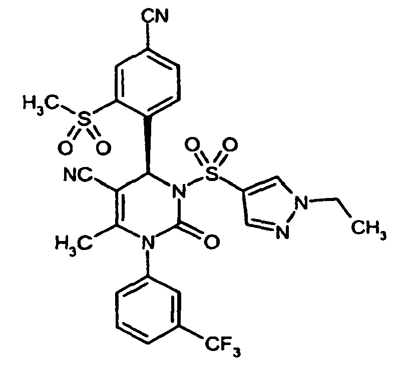 | MS (ESIpos): m/z = 619 (M+H)⁺; Rₜ = 2.16 min |
| **107** | 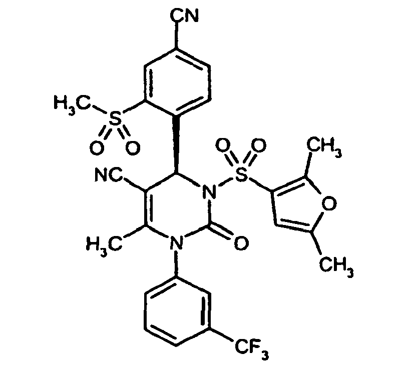 | MS (ESIpos): m/z = 619 (M+H)⁺, Rₜ = 2.27 min |
| **108** | 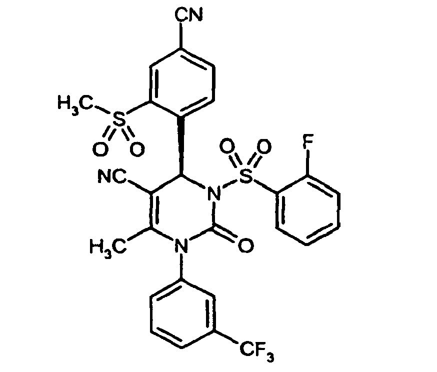 | MS (ESIpos): m/z = 619 (M+H)⁺; Rₜ = 2.21 min |
| **109** | 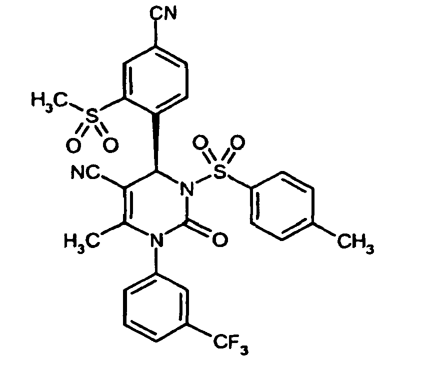 | MS (ESIpos): m/z = 615 (M+H)⁺; Rₜ = 2.27 min |
| **110** | 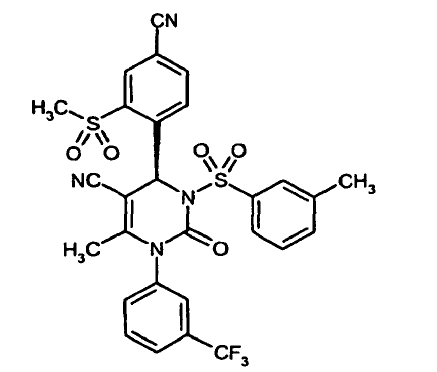 | MS (ESIpos): m/z = 615 (M+H)⁺; Rₜ = 2.26 min |
| **111** | 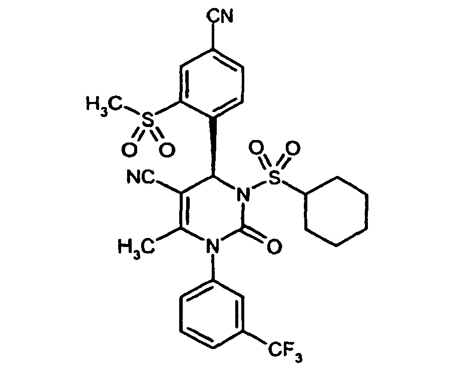 | MS (ESIpos): m/z = 607 (M+H)⁺ Rₜ = 2.28 min |
| **112** | 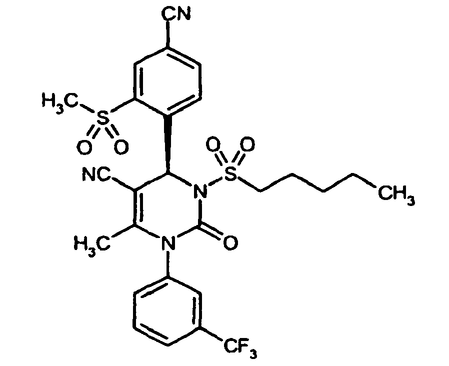 | MS (ESIpos): m/z = 594 (M+H)⁺; Rₜ = 2.28 min |
| **113** | 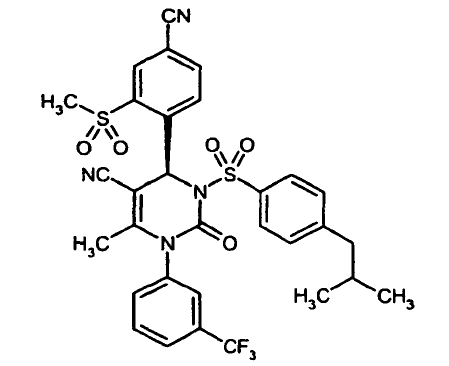 | MS (ESIpos): m/z = 657 (M+H)⁺; Rₜ = 2.38 min |
| **114** | 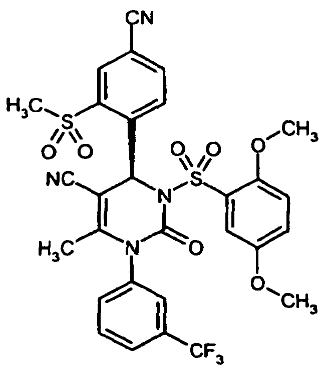 | MS (ESIpos): m/z = 661 (M+H)⁺; Rₜ = 2.22 min |

### Beispiel 115

(*rac*)-Ethyl 4-[4-cyano-2-(methylsulfonyl)phenyl]-6-methyl-2-oxo-1-[3-(trifluormethyl)phenyl]-1,2,3,4-tetrahydropyrimidin-5-carboxylat 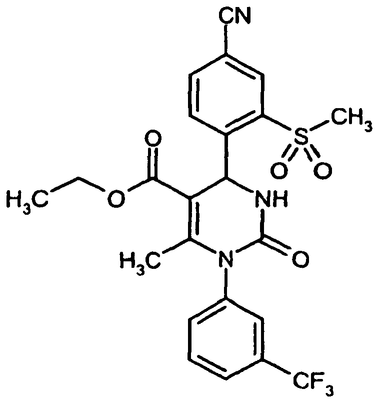 Die Reaktion wurde unter Argon durchgeführt. Phosphorsäuretriethylester (4.18 g, 22.9 mmol) und Diphosphorpentoxid (2.17 g, 15.3 mmol) wurden über Nacht bei 50°C gerührt. Dann wurde mit Methyl-*tert*.-butylether (60 ml) verdünnt, und 4-Formyl-3-(methylsulfonyl)benzonitril (4.00 g, 19.1 mmol; Beispiel 4A), 1-[3-(Trifluormethyl)phenyl]harnstoff (3.90 g, 19.1 mmol) sowie Acetessigsäureethylester (3.73 g, 28.7 mmol) wurden hinzugefügt. Das Gemisch wurde über Nacht unter Rückfluss gerührt. Der entstandene Niederschlag wurde abgesaugt und mit Diethylether (300 ml) gewaschen. Da die Umsetzung nicht vollständig verlaufen war, wurden nochmals Phosphorsäuretriethylester (5.36 g, 29.4 mmol) und Diphosphorpentoxid (2.71 g, 19.1 mol) über Nacht bei 50°C gerührt und dann mit dem zuvor isolierten Feststoff sowie Methyl-*tert*.-butylether (25 ml) eine weitere Nacht unter Rückfluss gerührt. Der entstandene Niederschlag wurde wiederum abgesaugt und mit Diethylether gewaschen. Es wurden so 5.93 g (61% d. Th.) der Zielverbindung erhalten.
HPLC (Methode 2): Rₜ = 4.56 min.
MS (DCI / NH₃): *m*/*z* = 508.1 [M+H]⁺, 525 [M+NH₄]⁺
¹H-NMR (400 MHz, DMSO-d₆): δ = 0.94 (t, 3H), 2.13 (s, 3H), 3.50 (s, 3H), 3.89-4.02 (q, 2H), 6.41 (s, 1H), 7.25 (s, 1H), 7.68-7.90 (m, 4H), 8.09 (d, 1H), 8.26 (d, 1H), 8.39 (s, 1H).

### Beispiel 116

(4*S*)-3-(Cyanomethyl)-4-[4-cyanco-2-(methylsulfonyl)phenyl]-6-methyl-2-oxo-1-[3-(trifluormethyl)phenyl]-1,2,3,4-tetrahydropyrimidin-5-carbonitril

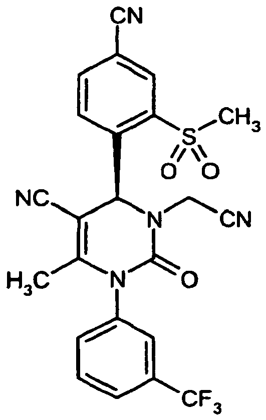

Der Ansatz wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (115 mg, 250 µmol) wurde bei 0°C in THF (1 ml) vorgelegt, mit Natriumhydrid (14 mg, 350 µmol; 1.4 eq.) versetzt und 20 min gerührt. Nach Zugabe von Bromacetonitril (50 mg, 376 µmol; 1.5 eq.) wurde die Mischung für 120 min bei RT gerührt. Das Reaktionsgemisch wurde danach direkt mittels präparativer HPLC aufgereinigt (Säule: Gromsil, C-18 5 µm; Eluent: Acetonitril/Wasser + 0.1% TFA 10:90 → 75:25). Man erhielt die Titelverbindung als einen farblosen Feststoff (85 mg, 68% d. Th.).
LC-MS (Methode 6): Rₜ = 2.39 min; MS (ESIpos): *m*/*z* (%) = 500.1 (100) [M+H]⁺; MS (ESIneg): m/z (%) = 498.0 (100) [M-H]⁻
¹H-NMR (400 MHz, DMSO-d₆): δ = 1.84 (s, 3H), 3.56 (s, 3H), 4.20 (d, 1H), 4.30 (d, 1H), 6.60 (s, 1H), 7.70-8.50 (m, 7H).

### Beispiel 117

(4*S*)-4-[4-Cyano-2-(methylsulfonyl)phenyl]-3-(4-cyanophenyl)-6-methyl-2-oxo-1-[3-(trifluor-v methyl)phenyl]-1,2,3,4-tetrahydropyrimidin-5-carbonitril

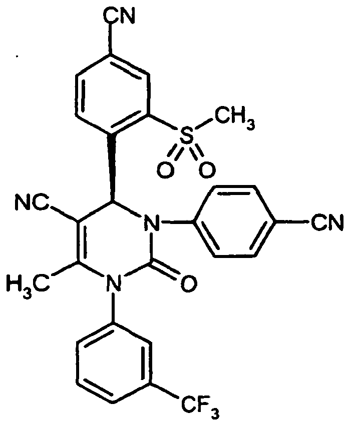

Der Ansatz wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol), 4-Cyanophenylboronsäure (127.7 mg, 869 µmol; 4 eq.), Kupfer(II)acetat (158 mg, 869 µmol; 4 eq.) und 4 Å-Molekularsieb (500 mg) wurden in Dichlormethan (5 ml) vorgelegt, mit Pyridin (281 µl; 16 eq.) und Triethylamin (121 µl; 4 eq.) versetzt und anschließend für 4 Tage bei RT gerührt. Das Reaktionsgemisch wurde danach über Kieselgur filtriert, welches mehrfach mit Methanol nachgewaschen wurde, und das Filtrat im Vakuum eingeengt. Das Rohprodukt wurde mittels präparativer HPLC gereinigt (Säule: Gromsil, C-18 5 µm; Eluent: Acetonitril/Wasser + 0.1 % TFA 10:90 → 80:20). Man erhielt die Titelverbindung als einen farblosen Feststoff (25 mg, 19.5% d. Th.).
LC-MS (Methode 10): Rₜ = 2.34 min; MS (ESIpos): m/z (%) = 562.0 (100) [M+H]⁺; MS (ESIneg): *m*/*z* (%) = 416.1 (100), 560.2 (50) [M-H]⁻
¹H-NMR (400 MHz, DMSO-d₆): δ = 1.90 (s, 3H), 3.00 (s, 3H), 7.10 (s, 1H), 7.40 (m, 2H), 7.70-8.70 (m, 9H).

### Beispiel 118

(4*S*)-4-[4-Cyano-2-(methylsulfonyl)phenyl]-6-methyl-2-oxo-1,3-bis[3-(trifluormethyl)phenyl)-1,2,3,4-tetrahydropyrimidin-5-carbonitril

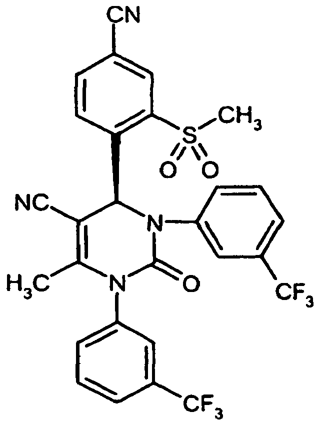

Der Ansatz wurde unter Argon durchgeführt. (4S)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol), 3-(Trifluormethyl)phenylboronsäure (165 mg, 869 µmol; 4 eq.), Kupfer(II)acetat (158 mg, 869 µmol; 4 eq.) und 4 Å-Molekularsieb (500 mg) wurden in Dichlormethan (5 ml) vorgelegt, mit Pyridin (281 µl; 16 eq.) und Triethylamin (121 µl; 4 eq.) versetzt und anschließend für 20 h bei RT gerührt. Das Reaktionsgemisch wurde danach über Kieselgur filtriert, welches mehrfach mit Methanol nachgewaschen wurde, und das Filtrat im Vakuum eingeengt. Das Rohprodukt wurde mittels präparativer HPLC gereinigt (Säule: Gromsil, C-18 5 µm; Eluent: Acetonitril/Wasser + 0.1% TFA 10:90 → 90:10). Man erhielt die Titelverbindung als einen farblosen Feststoff (34 mg, 26% d. Th.).
LC-MS (Methode 4): Rₜ = 1.39 min; MS (ESIpos): *m*/*z* (%) = 605.0 (100) [M+H]⁺
¹H-NMR (400 MHz, DMSO-d₆): δ = 1.90 (s, 3H), 3.00 (s, 3H), 7.10 (s, 1H), 7.30 (m, 1H), 7.50 (m, 1H), 7.60 (m, 1H), 7.70 (m, 1H), 7.70-8.70 (m, 7H).

### Beispiel 119

(4*S*)-4-[4-Cyano-2-(methylsulfonyl)phenyl]-3-(4-cyanophenyl)-6-methyl-2-oxo-1-[3-(trifluormethyl)phenyl]-1,2,3,4-tetrahydropyrimidin-5-carbonitril 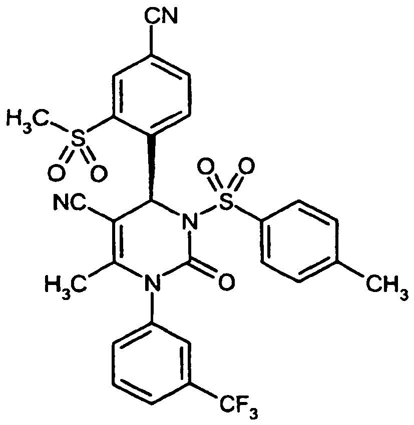 Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (278 mg, 604 µmol) wurde in trockenem THF (6 ml) vorgelegt und bei 0°C mit Natriumhydrid (60% in Mineralöl; 34 mg, 845 µmol; 1.4 eq.) versetzt. Es wurde auf RT erwärmt und 20 min gerührt. Anschließend wurde eine Lösung von *p*-Toluolsulfonylchlorid (161 mg, 845 µmol; 1.4 eq.) in THF (~2 ml) langsam zugetropft. Nach 16 h Reaktionszeit wurde das Reaktionsgemisch eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (358 mg, 96% d. Th.).
LC-MS (Methode 4): Rₜ = 1.38 min; MS (ESIpos): *mlz* (%) = 615.2 (100) [M+H]⁺
¹H-NMR (400 MHz, DMSO-d₆): δ = 1.75 (s, 3H), 2.40 (s, 3H), 3.50 (s, 3H), 7.30-7.40 (m, 4H), 7.60 (s, 1H), 7.70 (m, 2H), 7.85-7.90 (m, 2H), 8.13 (m, 1H), 8.25 (m, 1H), 8.65 (s, 1H).

### Beispiel 120

Benzyl 5-cyano-6-[4-cyano-2-(methylsulfonyl)phenyl]-4-methyl-2-oxo-3-[3-(trifluormethyl)-phenyl]-3,6-dihydropyrimidin-1(2*H*)-carboxylat

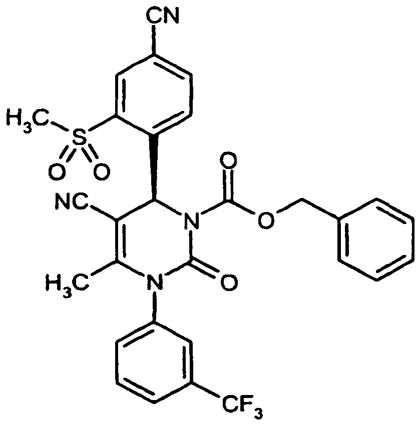

Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (115 mg, 250 µmol) wurde in trockenem THF (3 ml) vorgelegt und bei 0°C mit Natriumhydrid (60% in Mineralöl; 14 mg, 350 µmol; 1.4 eq.) versetzt. Es wurde auf RT erwärmt und 20 min gerührt. Anschließend wurde eine Lösung von Chlorameisensäurebenzylester (60 mg, 350 µmol; 1.4 eq.) in THF (1 ml) langsam zugetropft. Nach 1.5 h Reaktionszeit wurde das Reaktionsgemisch eingeengt und das Rohprodukt mittels präparativer HPLC aufgereinigt (Säule: Gromsil C-18 10 µm; Eluent: Acetonitril/Wasser + 0.1% TFA 10:90 → 90:10). Man erhielt die Titelverbindung als einen farblosen Feststoff (68 mg, 46% d. Th.).
LC-MS (Methode 9): Rₜ = 1.22 min; MS (ESIpos)**:** *m*/*z* (%) = 595 (10) [M+H]⁺; MS (ESIneg): *m*/*z* (%) = 416.2 (100), 593.4 (50) [M-H]⁻
¹H-NMR (400 MHz, DMSO-d₆): δ = 1.80 (s, 3H), 3.45 (s, 3H), 5.20 (s, 2H), 7.15 (m, 1H), 7.25-7.30 (m, 5H), 7.75-7.90 (m, 3H), 8.05 (br. s, 2H), 8.30 (dd, 1H), 8.50 (d, 1H).

### Beispiel 121

(4*S*)-4-[4-Cyano-2-(methylsulfonyl)phenyl]-3-[2-(diethylamino)ethyl]-6-methyl-2-oxo-1-[3-(trifluormethyl)phenyl]-1,2,3,4-tetrahydropyrimidin-5-carbonitril-Trifluoracetat

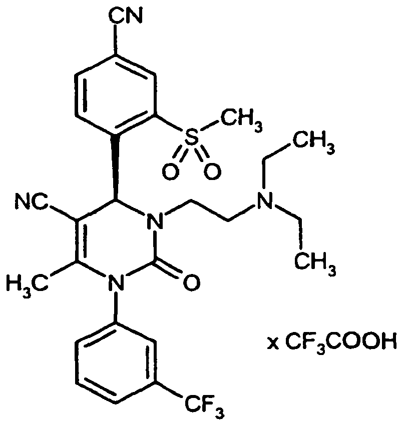

Der Ansatz wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril (100 mg, 217 µmol) wurde in trockenem THF (5 ml) vorgelegt, bei 0°C mit Natriumhydrid (60% in Mineralöl; 22 mg, 543 µmol; 2.5 eq.) versetzt und 20 min gerührt. Dann wurde 2-Brom-*N,N*-diethyl-ethanamin-Hydrobromid (85 mg, 326 µmol; 1.5 eq.) zugegeben und die Mischung 90 min bei RT gerührt. Das Reaktionsgemisch wurde danach auf gesättigte Kochsalz-Lösung (50 ml) gegeben und mit Essigsäureethylester (3 x 30 ml) extrahiert. Die vereinigten organischen Phasen wurden über festem Natriumsulfat getrocknet, filtriert und im Vakuum eingeengt. Das Rohprodukt wurde mittels präparativer HPLC aufgereinigt (Säule: Gromsil C-18 10 µm; Eluent: Acetonitril/Wasser + 0.1% TFA 10:90 → 75:25). Die Titelverbindung wurde als farbloser Feststoff erhalten (112 mg, 77% d. Th.).
LC-MS (Methode 5): Rₜ = 1.34 min; MS (ESIpos): *m*/*z* (%) = 560.2 (100) [M+H]⁺; MS (ESIneg): *m*/*z* (%) = 558.3 (100) [M-H]⁻
¹H-NMR (400 MHz, DMSO-d₆): δ = 1.12 (t, 6H), 1.85 (s, 3H), 3.10 (br. m, 6H), 3.30 (m, 1H), 3.55 (s, 3H), 3.70 (m, 1H), 6.55 (s, 1H), 7.70-8.40 (m, 6H), 8.51 (s, 1H), 9.10 (br. s, 1H).

### Beispiel 122

(4*S*)-4-[4-Cyano-2-(methylsulfonyl)phenyl)-3,6-dimethyl-2-oxo-1-[3-(trifluormethyl)phenyl]-1,2,3,4-tetrahydropyrimidin-5-carbonsäure-allylester 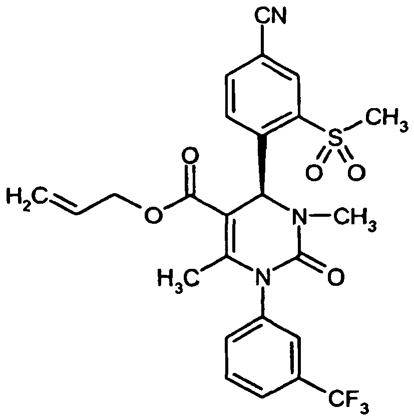 Der Ansatz wurde unter Argon durchgeführt. (4*S*)-4-(4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (260 mg, 0.5 mmol) wurde bei -78°C in THF (10 ml) vorgelegt und mit einer 1 M Lösung von Lithiumhexamethyldisilazid (LiHMDS) in THF (0.6 ml; 1.2 eq.) versetzt. Nach 20 min Rühren wurde Iodmethan (355 mg; 5 eq.) zugegeben und die Mischung für 16 h unter allmählicher Erwärmung von -78°C auf RT gerührt. Das Reaktionsgemisch wurde danach eingeengt und der Rückstand über Kieselgel flash-chromatographiert (Laufmittel: Cyclohexan → Cyclohexan/Essigsäureethylester 55:45). Man erhielt die Titelverbindung als Feststoff (157 mg, 59% d. Th.).
LC-MS (Methode 5): Rₜ = 2.30 min; MS (ESIpos): *m*/*z* (%) = 534.1 (100) [M+H]⁺; MS (ESIneg): *m*/*z* (%) = 532.1 (100) [M-H]⁻.

### Beispiel 123

4-(4*S*)-3,6-Dimethyl-2,5-dioxo-1-[3-(trifluormethyl)phenyl]-2,3,4,5,6,7-hexahydro-1*H*-pyrrolo-[3,4-d]pyrimidin-4-yl-3-(methylsulfonyl)benzonitril 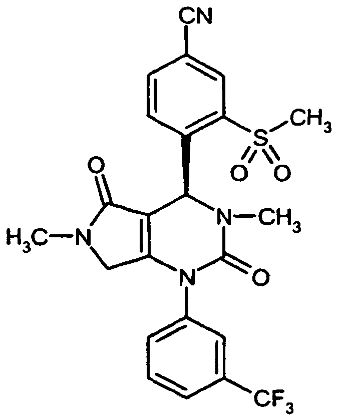 2,3-Dibrompropyl (4*S*)-6-(brommethyl)-4-[4-cyano-2-(methylsulfonyl)phenyl]-3-methyl-2-oxo-l-[3-(trifluormethyl)phenyl]-1,2,3,4-tetrahydropyrimidin-5-carboxylat (801 mg, 1.04 mmol) wurde unter Argon-Schutzgasatmosphäre mit einer 1 M Lösung von Methylamin in THF (20.7 ml, 20.7 mmol, 20 eq.) versetzt und zunächst 2 h bei RT und dann weitere 16 h bei 5°C gerührt. Das Reaktionsgemisch wurde danach im Vakuum eingeengt und das Rohprodukt mittels präparativer HPLC gereinigt (Säule: Gromsil C-18 10 µm; Eluent: AcetonitriVWasser + 0.1% TFA 10:90 → 75:25). Man erhielt die Titelverbindung als einen Feststoff (314 mg, 60% d. Th.).
LC-MS (Methode 5): Rₜ = 1.82 min; MS (ESIpos): *m*/*z* (%) = 505.1 (100) [M+H]⁺; MS (ESIneg): *m*/*z* (%) = 503.1 (100) [M-H]⁻
¹H-NMR (400 MHz, DMSO-d₆): δ = 2.70 (2s, 6H), 3.70 (s, 3H), 3.90 (m, 2H), 6.60 (s, 1H), 7.80 (m, 1H), 7.90 (m, 2H), 8.00 (br. s, 1H), 8.20 (m, 1H), 8.25 (m, 1H), 8.40 (m, 1H).

### Beispiel 124

(*rac)*-4-[4-Cyano-2-(ethylsulfonyl)phenyl]-6-methyl-2-oxo-1-[3-(trifluormethyl)phenyl]-1,2,3,4-tetrahydropyrimidin-5-carbonsäure-allylester 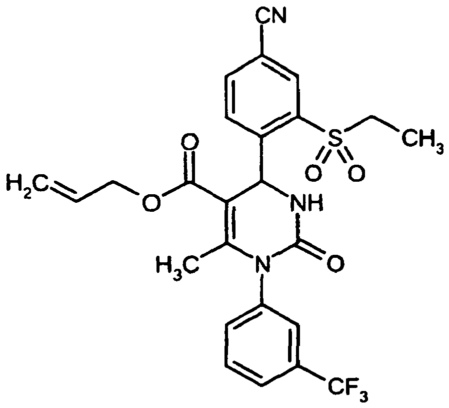 Die Reaktion wurde unter Argon durchgeführt. Phosphorsäuretriethylester (0.718 g, 3.9 mmol) und Diphosphorpentoxid (0.511 g) wurden über Nacht bei 40°C gerührt. Dann wurde mit MTBE (30 ml) verdünnt, und 4-Formyl-3-(ethylsulfonyl)benzonitril (0.88 g, 3.94 mmol), 1-[3-(Trifluormethyl)phenyl]harnstoff (0.805 g, 3.94 mmol) sowie Allylacetoacetat (0.841 g, 5.91 mmol; 1.5 eq.) wurden hinzugefügt. Das Gemisch wurde dann 4 h unter Rückfluss gerührt. Die Reaktionsmischung wurde danach durch Abdestillieren von MTBE aufkonzentriert und anschließend für weitere 2 h auf 90°C erhitzt. Zur Aufarbeitung wurde restliches Lösungsmittel im Vakuum entfernt und der Rückstand mit MTBE (20 ml) aufgeschlämmt und dann abgesaugt. Der Feststoff wurde mit MTBE (10 ml) gewaschen. Man erhielt so 1.34 g (42% d. Th.) der Titelverbindung.
LC-MS (Methode 4): Rₜ = 1.33 min; MS (ESIpos): *m*/*z* (%) = 534.1 (100) [M+H]⁺
¹H-NMR (400 MHz, DMSO-d₆): δ = 1.20 (t, 3H), 2.15 (s, 3H), 3.54 (m, 2H), 4.45 (m, 2H), 4.95 (d, 1H), 5.05 (d, 1H), 5.70 (m, 1H), 6.25 (d, 1H), 7.00 (d, 1H), 7.70-7.85 (m, 4H), 8.10 (br. d, 1H), 8.30 (m, 1H), 8.35 (s, 1H).

### Beispiel 125

(*rac*)-4-[4-Cyano-2-(ethylsulfonyl)phenyl]-6-methyl-2-oxo-1-[3-(trifluomethyl)phenyl]-1,2,3,4-tetrahydropyrimidin-5-carboxamid

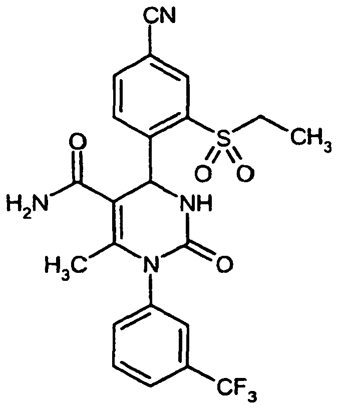

Die Reaktion wurde unter Argon durchgeführt. (*rac*)-4-[4-Cyano-2-(ethylsulfonyl)phenyl]-6-methyl-2-oxo-1-[3-(trifluormethyl)phenyl]-1,2,3,4-tetrahydropyrimidin-5-carbonsäure (1100 mg, 2.29 mmol) und HATU (2 eq., 1695 mg, 4.5 mmol) wurden in trockenem DMF (28 ml) bei 0°C vorgelegt und nach kurzem Rühren (20 min) mit einer 0.5 M Ammoniak-Lösung in Dioxan (6 eq., 23.6 ml, 13.4 mmol) sowie DIEA (2 eq., 576 mg, 4.5 mmol) versetzt. Das Gemisch wurde 3 h bei RT gerührt (HPLC-Kontrolle). Danach wurde das Reaktionsgemisch mit Essigsäureethylester (200 ml) verdünnt. Die organische Phase wurde nacheinander mit gesättigter Natriumhydrogencarbonat-Lösung (50 ml), 10%-iger Zitronensäure (3 x 50 ml) und gesättigter Kochsalz-Lösung (50 ml) gewaschen, über festem Natriumsulfat getrocknet, filtriert und eingeengt. Der Rückstand wurde an Kieselgel flash-chromatographiert (Laufmittel: Dichlormethan → Dichlormethan/Methanol 50:3). Es wurde ein farbloser Feststoff erhalten (1050 mg, 96% d. Th.).
LC-MS (Methode 5): Rₜ = 1.57 min; MS (ESIpos): *m*/*z* (%) = 493.0 (100) [M+H]⁺; MS (ESIneg): *m*/*z* (%) = 448.1 (100), 491.1 (90) [M-H]⁻
¹H-NMR (400 MHz, DMSO-d₆): δ = 1.16 (t, 3H), 1.80 (s, 3H), 3.57 (m, 2H), 6.25 (s, 1H), 7.20 (m, 1H), 7.30 (br. s, 1H), 7.45 (br. s, 1H), 7.65-7.80 (m, 4H), 8.10 (d, 1H), 8.25 (s, 1H), 8.35 (dd, 1H).

### Beispiel 126

(*rac*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril

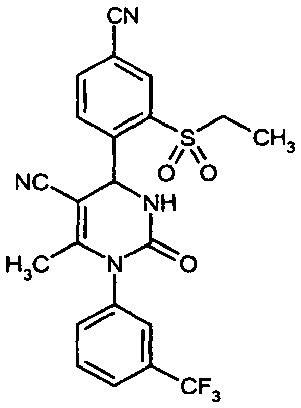

Die Reaktion wurde unter Argon durchgeführt. (*rac*)-4-[4-Cyano-2-(ethylsulfonyl)phenyl]-6-methyl-2-oxo-1-[3-(trifluormethyl)phenyl]-1,2,3,4-tetrahydropyrimidin-5-carboxamid (1000 mg, 2.03 mmol) wurde in trockenem THF (50 ml) vorgelegt, mit Methoxycarbonylsulfamoyl-triethylammoniumhydroxid (Burgess-Reagens; 726 mg, 3.046 mmol) versetzt und bei RT gerührt. Die HPLC-Kontrolle zeigte nach 75 min vollständigen Umsatz. Das Reaktionsgemisch wurde danach mit Wasser (20 ml) versetzt, eingeengt und der Rückstand in Essigsäureethylester (200 ml) aufgenommen. Die organische Phase wurde mit gesättigter Kochsalz-Lösung (3 x 50 ml) gewaschen, über festem Natriumsulfat getrocknet, filtriert und eingeengt. Der Rückstand wurde an Kieselgel flash-chromatographiert (Laufmittel: Cyclohexan → Cyclohexan/Essigsäureethylester 2:3). Die Titelverbindung wurde als farbloser Feststoff erhalten (890 mg, 92% d. Th.).
LC-MS (Methode 6): Rₜ = 2.34 min; MS (ESIpos): *m*/*z* (%) = 475.0 (100) [M+H]⁺; MS (ESIneg): *m*/*z* (%) = 473.1 (100) [M-H]⁻
¹H-NMR (400 MHz, DMSO-d₆): δ = 1.20 (t, 3H), 1.80 (s, 3H), 3.45 (m, 2H), 6.35 (s, 1H), 7.70-7.85 (m, 3H), 7.95 (br. s, 1H), 8.30-8.40 (m, 4H).

### Beispiel 127

(4*S*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril 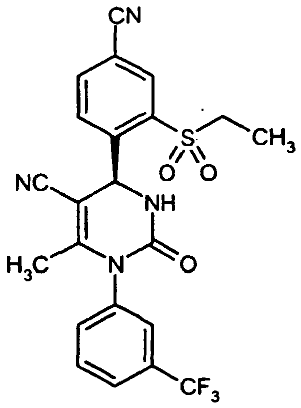 (*rac*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril (0.83 g) wurde durch präparative HPLC-Chromatographie an chiraler Phase in die Enantiomere aufgetrennt [Säule: chirale Kieselgel-Phase basierend auf dem Selektor Poly(*N*-methacryloyl-D-leucin-dicyclopropylmethylamid); Säulendimension: 670 mm x 40 mm; Probenvorbereitung: Lösung in Essigsäureethylester (30 ml); Injektionsvolumen: 6.1 ml; Eluent: Isohexan/Essigsäureethylester 3:7; Fluss: 50 ml/min; Temperatur: 24°C; Detektion: 260 nm]. Es wurden 0.379 g (89% d. Th.; >99.9% ee) des 4*S*-Enantiomeren erhalten.

Der Enantiomerenüberschuss (ee-Wert) wurde chromatographisch bestimmt [Säule: chirale Kieselgel-Phase basierend auf dem Selektor Poly(*N*-methacryloyl-D-leucin-dicyclopropyhnethylamid); Säulendimension: 250 mm x 4.6 mm; Eluent: Isohexan/Essigsäureethylester 1:1; Fluss: 2 ml/min; Detektion: 260 nm; Rₜ = 4.26 min (4R-Enantiomer: Rₜ = 8.87 min)].

LC-MS (Methode 4): Rₜ = 1.18 min; MS (ESIpos): *m*/*z* (%) = 475.1 (100) [M+H]⁺.

Für die ¹H-NMR-Daten siehe die racemische Verbindung (Beispiel 126).

### Beispiel 128

(4*S*)-4-[4-Cyano-2-(ethylsulfonyl)phenyl]-3,6-dimethyl-2-oxo-1-[3-(trifluormethyl)phenyl]-1,2,3,4-tetrahydropyrimidin-5-carbonitril 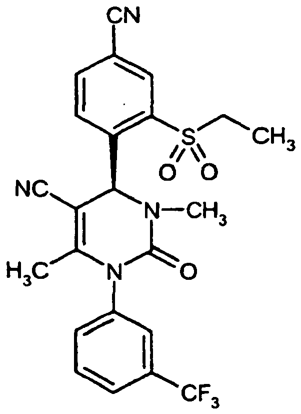 Der Ansatz wurde unter Argon durchgeführt. (4*S*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril (71.2 mg, 0.15 mmol) wurde in absolutem THF (6 ml) vorgelegt und bei -78°C mit einer 1 M Lösung von Lithiumhexamethyldisilazid (LiHMDS) in THF (180 µl; 1.2 eq.) versetzt. Nach 20 min Rühren wurde Iodmethan (47 µl; 5 eq.) zugegeben und die Mischung für 60 h unter allmählicher Erwärmung von -78°C auf RT gerührt. Das Reaktionsgemisch wurde danach direkt mittels präparativer HPLC gereinigt (Säule: Gromsil, C-18 10 µm; Eluent: Acetonitril/Wasser + 0.1% TFA 10:90 → 80:20). Man erhielt die Titelverbindung als einen farblosen Feststoff (60 mg, 82% d. Th.).
LC-MS (Methode 6): Rₜ = 2.42 min; MS (ESIpos): *m*/*z* (%) = 489.0 (100) [M+H]⁺; MS (ESIneg): *m*/*z* (%) = 487.0 (100) [M-H]⁻
¹H-NMR (400 MHz, DMSO-d₆): δ = 1.20 (t, 3H), 1.80 (s, 3H), 2.68 (s, 3H), 3.55 (m, 2H), 6.45 (s, 1H), 7.70-8.05 (m, 4H), 8.40 (m, 3H).

### Beispiel 129

(4*S*)-4-[4-Cyano-2-(ethylsulfonyl)phenyl]-6-methyl-3-(methylsulfonyl)-2-oxo-1-[3-(trifluormethyl)phenyl]-1,2,3,4-tetrahydropyrimidin-5-carbonitril

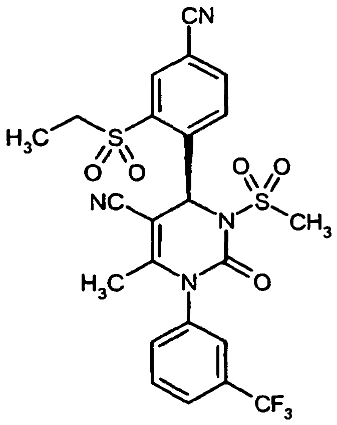

Die Reaktion wurde unter Argon durchgeführt. (4*S*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril (71.2 mg, 150 µmol) wurde in trockenem THF (3 ml) vorgelegt und bei 0°C mit Natriumhydrid (60% in Mineralöl; 8.4 mg, 210 µmol; 1.4 eq.) versetzt. Es wurde auf RT erwärmt und 20 min nachgerührt. Anschließend wurde eine Lösung von Methansulfonylchlorid (24.1 mg, 210 µmol; 1.4 eq.) in THF (1 ml) langsam zugetropft. Nach 20 h Reaktionszeit wurde das Reaktionsgemisch eingeengt und das Rohprodukt mittels präparativer HPLC aufgereinigt (Säule: Gromsil C-18 10 µm; Eluent: Acetonitril/Wasser + 0.1% TFA 10:90 → 80:20). Man erhielt die Titelverbindung als einen farblosen Feststoff (70 mg, 84% d. Th.).
LC-MS (Methode 6): Rₜ = 2.42 min; MS (ESIpos): *m*/*z* (%) = 553.0 (100) [M+H]⁺
¹H-NMR (400 MHz, DMSO-d₆): δ = 1.25 (t, 3H), 1.80 (s, 3H), 3.40 (s, 3H), 3.55 (m, 2H), 7.20 (s, 1H), 7.80-8.40 (m, 7H).

### Beispiel 130

(*rac*)-4-[4-Cyano-2-((2-hydroxyethyl)sulfonyl)phenyl]-6-methyl-2-oxo-1-[3-(trifluormethyl)-phenyl]-1,2,3,4-tetrahydropyrimidin-5-carbonsäure-allylester

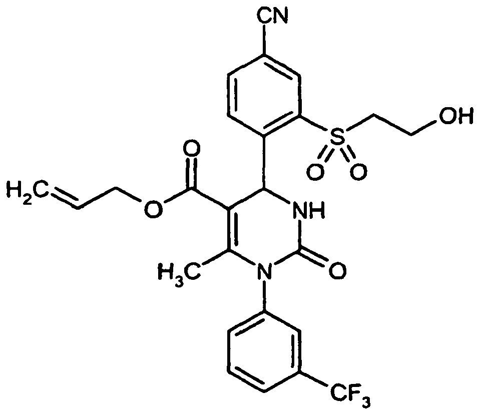

Die Reaktion wurde unter Argon durchgeführt. Phosphorsäuretriethylester (1.43 g, 7.9 mmol; 1.2 eq.) und Diphosphorpentoxid (0.745 g; 0.8 eq.) wurden über Nacht bei 40°C gerührt. Dann wurde mit MTBE (35 ml) verdünnt, und 3-[(2-Hydroxyethyl)sulfonyl]-4-formylbenzonitril (1.57 g, 6.6 mmol), 1-[3-(Trifluormethyl)phenyl]harnstoff (1.34 g, 6.6 mmol) sowie Allylacetoacetat (1.4 g, 9.84 mmol; 1.5 eq.) wurden hinzugefügt. Das Gemisch wurde 2 h unter Rückfluss gerührt. Die Reaktionsmischung wurde danach durch Abdestillieren von MTBE aufkonzentriert und anschließend für weitere 5 h auf 90°C erhitzt. Zur Aufarbeitung wurde restliches Lösungsmittel im Vakuum entfernt und der Rückstand in Essigsäureethylester (250 ml) aufgenommen. Die organische Phase wurde mit gesättigter Kochsalz-Lösung (3 x 50 ml) gewaschen, über festem Natriumsulfat getrocknet, filtriert und im Vakuum eingeengt. Der Rückstand wurde an Kieselgel flash-chromatographiert (Laufmittel: Cyclohexan → Cyclohexan/Essigsäureethylester 2:3). Die Titelverbindung wurde als farbloser Feststoff erhalten (1.22 g, 23% d. Th.).
LC-MS (Methode 4): Rₜ = 1.20 min; MS (ESIpos): *m*/*z* (%) = 550.1 (100) [M+H]⁺
¹H-NMR (400 MHz, DMSO-d₆): δ = 2.15 (s, 3H), 3.54 (m, 2H), 3.85 (m, 2H), 4.45 (m, 2H), 5.00 (d, 1H), 5.05 (d, 1H), 5.15 (t, 1H), 5.70 (m, 1H), 6.30 (d, 1H), 7.05 (d, 1H), 7.70-7.85 (m, 4H), 8.10 (br. d, 1H), 8.25 (m, 1H), 8.35 (m, 1H).

### Beispiel 131

(*rac*)-4-[4-Cyano-2-((2-hydroxyethyl)sulfonyl)phenyl]-6-methyl-2-oxo-1-[3-(trifluormethyl)-phenyl]-1,2,3,4-tetrahydropyrimidin-5-carboxamid 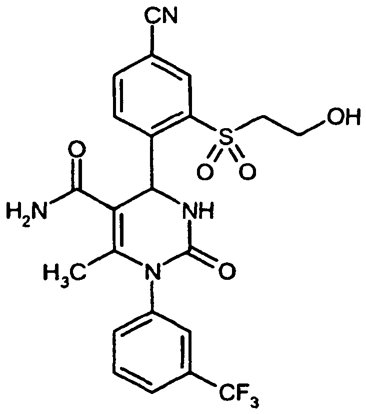 Die Reaktion wurde unter Argon durchgeführt. (*rac*)-4-[4-Cyano-2-((2-hydroxyethyl)sulfonyl)-phenyl]-6-methyl-2-oxo-1-[3-(trifluormethyl)phenyl]-1,2,3,4-tetrahydropyrimidin-5-carbonsäure (910 mg, 1.79 mmol) und HATU (5 eq., 3396 mg, 8.9 mmol) wurden in trockenem DMF (20 ml) bei 0°C vorgelegt und nach kurzem Rühren (20 min) mit Ammoniumchlorid (5 eq., 478 mg, 8.9 mmol) sowie DIEA (10 eq., 2309 mg, 17.9 mmol) versetzt. Das Gemisch wurde 16 h bei RT gerührt (HPLC-Kontrolle) und anschließend im Vakuum eingeengt. Das Rohprodukt wurde mittels präparativer HPLC aufgereinigt (Säule: Gromsil C-18; Eluent: Acetonitril/Wasser + 0.1% TFA 10:90 → 80:20). Die Titelverbindung wurde als Feststoff erhalten (740 mg, 81% d. Th.).
LC-MS (Methode 6): Rₜ = 1.84 min; MS (ESIpos): *m*/*z* (%) = 509.1 (100) [M+H]⁺; MS (ESIneg): *m*/*z* (%) = 464.3 (100), 507.1 (50) [M-H]⁻
¹H-NMR (400 MHz, DMSO-d₆): δ = 1.80 (s, 3H), 3.70 (m, 4H), 6.30 (s, 1H), 7.10 (m, 1H), 7.25 (br. s, 1H), 7.45 (br. s, 1H), 7.65-7.80 (m, 4H), 8.10 (d, 1H), 8.30 (m, 2H).

### Beispiel 132

(*rac*)-4-[4-Cyano-2-((2-hydroxyethyl)sulfonyl)phenyl]-6-methyl-2-oxo-1-[3-(trifluormethyl)-phenyl]-1,2,3,4-tetrahydropyrimidin-5-carbonitril 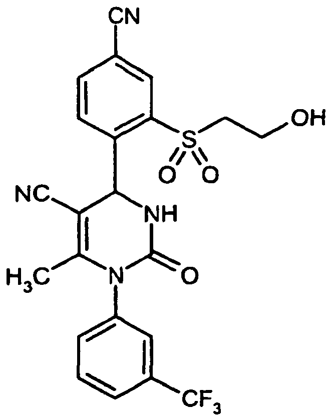 Der Ansatz wurde unter Argon durchgeführt. 4-{2-[(2-{[*tert*.-Butyl(dimethyl)silyl]oxy}ethyl)-sulfonyl]-4-cyanophenyl}-6-methyl-2-oxo-1-[3-(trifluormethyl)phenyl]-1,2,3,4-tetrahydropyrimidin-5-carbonitril (550 mg, 0.909 mmol) wurde in Dichlormethan (30 ml) vorgelegt, mit Trifluoressigsäure (30 ml) versetzt und die Mischung 5 h bei RT gerührt (HPLC-Kontrolle). Danach wurde ohne Erwärmung eingeengt und der Rückstand in Dichlormethan (200 ml) aufgenommen. Die organische Phase wurde mit gesättigter Natriumhydrogencarbonat-Lösung (2 x 50 ml) und mit gesättigter Kochsalz-Lösung (50 ml) gewaschen, über festem Natriumsulfat getrocknet, filtriert und im Vakuum eingeengt. Das erhaltene Öl wurde in wenig Acetonitril aufgenommen, mit Wasser versetzt und lyophilisiert. Die Titelverbindung wurde als Feststoff erhalten (450 mg, quant.).
LC-MS (Methode 6): Rₜ = 2.15 min; MS (ESIpos): *m*/*z* (%) = 491.0 (100) [M+H]⁺; MS (ESIneg): *m*/*z* (%) = 446.0 (100), 489.0 (40) [M-H]⁻.

### Beispiel 133

(4*S*)-4-[4-Cyano-2-((2-hydroxyethyl)sulfonyl)phenyl]-6-methyl-2-oxo-1-[3-(trifluorinethyl)-phenyl]-1,2,3,4-tetrahydropyrimidin-5-carbonitril 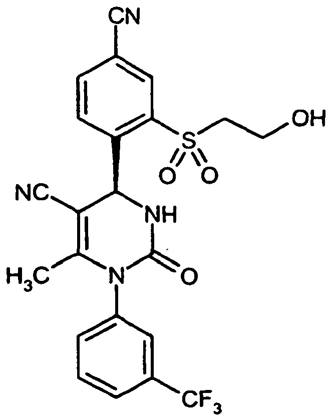 (*rac*)-4-[4-Cyano-2-((2-hydroxyethyl)sulfonyl)phenyl)-6-methyl-2-oxo-1-[3-(trifluormethyl)-phenyl]-1,2,3,4-tetrahydropyrimidin-5-carbonitril (0.13 g) wurde durch präparative HPLC-Chromatographie an chiraler Phase in die Enantiomere aufgetrennt [Säule: Daicel Chiralpak IC, 5 µm; Säulendimension: 250 mm x 20 mm; Probenvorbereitung: Lösung in Methanol (10 ml); Injektionsvolumen: 0.5 ml; Eluent: MTBE/Methanol 7:3; Fluss: 15 ml/min; Temperatur: 30°C; Detektion: 220 nm]. Es wurden 0.042 g (65% d. Th.; >99.5% ee) des 4S-Enantiomeren erhalten.
Der Enantiomerenüberschuss (ee-Wert) wurde chromatographisch bestimmt [Säule: Daicel Chiralpak IC, 5 µm; Säulendimension: 250 mm x 4.6 mm; Eluent: MTBE/Methanol 7:3; Fluss: 1 ml/min; Detektion: 220 nm; Rₜ = 4.00 min (4*R*-Enantiomer: Rₜ = 5.15 min)].
LC-MS (Methode 5): Rₜ = 1.72 min; MS (ESIpos): *m*/*z* (%) = 491.1 (100) [M+H]⁺; MS (ESIneg): *m*/*z* (%) = 446.1 (100), 489.2 (30) [M-H]⁻.

### Beispiel 134

(*rac*)-4-{4-Cyano-2-[(1-methylethyl)sulfonyl]phenyl}-6-methyl-2-oxo-1-[3-(trifluormethyl)-phenyl]-1,2,3,4-tetrahydropyrimidin-5-carbonsäure-allylester 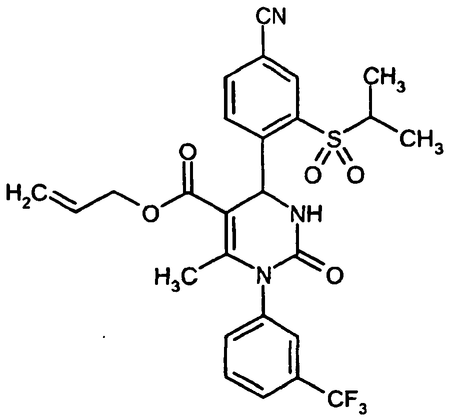 Die Reaktion wurde unter Argon durchgeführt. Phosphorsäuretriethylester (1.049 g, 5.76 mmol) und Diphosphorpentoxid (0.454 g) wurden über Nacht bei 40°C gerührt. Dann wurde mit MTBE (25 ml) verdünnt, und 4-Formyl-3-[(1-methylethyl)sulfonyl]benzonitril (0.759 g, 3.2 mmol), 1-[3-(Trifluormethyl)phenyl]harnstoff (0.653 g, 3.2 mmol) sowie Allylacetoacetat (0.682 g, 4.8 mmol; 1.5 eq.) wurden hinzugefügt. Das Gemisch wurde 6 h unter Rückfluss gerührt. Die Reaktionsmischung wurde danach durch Abdestillieren von MTBE aufkonzentriert und anschließend für weitere 2 h auf 90°C erhitzt. Zur Aufarbeitung wurde restliches Lösungsmittel im Vakuum entfernt und der Rückstand mit MTBE (20 ml) aufgeschlämmt und dann abgesaugt. Der Feststoff wurde mit MTBE (2 x 10 ml) gewaschen. Man erhielt 1.25 g (48% d. Th.) der Titelverbindung.
LC-MS (Methode 4): Rₜ = 1.37 min; MS (ESIpos): *m*/*z* (%) = 548.1 (100) [M+H]⁺
¹H-NMR (400 MHz, DMSO-d₆): δ = 1.13 (d, 3H), 1.40 (d, 3H), 2.15 (s, 3H), 3.71 (m, 1H), 4.50 (m, 2H), 4.95 (d, 1H), 5.05 (d, 1H), 5.70 (m, 1H), 6.15 (d, 1H), 6.80 (d, 1H), 7.70-7.85 (m, 4H), 8.15 (br. d, 1H), 8.30 (m, 2H).

### Beispiel 135

(*rac*)-4-{4-Cyano-2-[(1-methylethyl)sulfonyl]phenyl}-6-methyl-2-oxo-1-[3-(trifluormethyl)-phenyl]-1,2,3,4-tetrahydropyrimidin-5-carboxamid 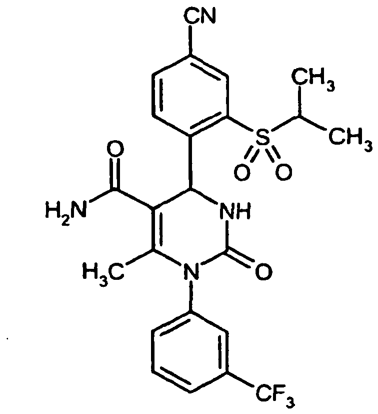 Die Reaktion wurde unter Argon durchgeführt. (*rac*)-4-{4-Cyano-2-[(1-methylethyl)sulfonyl]-phenyl}-6-methyl-2-oxo-1-[3-(trifluormethyl)phenyl]-1,2,3,4-tetrahydropyrimidin-5-carbonsäure (1000 mg, 1.97 mmol) und HATU (3 eq., 2248 mg, 5.9 mmol) wurden in trockenem DMF (25 ml) bei 0°C vorgelegt und nach kurzem Rühren (20 min) mit einer 0.5 M Ammoniak-Lösung in Dioxan (3 eq., 11.8 ml, 5.9 mmol) sowie DIEA (3 eq., 764 mg, 5.9 mmol) versetzt. Das Gemisch wurde 6 h bei RT gerührt (HPLC-Kontrolle) und anschließend im Vakuum eingeengt. Das Rohprodukt wurde mittels präparativer HPLC aufgereinigt (Säule: Gromsil C-18; Eluent: Acetonitril/ Wasser + 0.1 % TFA 10:90 → 80:20). Die Titelverbindung wurde als Feststoff erhalten (750 mg, 75% d. Th.).
LC-MS (Methode 5): Rₜ = 1.61 min; MS (ESIpos): *m*/*z* (%) = 507.2 (100) [M+H]⁺; MS (ESIneg): *m*/*z* (%) = 505.2 (100) [M-H]-
¹H-NMR (400 MHz, DMSO-d₆): δ = 1.07 (d, 3H), 1.35 (d, 3H), 1.80 (s, 3H), 3.85 (m, 1H), 6.20 (s, 1H), 7.05 (d, 1H), 7.30 (br. s, 1H), 7.45 (br. s, 1H), 7.65-7.80 (m, 4H), 8.15 (d, 1H), 8.25 (d, 1H), 8.35 (dd, 1H).

### Beispiel 136

(*rac*)-4-{4-Cyano-2-[(1-methylethyl)sulfonyl]phenyl}-6-methyl-2-oxo-1-[3-(trifluormethyl)-phenyl]-1,2,3,4-tetrahydropyrimidin-5-carbonitril 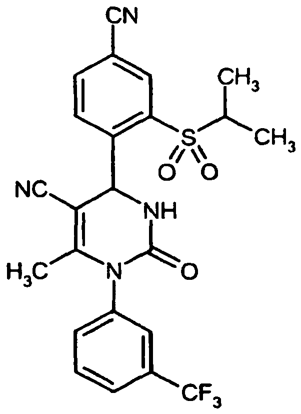 Die Reaktion wurde unter Argon durchgeführt. (*rac*)-4-{4-Cyano-2-[(1-methylethyl)sulfonyl]-phenyl}-6-methyl-2-oxo-1-[3-(trifluormethyl)phenyl]-1,2,3,4-tetrahydropyrimidin-5-carboxamid (730 mg, 1.44 mmol) wurde in trockenem THF (35 ml) vorgelegt, mit Methoxycarbonylsulfamoyl-triethylammoniumhydroxid (Burgess-Reagens; 687 mg, 2.88 mmol; 2 eq.) versetzt und bei RT gerührt. Die HPLC-Kontrolle zeigte nach 75 min vollständigen Umsatz. Das Reaktionsgemisch wurde danach mit Wasser (20 ml) versetzt, eingeengt und der Rückstand in Essigsäureethylester (200 ml) aufgenommen. Die organische Phase wurde mit gesättigter Kochsalz-Lösung (3 x 30 ml) gewaschen, über festem Natriumsulfat getrocknet, filtriert und eingeengt. Der Rückstand wurde an Kieselgel flash-chromatographiert (Laufmittel: Cyclohexan → Cyclohexan/Essigsäureethylester 1:1). Die Titelverbindung wurde als farbloser Feststoff erhalten (700 mg, 99% d. Th.).
LC-MS (Methode 4): Rₜ = 1.22 min; MS (ESIpos): *m*/*z* (%) = 489.1 (100) [M+H]⁺
¹H-NMR (400 MHz, DMSO-d₆): δ = 1.20 (d, 3H), 1.30 (d, 3H), 1.80 (s, 3H), 3.55 (m, 1H), 6.30 (s, 1H), 7.70-7.85 (m, 3H), 7.95 (br. s, 1H), 8.30-8.40 (m, 4H).

### Beispiel 137

(4*S*)-4-(4-Cyano-2-[(1-methylethyl)sulfonyl]phenyl}-6-methyl-2-oxo-1-[3-(trifluormethyl)phenyl]-1,2,3,4-tetrahydropyrimidin-5-carbonitril 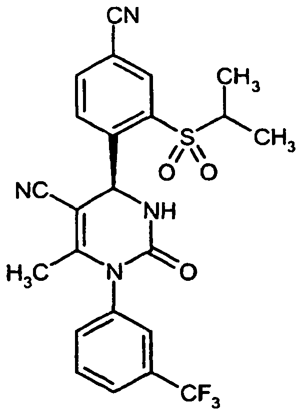 (*rac*)-4-{4-Cyano-2-[(1-methylethyl)sulfonyl]phenyl}-6-methyl-2-oxo-1-[3-(trifluormethyl)-phenyl]-1,2,3,4-tetrahydropyrimidin-5-carbonitril (0.70 g) wurde durch präparative HPLC-Chromatographie an chiraler Phase in die Enantiomere aufgetrennt [Säule: Daicel Chiralpak IC, 5 µm; Säulendimension: 250 mm x 20 mm; Probenvorbereitung: Lösung in Methanol/Acetonitril (1:6, 35 ml); Injektionsvolumen: 0.5 ml; Eluent: MTBE/Methanol 7:3; Fluss: 15 ml/min; Temperatur: 30°C; Detektion: 220 nm]. Es wurden 0.334 g (95% d. Th.; >98.5% ee) des 4S-Enantiomeren erhalten.
Der Enantiomerenüberschuss (ee-Wert) wurde chromatographisch bestimmt [Säule: Daicel Chiralpak IA, 5 µm; Säulendimension: 250 mm x 4.6 mm; Eluent: MTBE/Acetonitril 7:3; Fluss: 1 ml/min; Detektion: 220 nm; Rₜ = 3.46 min (4R-Enantiomer: Rₜ = 4.905 min)].
LC-MS (Methode 5): Rₜ = 1.97 min; MS (ESIpos): *m*/*z* (%) = 489.2 (100) [M+H]⁺; MS (ESIneg): *m*/*z* (%) = 444.2 (100), 487.2 (70) [M-H]⁻.
Für die ¹H-NMR-Daten siehe die racemische Verbindung (Beispiel 136).

### Beispiel 138

(rac)-4-[4-Cyano-2-(phenylsulfonyl)phenyl]-6-methyl-2-oxo-1-[3-(trifluormethyl)phenyl]-1,2,3,4-tetrahydropyrimidin-5-carbonsäure-allylester 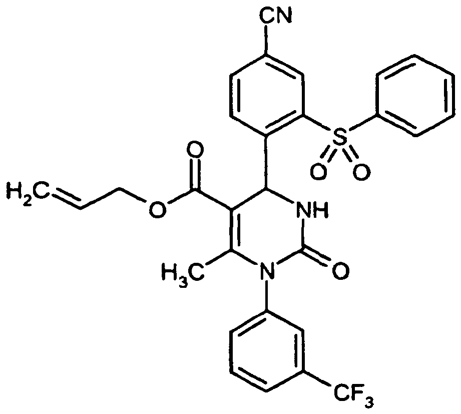 Die Reaktion wurde unter Argon durchgeführt. Phosphorsäuretriethylester (172 mg, 0.947 mmol) und Diphosphorpentoxid (90 mg) wurden über Nacht bei 40°C gerührt. Dann wurde mit MTBE (9.5 ml) verdünnt, und 4-Formyl-3-(phenylsulfonyl)benzonitril (214 mg, 0.789 mmol), 1-[3-(Trifluormethyl)phenyl]harnstoff (0.161 g, 0.789 mmol) sowie Allylacetoacetat (0.168 g, 1.18 mmol; 1.5 eq.) wurden hinzugefügt. Das Gemisch wurde 16 h unter Rückfluss gerührt. Die Reaktionsmischung wurde danach durch Abdestillieren von MTBE aufkonzentriert und anschließend für weitere 2 h auf 90°C erhitzt. Zur Aufarbeitung wurde restliches Lösungsmittel im Vakuum entfernt und der Rückstand in Essigsäureethylester (150ml) aufgenommen. Die organische Phase wurde mit gesättigter Kochsalz-Lösung (2 x 30 ml) gewaschen, über festem Natriumsulfat getrocknet, filtriert und im Vakuum eingeengt. Das Rohprodukt wurde mittels präparativer HPLC aufgereinigt (Säule: Gromsil C-18; Eluent: Acetonitril/Wasser + 0.1% TFA 10:90 → 90:10). Die Titelverbindung wurde als Feststoff erhalten (230 mg, 33% d. Th.).
LC-MS (Methode 4): Rₜ = 1.40 min; MS (ESIpos): *m*/*z* (%) = 582.1 (100) [M+H]⁺
¹H-NMR (400 MHz, DMSO-d₆): δ = 2.10 (s, 3H), 3.45 (m, 1H), 4.10 (m, 1H), 4.50 (m, 2H), 4.75 (d, 1H), 5.15 (m, 1H), 6.15 (d, 1H), 6.95 (d, 1H), 7.65-7.80 (m, 7H), 8.05 (br. d, 1H), 8.15 (d, 2H), 8.25 (dd, 1H), 8.60 (d, 1H).

### Beispiel 139

(*rac*)-4-{4-Cyano-2-[phenylsulfonyl]phenyl}-6-methyl-2-oxo-1-(3-(trifluormethyl)phenyl]-1,2,3,4-tetrahydropyrimidin-5-carboxamid 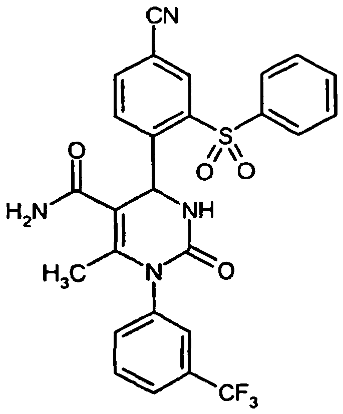 Die Reaktion wurde unter Argon durchgeführt. (*rac*)-4-(4-Cyano-2-[phenylsulfonyl]phenyl)-6-methyl-2-oxo-1-[3-(trifluormethyl)phenyl]-1,2,3,4-tetrahydropyrimidin-5-carbonsäure (166 mg, 0.307 mmol) und HATU (5 eq., 583 mg, 1.53 mmol) wurden in trockenem DMF (3.5 ml) bei 0°C vorgelegt und nach kurzem Rühren (20 min) mit Ammoniumchlorid (5 eq., 82 mg, 1.53 mmol) [alternativ: 1.23 ml einer 0.5 M Ammoniak-Lösung in Dioxan] sowie DIEA (10 eq., 369 mg, 3.07 mmol) versetzt. Das Gemisch wurde 6 h bei RT gerührt (HPLC-Kontrolle) und anschließend im Vakuum eingeengt. Das Rohprodukt wurde mittels präparativer HPLC aufgereinigt (Säule: Gromsil C-18; Eluent: AcetonitriVWasser + 0.1% TFA 10:90 → 90:10). Man erhielt die Titelverbindung als einen Feststoff (147 mg, 89% d. Th.).
LC-MS (Methode 6): Rₜ = 2.18 min; MS (ESIpos): *m*/*z* (%) = 541.0 (100) [M+H]⁺
¹H-NMR (400 MHz, DMSO-d₆): δ = 1.75 (s, 3H), 6.20 (s, 1H), 6.85 (d, 1H), 7.05 (br. s, 1H), 7.30 (br. s, 1H), 7.60-7.80 (m, 7H), 8.15 (m, 3H), 8.25 (d, 1H), 8.55 (s, 1H).

### Beispiel 140

(*rac*)-4-{4-Cyano-2-[phenylsulfonyl]phenyl}-6-methyl-2-oxo-1-[3-(trifluormethyl)phenyl]-1,2,3,4-tetrahydropyrimidin-5-carbonitril 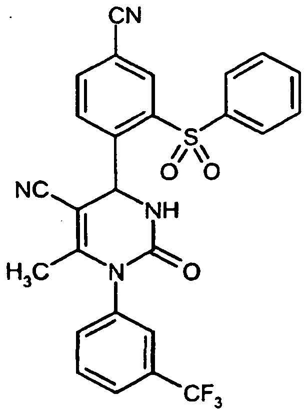 Die Reaktion wurde unter Argon durchgeführt. (*rac*)-4-{4-Cyano-2-[phenylsulfonyl]phenyl}-6-methyl-2-oxo-1-[3-(trifluormethyl)phenyl]-1,2,3,4-tetrahydropyrimidin-5-carboxamid (137 mg, 253 µmol) wurde in trockenem THF (8 ml) vorgelegt, mit Methoxycarbonylsulfamoyl-triethylammoniumhydroxid (Burgess-Reagens; 121 mg, 507 µmol; 2 eq.) versetzt und 8 h bei RT gerührt (HPLC-Kontrolle). Das Reaktionsgemisch wurde danach im Vakuum eingeengt und der Rückstand mittels präparativer HPLC aufgereinigt (Säule: Gromsil C-18; Eluent: Acetonitril/Wasser + 0.1 % TFA 10:90 → 90:10). Man erhielt die Titelverbindung als einen Feststoff (106 mg, 80% d. Th.).
LC-MS (Methode 5): Rₜ = 2.07 min; MS (ESIpos): *m*/*z* (%) = 523.1 (100) [M+H]⁺; MS (ESIneg): *m*/*z* (%) = 478.1 (100), 521.2 (20) [M-H]-
¹H-NMR (400 MHz, DMSO-d₆): δ = 1.75 (s, 3H), 6.35 (s, 1H), 7.65-8.35 (m, 12H), 8.60 (s, 1H).

### Beispiel 141

(4*S*)-4-{4-Cyano-2-[phenylsulfonyl]phenyl}-6-methyl-2-oxo-1-[3-(trifluormethyl)phenyl]-1,2,3,4-tetrahydropyrimidin-5-carbonitril 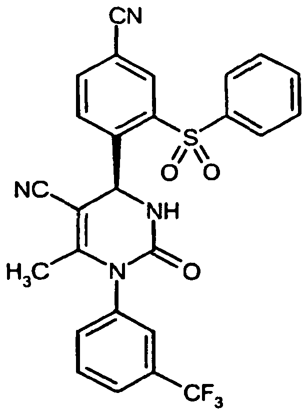 (*rac*)-4-{4-Cyano-2-[phenylsulfonyl)phenyl}-6-methyl-2-oxo-1-[3-(trifluormethyl)phenyl]-1,2,3,4-tetrahydropyrimidin-5-carbonitril (90 mg) wurde durch präparative HPLC-Chromatographie an chiraler Phase in die Enantiomere aufgetrennt [Säule: Daicel Chiralpak IC, 5 µm; Säulendimension: 250 mm x 20 mm; Probenvorbereitung: Lösung in Methanol/Acetonitril (1:1, 18 ml); Eluent: MTBE/Methanol 7:3; Fluss: 15 ml/min; Temperatur: 30°C; Detektion: 220 nm]. Es wurden 39 mg (87% d. Th.; >97.5% ee) des 4*S*-Enantiomeren erhalten.
Der Enantiomerenüberschuss (ee-Wert) wurde chromatographisch bestimmt [Säule: Daicel Chiralpak IA, 5 µm; Säulendimension: 250 mm x 4.6 mm; Eluent: MTBE/Methanol 7:3; Fluss: 1 ml/min; Detektion: 220 nm; Rₜ = 3.49 min (4*R*-Enantiomer: Rₜ = 5.95 min)].
LC-MS (Methode 6): Rₜ = 2.53 min; MS (ESIpos): *m*/*z* (%) = 523.2 (100) [M+H]⁺; MS (ESIneg): *m*/*z* (%) = 478.1 (100), 521.2 (20) [M-H]⁻.
Für die ¹H-NMR-Daten siehe die racemische Verbindung (Beispiel 140).

### Beispiel 142

2-Hydroxyethyl (4*S*)-4-[4-cyano-2-(methylsulfonyl)phenyl]-6-methyl-2-oxo-1-[3-(trifluormethyl)-phenyl]-1,2,3,4-tetrahydropyrimidin-5-carboxylat 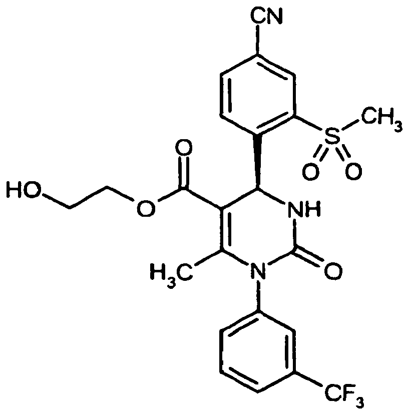 Die Reaktion wurde unter Argon durchgeführt. (4S)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure (170 mg, 355 µmol; Beispiel 5A) wurde in trockenem DMF (4 ml) vorgelegt, mit 2-Bromethanol (177 mg, 1.42 mmol; 4 eq.) sowie Triethylamin (72 mg, 709 µmol; 2 eq.) versetzt und 10 h bei 70°C gerührt. Das Reaktionsgemisch wurde danach im Vakuum eingeengt und der Rückstand in Essigsäureethylester (50 ml) aufgenommen. Die organische Phase wurde mit gesättigter Natriumhydrogencarbonat-Lösung (2 x 20 ml) und gesättigter Kochsalz-Lösung (20 ml) gewaschen, über festem Natriumsulfat getrocknet, filtriert und im Vakuum eingeengt. Das Rohprodukt wurde mittels präparativer HPLC aufgereinigt (Säule: Gromsil C-18; Eluent: Acetonitril/Wasser + 0.1% TFA 10:90 → 90:10). Man erhielt die Titelverbindung als einen Feststoff (175 mg, 94% d. Th.).
LC-MS (Methode 5): Rₜ = 1.74 min; MS (ESIpos): *m*/*z* (%) = 524.1 (100) [M+H]⁺; MS (ESIneg): *m*/*z* (%) = 522.2 (100) [M-H]⁻
¹H-NMR (400 MHz, DMSO-d₆): δ = 2.15 (s, 3H), 3.35 (m, 2H), 3.50 (s, 3H), 3.90 (m, 1H), 4.00 (m, 1H), 4.65 (br. s, 1H), 6.30 (s, 1H), 7.10 (d, 1H), 7.70-7.85 (m, 4H), 8.10 (br. d, 1H), 8.25 (dd, 1H), 8.35 (d, 1H).

### Beispiel 143

(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonsäure-allylester 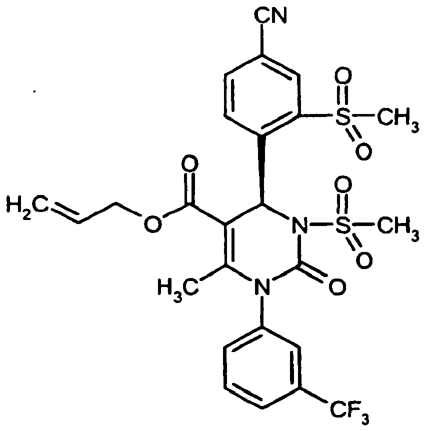 Die Reaktion wurde unter Argon durchgeführt. (4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonsäure-allylester (260 mg, 500 µmol) wurde in trockenem THF (10 ml) vorgelegt und bei 0°C mit Natriumhydrid (60% in Mineralöl; 28 mg, 700 µmol; 1.4 eq.) versetzt. Es wurde auf RT erwärmt und 20 min nachgerührt. Anschließend wurde eine Lösung von Methansulfonylchlorid (80 mg, 700 µmol; 1.4 eq.) in THF (2 ml) langsam zugetropft. Nach 20 h Reaktionszeit wurde das Reaktionsgemisch eingeengt und der Rückstand an Kieselgel flash-chromatographiert (Laufmittel: Cyclohexan → Cyclohexan/Essigsäureethylester 55:45). Die Titelverbindung wurde als farbloser Feststoff erhalten (288 mg, 96% d. Th.).
LC-MS (Methode 5): Rₜ = 2.24 min; MS (ESIpos): *m*/*z* (%) = 540.1 (100), 598.1 (40) [M+H]⁺; MS (ESIneg): *m*/*z* (%) = 475.1 (80), 596.1 (100) [M-H]⁻.

### Beispiel 144

2-Hydroxyethyl (4*S*)-4-[4-cyano-2-(methylsulfonyl)phenyl]-6-methyl-3-(methylsulfonyl)-2-oxo-1 - [3-(trifluormethyl)phenyl]-1,2,3,4-tetrahydropyrimidin-5-carboxylat 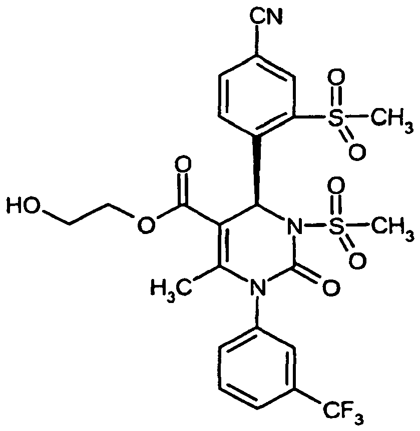 Die Reaktion wurde unter Argon durchgeführt. (4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonsäure (84 mg, 150 µmol) wurde in trockenem DMF (2 ml) vorgelegt, mit 2-Bromethanol (75 mg, 600 µmol; 4 eq.) sowie Triethylamin (31 mg, 300 µmol; 2 eq.) versetzt und 8 h bei 70°C gerührt. Das Reaktionsgemisch wurde danach im Vakuum eingeengt und der Rückstand in Essigsäureethylester (50 ml) aufgenommen. Die organische Phase wurde mit gesättigter Natriumhydrogencarbonat-Lösung (2 x 20 ml) und gesättigter Kochsalz-Lösung (20 ml) gewaschen, über festem Natriumsulfat getrocknet, filtriert und im Vakuum eingeengt. Das Rohprodukt wurde an Kieselgel flash-chromatographiert (Laufmittel: Cyclohexan → Cyclohexan/Essigsäureethylester 1:3). Die Titelverbindung wurde als farbloser Feststoff erhalten (56 mg, 62% d. Th.).
LC-MS (Methode 4): Rₜ = 1.13 min; MS (ESIpos): *m*/*z* (%) = 539.9 (100), 602 (20) [M+H]⁺; MS (ESIneg): *m*/*z* (%) = 479.0 (100), 600.0 (80) [M-H]⁻
¹H-NMR (400 MHz, DMSO-d₆): δ = 1.75 (s, 3H), 3.40 (s, 3H), 3.50 (m, 2H), 4.05 (m, 2H), 4.65 (t, 1H), 7.30 (s, 1H), 7.75-7.90 (m, 3H), 8.05 (m, 2H), 8.25 (dd, 1H), 8.50 (d, 1H) [ein Methylsignal vom Lösungsmittel-Peak überlagert].

### Beispiel 145

(4*S*)-4-[4-Cyano-2-(methylsulfonyl)phenyl]-3,6-dimethyl-2-oxo-1-[3-(trifluonnethyl)phenyl]-1,2,3,4-tetrahydropyrimidin-5-carbonsäure-allylester 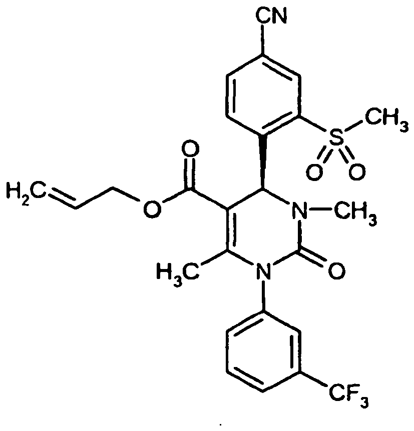 Die Reaktion wurde unter Argon durchgeführt. (4*S*)-4-[4-Cyano-2-(methylsulfonyl)phenyl]-6-methyl-2-oxo-1-[3-(trifluormethyl)phenyl)-1,2,3,4-tetrahydropyrimidin-5-carbonsäure-allylester (260 mg, 0.5 mmol) wurde in absolutem THF (10 ml) vorgelegt und bei -78°C mit einer 1M Lösung von Lithiumhexamethyldisilazid (LiHMDS) in THF (0.6 ml, 600 µmol; 1.2 eq.) versetzt. Nach 20 min Rühren wurde Iodmethan (355 mg, 2.5 mmol; 5 eq.) zugegeben und die Mischung für 16 h unter allmählicher Erwärmung von -78°C auf RT gerührt. Das Reaktionsgemisch wurde dann im Vakuum eingeengt und der Rückstand an Kieselgel flash-chromatographiert (Laufmittel: Cyclohexan → CyclohexanlEssigsäureethylester 55:45). Die Titelverbindung wurde als farbloser Feststoff erhalten (157 mg, 59% d. Th.).
LC-MS (Methode 5): Rₜ = 2.30 min; MS (ESIpos): *m*/*z* (%) = 534.1 (100) [M+H]⁺; MS (ESIneg): *m*/*z* (%) = 532.2 (100) [M-H]⁻.

### Beispiel 146

2-Hydroxyethyl (4*S*)-4-[4-cyano-2-(methylsulfonyl)phenyl]-3,6-dimethyl-2-oxo-1-[3-(trifluormethyl)phenyl]-1,2,3,4-tetrahydropyrimidin-5-carboxylat 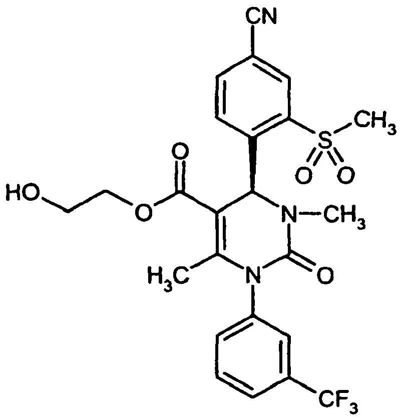 Die Reaktion wurde unter Argon durchgeführt. (4S)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonsäure (74 mg, 150 µmol) wurde in trockenem DMF (2 ml) vorgelegt, mit 2-Bromethanol (75 mg, 600 µmol; 4 eq.) sowie Triethylamin (31 mg, 300 µmol; 2 eq.) versetzt und 8 h bei 70°C gerührt. Das Reaktionsgemisch wurde danach im Vakuum eingeengt und der Rückstand in Essigsäureethylester (50 ml) aufgenommen. Die organische Phase wurde mit gesättigter Natriumhydrogencarbonat-Lösung (2 x 20 ml) und gesättigter Kochsalz-Lösung (20 ml) gewaschen, über festem Natriumsulfat getrocknet, filtriert und im Vakuum eingeengt. Das Rohprodukt wurde an Kieselgel flash-chromatographiert (Laufmittel: Cyclohexan → Cyclohexan/Essigsäureethylester 1:3). Die produkthaltigen Fraktionen wurden vereinigt, eingeengt, der Rückstand in wenig Acetonitril aufgenommen, mit Wasser versetzt und lyophilisiert. Die Titelverbindung wurde als farbloser Feststoff erhalten (63 mg, 78% d. Th.).
LC-MS (Methode 4): Rₜ = 1.13 min; MS (ESIpos): *m*/*z* (%) = 538.1 (100) [M+H]⁺; MS (ESIneg): *m*/*z* (%) = 536.2 (100) [M-H]-
¹H-NMR (400 MHz, DMSO-d₆): δ = 2.05 (s, 3H), 2.75 (s, 3H), 3.50 (m, 2H), 3.55 (s, 3H), 4.05 (m, 2H), 4.70 (t, 1H), 6.75 (s, 1H), 7.70-7.85 (m, 3H), 7.95 (br. s, 1H), 8.15 (br. d, 1H), 8.25 (dd, 1H), 8.50 (d, 1H).

### Beispiel 147

(4*S*)-4-{4-Cyano-2-[melhylsulfinyl]phenyl}-6-methyl-3-(methylsulfonyl)-2-oxo-1-[3-(trifluormethyl)phenyl]-1,2,3,4-tetrahydropyrimidin-5-carbonitril 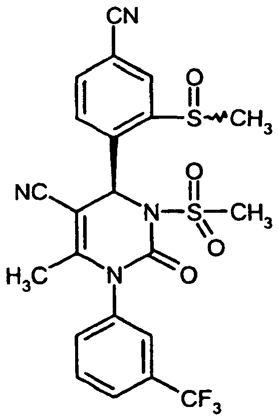 Die Reaktion wurde unter Argon durchgeführt. (4*S*)-4-{4-Cyano-2-[methylsulfinyl]phenyl}-6-methyl-2-oxo-1-[3-(trifluormethyl)phenyl]-1,2,3,4-tetrahydropyrimidin-5-carbonitril (444 mg, 1 mmol) wurde in trockenem THF (10 ml) vorgelegt und bei 0°C mit Natriumhydrid (60% in Mineralöl; 56 mg, 1.4 mmol; 1.4 eq.) versetzt. Es wurde auf RT erwärmt und 20 min nachgerührt. Anschließend wurde eine Lösung von Methansulfonylchlorid (160 mg, 1.4 mmol; 1.4 eq.) in THF (5 ml) langsam zugetropft. Nach 16 h Reaktionszeit wurde das Reaktionsgemisch mit Ammoniumchlorid-Lösung (50 ml) versetzt und das Gemisch mit Essigsäureethylester (3 x 30 ml) extrahiert. Die vereinigten organischen Phasen wurden über Natriumsulfat getrocknet, filtriert und im Vakuum eingeengt. Der Rückstand wurde mittels präparativer HPLC aufgereinigt (Säule: Gromsil C-18; Eluent: Acetonitril/Wasser + 0.1 % TFA 10:90 → 80:20). Man erhielt die Titelverbindung als einen Feststoff (210 mg, 40% d. Th.).
LC-MS (Methode 4): Rₜ= 1.15 min; MS (ESIpos): *m*/*z* (%) = 523.0 (100) [M+H]⁺; MS (ESIneg): *m*/*z* (%) = 441.1 (100), 521.0 (100) [M-H]-
¹H-NMR (400 MHz, DMSO-d₆): δ = 1.80 (s, 3H), 2.70 (s, 3H), 3.30 (s, 3H), 6.75 (s, 1H), 7.70-8.32 (m, 6H), 8.50 (s, 1H).

### Beispiel 148

(4*S*)-4-(4-Cyano-2-[(cyclobutylmethyl)sulfonyl]phenyl)-3,6-dimethyl-2-oxo-1-[3-(trifluormethyl)-phenyl]-1,2,3,4-tetrahydropyrimidin-5-carbonitril 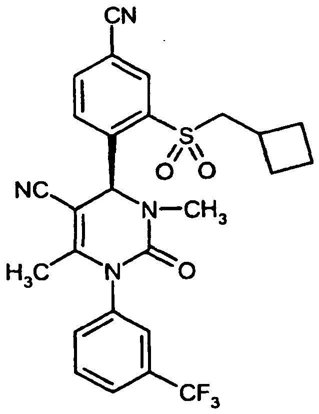 Die Reaktion wurde unter Argon durchgeführt. Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat (150 mg, 101 µmol; 32% Reinheit) wurde in DMF (1 ml) suspendiert. Anschließend wurde 1-(Brommethyl)-cyclobutan (164.1 mg) hinzugegeben und das Gemisch in einem geschlossenen Rohr für 72 h auf 110°C erhitzt. Der Ansatz wurde danach filtriert und das Filtrat im Vakuum eingeengt. Der Rückstand wurde mittels präparativer HPLC gereinigt (Säule: Gromsil C-18 10 µm; Eluent: Acetonitril/ Wasser + 0.1 % TFA 10:90 → 90:10). Die Titelverbindung wurde als Feststoff erhalten (40.2 mg, 75% d. Th.).
LC-MS (Methode 4): Rₜ = 1.42 min; MS (ESIpos): *m*/*z* (%) = 529.3 (100) [M+H]⁺; MS (ESIneg): *m*/*z* (%) = 527.3 (100) [M-H]⁻
¹H-NMR (400 MHz, DMSO-d₆): δ = 1.76-2.10 (m, 9H), 2.65 (s, 3H), 2.70 (m, 1H), 3.65 (dd, 1H), 3.75 (dd, 1H), 6.45 (s, 1H), 7.70-8.40 (m, 7H).

### Beispiel 149

(4*S*)-4-{4-Cyano-2-[(cyclopropylmethyl)sulfonyl]phenyl}-3,6-dimethyl-2-oxo-1-[3-(trifluormethyl)phenyl]-1,2,3,4-tetrahydropyrimidin-5-carbonitril 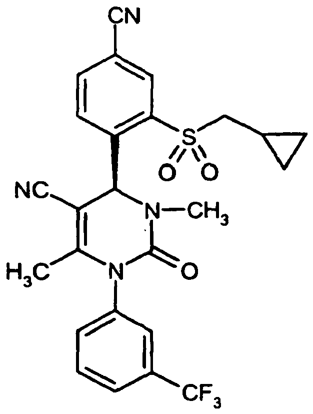 Die Reaktion wurde unter Argon durchgeführt. Natrium 5-cyano-2-{(4S)-5-cyano-3,6-dimethyl-2-oxo-1-[3-(trifluormethyl)phenyl]-1,2,3,4-tetrahydropyrimidin-4-yl}benzolsulfinat (100 mg, 67 µmol; 32% Reinheit) wurde in DMF (2000 µl) suspendiert. Anschließend wurde 1-(Brommethyl)cyclopropan (278 mg, 2.06 mmol) hinzugegeben und das Gemisch über Nacht in einem geschlossenen Rohr auf 130°C erhitzt. Die Reaktionsmischung wurde danach mit Molekularsieb (4 Å), Kaliumiodid (110 mg, 0.66 mmol) und weiterem 1-(Brommethyl)cyclopropan (89 mg, 0.66 mmol) versetzt und erneut für 6 h in einem geschlossenen Rohr auf 100°C erhitzt. Der Ansatz wurde dann filtriert und das Filtrat im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90:10). Die Titelverbindung wurde als Feststoff erhalten (16.2 mg, 44% d. Th.).
LC-MS (Methode 9): Rₜ = 1.16 min; MS (ESIpos): *m*/*z* (%) = 515.2 (100) [M+H]⁺; MS (ESIneg): *m*/*z* (%) = 513.2 (80) [M-H]⁻
¹H-NMR (400 MHz, DMSO-d₆): δ = 0.20 (m, 1H), 0.30 (m, 1H), 0.50 (m, 1H), 0.60 (m, 1H), 1.00 (m, 1H), 1.80 (s, 3H), 2.70 (s, 3H), 3.45 (dd, 1H), 3.60 (dd, 1H), 6.50 (s, 1H), 7.75-8.45 (m, 7H).

### Beispiel 150

(4*S*)-4-{4-Cyano-2-[(3,3,3-trifluorpropyl)sulfonyl]phenyl}-3,6-dimethyl-2-oxo-1-[3-(trifluormethyl)phenyl]-1,2,3,4-tetrahydropyrimidin-5-carbonitril 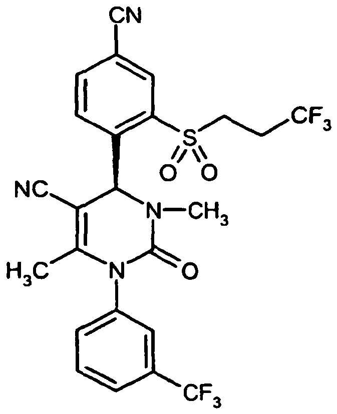 Die Reaktion wurde unter Argon durchgeführt. Natrium 5-cyano-2-{(4S)-5-cyano-3,6-dimethyl-2-oxo-1-[3-(trifluormethyl)phenyl]-1,2,3,4-tetrahydropyrimidin-4-yl}benzolsulfinat (66 mg, 44 µmol; 32% Reinheit) wurde in DMF (600 µl) suspendiert. Anschließend wurde 1,1,1-Trifluor-3-iodpropan (144 mg, 643 µmol; 14.6 eq.) hinzugegeben und das Gemisch über Nacht in einem geschlossenen Rohr auf 120°C erhitzt. Der Ansatz wurde danach filtriert und das Filtrat im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90:10). Die Titelverbindung wurde als Feststoff erhalten (7.4 mg, 30% d. Th.).
LC-MS (Methode 9): Rₜ = 1.18 min; MS (ESIpos): *m*/*z* (%) = 557.2 (100) [M+H]⁺; MS (ESIneg): *m*/*z* (%) = 555.2 (80) [M-H]⁻
¹H-NMR (400 MHz, DMSO-d₆): δ = 1.80 (s, 3H), 2.70 (s, 3H), 2.80 (m, 2H), 3.90 (t, 2H), 6.45 (s, 1H), 7.65-8.40 (br. m, 5H), 8.45 (m, 1H), 8.55 (s, 1H).

### Beispiel 151

(4*S*)-4-{4-Cyano-2-[(trifluormethyl)sulfonyl]phenyl}-3,6-dimethyl-2-oxo-1-[3-(trifluormethyl)-phenyl]-1,2,3,4-tetrahydropyrimidin-5-carbonitril

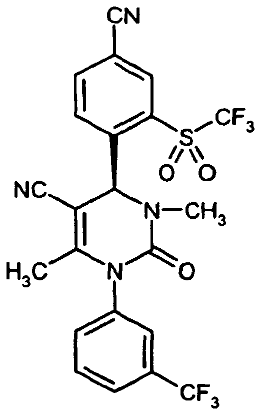

Die Reaktion wurde unter Argon durchgeführt. Tris(dimethylamino)sulfonium-difluortrimethylsilicat (TASF; 18.4 mg, 66.7 µmol; 1.1 eq.) und (Trifluormethyl)trimethylsilan (2 M Lösung in THF, 60.6 µl, 121 µmol) wurden bei 0°C in THF (1.45 ml) vorgelegt. 5-Cyano-2-{(4*S*)-5-cyano-3,6-dimethyl-2-oxo-1-[3-(trifluormethyl)phenyl]-1,2,3,4-tetrahydropyrimidin-4-yl}benzolsulfonylchlorid (30 mg, 60.6 µmol) wurde zugegeben, und die Mischung wurde über Nacht bei RT gerührt. Danach wurden nochmals bei 0°C Tris(dimethylamino)sulfonium-difluortrimethylsilicat (TASF; 16.7 mg, 60.6 µmol; 1.0 eq.) sowie (Trifluormethyl)trimethylsilan (2 M Lösung in THF, 65 µl, 130 µmol) zugesetzt, und die Mischung wurde erneut für 15 h unter allmählicher Erwärmung auf RT gerührt. Der Ansatz wurde dann filtriert und das Filtrat im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90: 10). Die Titelverbindung wurde als Feststoff erhalten (3.3 mg, 10% d. Th.).
LC-MS (Methode 9): Rₜ = 1.19 min; MS (ESIpos): *m*/*z* (%) = 529.2 (100) [M+H]⁺; MS (ESIneg): *m*/*z* (%) = 527.1 (80) [M-H]⁻.
¹H-NMR (400 MHz, DMSO-d₆): δ = 1.80 (s, 3H), 2.65 (s, 3H), 6.25 (s, 1H), 7.75-8.55 (m, 5H), 8.65 (dd, 1H), 8.80 (s, 1H).

### Beispiel 152

(4*S*)-4-[2-(Benzylsulfonyl)-4-cyanophenyl]-3,6-dimethyl-2-oxo-1-[3-(trifluormethyl)phenyl)-1,2,3,4-tetrahydropyrimidin-5-carbonitril 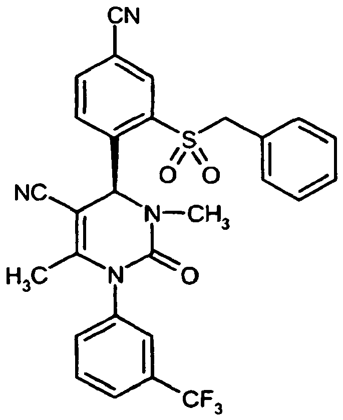 Die Reaktion wurde unter Argon durchgeführt. Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat (150 mg, 101 µmol; 32% Reinheit) wurde in DMF (1 ml) suspendiert. Anschließend wurde Benzylbromid (173 mg, 1.01 mmol; 10 eq.) hinzugegeben und das Gemisch für 24 h in einem geschlossenen Rohr auf 110°C erhitzt. Die Reaktionsmischung wurde danach mit Molekularsieb (4 Å), Kaliumiodid (110 mg, 0.66 mmol) und weiterem Benzylbromid (173 mg, 1.01 mmol; 10 eq.) versetzt und erneut für 6 h in einem geschlossenen Rohr auf 100°C erhitzt. Der Ansatz wurde dann filtriert und das Filtrat im Vakuum eingeengt. Der Rückstand wurde mittels präparativer HPLC gereinigt (Säule: Gromsil C-18 10 µm, Eluent: Acetonitril/Wasser + 0.1% TFA 10:90 → 90:10). Die Titelverbindung wurde als Feststoff erhalten (49.3 mg, 89% d. Th.).
LC-MS (Methode 9): Rₜ = 1.22 min; MS (ESIpos): *m*/*z* (%) = 551.3 (100) [M+H]⁺; MS (ESIneg): *m*/*z* (%) = 549.2 (100) [M-H]⁻
¹H-NMR (400 MHz, DMSO-d₆): δ = 1.80 (s, 3H), 2.45 (s, 3H), 4.90 (q, 2H), 6.55 (s, 1H), 7.30-7.40 (m, 5H), 7.70-8.40 (m, 7H).

### Beispiel 153

(4*S*)-4-{4-Cyano-2-[(2-methoxyethyl)sulfonyl]phenyl}-3,6-dimethyl-2-oxo-1-[3-(trifluormethyl)-phenyl]-1,2,3,4-tetrahydropyrimidin-5-carbonitril

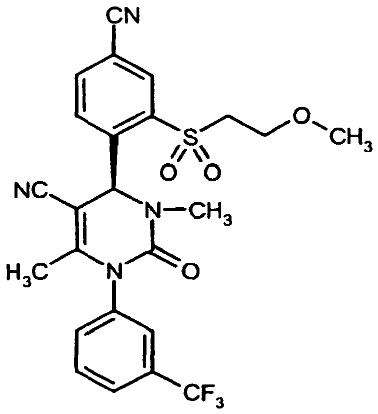

Die Reaktion wurde unter Argon durchgeführt. Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat (100 mg, 73 µmol; 32% Reinheit) wurde in DMF (0.5 ml) suspendiert. Anschließend wurde 2-Bromethylmethylether (148 mg, 1.06 mmol) hinzugegeben und das Gemisch für 15 h in einem geschlossenen Rohr auf 115°C erhitzt. Der Ansatz wurde danach filtriert und das Filtrat im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90:10). Die Titelverbindung wurde als Feststoff erhalten (27.3 mg, 73% d. Th.).
LC-MS (Methode 9): Rₜ= 1.11 min; MS (ESIpos): *m*/*z* (%) = 519.2 (100) [M+H]⁺; MS (ESIneg): *m*/*z* (%) = 517.2 (100) [M-H]⁻
¹H-NMR (400 MHz, DMSO-d₆): δ = 1.80 (s, 3H), 2.70 (s, 3H), 3.15 (s, 3H), 3.70-3.87 (m, 4H), 6.45 (s, 1H), 7.30-8.40 (m, 6H), 8.45 (m, 1H).

### Beispiel 154

(4*S*)-4-{4-Cyano-2-[(1,3-thiazol-4-ylmethyl)sulfonyl]phenyl}-3,6-dimethyl-2-oxo-1-[3-(trifluormethyl)phenyl]-1,2,3,4-tetrahydropyrimidin-5-carbonitril 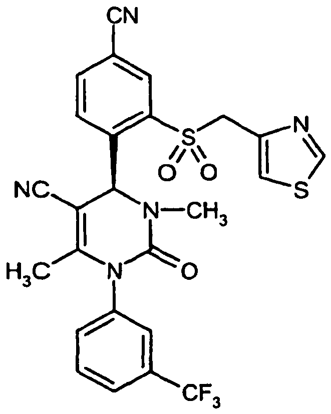 Die Reaktion wurde unter Argon durchgeführt. 4-(Chlormethyl)thiazol-Hydrochlorid (86 mg, 505 µmol; 5.0 eq.), Natrium 5-cyano-2-{(4*S*)-5-cyano-3,6-dimethyl-2-oxo-1-[3-(trifluormethyl)-phenyl]-1,2,3,4-tetrahydropyrimidin-4-yl)benzolsulfinat (150 mg, 101 µmol; 32% Reinheit), Kaliumcarbonat (77 mg, 556 µmol; 5.5 eq.), Kaliumiodid (84 mg, 505 µmol; 5.0 eq.) sowie 18-Krone-6 (13.4 mg, 51 µmol; 0.5 eq.) wurden in DMF (3 ml) suspendiert. Anschließend wurde das Gemisch für 15 h in einem geschlossenen Rohr auf 110°C erhitzt. Die Reaktionsmischung wurde danach mit Molekularsieb (4 Å), Kaliumiodid (84 mg, 505 µmol; 5.0 eq.) und weiterem 4-(Chlormethyl)thiazol-Hydrochlorid (86 mg, 505 µmol; 5.0 eq.) versetzt und erneut für 2 h in einem geschlossenen Rohr auf 100°C erhitzt. Der Ansatz wurde dann filtriert und das Filtrat im Vakuum eingeengt. Der Rückstand wurde mittels präparativer HPLC gereinigt (Säule: Gromsil C-18 10 µm; Eluent: Acetonitril/Wasser + 0.1% TFA 10:90 → 90:10). Die Titelverbindung wurde als Feststoff erhalten (37 mg, 66% d. Th.).
LC-MS (Methode 4): Rₜ = 1.25 min; MS (ESIpos): *m*/*z* (%) = 558.1 (100) [M+H]⁺; MS (ESIneg): *m*/*z* (%) = 492.3 (100), 557.0 (100) [M-H]⁻
¹H-NMR (400 MHz, DMSO-d₆): δ = 1.80 (s, 3H), 2.60 (s, 3H), 5.15 (m, 2H), 6.50 (s, 1H), 7.70-8.40 (br. m, 8H), 9.0 (s, 1H).

### Beispiel 155

(4*S*)-4-[2-(Cyclobutylsulfonyl)-4-cyanophenyl]-3,6-dimethyl-2-oxo-1-[3-(trifluormethyl)phenyl]-1,2,3,4-tetrahydropyrimidin-5-carbonitril

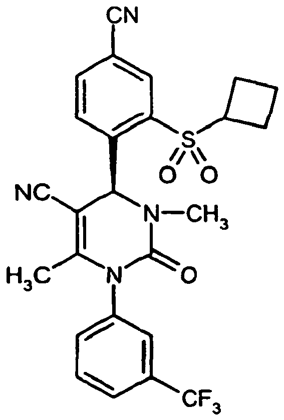

Natrium 5-cyano-2-{(4*S*)-5-cyano-3,6-dimethyl-2-oxo-1-[3-(trifluormethyl)phenyl]-1,2,3,4-tetrahydropyrimidin-4-yl}benzolsulfinat (150 mg, 65 µmol; ca. 21% Reinheit) wurde unter Argon in einem druckfesten Glasrohr in DMF (1 ml) suspendiert. Molekularsieb (4 Å, 20 mg) und Bromcyclobutan (100 µl, 1088 µmol, 16.7 eq.) wurden zugegeben. Das versiegelte Rohr wurde für 15 h auf 115°C erhitzt. Die Reaktionsmischung wurde danach filtriert und das Filtrat im Vakuum eingeengt. Der Rückstand wurde mittels präparativer HPLC gereinigt (Gromsil C18-Säule; Eluent: Acetonitril/Wasser + 0.1% TFA). Nach Lyophilisation erhielt man die Titelverbindung als Feststoff (15.5 mg, 90% Reinheit nach LC-MS, 41% d. Th.).

LC-MS (Methode 9): Rₜ = 1.20 min; MS (ESIpos): m/z (%) = 515.3 (100) [M+H]⁺; MS (ESIneg): *m*/*z* (%) = 513.2 (100) [M-H]⁻.

### Beispiel 156

(4*S*)-4-{4-Cyano-2-[(trifluormethyl)sulfanyl]phenyl}-3,6-dimethyl-2-oxo-1-[3-(trifluormethyl)-phenyl]-1,2,3,4-tetrahydropyrimidin-5-carbonitril 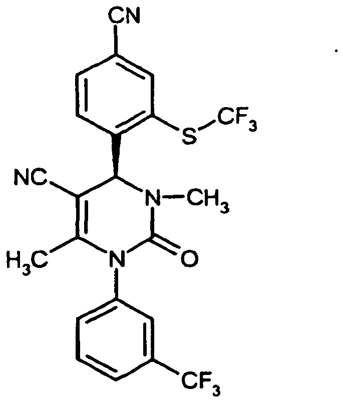 (4*S*)-4-(4-Cyano-2-sulfanylphenyl)-3,6-dimethyl-2-oxo-1-[3-(trifluormethyl)phenyl]-1,2,3,4-tetrahydropyrimidin-5-carbonitril (45 mg, 50% Reinheit, 53 µmol) wurde unter Argon in Dichlormethan (1 ml) gelöst. Bei -78°C wurde 3,3-Dimethyl-1-(trifluormethyl)-1,3-dihydro-1λ³,2-benziodoxol (26 mg, 79 µmol; 1.5 eq.) zugegeben und das Gemisch 2 h lang gerührt. Die Reaktionslösung wurde danach im Vakuum eingeengt und der Rückstand mittels präparativer HPLC gereinigt (Kromasil C18-Säule, 20 x 50 mm; Eluent: Acetonitril/Wasser + 0.1% TFA). Nach Lyophilisation wurde die Titelverbindung als Feststoff erhalten (11.4 mg, 44% d. Th.).
LC-MS (Methode 9): Rₜ = 1.24 min; MS (ESIpos): *m*/*z* (%) = 497.3 (100) [M+H]⁺; MS (ESIneg): *m*/*z* (%) = 495.2 (100) [M-H]⁻
¹H-NMR (400 MHz, DMSO-d₆): δ = 1.75 (s, 3H), 2.70 (s, 3H), 6.00 (s, 1H), 7.75 (m, 2H), 7.85 (m, 2H), 8.05 (br. s, 1H), 8.25 (m, 1H), 8.45 (s, 1H).

### B. Bewertung der pharmakologischen Wirksamkeit

Die pharmakologische Wirkung der erfindungsgemäßen Verbindungen kann in den nachstehend beschriebenen Assays gezeigt werden:

### Abkürzungen:

- AMC: 7-Amido-4-methylcumarin
- BNP: brain natriuretic peptide
- BSA: bovine serum albumin
- HEPES: *N*-(2-Hydroxyethyl)piperazin-*N*'-2-ethansulfonsäure
- HNE: humane neutrophile Elastase
- IC: Inhibitionskonzentration
- MeOSuc: Methoxysuccinyl
- NADP: Nikotinamid-Adenin-Dinukleotid-Phosphat
- v/v: Volumen zu Volumen-Verhältnis (einer Lösung)
- w/v: Gewicht zu Volumen-Verhältnis (einer Lösung)

### B-1. In vitro HNE-Inhibitionstest

Die Wirkstärke der erfindungsgemäßen Verbindungen wird in einem *in vitro*-Hemmtest ermittelt. Die HNE-vermittelte amidolytische Spaltung eines geeigneten Peptidsubstrates führt hierbei zu einer Fluoreszenzlichtzunahme. Die Signalintensität des Fluoreszenzlichtes ist direkt proportional zur Enzymaktivität. Die Wirkkonzentration einer Testverbindung, bei der die Hälfte des Enzyms inhibiert ist (50% Signalintensität des Fluoreszenzlichtes), wird als IC₅₀-Wert angegeben.

### Ausführung:

In einer 384 Loch-Mikrotiterplatte werden in einem Testvolumen von insgesamt 50 µl Testpuffer (0.1 M HEPES pH 7.4, 0.5 M NaCl, 0.1% w/v BSA, 1% v/v DMSO), Enzym (80 pM HNE; Fa. Serva, Heidelberg) und Substrat (20 µM MeOSuc-Ala-Ala-Pro-Val-AMC; Fa. Bachem, Weil am Rhein) bei An- und Abwesenheit der Testsubstanz zwei Stunden bei 37°C inkubiert. Die Fluoreszenzlichtintensität der Testansätze wird gemessen (Ex. 380 nm, Em. 460 nm). Die IC₅₀-Werte werden durch eine Auftragung der Fluoreszenzlichtintensität gegenüber der Wirkstoffkonzentration ermittelt.

Für die erfindungsgemäßen Verbindungen repräsentative IC₅₀-Werte (bei einer HNE-Konzentration von 80 pM) sind in der folgenden Tabelle A wiedergegeben:

**Tabelle A: Hemmung der humanen neutrophilen Elastase (HNE)**

| **Ausführungsbeispiel Nr.** | **IC₅₀ [nM]** |
|---|---|
| 2 | 10.0 |
| 6 | 0.5 |
| 10 | < 0.3 |
| 12 | < 0.3 |
| 14 | < 0.3 |
| 18 | < 0.3 |
| 20 | 1.9 |
| 22 | 0.45 |
| 27 | < 0.3 |
| 32 | < 0.3 |
| 33 | < 0.3 |
| 36 | < 0.3 |
| 41 | < 0.3 |
| 43 *(Diastereomer 1)* | 0.3 |
| 50 | 1.6 |
| 51 | < 0.3 |
| 55 | < 0.3 |
| 69 | 0.9 |
| 80 | < 0.3 |
| 85 | < 0.3 |
| 91 | < 0.3 |
| 96 | < 0.3 |
| 103 | < 0.3 |
| 116 | < 0.3 |
| 120 | < 0.3 |
| 128 | < 0.3 |
| 132 | 1.1 |
| 141 | < 0.3 |
| 144 | < 0.3 |

### B-2. Tiermodell der pulmonalen arteriellen Hypertonie

Die Monocrotalin-induzierte pulmonale Hypertonie der Ratte ist ein weit verbreitetes Tiermodell für die pulmonale arterielle Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de novo*-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere sowie eine Ermittlung der arteriellen und zentralvenösen Sauerstoffsättigung. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver endexspiratorischer Druck: 1 cm H₂O; Atemzugvolumen: 10 ml/kg Körpergewicht; FIO₂: 0.5). Die Narkose wird durch Isofluran-Inhalationsnarkose aufrechterhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldruckes. Das Herzminutenvolumen wird mittels Thermodilution ermittelt. Im Anschluß an die Hämodynamik wird das Herz entnommen und das Verhältnis rechter zu linker Ventrikel inklusive Septum bestimmt. Weiterhin werden Plasmaproben zur Bestimmung von Biomarkern (zum Beispiel proBNP) und Plasma-Substanzspiegeln gewonnen.

### B-3. Tiermodell des akuten Lungenversagens

Elastase-induziertes Lungenversagen an Maus, Ratte oder Hamster ist ein weit verbreitetes Tiermodell für akutes Lungenversagen (auch: "acute lung injury", "acute respiratory distress syndrome") [Tremblay et al., Chest 121, 582-588 (2002); Kuraki et al., Am. J. Resp. Crit. Care Med. 166, 596-500 (2002)]. Die Behandlung der Tiere erfolgt 1 Stunde vor orotrachealer Instillation der humanen neutrophilen Elastase (HNE). 2 Stunden nach der orotrachealen HNE-Instillation wird eine bronchoalveolare Lavage durchgeführt und der Hämoglobingehalt sowie das Differentialzellbild in der Lavage bestimmt.

### B-4. Tiermodell des Lungenemphysems

Elastase-induziertes Lungenemphysem an Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird die Lungen-Compliance bestimmt und eine Alveolarmorphometrie durchgeführt.

### B-5. CYP-Inhibitionstest

Die Fähigkeit von Substanzen, CYP1A2, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-spezifische Metaboliten bilden. Die Inhibitionseffekte werden bei sechs verschiedenen Konzentrationen der Testverbindungen untersucht [2.8, 5.6, 8.3, 16.7, 20 (oder 25) sowie 50 µM], mit dem Ausmaß der CYP-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, wird immer mit inkubiert, um Ergebnisse zwischen verschiedenen Serien vergleichbar zu machen.

### Durchführung:

Die Inkubation von Phenacetin, Diclofenac, Tolbutamid, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.3 mM NADP, 3.3 mM MgCl₂ x 6 H₂O. 3.3 mM Glukose-6-phosphat, Glukose-6-phosphat-Dehydrogenase (0.4 U/ml) und 100 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

### B-6. Hepatozytenassay zur Bestimmung der metabolischen Stabilität

Die metabolische Stabilität von Testverbindungen gegenüber Hepatozyten wird bestimmt, indem die Verbindungen bei niedrigen Konzentrationen (bevorzugt unter oder um 1 µM) und bei niedrigen Zellzahlen (bevorzugt bei 1*10⁶ Zellen/ml) inkubiert werden, um möglichst lineare kinetische Bedingungen im Versuch sicherzustellen. Sieben Proben aus der Inkubationslösung werden in einem festgelegten Zeitraster für die LC-MS-Analytik entnommen, um die Halbwertszeit (d.h. den Abbau) der jeweiligen Verbindung zu bestimmen. Aus dieser Halbwertszeit werden unterschiedliche "Clearance"-Parameter (CL) und "Fₘₐₓ"- Werte berechnet (s.u.).

Die CL- und Fₘₐₓ-Werte stellen ein Maß für den Phase 1- und Phase 2-Metabolismus der Verbindungen in den Hepatozyten dar. Um den Einfluss des organischen Lösungsmittels auf die Enzyme in den Inkubationsansätzen möglichst klein zu halten, wird dessen Konzentration im Allgemeinen auf 1% (Acetonitril) bzw. 0.1 % (DMSO) begrenzt.

Für alle Spezies und Rassen wird mit einer Hepatozyten-Zellzahl in der Leber von 1.1 * 10⁸ Zellen/g Leber gerechnet. CL-Parameter, deren Berechnung auf Halbwertszeiten beruhen, die wesentlich über die Inkubationszeit hinausgehen (üblicherweise 90 Minuten), können nur als grobe Richtwerte angesehen werden.

Die berechneten Parameter und deren Bedeutung sind:
- **Fₘₐₓ well-stirred [%]**: maximal mögliche Bioverfügbarkeit nach oraler Applikation
- *Berechnung:*: (1-CL_{blood} well-stirred/QH) * 100
- **CL_{blood} well-stirred [L/(h*kg)]**: berechnete Blut-Clearance (well stirred-Modell)
- *Berechnung:*: (QH *CL'_{intrinsic}) / (QH + CL'_{intrinsic})
- **CL**'_{intrinsic} **[ml/(min*kg)]**: maximale Fähigkeit der Leber (der Hepatozyten), eine Verbindung zu metabolisieren (unter der Annahme, dass der Leberblutfluss nicht geschwindigkeitslimitierend ist)
- *Berechnung:*: CL'_{intrinsic, apparent}* speziesspezifische Hepatozytenzahl [1.1 * 10⁸/g Leber] * speziesspezifisches Lebergewicht [g/kg]
- ***CL'*_{*i*ntrinsic}, ₐₚₚₐᵣₑₙₜ [ml/(min*mg)]**: normiert die Eliminationskonstante, indem diese durch die eingesetzte Hepatozyten-Zellzahl x (x * 10⁶/ml) dividiert wird
- *Berechnung:*: kₑₗ [1/min] / (Zellzahl [x * 10⁶] / Inkubationsvolumen [ml])
(QH = speziesspezifischer Leberblutfluss).

Für die erfindungsgemäßen Verbindungen repräsentative Werte aus diesem Assay nach Inkubation der Verbindungen mit Ratten-Hepatozyten sind in der folgenden Tabelle B wiedergegeben:

**Tabelle B: berechnete Blut-Clearance und Bioverfügbarkeit nach Inkubation mit Ratten-Hepatozyten**

| **Ausführungsbeispiel Nr.** | **CL_{blood} [L/(h*kg)]** | **Fₘₐₓ [%]** |
|---|---|---|
| 5 | 0.0 | 100 |
| 6 | 0.2 | 96 |
| 10 | 1.5 | 65 |
| 12 | 1.7 | 59 |
| 22 | 0.2 | 95 |
| 25 | 0.3 | 93 |
| 27 | 0.4 | 91 |
| 29 | 0.4 | 90 |
| 31 | 0.4 | 91 |
| 32 | 0.1 | 97 |
| 33 | 0.6 | 87 |
| 36 | 0.4 | 89 |
| 38 | 0.2 | 96 |
| 43 *(Diastereomer 1)* | 1.3 | 69 |
| 91 | 0.7 | 83 |
| 92 | 0.7 | 83 |
| 116 | 0.4 | 91 |
| 117 | 0.1 | 97 |
| 118 | 0.6 | 86 |
| 123 | 1.0 | 75 |
| 127 | 0.2 | 94 |
| 128 | 0.9 | 78 |
| 129 | 0.9 | 79 |
| 144 | 1.2 | 72 |

### C. Ausführungsbeispiele für pharmazeutische Zusammensetzungen

Die erfmdungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 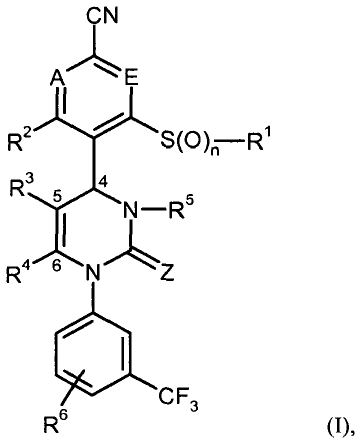 in welcher
A und E beide für C-R⁷ stehen oder eines der beiden Ringglieder A und E für N und das andere für C-R⁷ steht, worin R⁷ jeweils Wasserstoff, Fluor oder Chlor bedeutet,
Z für O oder S steht,
n für die Zahl 0, 1 oder 2 steht,
R¹ für (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl, Phenyl oder 5- oder 6-gliedrigem Heteroaryl oder bis zu fünffach mit Fluor substituiert sein kann, oder für (C₂-C₆)-Alkenyl, (C₃-C₆)-Cycloalkyl, Phenyl oder 5- oder 6-gliedriges Heteroaryl steht, wobei die genannten (C₃-C₆)-Cycloalkyl-Gruppen bis zu zweifach, gleich oder verschieden, mit (C₁-C₄)-Alkyl, Hydroxy und/oder (C₁-C₄)-Alkoxy substituiert sein können und die genannten Phenyl- und Heteroaryl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und/oder Trifluormethoxy substituiert sein können,
R² für Wasserstoff, Fluor oder Chlor steht,
R³ für Cyano oder eine Gruppe der Formel -C(=O)-R⁸, -C(=O)-O-R⁸, -C(=O)-NH₂ oder -C(=O)-N-H-R⁸ steht, worin R⁸ (C₁-C₆)-Alkyl, (C₃-C₆)-Alkenyl oder (C₃-C₆)-Cycloalkyl bedeutet, wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und/oder Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
R⁴ für Methyl oder Ethyl steht
oder R³ und R⁴ miteinander verbunden sind und zusammen eine anellierte Gruppe der Formel 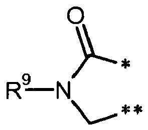 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₆)-Alkyl oder (C₃-C₆)-Cycloalkyl bedeutet, wobei (C₁-C₆)-Alkyl mit Hydroxy, (C₁-C₄)-Alkoxy, Aminocarbonyl, Aminocarbonylamino, (C₁-C₄)-Acylarnino oder (C₃-C₆)-Cycloalkyl substituiert sein kann,
R⁵ für Wasserstoff oder (C₁-C₆)-Allcyl, das mit Cyano, Hydroxy, (C₁-C₄)-Alkoxy, Amino, Mono- oder Di-(C₁-C₄)-alkylamino oder (C₃-C₆)-Cycloalkyl oder bis zu dreifach mit Fluor substituiert sein kann, oder für Phenyl, Pyridyl oder Pyrimidinyl steht, wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
oder R⁵ für eine Gruppe der Formel -C(=O)-O-R¹⁰, -L¹-C(=O)-O-R¹¹, -L²-C(=O)-NR¹²R¹³, -L²-SO₂-NR¹²R¹³, -L²-C(=O)-NR¹⁴-NR¹²R¹³ oder -L²-SO₂-R¹⁵ steht, worin L¹ (C₁-C₆)-Alkandiyl bedeutet, L² eine Bindung oder (C₁-C₆)-Alkandiyl bedeutet, R¹⁰ (C₁-C₆)-Alkyl, das mit (C₃-C₆)-Cycloalkyl oder Phenyl substituiert sein kann, bedeutet, R¹¹ Wasserstoff oder (C₁-C₆)-Alkyl, das mit (C₃-C₆)-Cycloalkyl oder Phenyl substituiert sein kann, bedeutet, R¹² und R¹³ gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten, wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und/oder Aminocarbonyl substituiert sein können und in (C₁-C₆)-Alkyl eine CH₂-Gruppe, soweit in einer chemisch stabilen Verbindung resultierend, gegen ein O-Atom ausgetauscht sein kann und (C₃-C₆)-Cycloalkyl und 4- bis 6-gliedriges Heterocyclyl darüber hinaus bis zu zweifach, gleich oder verschieden, mit (C₁-C₄)-Alkyl substituiert sein können, welches seinerseits mit Hydroxy, (C₁-C₄)-Alkoxy oder Hydroxycarbonyl substituiert sein kann, oder R¹² und R¹³ zusammen mit dem Stickstoffatom, an das sie gebunden sind, einen 4-bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- oder Di-(C₁-C₄)-alkylamino, Hydroxycarbonyl, Aminocarbonyl, (C₃-C₆)-Cycloalkyl, 4- bis 6-gliedrigem Heterocyclyl und/oder 5- oder 6-gliedrigem Heteroaryl substituiert sein kann, wobei (C₁-C₄)-Alkyl seinerseits mit Hydroxy, (C₁-C₄)-Alkoxy oder Hydroxycarbonyl substituiert sein kann, R¹⁴ Wasserstoff oder (C₁-C₄)-Alkyl bedeutet und R¹⁵ (C₁-C₆)-Alkyl, (C₃-C₆)-Cycloalkyl, Phenyl oder 5- oder 6-gliedriges Heteroaryl bedeutet, wobei (C₁-C₆)-Alkyl mit Chlor, Hydroxy, (C₁-C₄)-Alkoxy, Mono- oder Di-(C₁-C₄)-alkylamino oder (C₃-C₆)-Cycloalkyl oder bis zu dreifach mit Fluor substituiert sein kann und Phenyl und 5- oder 6-gliedriges Heteroaryl ihrerseits bis zu zweifach, gleich oder verschieden, mit Fluor, Chlor, Cyano, (C₁-C₄)-Alkyl, Difluormethyl, Trifluormethyl, (C₁-C₄)-Alkoxy und/oder Trifluormethoxy substituiert sein können,
und R⁶ für Wasserstoff, Fluor oder Chlor steht,
sowie ihre pharmazeutisch verträglichen Salze, Solvate und Solvate der Salze.

2. Verbindung der Formel (I) nach Anspruch 1, in welcher
A und E beide für C-R⁷ stehen oder eines der beiden Ringglieder A und E für N und das andere für C-R⁷ steht, worin R⁷ jeweils Wasserstoff, Fluor oder Chlor bedeutet,
Z für O oder S steht,
n für die Zahl 0, 1 oder 2 steht,
R¹ für (C₁-C₆)-Alkyl, das mit Hydroxy, (C₁-C₄)-Alkoxy, Amino, Mono- oder Di-(C₁-C₄)-alkylamino, Hydroxycarbonyl, Aminocarbonyl, (C₃-C₆)-Cycloalkyl oder Phenyl substituiert sein kann, oder für (C₂-C₆)-Alkenyl, (C₃-C₆)-Cycloalkyl oder Phenyl steht, wobei die genannten (C₃-C₆)-Cycloalkyl-Gruppen bis zu zweifach, gleich oder verschieden, mit (C₁-C₄)-Alkyl, Hydroxy und/oder (C₁-C₄)-Alkoxy substituiert sein können und die genannten Phenyl-Gruppen bis zu zweifach, gleich oder verschieden, mit Fluor, Chlor, Cyano, (C₁-C₄)-Alkyl, Difluormethyl, Trifluormethyl, (C₁-C₄)-Alkoxy, Difluormethoxy und/oder Trifluormethoxy substituiert sein können,
R² für Wasserstoff, Fluor oder Chlor steht,
R³ für Cyano oder eine Gruppe der Formel -C(=O)-R⁸, -C(=O)-O-R⁸, -C(=O)-NH₂ oder -C(=O)-NH-R⁸ steht, worin R⁸ (C₁-C₆)-Alkyl, (C₃-C₆)-Alkenyl oder (C₃-C₆)-Cycloalkyl bedeutet, wobei (C₁-C₆)-Alkyl und (C₃-C₆)-Cycloalkyl ihrerseits bis zu zweifach, gleich oder verschieden, mit Hydroxy, (C₁-C₄)-Alkoxy, Hydroxycarbonyl, (C₁-C₄)-Alkoxycarbonyl, Amino, Mono- und/oder Di-(C₁-C₄)-alkylamino substituiert sein können und in (C₁-C₆)-Alkyl und (C₃-C₆)-Cycloalkyl jeweils eine CH₂-Gruppe, soweit in einer chemisch stabilen Verbindung resultierend, gegen ein O-Atom ausgetauscht sein kann,
R⁴ für Methyl oder Ethyl steht
oder R³ und R⁴ miteinander verbunden sind und zusammen eine anellierte Gruppe der Formel 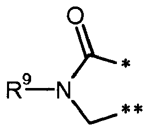 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und
R⁹ Wasserstoff, (C₁-C₆)-Alkyl oder (C₃-C₆)-Cycloalkyl bedeutet, wobei (C₁-C₆)-Alkyl mit Hydroxy, (C₁-C₄)-Alkoxy, Aminocarbonyl, (C₁-C₄)-Acylamino oder (C₃-C₆)-Cycloalkyl substituiert sein kann,
R⁵ für Wasserstoff oder (C₁-C₆)-Alkyl, das bis zu dreifach mit Fluor substituiert sein kann, oder für Phenyl, Pyridyl oder Pyrimidinyl steht, wobei Phenyl, Pyridyl und Pyrimidinyl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
oder R⁵ für eine Gruppe der Formel -C(=O)-O-R¹⁰, -L¹-C(=O)-O-R¹¹, -L²-C(=O)-NR¹²R¹³, -L²-SO₂-NR¹²R¹³, -L²-C(=O)-NR¹⁴-NR¹²R¹³ oder L²-SO₂-R¹⁵ steht, worin
L¹ (C₁-C₆)-Alkandiyl bedeutet,
L² eine Bindung oder (C₁-C₆)-Alkandiyl bedeutet,
R¹⁰ (C₁-C₆)-Alkyl bedeutet,
R¹¹ Wasserstoff oder (C₁-C₆)-Alkyl bedeutet,
R¹² und R¹³ gleich oder verschieden sind und unabhängig voneinander Wasserstoff, (C₁-C₆)-Alkyl, (C₃-C₆)-Cycloalkyl oder 4- bis 6-gliedriges Heterocyclyl bedeuten, wobei (C₁-C₆)-Alkyl, (C₃-C₆)-Cycloalkyl und 4- bis 6-gliedriges Heterocyclyl ihrerseits bis zu zweifach, gleich oder verschieden, mit Fluor, Hydroxy, (C₁-C₄)-Alkoxy, Oxo, Amino, Mono- oder Di-(C₁-C₄)-alkylamino, Hydroxycarbonyl, (C₁-C₄)-Alkoxycarbonyl und/oder Aminocarbonyl substituiert sein können und in (C₁-C₆)-Alkyl eine CH₂-Gruppe, soweit in einer chemisch stabilen Verbindung resultierend, gegen ein O-Atom ausgetauscht sein kann,
oder R¹² und R¹³ zusammen mit dem Stickstoffatom, an das sie gebunden sind, einen 4-bis 6-gliedrigen Heterocyclus bilden, der ein weiteres Ring-Heteroatom aus der Reihe N, O, S, SO oder SO₂ enthalten und bis zu zweifach, gleich oder verschieden, mit (C₁-C₄)-Alkyl, Hydroxy, (C₁-C₄)-Alkoxy, Oxo, Amino, Mono- und/oder Di-(C₁-C₄)-alkylamino substituiert sein kann, wobei (C₁-C₄)-Alkyl seinerseits mit Hydroxy oder (C₁-C₄)-Alkoxy substituiert sein kann,
R¹⁴ Wasserstoff oder (C₁-C₄)-Alkyl bedeutet
und R¹⁵ (C₁-C₆)-Alkyl, (C₃-C₆)-Cycloalkyl, Phenyl oder 5- oder 6-gliedriges Heteroaryl bedeutet, wobei (C₁-C₆)-Alkyl mit Fluor, Chlor, Hydroxy, (C₁-C₄)-Alkoxy, Mono- oder Di-(C₁-C₄)-alkylamino substituiert sein kann und Phenyl und 5- oder 6-gliedriges Heteroaryl ihrerseits bis zu zweifach, gleich oder verschieden, mit Fluor, Chlor, Cyano, (C₁-C₄)-Alkyl, Trifluormethyl, (C₁-C₄)-Alkoxy und/oder Trifluormethoxy substituiert sein können,
und R⁶ für Wasserstoff, Fluor oder Chlor steht,
sowie ihre pharmazeutisch verträglichen Salze, Solvate und Solvate der Salze.

3. Verbindung der Formel (I) nach Anspruch 1 oder 2, in welcher
A und E beide für CH stehen,
Z fur O steht,
n für die Zahl 0 oder 2 steht,
R¹ für (C₁-C₄)-Alkyl, das mit Hydroxy, (C₁-C₄)-Alkoxy, Hydroxycarbonyl, Aminocarbonyl, (C₃-C₆)-Cycloalkyl, Phenyl oder 5-gliedrigem Heteroaryl oder bis zu dreifach mit Fluor substituiert sein kann, oder für (C₃-C₆)-Cycloalkyl, Phenyl oder 5-gliedriges Heteroaryl steht, wobei die genannten Phenyl- und Heteroaryl-Gruppen bis zu zweifach, gleich oder verschieden, mit Fluor, Chlor, Cyano, Methyl, Trifluormethyl, Methoxy und/oder Trifluormethoxy substituiert sein können,
R² für Wasserstoff steht,
R³ für Cyano, Acetyl oder (2-Hydroxyethoxy)carbonyl steht,
R⁴ für Methyl steht
oder R³ und R⁴ miteinander verbunden sind und zusammen eine anellierte Gruppe der Formel 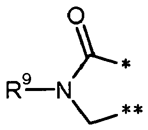 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₄)-Alkyl oder (C₃-C₆)-Cycloalkyl bedeutet, wobei (C₁-C₄)-Alkyl mit Hydroxy oder (C₁-C₄)-Alkoxy substituiert sein kann,
R⁵ für Wasserstoff oder (C₁-C₄)-Alkyl, das mit Cyano oder Di-(C₁-C₄)-alkylamino substituiert sein kann, oder für eine Gruppe der Formel -L²-C(=O)-N-R¹²R¹³ -L²-C(=O)-NH-NR¹²R¹³ oder -L²-SO₂-R¹⁵ steht, worin L² eine Bindung, -CH₂-, -CH₂CH₂- oder -CH(CH₃)- bedeutet, R¹² Wasserstoff oder (C₁-C₄)-Alkyl, das mit Hydroxy oder (C₁-C₄)-Alkoxy substituiert sein kann, bedeutet, R¹³ Wasserstoff, (C₁-C₆)-Alkyl oder (C₃-C₆)-Cycloalkyl bedeutet, wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und/oder Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann, oder R¹² und R¹³ zusammen mit dem Stickstoffatom, an das sie gebunden sind, einen 5-oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy, Oxo, Hydroxycarbonyl, Aminocarbonyl, 4- bis 6-gliedrigem Heterocyclyl oder 5- oder 6-gliedrigem Heteroaryl substituiert sein kann, wobei (C₁-C₄)-Alkyl seinerseits mit Hydroxy, (C₁-C₄)-Alkoxy oder Hydroxycarbonyl substituiert sein kann, und R¹⁵ (C₁-C₄)-Alkyl, (C₃-C₆)-Cycloalkyl oder Phenyl bedeutet, wobei (C₁-C₄)-Alkyl mit (C₃-C₆)-Cycloalkyl substituiert sein kann und Phenyl bis zu zweifach, gleich oder verschieden, mit Fluor, Chlor, Cyano, Methyl, Trifluormethyl, Methoxy und/oder Trifluormethoxy substituiert sein kann,
und R⁶ für Wasserstoff oder Fluor steht,
sowie ihre pharmazeutisch verträglichen Salze, Solvate und Solvate der Salze.

4. Verbindung der Formel (I) nach einem oder mehreren der Ansprüche 1 bis 3, in welcher
A und E beide für CH stehen,
Z für O steht,
n für die Zahl 0 oder 2 steht,
R¹ für (C₁-C₄)-Alkyl, das mit Hydroxy, (C₁-C₄)-Alkoxy, Hydroxycarbonyl, Aminocarbonyl, (C₃-C₆)-Cycloalkyl oder Phenyl substituiert sein kann, oder für (C₃-C₆)-Cycloalkyl oder Phenyl steht, wobei die genannten Phenyl-Gruppen bis zu zweifach, gleich oder verschieden, mit Fluor, Chlor, Cyano, Methyl, Trifluormethyl, Methoxy und/oder Trifluormethoxy substituiert sein können,
R² für Wasserstoff steht,
R³ für Cyano oder Acetyl steht,
R⁴ für Methyl steht
oder R³ und R⁴ miteinander verbunden sind und zusammen eine anellierte Gruppe der Formel 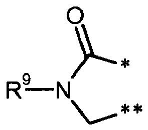 bilden, worin * die Verknüpfungsstelle mit der in Formel (I) abgebildeten 5-Position des Dihydropyrimidin-Rings und ** die Verknüpfungsstelle mit der in Formel (I) abgebildeten 6-Position des Dihydropyrimidin-Rings bedeuten und R⁹ Wasserstoff, (C₁-C₄)-Alkyl oder (C₃-C₆)-Cycloalkyl bedeutet, wobei (C₁-C₄)-Alkyl mit Hydroxy oder (C₁-C₄)-Alkoxy substituiert sein kann,
R⁵ für Wasserstoff, (C₁-C₄)-Alkyl oder eine Gruppe der Formel -L²-C(=O)-NR¹²R¹³, -L²-C(=O)-NH-NR¹²R¹³ oder -L²-SO₂-R¹⁵ steht, worin L² eine Bindung, -CH₂-, -CH₂CH₂- oder -CH(CH₃)- bedeutet, R¹² Wasserstoff oder (C₁-C₄)-Alkyl, das mit Hydroxy oder (C₁-C₄)-Alkoxy substituiert sein kann, bedeutet, R¹³ Wasserstoff, (C₁-C₆)-Alkyl oder (C₃-C₆)-Cycloalkyl bedeutet, wobei (C₁-C₆)-Alkyl bis zu zweifach, gleich oder verschieden, mit Hydroxy, (C₁-C₄)-Alkoxy, Hydroxycarbonyl, (C₁-C₄)-Alkoxycarbonyl und/oder Aminocarbonyl substituiert sein kann und in (C₁-C₆)-Alkyl eine CH₂-Gruppe, soweit in einer chemisch stabilen Verbindung resultierend, gegen ein O-Atom ausgetauscht sein kann, oder R¹² und R¹³ zusammen mit dem Stickstoffatom, an das sie gebunden sind, einen 5-oder 6-gliedrigen Heterocyclus bilden, der ein weiteres Ring-Heteroatom aus der Reihe N, O oder S enthalten und mit (C₁-C₄)-Alkyl, Hydroxy, (C₁-C₄)-Alkoxy oder Oxo substituiert sein kann, wobei (C₁-C₄)-Alkyl seinerseits mit Hydroxy oder (C₁-C₄)-Alkoxy substituiert sein kann, und R¹⁵ (C₁-C₄)-Alkyl, (C₃-C₆)-Cycloalkyl oder Phenyl bedeutet, wobei Phenyl bis zu zweifach, gleich oder verschieden, mit Fluor, Chlor, Cyano, Methyl, Trifluormethyl, Methoxy und/oder Trifluormethoxy substituiert sein kann,
und R⁶ für Wasserstoff oder Fluor steht,
sowie ihre pharmazeutisch verträglichen Salze, Solvate und Solvate der Salze.

5. Verbindung der Formel (I) nach einem oder mehreren der Ansprüche 1 bis 4, in welcher
A und E beide für CH stehen,
Z für O steht,
n für die Zahl 2 steht,
R¹ für (C₁-C₄)-Alkyl, das mit Hydroxy, (C₁-C₄)-Alkoxy, Cyclopropyl, Cyclobutyl oder Phenyl oder bis dreifach mit Fluor substituiert sein kann, steht,
R² für Wasserstoff steht,
R³ für Cyano oder (2-Hydroxyethoxy)carbonyl steht,
R⁴ für Methyl steht,
R⁵ für Wasserstoff, (C₁-C₄)-Alkyl oder eine Gruppe der Formel -L²-C(=O)-NH-R¹³ oder -SO₂-R¹⁵ steht, worin L² eine Bindung oder -CH₂- bedeutet, R¹³ Wasserstoff oder (C₁-C₄)-Alkyl, das mit Hydroxy oder (C₁-C₄)-Alkoxy substituiert sein kann, oder (C₃-C₆)-Cycloalkyl bedeutet und R¹⁵ (C₁-C₄)-Alkyl oder (C₃-C₆)-Cycloalkyl bedeutet,
und R⁶ für Wasserstoff steht,
sowie ihre pharmazeutisch verträglichen Salze, Solvate und Solvate der Salze.

6. Verbindung der Formel (I) nach einem oder mehreren der Ansprüche 1 bis 5, in welcher
A und E beide für CH stehen,
Z für O steht,
n für die Zahl 2 steht,
R¹ für (C₁-C₄)-Alkyl, das mit Hydroxy oder (C₁-C₄)-Alkoxy substituiert sein kann, steht,
R² für Wasserstoff steht,
R³ für Cyano steht,
R⁴ für Methyl steht,
R⁵ für Wasserstoff, (C₁-C₄)-Alkyl oder eine Gruppe der Formel -CH₂-C(=O)-NH-R¹³ oder -SO₂-R¹⁵ steht, worin
R¹³ Wasserstoff oder (C₁-C₄)-Alkyl, das mit Hydroxy oder (C₁-C₄)-Alkoxy substituiert sein kann, bedeutet und R¹⁵ (C₁-C₄)-Alkyl oder (C₃-C₆)-Cycloalkyl bedeutet,
und R⁶ für Wasserstoff steht,
sowie ihre pharmazeutisch verträglichen Salze, Solvate und Solvate der Salze.

7. Verbindung gemäss der Formel (I) mit der in Formel *(I-ent)* wiedergegebenen Konfiguration an der 4-Position des Dihydropyrimidin-Rings nach einem oder mehreren der Ansprüche 1 bis 6, 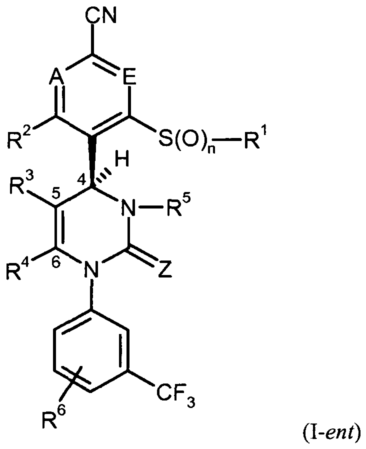 in welcher
A und E beide für CH stehen,
Z für O steht,
n für die Zahl 2 steht,
R¹ für (C₁-C₄)-Alkyl, das mit Hydroxy oder (C₁-C₄)-Alkoxy substituiert sein kann, steht,
R² für Wasserstoff steht,
R³ für Cyano steht,
R⁴ für Methyl steht,
R⁵ für Wasserstoff, (C₁-C₄)-Alkyl oder eine Gruppe der Formel -CH₂-C(=O)-NH-R¹³ oder -SO₂-R¹⁵ steht, worin R¹³ Wasserstoff oder (C₁-C₄)-Alkyl, das mit Hydroxy oder (C₁-C₄)-Alkoxy substituiert sein kann, bedeutet und R¹⁵ (C₁-C₄)-Alkyl oder (C₃-C₆)-Cycloalkyl bedeutet,
und R⁶ für Wasserstoff steht,
sowie ihre pharmazeutisch verträglichen Salze, Solvate und Solvate der Salze.

8. Verbindung der Formel (I) nach einem oder mehreren der Ansprüche 1 bis 7, ausgewählt aus folgender Gruppe:
4- {(4*S*)-5-Acetyl-6-methyl-2-oxo-1-[3-(trifluormethyl)phenyl]-1,2,3,4-tetrahydropyrimidin-4-yl}-3-(methylsulfonyl)benzonitril
(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril
(6*S*)-5-Cyano-6-[4-cyano-2-(methylsulfonyl)phenyl]-*N*-(2-hydroxyethyl)-4-methyl-2-oxo-3-[3-(trifluormethyl)phenyl]-3,6-dihydropyrimidin-1(2*H*)-carboxamid
(6*S*)-5-Cyano-6-[4-cyano-2-(methylsulfonyl)phenyl]-*N'*,*N'*-bis(2-hydroxyethyl)-4-methyl-2-oxo-3-[3-(trifluormethyl)phenyl]-3,6-dihydropyrimidin-1 (2*H*)-carbohydrazid
(6*S*)-5-Cyano-6-[4-cyano-2-(methylsulfonyl)phenyl]-*N*-[2-(2-hydroxyethoxy)ethyl]-4-methyl-2-oxo-3-[3-(trifluormethyl)phenyl]-3,6-dihydropyrimidin-1(2*H*)-carboxamid
(4*S*)-4-[4-Cyano-2-(methylsulfonyl)phenyl]-3-{[(3*S*)-3-hydroxypyrrolidin-1-yl]carbonyl}-6-methyl-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-3-{[4-(2-hydroxyethyl)piperazin-1-yl]-carbonyl}-6-methyl-2-oxo-1-[3-(trifluormethyl)phenyl]-1,2,3,4-tetrahydropyrimidin-5-carbonitril
2-[(6*S*)-5-Cyano-6-[4-cyano-2-(methylsulfonyl)phenyl]-4-methyl-2-oxo-3-[3-(trifluormethyl)phenyl]-3,6-dihydropyrimidin-1(2*H*)-yl]acetamid
(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-3-(cyclopropylsulfonyl)-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonitril
4- {(4*S*)-6-Methyl-3-(methylsulfonyl)-2,5-dioxo-1-[3-(trifluormethyl)phenyl]-2,3,4,5,6,7-hexahydro-1*H*-pyrrolo[3,4-d]pyrimidin-4-yl}-3-(methylsulfonyl)benzonitril
(4*S*)-4-[4-Cyano-2-(methylsulfanyl)phenyl]-6-methyl-2-oxo-1-[3-(trifluormethyl)phenyl]-1,2,3,4-tetrahydropyrimidin-5-carbonitril
(4*S*)-4-{4-Cyano-2-[(*S*)-methylsulfinyl]phenyl}-6-methyl-2-oxo-1-[3-(trifluormethyl)-phenyl]-1,2,3,4-tetrahydropyrimidin-5-carbonitril
(*rac*)-1-(2-{4-[4-Cyano-2-(methylsulfonyl)phenyl]-2,5-dioxo-1-[3-(trifluormethyl)phenyl]-1,2,3,4,5,7-hexahydro-6*H*-pyrrolo[3,4-d]pyrimidin-6-yl}ethyl)harnstoff
(4*S*)-4-[4-Cyano-2-(methylsulfonyl)phenyl]-3-[(3-hydroxyazetidin-1-yl)carbonyl]-6-methyl-2-oxo-1-[3-(trifluormethyl)phenyl]-1,2,3,4-tetrahydropyrimidin-5-carbonitril
(4*S*)-3-[(3*R*)-3-Aminopiperidin-1-yl]carbonyl-4-[4-cyano-2-(methylsulfonyl)phenyl]-6-methyl-2-oxo-1-[3-(trifluormethyl)phenyl]-1,2,3,4-tetrahydropyrimidin-5-carbonitril
(4S)-4-[4-Cyan-2-(methylsulfonyl)phenyl]-6-methyl-2-oxo-3-{[4-(pyridin-2-yl)piperazin-1-yl]carbonyl}-1-[3-(trifluormethyl)phenyl]-1,2,3,4-tetrahydropyrimidin-5-carbonitril
(6S)-5-Cyan-6-[4-cyan-2-(methylsulfonyl)phenyl]-N,N-bis(2-hydroxypropyl)-4-methyl-2-oxo-3-[3-(trifluormethyl)phenyl]-3,6-dihydropyrimidin-1(2H)-carboxamid
(6S)-5-Cyan-6-[4-cyan-2-(methylsulfonyl)phenyl]-N-(1-hydroxy-2-methylpropan-2-yl)-4-methyl-2-oxo-3-[3-(trifluormethyl)phenyl]-3,6-dihydropyrimidin-1(2H)-carboxamid
(4S)-4-[4-Cyan-2-(methylsulfonyl)phenyl]-3-[(4-fluorphenyl)sulfonyl]-6-methyl-2-oxo-1-[3-(trifluormethyl)phenyl]-1,2,3,4-tetrahydropyrimidin-5-carbonitril
(4S)-4-[4-Cyan-2-(methylsulfonyl)phenyl]-3-{[1-(difluormethyl)-5-methyl-1H-pyrazol-4-yl]sulfonyl}-6-methyl-2-oxo-1-[3-(trifluormethyl)phenyl]-1,2,3,4-tetrahydropyrimidin-5-carbonitril
(4S)-4-[4-Cyan-2-(methylsulfonyl)phenyl]-3-[(2-cyanphenyl)sulfonyl]-6-methyl-2-oxo-1-[3-(trifluormethyl)phenyl]-1,2,3,4-tetrahydropyrimidin-5-carbonitril
(4*S*)-3-(Cyanomethyl)-4-[4-cyano-2-(methylsulfonyl)phenyl]-6-methyl-2-oxo-1-[3-(trifluormethyl)phenyl]-1,2,3,4-tetrahydropyrimidin-5-carbonitril
Benzyl 5-cyano-6-[4-cyano-2-(methylsulfonyl)phenyl]-4-methyl-2-oxo-3-[3-(trifluormethyl)-phenyl]-3,6-dihydropyrimidin-1(2*H*)-carboxylat
(4*S*)-4-[4-Cyano-2-(ethylsulfonyl)phenyl]-3,6-dimethyl-2-oxo-1-[3-(trifluormethyl)phenyl]-1,2,3,4-tetrahydropyrimidin-5-carbonitril
(*rac*)-4-[4-Cyano-2-((2-hydroxyethyl)sulfonyl)phenyl]-6-methyl-2-oxo-1-[3-(trifluormethyl)-phenyl]-1,2,3,4-tetrahydropyrimidin-5-carbonitril
(4*S*)-4-{4-Cyano-2-[phenylsulfonyl]phenyl}-6-methyl-2-oxo-1-[3-(trifluormethyl)phenyl]-1,2,3,4-tetrahydropyrimidin-5-carbonitril
2-Hydroxyethyl (4*S*)-4-[4-cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carboxylat
sowie ihre pharmazeutisch verträglichen Salze, Solvate und Solvate der Salze.

9. Verbindung der Formel (I) nach einem oder mehreren der Ansprüche 1 bis 7, ausgewählt aus folgender Gruppe:
(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carboxamid
(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril
(6*S*)-5-Cyano-6-[4-cyano-2-(methylsulfonyl)phenyl]-N-(2-hydroxyethyl)-4-methyl-2-oxo-3-[3-(trifluormethyl)phenyl]-3,6-dihydropyrimidin-1(2*H*)-carboxamid
(6*S*)-5-Cyano-6-[4-cyano-2-(methylsulfonyl)phenyl]-*N*',*N*'-bis(2-hydroxyethyl)-4-methyl-2-oxo-3-[3-(trifluormethyl)phenyl]-3,6-dihydropyrimidin-1(2*H*)-carbohydrazid 2-[(6*S*)-5-Cyano-6-[4-cyano-2-(methylsulfonyl)phenyl]-4-methyl-2-oxo-3-[3-(trifluormethyl)phenyl]-3,6-dihydropyrimidin-1(2*H*)-yl]acetamid
(4*S*)-4-[4-Cyano-2-(methylsulfonyl)phenyl]-6-methyl-2-oxo-3-[2-oxo-2-(3-oxopiperazin-1-yl)ethyl]-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-3-{[2-(trifluormethoxy)phenyl]sulfonyl}-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-3-(ethylsulfonyl)-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-3-(cyclopropylsulfonyl)-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonitril
4- {(4*S*)-6-Methyl-3-(methylsulfonyl)-2,5-dioxo-1-[3-(trifluormethyl)phenyl]-2,3,4,5,6,7-hexahydro-1*H-*pyrrolo[3,4-d]pyrimidin-4-yl}-3-(methylsulfonyl)benzonitril
(*rac*)-4-[4-Cyano-2-(methylsulfonyl)phenyl]-*N*-(2-hydroxyethyl)-6-methyl-2,5-dioxo-1-[3-(trifluormethyl)phenyl]-1,2,4,5,6,7-hexahydro-3*H*-pyrrolo[3,4-d]pyrimidin-3-carboxamid
(4*S*)-4-{4-Cyano-2-[(*S*)-methylsulfinyl]phenyl}-6-methyl-2-oxo-1-[3-(trifluormethyl)phenyl]-1,2,3,4-tetrahydropyrimidin-5-carbonitril
(4S)-4-[4-Cyan-2-(methylsulfonyl)phenyl]-3-[(4-fluorphenyl)sulfonyl]-6-methyl-2-oxo-1-[3-(trifluormethyl)phenyl]-1,2,3,4-tetrahydropyrimidin-5-carbonitril
(4S-4-[4-Cyan-2-(methylsulfonyl)phenyl]-3-[(1,2-dimethyl-1H-imidazol-4-yl)sulfonyl]-6-methyl-2-oxo-1-[3-(trifluormethyl)phenyl]-1,2,3,4-tetrahydropyrimidin-5-carbonitril
(4*S*)-3-(Cyanomethyl)-4-[4-cyano-2-(methylsulfonyl)phenyl]-6-methyl-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-3-(4-cyanophenyl)-6-methyl-2-oxo-1-[3-(trifluormethyl)phenyl]-1,2,3,4-tetrahydropyrimidin-5-carbonitril
(4*S*)-4-[4-Cyano-2-(methylsulfonyl)phenyl]-6-methyl-2-oxo-1,3-bis[3-(trifluormethyl)phenyl]-1,2,3,4-tetrahydropyrimidin-5-carbonitril
4-(4*S*)-3,6-Dimethyl-2,5-dioxo-1-[3-(trifluormethyl)phenyl]-2,3,4,5,6,7-hexahydro-*1H-*pyrrolo[3,4-d]pyrimidin-4-yl-3-(methylsulfonyl)benzonitril
(4*S*)-4-[4-Cyano-2-(ethylsulfonyl)phenyl]-6-methyl-2-oxo-1-[3-(trifluormethyl)phenyl]-1,2,3,4-tetrahydropyrimidin-5-carbonitril
(4*S*)-4-[4-Cyano-2-(ethylsulfonyl)phenyl]-3,6-dimethyl-2-oxo-1-[3-(trifluormethyl)phenyl]-1,2,3,4-tetrahydropyrimidin-5-carbonitril
(4*S*)-4-[4-Cyano-2-(ethylsulfonyl)phenyl]-6-methyl-3-(methylsulfonyl)-2-oxo-1-[3-(trifluormethyl)phenyl]-1,2,3,4-tetrahydropyrimidin-5-carbonitril
2-Hydroxyethyl (4*S*)-4-[4-cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carboxylat
sowie ihre pharmazeutisch verträglichen Salze, Solvate und Solvate der Salze.

10. Verbindung der Formel (I) nach einem oder mehreren der Ansprüche 1 bis 7 mit der folgenden Struktur
(4*S*)-4-[4-Cyano-2-(methylsulfonyl)phenyl]-3,6-dimethyl-2-oxo-1-[3-(trifluormethyl)phenyl]-1,2,3,4-tetrahydropyrimidin-5-carbonitril
sowie ihre pharmazeutisch verträglichen Salze, Solvate und Solvate der Salze.

11. Verbindung der Formel (I) nach einem oder mehreren der Ansprüche 1 bis 7 mit der folgenden Struktur
(4*S*)-4-[4-Cyano-2-(methylsulfonyl)phenyl]-6-methyl-3-(methylsulfonyl)-2-oxo-1-[3-(trifluormethyl)phenyl]-1,2,3,4-tetrahydropyrimidin-5-carbonitril
sowie ihre pharmazeutisch verträglichen Salze, Solvate und Solvate der Salze.

12. Verbindung der Formel (I) nach einem oder mehreren der Ansprüche 1 bis 7 mit der folgenden Struktur
(4*S*)-4-[4-Cyano-2-(methylsulfonyl)phenyl]-6-methyl-2-oxo-1-[3-(trifluormethyl)phenyl]-1,2,3,4-tetrahydropyrimidin-5-carbonitril
sowie ihre pharmazeutisch verträglichen Salze, Solvate und Solvate der Salze.

13. Verfahren zur Herstellung von Verbindungen der Formel (I), wie in den Ansprüchen 1 bis 12 definiert, **dadurch gekennzeichnet, dass** man eine Verbindung der Formel (II) 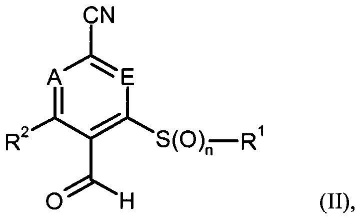 in welcher A, E, n, R¹ und R² die in den Ansprüchen 1 bis 12 angegebenen Bedeutungen haben,
in Gegenwart einer Säure oder eines Säureanhydrids in einer 3-Komponenten-Eintopf-Reaktion oder sequentiell mit einer Verbindung der Formel (III) 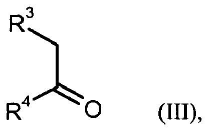 in welcher R³ und R⁴ die in den Ansprüchen 1 bis 8 angegebenen Bedeutungen haben, und einer Verbindung der Formel (IV) 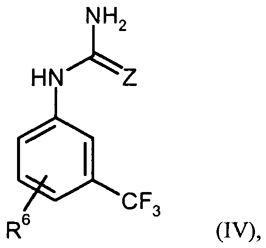 in welcher Z und R⁶ die in den Ansprüchen 1 bis 12 angegebenen Bedeutungen haben, zu einer Verbindung der Formel (I-A) 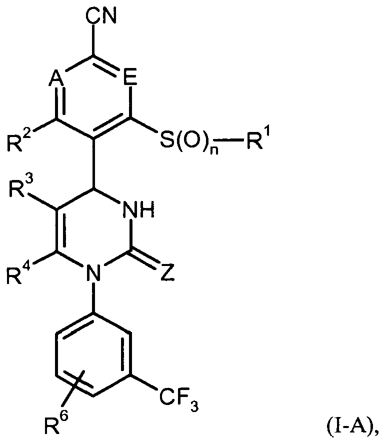 in welcher A, E, Z, n, R¹, R², R³, R⁴ und R⁶ jeweils die oben angegebenen Bedeutungen haben,
umsetzt und diese im Falle, dass R⁵ in Formel (I) nicht für Wasserstoff steht, in Gegenwart einer Base mit einer Verbindung der Formel (V)
R^{5A}-X , (V)
in welcher
R^{5A} die in den Ansprüchen 1 bis 12 angegebene Bedeutung von R⁵ hat, jedoch nicht für Wasserstoff steht,
und
X für eine Abgangsgruppe wie beispielsweise Halogen, Mesylat, Tosylat oder Triflat steht,
zu einer Verbindung der Formel (I-B) 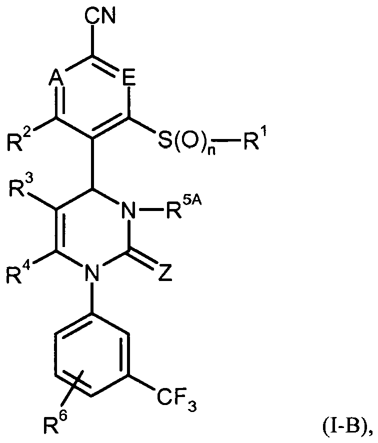 in welcher A, E, Z, n, R¹, R², R³, R⁴, R^{5A} und R⁶ jeweils die oben angegebenen Bedeutungen aufweisen,
reagiert
und gegebenenfalls die so erhaltenen Verbindungen der Formel (I-A) bzw. (I-B) nach dem Fachmann bekannten Methoden in ihre Enantiomere und/oder Diastereomere trennt und/oder mit den entsprechenden (i) Lösungsmitteln und/oder (ii) Basen oder Säuren in ihre Solvate, Salze und/oder Solvate der Salze überführt.

14. Verbindung, wie in einem oder mehreren der Ansprüche 1 bis 12 definiert, zur Behandlung und/oder Prävention von Krankheiten.

15. Verbindung, wie in einem oder mehreren der Ansprüche 1 bis 12 definiert, zur Verwendung in einem Verfahren zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

16. Verbindung, wie in einem oder mehreren der Ansprüche 1 bis 12 definiert, zur Verwendung in einem Verfahren zur Behandlung und/oder Prävention von Bronchiektasie.

17. Verwendung einer Verbindung, wie in einem oder mehreren der Ansprüche 1 bis 12 definiert, zur Herstellung eines Arzneimittels zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

18. Arzneimittel enthaltend eine Verbindung, wie in einem oder mehreren der Ansprüche 1 bis 12 definiert, in Kombination mit einem oder mehreren inerten, nicht-toxischen, pharmazeutisch geeigneten Hilfsstoffen.

19. Arzneimittel enthaltend eine Verbindung, wie in einem oder mehreren der Ansprüche 1 bis 12 definiert, in Kombination mit einem oder mehreren weiteren Wirkstoffen ausgewählt aus der Gruppe bestehend aus Kinase-Inhibitoren, Matrixmetalloprotease-Inhibitoren, Stimulatoren und Aktivatoren der löslichen Guanylatcyclase, Prostacyclin-Analoga, Endothelinrezeptor-Antagonisten, Phosphodiesterase-Inhibitoren, beta-adrenerge Rezeptor-Agonisten, Anticholinergika und Glucocorticoide.

20. Arzneimittel nach Anspruch 18 oder 19 zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF).

21. Arzneimittel nach Anspruch 18 oder 19 zur Behandlung und/oder Prävention von Bronchiektasie.

22. Verwendung eines Arzneimittels, wie in einem oder mehreren der Ansprüche 18 bis 20 definiert, zur Herstellung eines Arzneimittels zur Behandlung und/oder Prävention von pulmonaler arterieller Hypertonie (PAH) und anderen Formen der pulmonalen Hypertonie (PH), von chronisch-obstruktiven Lungenerkrankungen (COPD), der akuten Lungenschädigung (ALI), des akuten Atemwegssyndroms (ARDS), des Lungenemphysems, der alpha-1-Antitrypsin-Defizienz (AATD) und der zystischen Fibrose (CF), oder eines Arzneimittels.

## Claims

1. A compound of the formula (I) 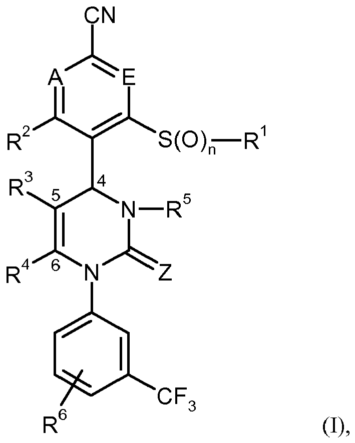 in which
A and E both represent C-R⁷ or one of the two ring members A and E represents N and the other represents C-R⁷, in which R⁷ represents in each case hydrogen, fluorine or chlorine,
Z represents O or S,
n represents the number 0, 1 or 2,
R¹ represents (C₁-C₆)-alkyl which may be substituted by hydroxyl, (C₁-C₄)-alkoxy, amino, mono- or di-(C₁-C₄)-alkylamino, hydroxycarbonyl, aminocarbonyl, (C₃-C₆)-cycloalkyl, phenyl or 5- or 6-membered heteroaryl or up to five times by fluorine, or represents (C₂-C₆)-alkenyl, (C₃-C₆)-cycloalkyl, phenyl or 5- or 6-membered heteroaryl, where the (C₃-C₆)-cycloalkyl groups mentioned may be substituted up to two times by identical or different substituents from the group consisting of (C₁-C₄)-alkyl, hydroxyl and (C₁-C₄)-alkoxy and the phenyl and heteroaryl groups mentioned may be substituted up to two times by
R² identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy, represents hydrogen, fluorine or chlorine,
R³ represents cyano or a group of the formula -C(=O)-R⁸, -C(=O)-O-R⁸, -C(=O)-NH₂ or -C(=O)-NH-R⁸, in which R⁸ represents (C₁-C₆)-alkyl, (C₃-C₆)-alkenyl or (C₃-C₆)-cycloalkyl, where (C₁-C₆)-alkyl and (C₃-C₆)-cycloalkyl for their part may be substituted up to two times by identical or different substituents from the group consisting of hydroxyl, (C₁-C₄)-alkoxy, hydroxycarbonyl, (C₁-C₄)-alkoxycarbonyl, amino, mono- and di-(C₁-C₄)-alkylamino and in (C₁-C₆)-alkyl and (C₃-C₆)-cycloalkyl in each case one CH₂ group may be replaced by an oxygen atom, if this results in a chemically stable compound,
R⁴ represents methyl or ethyl
or R³ and R⁴ are attached to one another and together form a fused group of the formula 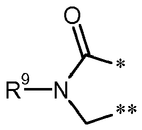 in which * denotes the point of attachment to the 5-position, shown in formula (I), of the dihydropyrimidine ring and ** denotes the point of attachment to the 6-position, shown in formula (I), of the dihydropyrimidine ring and R⁹ represents hydrogen, (C₁-C₆)-alkyl or (C₃-C₆)-cycloalkyl, where (C₁-C₆)-alkyl may be substituted by hydroxyl, (C₁-C₄)-alkoxy, aminocarbonyl, aminocarbonylamino, (C₁-C₄)-acylamino or (C₃-C₆)-cycloalkyl,
R⁵ represents hydrogen or (C₁-C₆)-alkyl which may be substituted by cyano, hydroxyl, (C₁-C₄)-alkoxy, amino, mono- or di-(C₁-C₄)-alkylamino or (C₃-C₆)-cycloalkyl or up to three times by fluorine, or represents phenyl, pyridyl or pyrimidinyl, where phenyl, pyridyl and pyrimidinyl for their part may be substituted up to two times by identical or different substituents from the group consisting of fluorine, chlorine, cyano, (C₁-C₄)-alkyl, trifluoromethyl, (C₁-C₄)-alkoxy and trifluoromethoxy,
or R⁵ represents a group of the formula -C(=O)-O-R¹⁰, -L¹-C(=O)-O-R¹¹, -L²-C(=O)-NR¹²R¹³, -L²-SO₂-NR¹²R¹³, -L²-C(=O)-NR¹⁴-NR¹²R¹³ or -L²-SO₂-R¹⁵, in which
L¹ represents (C₁-C₆)-alkanediyl,
L² represents a bond or (C₁-C₆)-alkanediyl,
R¹⁰ represents (C₁-C₆)-alkyl which may be substituted by (C₃-C₆)-cycloalkyl or phenyl,
R¹¹ represents hydrogen or (C₁-C₆)-alkyl which may be substituted by (C₃-C₆)-cycloalkyl or phenyl,
R¹² and R¹³ are identical or different and independently of one another represent hydrogen, (C₁-C₆)-alkyl, (C₃-C₆)-cycloalkyl or 4- to 6-membered heterocyclyl, where (C₁-C₆)-alkyl, (C₃-C₆)-cycloalkyl and 4- to 6-membered heterocyclyl for their part may be substituted up to two times by identical or different substituents from the group consisting of fluorine, hydroxyl, (C₁-C₄)-alkoxy, oxo, amino, mono- or di-(C₁-C₄)-alkylamino, hydroxycarbonyl, (C₁-C₄)-alkoxycarbonyl and aminocarbonyl and in (C₁-C₆)-alkyl a CH₂ group may be replaced by an oxygen atom, if this results in a chemically stable compound, and (C₃-C₆)-cycloalkyl and 4- to 6-membered heterocyclyl may additionally be substituted up to two times by identical or different (C₁-C₄)-alkyl radicals, which for their part may be substituted by hydroxyl, (C₁-C₄)-alkoxy or hydroxycarbonyl,
or R¹² and R¹³ together with the nitrogen atom to which they are attached form a 4- to 6-membered heterocycle which may contain a further ring heteroatom from the group consisting of N, O, S, SO and SO₂ and which may be substituted up to two times by identical or different substituents from the group consisting of (C₁-C₄)-alkyl, hydroxyl, (C₁-C₄)-alkoxy, oxo, amino, mono- or di-(C₁-C₄)-alkylamino, hydroxycarbonyl, aminocarbonyl, (C₃-C₆)-cycloalkyl, 4- to 6-membered heterocyclyl and 5- or 6-membered heteroaryl, where (C₁-C₄)-alkyl for its part may be substituted by hydroxyl, (C₁-C₄)-alkoxy or hydroxycarbonyl,
R¹⁴ represents hydrogen or (C₁-C₄)-alkyl
and R¹⁵ represents (C₁-C₆)-alkyl, (C₃-C₆)-cycloalkyl, phenyl or 5- or 6-membered heteroaryl, where (C₁-C₆)-alkyl may be substituted by chlorine, hydroxyl, (C₁-C₄)-alkoxy, mono- or di-(C₁-C₄)-alkylamino or (C₃-C₆)-cycloalkyl or up to three times by fluorine and phenyl and 5-or 6-membered heteroaryl for their part may be substituted up to two times by identical or different substituents from the group consisting of fluorine, chlorine, cyano, (C₁-C₄)-alkyl, difluoromethyl, trifluoromethyl, (C₁-C₄)-alkoxy and trifluoromethoxy,
and R⁶ represents hydrogen, fluorine or chlorine,
or a pharmaceutical acceptable salt, a solvate or a solvate of a salt thereof.

2. The compound of the formula (I) as claimed in claim 1, in which
A and E both represent C-R⁷ or one of the two ring members A and E represents N and the other represents C-R⁷, in which R⁷ represents in each case hydrogen, fluorine or chlorine,
Z represents O or S,
n represents the number 0, 1 or 2,
R¹ represents (C₁-C₆)-alkyl which may be substituted by hydroxyl, (C₁-C₄)-alkoxy, amino, mono- or di-(C₁-C₄)-alkylamino, hydroxycarbonyl, aminocarbonyl, (C₃-C₆)-cycloalkyl or phenyl, or represents (C₂-C₆)-alkenyl, (C₃-C₆)-cycloalkyl or phenyl, where the (C₃-C₆)-cycloalkyl groups mentioned may be substituted up to two times by identical or different substituents from the group consisting of (C₁-C₄)-alkyl, hydroxyl and (C₁-C₄)-alkoxy and the phenyl groups mentioned may be substituted up to two times by identical or different substituents from the group consisting of fluorine, chlorine, cyano, (C₁-C₄)-alkyl, difluoromethyl, trifluoromethyl, (C₁-C₄)-alkoxy, difluoromethoxy and trifluoromethoxy,
R² represents hydrogen, fluorine or chlorine,
R³ represents cyano or a group of the formula -C(=O)-R⁸, -C(=O)-O-R⁸, -C(=O)-NH₂ or -C(=O)-NH-R⁸, in which R⁸ represents (C₁-C₆)-alkyl, (C₃-C₆)-alkenyl or (C₃-C₆)-cycloalkyl, where (C₁-C₆)-alkyl and (C₃-C₆)-cycloalkyl for their part may be substituted up to two times by identical or different substituents from the group consisting of hydroxyl, (C₁-C₄)-alkoxy, hydroxycarbonyl, (C₁-C₄)-alkoxy- carbonyl, amino, mono- and di-(C₁-C₄)-alkylamino and in (C₁-C₆)-alkyl and (C₃-C₆)-cycloalkyl in each case one CH₂ group may be replaced by an oxygen atom, if this results in a chemically stable compound,
R⁴ represents methyl or ethyl
or R³ and R⁴ are attached to one another and together form a fused group of the formula 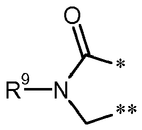 in which * denotes the point of attachment to the 5-position, shown in formula (I), of the dihydropyrimidine ring and ** denotes the point of attachment to the 6-position, shown in formula (I), of the dihydropyrimidine ring and R⁹ represents hydrogen, (C₁-C₆)-alkyl or (C₃-C₆)-cycloalkyl, where (C₁-C₆)-alkyl may be substituted by hydroxyl, (C₁-C₄)-alkoxy, aminocarbonyl, (C₁-C₄)-acylamino or (C₃-C₆)-cycloalkyl,
R⁵ represents hydrogen or (C₁-C₆)-alkyl which may be substituted up to three times by fluorine, or represents phenyl, pyridyl or pyrimidinyl, where phenyl, pyridyl and pyrimidinyl for their part may be substituted up to two times by identical or different substituents from the group consisting of fluorine, chlorine, cyano, (C₁-C₄)-alkyl, trifluoromethyl, (C₁-C₄)-alkoxy and trifluoromethoxy,
or R⁵ represents a group of the formula -C(=O)-O-R¹⁰, -L¹-C(=O)-O-R¹¹, -L²-C(=O)-
NR¹²R¹³, -L²-SO₂-NR¹²R¹³ -L²-C(=O)-NR¹⁴-NR¹²R¹³ or -L²-SO₂-R¹⁵, in which
L¹ represents (C₁-C₆)-alkanediyl,
L² represents a bond or (C₁-C₆)-alkanediyl,
R¹⁰ represents (C₁-C₆)-alkyl,
R¹¹ represents hydrogen or (C₁-C₆)-alkyl,
R¹² and R¹³ are identical or different and independently of one another represent hydrogen, (C₁-C₆)-alkyl, (C₃-C₆)-cycloalkyl or 4- to 6-membered heterocyclyl, where (C₁-C₆)-alkyl, (C₃-C₆)-cycloalkyl and 4- to 6-membered heterocyclyl for their part may be substituted up to two times by identical or different substituents from the group consisting of fluorine, hydroxyl, (C₁-C₄)-alkoxy, oxo, amino, mono- or di-(C₁-C₄)-alkylamino, hydroxycarbonyl, (C₁-C₄)-alkoxycarbonyl and aminocarbonyl and in (C₁-C₆)-alkyl a CH₂ group may be replaced by an oxygen atom, if this results in a chemically stable compound,
or R¹² and R¹³ together with the nitrogen atom to which they are attached form a 4- to 6-membered heterocycle which may contain a further ring heteroatom from the group consisting of N, O, S, SO and SO₂ and may be substituted up to two times by identical or different substituents from the group consisting of (C₁-C₄)-alkyl, hydroxyl, (C₁-C₄)-alkoxy, oxo, amino, mono- and di-(C₁-C₄)-alkylamino, where (C₁-C₄)-alkyl for its part may be substituted by hydroxyl or (C₁-C₄)-alkoxy,
R¹⁴ represents hydrogen or (C₁-C₄)-alkyl
and R¹⁵ represents (C₁-C₆)-alkyl, (C₃-C₆)-cycloalkyl, phenyl or 5- or 6-membered heteroaryl, where (C₁-C₆)-alkyl may be substituted by fluorine, chlorine, hydroxyl, (C₁-C₄)-alkoxy, mono- or di-(C₁-C₄)-alkylamino and phenyl and 5- or 6-membered heteroaryl for their part may be substituted up to two times by identical or different substituents from the group consisting of fluorine, chlorine, cyano, (C₁-C₄)-alkyl, trifluoromethyl, (C₁-C₄)-alkoxy and trifluoromethoxy,
and R⁶ represents hydrogen, fluorine or chlorine,
or a pharmaceutical acceptable salt, a solvate or a solvate of a salt thereof.

3. The compound of the formula (I) as claimed in claim 1 or 2, in which
A and E both represent CH,
Z represents O,
n represents the number 0 or 2,
R¹ represents (C₁-C₄)-alkyl which may be substituted by hydroxyl, (C₁-C₄)-alkoxy, hydroxycarbonyl, aminocarbonyl, (C₃-C₆)-cycloalkyl, phenyl or 5-membered heteroaryl or up to three times by fluorine, or represents (C₃-C₆)-cycloalkyl, phenyl or 5-membered heteroaryl, where the phenyl and heteroaryl groups mentioned may be substituted up to two times by identical or different substituents from the group consisting of fluorine, chlorine, cyano, methyl, trifluoromethyl, methoxy and trifluoromethoxy,
R² represents hydrogen,
R³ represents cyano, acetyl or (2-hydroxyethoxy)carbonyl,
R⁴ represents methyl
or R³ and R⁴ are attached to one another and together form a fused group of the formula 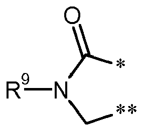 in which * denotes the point of attachment to the 5-position, shown in formula (I), of the dihydropyrimidine ring and ** denotes the point of attachment to the 6-position, shown in formula (I), of the dihydropyrimidine ring and R⁹ represents hydrogen, (C₁-C₄)-alkyl or (C₃-C₆)-cycloalkyl, where (C₁-C₄)-alkyl may be substituted by hydroxyl or (C₁-C₄)-alkoxy,
R⁵ represents hydrogen or (C₁-C₄)-alkyl which may be substituted by cyano or di-(C₁-C₄)-alkylamino, or represents a group of the formula -L²-C(=O)-NR¹²R¹³, -L²-C(=O)-NH-NR¹²R¹³ or -L²-SO₂-R¹⁵, in which L² represents a bond, -CH₂-, -CH₂CH₂- or -CH(CH₃)-, R¹² represents hydrogen or (C₁-C₄)-alkyl which may be substituted by hydroxyl or (C₁-C₄)-alkoxy, R¹³ represents hydrogen, (C₁-C₆)-alkyl or (C₃-C₆)-cycloalkyl, where (C₁-C₆)-alkyl may be substituted up to two times by identical or different substituents from the group consisting of hydroxyl, (C₁-C₄)-alkoxy, hydroxycarbonyl, (C₁-C₄)-alkoxycarbonyl and aminocarbonyl and in (C₁-C₆)-alkyl a CH₂ group may be replaced by an oxygen atom, if this results in a chemically stable compound, or R¹² and R¹³ together with the nitrogen atom to which they are attached form a 5- or 6-membered heterocycle which may contain a further ring heteroatom from the group consisting of N, O and S and may be substituted by (C₁-C₄)-alkyl, hydroxyl, (C₁-C₄)-alkoxy, oxo, hydroxycarbonyl, aminocarbonyl, 4- to 6-membered heterocyclyl or 5- or 6-membered heteroaryl, where (C₁-C₄)-alkyl for its part may be substituted by hydroxyl, (C₁-C₄)-alkoxy or hydroxycarbonyl, and R¹⁵ represents (C₁-C₄)-alkyl, (C₃-C₆)-cycloalkyl or phenyl, where (C₁-C₄)-alkyl may be substituted by (C₃-C₆)-cycloalkyl and phenyl may be substituted up to two times by identical or different substituents from the group consisting of fluorine, chlorine, cyano, methyl, trifluoromethyl, methoxy and trifluoromethoxy,
and R⁶ represents hydrogen or fluorine,
or a pharmaceutical acceptable salt, a solvate or a solvate of a salt thereof.

4. The compound of the formula (I) as claimed in one or more of claims 1 to 3, in which
A and E both represent CH,
Z represents O,
n represents the number 0 or 2,
R¹ represents (C₁-C₄)-alkyl which may be substituted by hydroxyl, (C₁-C₄)-alkoxy, hydroxycarbonyl, aminocarbonyl, (C₃-C₆)-cycloalkyl or phenyl, or represents (C₃-C₆)-cycloalkyl or phenyl, where the phenyl groups mentioned may be substituted up to two times by identical or different substituents from the group consisting of fluorine, chlorine, cyano, methyl, trifluoromethyl, methoxy and trifluoromethoxy,
R² represents hydrogen,
R³ represents cyano or acetyl,
R⁴ represents methyl
or R³ and R⁴ are attached to one another and together form a fused group of the formula 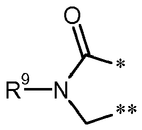 in which * denotes the point of attachment to the 5-position, shown in formula (I), of the dihydropyrimidine ring and ** denotes the point of attachment to the 6-position, shown in the formula (I), of the dihydropyrimidine ring and R⁹ represents hydrogen, (C₁-C₄)-alkyl or (C₃-C₆)-cycloalkyl, where (C₁-C₄)-alkyl may be substituted by hydroxyl or (C₁-C₄)-alkoxy,
R⁵ represents hydrogen, (C₁-C₄)-alkyl or a group of the formula -L²-C(=O)-NR¹²R¹³, -L²-C(=O)-NH-NR¹²R¹³ or L²-SO₂R¹⁵, in which L² represents a bond, -CH₂-, -CH₂CH₂- or -CH(CH₃)-, R¹² represents hydrogen or (C₁-C₄)-alkyl which may be substituted by hydroxyl or (C₁-C₄)-alkoxy, R¹³ represents hydrogen, (C₁-C₆)-alkyl or (C₃-C₆)-cycloalkyl, where (C₁-C₆)-alkyl may be substituted up to two times by identical or different substituents from the group consisting of hydroxyl, (C₁-C₄)-alkoxy, hydroxycarbonyl, (C₁-C₄)-alkoxycarbonyl and aminocarbonyl and in (C₁-C₆)-alkyl a CH₂ group may be replaced by an oxygen atom, if this results in a chemically stable compound, or R¹² and R¹³ together with the nitrogen atom to which they are attached form a 5- or 6-membered heterocycle which may contain a further ring heteroatom from the group consisting ofN, O and S and may be substituted by (C₁-C₄)-alkyl, hydroxyl, (C₁-C₄)-alkoxy or oxo, where (C₁-C₄)-alkyl for its part may be substituted by hydroxyl or (C₁-C₄)-alkoxy, and R¹⁵ represents (C₁-C₄)-alkyl, (C₃-C₆)-cycloalkyl or phenyl, where phenyl may be substituted up to two times by identical or different substituents from the group consisting of fluorine, chlorine, cyano, methyl, trifluoromethyl, methoxy and trifluoromethoxy,
and R⁶ represents hydrogen or fluorine,
or a pharmaceutical acceptable salt, a solvate or a solvate of a salt thereof.

5. The compound of the formula (I) as claimed in one or more of claims 1 to 4, in which
A and E both represent CH,
Z represents O,
n represents the number 2,
R¹ represents (C₁-C₄)-alkyl which may be substituted by hydroxyl, (C₁-C₄)-alkoxy, cyclopropyl, cyclobutyl or phenyl or up to three times by fluorine,
R² represents hydrogen,
R³ represents cyano or (2-hydroxyethoxy)carbonyl,
R⁴ represents methyl,
R⁵ represents hydrogen, (C₁-C₄)-alkyl or a group of the formula -L²-C(=O)-NH-R¹³ or -SO₂-R¹⁵, in which L² represents a bond or -CH₂-, R¹³ represents hydrogen or (C₁-C₄)-alkyl which may be substituted by hydroxyl or (C₁-C₄)-alkoxy, or (C₃-C₆)-cycloalkyl and R¹⁵ represents (C₁-C₄)-alkyl or (C₃-C₆)-cycloalkyl,
and R⁶ represents hydrogen,
or a pharmaceutical acceptable salt, a solvate or a solvate of a salt thereof.

6. The compound of the formula (I) as claimed in one or more of claims 1 to 5, in which
A and E both represent CH,
Z represents O,
n represents the number 2,
R¹ represents (C₁-C₄)-alkyl which may be substituted by hydroxyl or (C₁-C₄)-alkoxy,
R² represents hydrogen,
R³ represents cyano,
R⁴ represents methyl,
R⁵ represents hydrogen, (C₁-C₄)-alkyl or a group of the formula -CH₂-C(=O)-NH-R¹³ or -SO₂-R¹⁵, in which
R¹³ represents hydrogen or (C₁-C₄)-alkyl which may be substituted by hydroxyl or (C₁-C₄)-alkoxy
and R¹⁵ represents (C₁-C₄)-alkyl or (C₃-C₆)-cycloalkyl,
and R⁶ represents hydrogen,
or a pharmaceutical acceptable salt, a solvate or a solvate of a salt thereof.

7. The compound of the formula (I) having the configuration shown in formula (I-*ent*) at the 4-position of the dihydropyrimidine ring as claimed in one or more of claims 1 to 6, 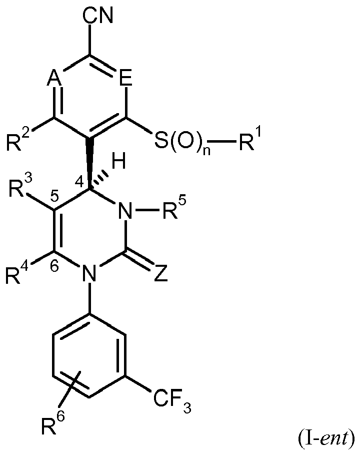 in which
A and E both represent CH,
Z represents O,
n represents the number 2,
R¹ represents (C₁-C₄)-alkyl which may be substituted by hydroxyl or (C₁-C₄)-alkoxy,
R² represents hydrogen,
R³ represents cyano,
R⁴ represents methyl,
R⁵ represents hydrogen, (C₁-C₄)-alkyl or a group of the formula -CH₂-C(=O)-NH-R¹³ or -SO₂-R¹⁵, in which R¹³ represents hydrogen or (C₁-C₄)-alkyl which may be substituted by hydroxyl
or (C₁-C₄)-alkoxy and
R¹⁵ represents (C₁-C₄)-alkyl or (C₃-C₆)-cycloalkyl, and
R⁶ represents hydrogen,
or a pharmaceutical acceptable salt, a solvate or a solvate of a salt thereof.

8. The compound of the formula (I) as claimed in one or more of claims 1 to 7, selected from the group consisting of:
4-{(4*S*)-5-Acetyl-6-methyl-2-oxo-1-[3-(trifluoromethyl)phenyl]-1,2,3,4-tetrahydropyrimidin-4-yl}-3-(methylsulfonyl)benzonitrile
(4*S*)-4-[4-Cyano-2-(methylsulfonyl)phenyl]-6-methyl-2-oxo-1-[3-(trifluoromethyl)phenyl]-1,2,3,4-tetrahydropyrimidine-5-carbonitrile
(6*S*)-5-Cyano-6-[4-cyano-2-(methylsulfonyl)phenyl]-*N*-(2-hydroxyethyl)-4-methyl-2-oxo-3-[3-(trifluoromethyl)phenyl]-3,6-dihydropyrimidine-1(2*H*)-carboxamide
(6*S*)-5-Cyano-6-[4-cyano-2-(methylsulfonyl)phenyl]-*N*',*N*'-bis(2-hydroxyethyl)-4-methyl-2-oxo-3-[3-(trifluoromethyl)phenyl]-3,6-dihydropyrimidine-1(2*H*)-carbohydrazide
(6*S*)-5-Cyano-6-[4-cyano-2-(methylsulfonyl)phenyl]-*N*-[2-(2-hydroxyethoxy)ethyl]-4-methyl-2-oxo-3-[3-(trifluoromethyl)phenyl]-3,6-dihydropyrimidine-1(2*H*)-carboxamide
(4*S*)-4-[4-Cyano-2-(methylsulfonyl)phenyl]-3-{[(3*S*)-3-hydroxypyrrolidin-1-yl]carbonyl}-6-methyl-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-3-{[4-(2-hydroxyethyl)piperazin-1-yl]carbonyl}-6-methyl-2-oxo-1-[3-(trifluoromethyl)phenyl]-1,2,3,4-tetrahydropyrimidine-5-carbonitrile
2- [(6*S*)-5-Cyano-6-[4-cyano-2-(methylsulfonyl)phenyl]-4-methyl-2-oxo-3-[3-(trifluoromethyl)phenyl]-3,6-dihydropyrimidin-1(2*H*)-yl]acetamide
(4*S*)-4-[4-Cyano-2-(methylsulfonyl)phenyl]-6-methyl-3-(methylsulfonyl)-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-6-methyl-3-(cyclopropylsulfonyl)-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-3,6-dimethyl-2-oxo-1-[3-(trifluoromethyl)phenyl]-1,2,3,4-tetrahydropyrimidine-5-carbonitrile
4- {(4*S*)-6-Methyl-3-(methylsulfonyl)-2,5-dioxo-1-[3-(trifluoromethyl)phenyl]-2,3,4,5,6,7-hexahydro-1*H*-pyrrolo[3,4-d]pyrimidin-4-yl}-3-(methylsulfonyl)benzonitrile
(4*S*)-4-[4-Cyano-2-(methylsulfanyl)phenyl]-6-methyl-2-oxo-1-[3-(trifluoromethyl)phenyl]-1,2,3,4-tetrahydropyrimidine-5-carbonitrile
(4*S*)-4-{4-Cyano-2-[(*S*)-methylsulfinyl]phenyl} -6-methyl-2-oxo-1-[3-(trifluoromethyl)phenyl]-1,2,3,4-tetrahydropyrimidine-5-carbonitrile
(*rac*)-1-(2*-*{4-[4-Cyano-2-(methylsulfonyl)phenyl]-2,5-dioxo-1-[3-(trifluoromethyl)phenyl]-1,2,3,4,5,7-hexahydro-6*H*-pyrrolo[3,4-d]pyrimidin-6-yl}ethyl)urea
(4*S*)-4-[4-Cyano-2-(methylsulfonyl)phenyl]-3-[(3-hydroxyazetidin-1-yl)carbonyl]-6-methyl-2-oxo-1-[3-(trifluoromethyl)phenyl]-1,2,3,4-tetrahydropyrimidine-5-carbonitrile
(4*S*)-3*-*[(3*R*)*-*3-Aminopiperidin-1-yl]carbonyl-4-[4-cyano-2-(methylsulfonyl)phenyl]-6-methyl-2-oxo-1-[3-(trifluoromethyl)phenyl]-1,2,3,4-tetrahydropyrimidine-5-carbonitrile
(4S)-4-[4-cyano-2-(methylsulfonyl)phenyl]-6-methyl-2-oxo-3-{[4-(pyridin-2-yl)piperazin-1-yl]carbonyl}-1-[3-(trifluoromethyl)phenyl]-1,2,3,4-tetrahydropyrimidine-5-carbonitrile
(6S)-5-cyano-6-[4-cyano-2-(methylsulfonyl)phenyl]-N,N-bis(2-hydroxypropyl)-4-methyl-2-oxo-3-[3-(trifluoromethyl)phenyl]3,6-dihydropyrimidine-1(2H)-carboxamide
(6S)-5-cyano-6-[4-cyano-2-(methylsulfonyl)phenyl]-N-(1-hydroxy-2-methylpropan-2-yl)-4-methyl-2-oxo-3-[3-(trifluoromethyl)phenyl]3,6-dihydropyrimidine-1(2H)-carboxamide
(4S)-4-[4-cyano-2-(methylsulfonyl)phenyl]-3-[(4-fluorophenyl)sulfonyl]-6-methyl-2-oxo-1-[3-(trifluoromethyl)phenyl]-1,2,3,4-tetrahydropyrimidine-5-carbonitrile
(4 S)-4-[4-cyano-2-(methylsulfonyl)phenyl]-3-{[1-(difluoromethyl)-5-methyl-1H-pyrazol-4-yl]sulfonyl}-6-methyl-2-oxo-1-[3-(trifluoromethyl)phenyl]-1,2,3,4-tetrahydropyrimidin-5-carbonitrile
(4S)-4-[4-cyano-2-(methylsulfonyl)phenyl]-3-[(2-cyanophenyl)sulfonyl]-6-methyl-2-oxo-1-[3-(trifluoromethyl)phenyl]-1,2,3,4-tetrahydropyrimidine-5-carbonitrile
(4S)-3-(Cyanomethyl)-4-[4-cyano-2-(methylsulfonyl)phenyl]-6-methyl-2-oxo-1-[3-(trifluoromethyl)-phenyl]-1,2,3,4-tetrahydropyrimidine-5-carbonitrile
Benzyl 5-cyano-6-[4-cyano-2-(methylsulfonyl)phenyl]-4-methyl-2-oxo-3-[3-(trifluoromethyl)-phenyl]-3,6-dihydropyrimidine-1(2*H*)-carboxylate
(4S)-4-[4-Cyano-2-(ethylsulfonyl)phenyl]-3,6-dimethyl-2-oxo-1-[3-(trifluoromethyl)phenyl]-1,2,3,4-tetrahydropyrimidine-5-carbonitrile
(*rac*)-4-[4-Cyano-2-((2-hydroxyethyl)sulfonyl)phenyl]-6-methyl-2-oxo-1-[3-(trifluoromethyl)-phenyl]-1,2,3,4-tetrahydropyrimidine-5-carbonitrile
(4*S*)-4-{4-Cyano-2-[phenylsulfonyl]phenyl}-6-methyl-2-oxo-1-[3-(trifluoromethyl)phenyl]-1,2,3,4-tetrahydropyrimidine-5-carbonitrile
2-Hydroxyethyl (4*S*)-4-[4-cyano-2-(methylsulfonyl)phenyl]-6-methyl-3-(methylsulfonyl)-2-oxo-1-[3-(trifluoromethyl)phenyl]-1,2,3,4-tetrahydropyrimidine-5-carboxylate
or a pharmaceutical acceptable salt, a solvate or a solvate of a salt thereof.

9. The compound of the formula (I) as claimed in one or more of claims 1 to 7, selected from
(4*S*)-4-[4-Cyano-2-(methylsulfonyl)phenyl]-6-methyl-2-oxo-1-[3-(trifluoromethyl)phenyl]-1,2,3,4-tetrahydropyrimidine-5-carboxamide
(4*S*)-4-[4-Cyano-2-(methylsulfonyl)phenyl]-6-methyl-2-oxo-1-[3-(trifluoromethyl)phenyl]-1,2,3,4-tetrahydropyrimidine-5-carbonitrile
(6*S*)-5-Cyano-6-[4-cyano-2-(methylsulfonyl)phenyl]-*N*-(2-hydroxyethyl)-4-methyl-2-oxo-3-[3-(trifluoromethyl)phenyl]-3,6-dihydropyrimidine-1(2*H*)-carboxamide
(6*S*)-5-Cyano-6-[4-cyano-2-(methylsulfonyl)phenyl]-*N*',*N*'-bis(2-hydroxyethyl)-4-methyl-2-oxo-3-[3-(trifluoromethyl)phenyl]-3,6-dihydropyrimidine-1(2*H*)-carbohydrazide
2-[(6*S*)-5-Cyano-6-[4-cyano-2-(methylsulfonyl)phenyl]-4-methyl-2-oxo-3-[3-(trifluoromethyl)-phenyl]-3,6-dihydropyrimidin-1(2*H*)-yl]acetamide
(4*S*)-4-[4-Cyano-2-(methylsulfonyl)phenyl]-6-methyl-2-oxo-3-[2-oxo-2-(3-oxopiperazin-1-yl)ethyl]-1-[3-(trifluoromethyl)phenyl]-1,2,3,4-tetrahydropyrimidine-5-carbonitrile
(4*S*-4-[4-Cyano-2-(methylsulfonyl)phenyl]-6-methyl-3-(methylsulfonyl)-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-6-methyl-3-{[2-(trifluoromethoxy)phenyl]sulfonyl}-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-6-methyl-3-(ethylsulfonyl)-2-oxo-1-[3-(trifluoromethyl)-phenyl]-1,2,3,4-tetrahydropyrimidine-5-carbonitrile
(4*S*)-4-[4-Cyano-2-(methylsulfonyl)phenyl]-6-methyl-3-(cyclopropylsulfonyl)-2-oxo-1-[3-(trifluoromethyl)phenyl]-1,2,3,4-tetrahydropyrimidine-5-carbonitrile
(4*S*)-4-[4-Cyano-2-(methylsulfonyl)phenyl]-3,6-dimethyl-2-oxo-1-[3-(trifluoromethyl)phenyl]-1,2,3,4-tetrahydropyrimidine-5-carbonitrile
4- {(4*S*)-6-Methyl-3-(methylsulfonyl)-2,5-dioxo-1-[3-(trifluoromethyl)phenyl]-2,3,4,5,6,7-hexahydro-1*H*-pyrrolo[3,4-d]pyrimidin-4-yl}-3-(methylsulfonyl)benzonitrile
(rac)-4-[4-Cyano-2-(methylsulfonyl)phenyl]-*N*-(2-hydroxyethyl)-6-methyl-2,5-dioxo-1-[3-(trifluoromethyl)phenyl]-1,2,4,5,6,7-hexahydro-3*H*-pyrrolo[3,4-d]pyrimidine-3-carboxamide
(4*S*)-4-{4-Cyano-2-[(*S*)-methylsulfinyl]phenyl}-6-methyl-2-oxo-1-[3-(trifluoromethyl)phenyl]-1,2,3,4-tetrahydropyrimidine-5-carbonitrile
(4*S*)-4-[4-cyano-2-(methylsulfonyl)phenyl]-3-[(4-fluorophenyl)sulfonyl]-6-methyl-2-oxo-1-[3-(trifluoromethyl)phenyl]-1,2,3,4-tetrahydropyrimidine-5-carbonitrile
(4S)-4-[4-cyano-2-(methylsulfonyl)phenyl]-3-[(1,2-dimethyl-1H-imidazol-4-yl)sulfonyl]-6-methyl-2-oxo-1-[3-(trifluoromethyl)phenyl]-1,2,3,4-tetrahydropyrimidine-5-carbonitrile
(4S)-3-(Cyanomethyl)-4-[4-cyano-2-(methylsulfonyl)phenyl] -6-methyl-2-oxo-1-[3-(trifluoromethyl)-phenyl]-1,2,3,4-tetrahydropyrimidine-5-carbonitrile
(4S)-4-[4-Cyano-2-(methylsulfonyl)phenyl]-3-(4-cyanophenyl)-6-methyl-2-oxo-1-[3-(trifluoromethyl)phenyl]-1,2,3,4-tetrahydropyrimidine-5-carbonitrile
(4S)-4- [4-Cyano-2-(methylsulfonyl)phenyl]-6-methyl-2-oxo-1,3-bis [3-(trifluoromethyl)phenyl]-1,2,3,4-tetrahydropyrimidine-5-carbonitrile
4-(4*S*)-3,6-Dimethyl-2,5-dioxo-1-[3-(trifluoromethyl)phenyl]-2,3,4,5,6,7-hexahydro-1*H*-pyrrolo-[3,4-d]pyrimidin-4-yl-3-(methylsulfonyl)benzonitrile
(4*S*)-4-[4-Cyano-2-(ethylsulfonyl)phenyl]-6-methyl-2-oxo-1-[3-(trifluoromethyl)phenyl]-1,2,3,4-tetrahydropyrimidine-5-carbonitrile
(4*S*)-4-[4-Cyano-2-(ethylsulfonyl)phenyl]-3,6-dimethyl-2-oxo-1-[3-(trifluoromethyl)phenyl]-1,2,3,4-tetrahydropyrimidine-5-carbonitrile
(4*S*)-4-[4-Cyano-2-(ethylsulfonyl)phenyl]-6-methyl-3-(methylsulfonyl)-2-oxo-1-[3-(trifluoromethyl)-phenyl]-1,2,3,4-tetrahydropyrimidine-5-carbonitrile
2-Hydroxyethyl (4*S*)-4-[4-cyano-2-(methylsulfonyl)phenyl]-6-methyl-3-(methylsulfonyl)-2-oxo-1-[3-(trifluoromethyl)phenyl]-1,2,3,4-tetrahydropyrimidine-5-carboxylate
or a pharmaceutical acceptable salt, a solvate or a solvate of a salt thereof.

10. The compound of the formula (I) as claimed in one or more of claims 1 to 7, with the structure
(4*S*)-4-[4-Cyano-2-(methylsulfonyl)phenyl]-3,6-dimethyl-2-oxo-1-[3-(trifluoromethyl)phenyl]-1,2,3,4-tetrahydropyrimidine-5-carbonitrile

11. The compound of the formula (I) as claimed in one or more of claims 1 to 7, with the structure
(4*S*)-4-[4-Cyano-2-(methylsulfonyl)phenyl]-6-methyl-3-(methylsulfonyl)-2-oxo-1-[3-(trifluoromethyl)phenyl]-1,2,3,4-tetrahydropyrimidine-5-carbonitrile
or a pharmaceutical acceptable salt, a solvate or a solvate of a salt thereof.

12. The compound of the formula (I) as claimed in one or more of claims 1 to 7, with the structure
(4*S*)-4-[4-Cyano-2-(methylsulfonyl)phenyl]-6-methyl-2-oxo-1-[3-(trifluoromethyl)phenyl]-1,2,3,4-tetrahydropyrimidine-5-carbonitrile
or a pharmaceutical acceptable salt, a solvate or a solvate of a salt thereof.

13. A process for preparing a compound of the formula (I) as defined in one or more of claims 1 to 12, **characterized in that** a compound of the formula (II) 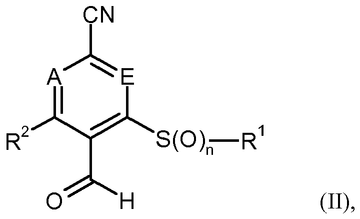 in which A, E, n, R¹ and R² have the meanings given in claims 1 to 12,
is reacted in the presence of an acid or an acid anhydride in a 3-component one-pot reaction or sequentially with a compound of the formula (III) 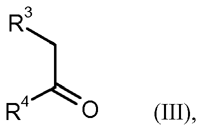 in which R³ and R⁴ have the meanings given in claims 1 to 12 and a compound of the formula (IV) 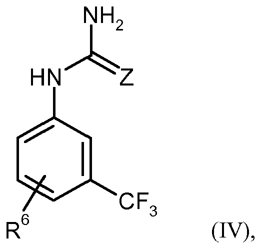 in which Z and R⁶ have the meanings given in claims 1 to 12, to give a compound of the formula (I-A) 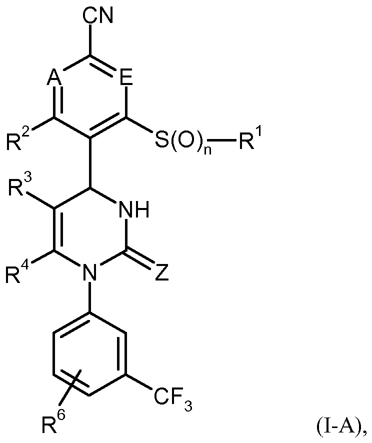 in which A, E, Z, n, R¹, R², R³, R⁴ and R⁶ each have the meanings given above,
and this compound is, in the case that R⁵ in formula (I) does not represent hydrogen, reacted in the presence of a base with a compound of the formula (V)
R^{5A}-X , (V)
in which
R^{5A} has the meaning of R⁵ given in claims 1 to 12, but does not represent hydrogen,
and X represents a leaving group, such as, for example, halogen, mesylate, tosylate or triflate,
to give a compound of the formula (I-B) 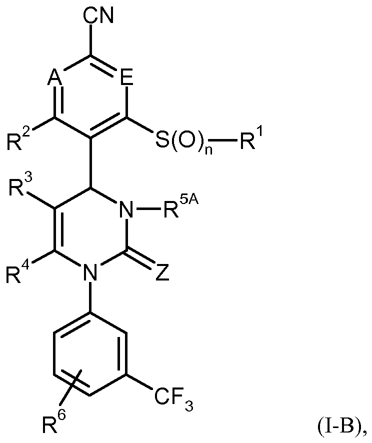 in which A, E, Z, n, R¹, R², R³, R⁴, R^{5A} and R⁶ each have the meanings given above,
and the compound of the formula (I-A) or (I-B) obtained in this manner is, if appropriate, separated by methods known to the person skilled in the art into its enantiomers and/or diastereomers and/or converted with the appropriate (i) solvents and/or (ii) bases or acids into its solvates, salts and/or solvates of the salts.

14. The compound as defined in one or more of of claims 1 to 12 for the treatment and/or prevention of diseases.

15. The compound as defined in one or more of claims 1 to 12 for use in a method for the treatment and/or prevention of pulmonary arterial hypertension (PAH) and other forms of pulmonary hypertension (PH), of chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

16. The compound as defined in one or more of of claims 1 to 12 for the treatment and/or prevention of bronchiectasy.

17. The use of a compound as defined in one or more of claims 1 to 12 for preparing a medicament for the treatment and/or prevention of pulmonary arterial hypertension (PAH) and other forms of pulmonary hypertension (PH), or chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

18. A medicament comprising a compound as defined in one or more of claims 1 to 12 in combination with one or more inert non-toxic pharmaceutically acceptable auxiliaries.

19. A medicament comprising a compound as defined in one or more of claims 1 to 12 in combination with one or more further active compounds selected from the group of the kinase inhibitors, the matrix metalloprotease inhibitors, stimulators and activators of soluble guanylate cyclase, prostacyclin analogs, endothelin receptor antagonists, phosphodiesterase inhibitors, beta-adrenergic receptor agonists, anticholinergics and glucocorticoids.

20. The medicament as claimed in claim 18 or 19 for the treatment and/or prevention of pulmonary arterial hypertension (PAH) and other forms of pulmonary hypertension (PH), or chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF).

21. The medicament as claimed in claim 18 or 19 for the treatment and/or prevention of bronchiectasy.

22. Use of at least one medicament as defined in one or more of claims 18 to 20 for the production of a medicament for the treatment and/or prevention of pulmonary arterial hypertension (PAH) and other forms of pulmonary hypertension (PH), or chronic-obstructive pulmonary diseases (COPD), of acute lung injury (ALI), of acute respiratory distress syndrome (ARDS), of pulmonary emphysema, of alpha-1 antitrypsin deficiency (AATD) and of cystic fibrosis (CF) in humans and animals.

## Revendications

1. Composé de formule (I) 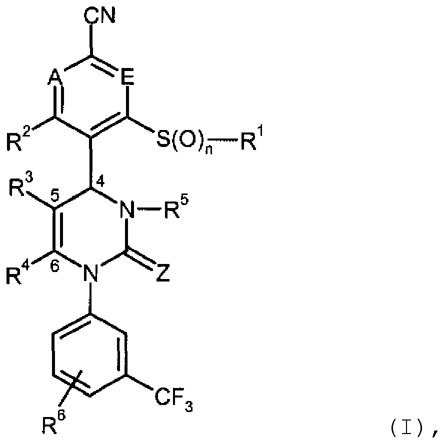 dans laquelle
A et E représentent l'un et l'autre C-R⁷ ou l'un des deux chaînons de cycle A et E représente N et l'autre représente C-R⁷, où R⁷ représente chaque fois un atome d'hydrogène, de fluor ou de chlore,
Z représente 0 ou S,
n représente le nombre 0, 1 ou 2,
R¹ représente un groupe alkyle en C₁-C₆, qui peut être substitué par hydroxy, alcoxy en C₁-C₄, amino, mono- ou di-[alkyl(C₁-C₄)]amino, hydroxycarbonyle, aminocarbonyle, cycloalkyle en C₃-C₆, phényle ou hétéroaryle à 5 ou 6 chaînons ou jusqu'à cinq fois par le fluor, ou représente un groupe alcényle en C₂-C₆, cycloalkyle en C₃-C₆, phényle ou hétéroaryle à 5 ou 6 chaînons, les groupes cycloalkyle en C₃-C₆ nommés pouvant porter jusqu'à deux substituants, identiques ou différents, alkyle en C₁-C₄, hydroxy et/ou alcoxy en C₁-C₄ et les groupes phényle et hétéroaryle nommés pouvant porter jusqu'à deux substituants, identiques ou différents, fluoro, chloro, cyano, alkyle en C₁-C₄, difluorométhyle, trifluorométhyle, alcoxy en C₁-C₄, difluorométhoxy et/ou trifluorométhoxy,
R² représente un atome d'hydrogène, de fluor ou de chlore,
R³ représente un groupe cyano ou un groupe de formule -C(=O)-R⁸, -C (=O)-O-R⁸, -C (=O)-NH₂ ou -C(=O)-NH-R⁸, où R⁸ représente un groupe alkyle en C₁-C₆, alcényle en C₃-C₆ ou cycloalkyle en C₃-C₆, les groupes alkyle en C₁-C₆ et cycloalkyle en C₃-C₆ pouvant pour leur part porter jusqu'à deux substituants, identiques ou différents, hydroxy, alcoxy en C₁-C₄, hydroxycarbonyle, alcoxy(C₁-C₄)carbonyle, amino, mono- et/ou di-[alkyl(C₁-C₄)]amino et dans les groupes alkyle en C₁-C₆ et cycloalkyle en C₃-C₆ chaque fois un groupe CH₂ pouvant, tant qu'il en résulte un composé chimiquement stable, être échangé contre un atome d'oxygène,
R⁴ représente le groupe méthyle ou éthyle
ou R³ et R⁴ sont liés l'un à l'autre et forment ensemble un groupe condensé de formule 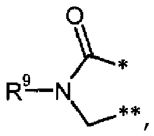 dans laquelle * désigne le point de liaison avec la position 5, indiquée dans la formule (I), du cycle dihydropyrimidine et ** désigne le point de liaison avec la position 6, indiquée dans la formule (I), du cycle dihydropyrimidine et R⁹ représente un atome d'hydrogène, un groupe alkyle en C₁-C₆ ou cycloalkyle en C₃-C₆, le groupe alkyle en C₁-C₆ pouvant être substitué par hydroxy, alcoxy en C₁-C₄, aminocarbonyle, aminocarbonylamino, acyl(C₁-C₄)amino ou cycloalkyle en C₃-C₆,
R⁵ représente un atome d'hydrogène ou un groupe alkyle en C₁-C₆, qui peut être substitué par cyano, hydroxy, alcoxy en C₁-C₄, amino, mono- ou di-[alkyl(C₁-C₄)]amino ou cycloalkyle en C₃-C₆ ou jusqu'à trois fois par le fluor, ou représente un groupe phényle, pyridyle ou pyrimidinyle, les groupes phényle, pyridyle et pyrimidinyle pouvant pour leur part porter jusqu'à deux substituants, identiques ou différents, fluoro, chloro, cyano, alkyle en C₁-C₄, trifluorométhyle, alcoxy en C₁-C₄ et/ou trifluorométhoxy,
ou R⁵ représente un groupe de formule -C(=O)-O-R₁₀, -L¹-C(=O)-O-R¹¹, -L²-C(=O)-NR¹²R¹³, -L²-SO₂-NR¹²R¹³, -L²-C (=O)-NR₁₄-NR¹²R¹³ ou -L²-SO₂-R¹⁵, où L¹ représente un groupe alcane(C₁-C₆)diyle, L² représente une liaison ou un groupe alcane (C₁-C₆) diyle, R¹⁰ représente un groupe alkyle en C₁-C₆, qui peut être substitué par cycloalkyle en C₃-C₆ ou phényle, R¹¹ représente un atome d'hydrogène ou un groupe alkyle en C₁-C₆, qui peut être substitué par cycloalkyle en C₃-C₆ ou phényle, R¹² et R¹³ sont identiques ou différents et représentent, indépendamment l'un de l'autre, un atome d'hydrogène, un groupe alkyle en C₁-C₆, cycloalkyle en C₃-C₆ ou hétérocyclyle à 4-6 chaînons, les groupes alkyle en C₁-C₆, cycloalkyle en C₃-C₆ et hétérocyclyle à 4-6 chaînons pouvant pour leur part porter jusqu'à deux substituants, identiques ou différents, fluoro, hydroxy, alcoxy en C₁-C₄, oxo, amino, mono- ou di-[alkyl(C₁-C₄)] amino, hydroxycarbonyle, alcoxy(C₁-C₄)carbonyle et/ou aminocarbonyle et dans le groupe alkyle en C₁-C₆ un groupe CH₂ pouvant, tant qu'il en résulte un composé chimiquement stable, être échangé contre un atome d'oxygène, et les groupes cycloalkyle en C₃-C₆ et hétérocyclyle à 4-6 chaînons pouvant en outre être jusqu'à deux fois substitués par alkyle en C₁-C₄, le même ou différent, qui pour sa part peut être substitué par hydroxy, alcoxy en C₁-C₄ ou hydroxycarbonyle,
ou R¹² et R¹³ forment ensemble, avec l'atome d'azote auquel ils sont fixés, un hétérocycle à 4-6 chaînons, qui peut contenir un autre hétéroatome formant le cycle, choisi dans la série constituée par N, O, S, SO ou SO₂ et porter jusqu'à deux substituants, identiques ou différents, alkyle en C₁-C₄, hydroxy, alcoxy en C₁-C₄, oxo, amino, mono- ou di-[alkyl(C₁-C₄)] amino, hydroxycarbonyle, aminocarbonyle, cycloalkyle en C₃-C₆, hétérocyclyle à 4-6 chaînons et/ou hétéroaryle à 5 ou 6 chaînons, le groupe alkyle en C₁-C₄ pouvant pour sa part être substitué par hydroxy, alcoxy en C₁-C₄ ou hydroxycarbonyle, R¹⁴ représente un atome d'hydrogène ou un groupe alkyle en C₁-C₄ et R¹⁵ représente un groupe alkyle en C₁-C₆, cycloalkyle en C₃-C₆, phényle ou hétéroaryle à 5 ou 6 chaînons, le groupe alkyle en C₁-C₆ pouvant être substitué par chloro, hydroxy, alcoxy en C₁-C₄, mono- ou di-[alkyl(C₁-C₄)] amino ou cycloalkyle en C₃-C₆ ou jusqu'à trois fois par le fluor et les groupes phényle et hétéroaryle à 5 ou 6 chaînons pouvant pour leur part porter jusqu'à deux substituants, identiques ou différents, fluoro, chloro, cyano, alkyle en C₁-C₄, difluorométhyle, trifluorométhyle, alcoxy en C₁-C₄ et/ou trifluorométhoxy,
et R⁶ représente un atome d'hydrogène, de fluor ou de chlore,
ainsi que ses sels, produits de solvatation et produits de solvatation des sels, pharmaceutiquement acceptables.

2. Composé de formule (I) selon la revendication 1, dans lequel
A et E représentent l'un et l'autre C-R⁷ ou l'un des deux chaînons de cycle A et E représente N et l'autre représente C-R⁷, où R⁷ représente chaque fois un atome d'hydrogène, de fluor ou de chlore,
Z représente 0 ou S,
n représente le nombre 0, 1 ou 2,
R¹ représente un groupe alkyle en C₁-C₆, qui peut être substitué par hydroxy, alcoxy en C₁-C₄, amino, mono- ou di-[alkyl(C₁-C₄)]amino, hydroxycarbonyle, aminocarbonyle, cycloalkyle en C₃-C₆ ou phényle, ou représente un groupe alcényle en C₂-C₆, cycloalkyle en C₃-C₆ ou phényle, les groupes cycloalkyle en C₃-C₆ nommés pouvant porter jusqu'à deux substituants, identiques ou différents, alkyle en C₁-C₄, hydroxy et/ou alcoxy en C₁-C₄ et les groupes phényle nommés pouvant porter jusqu'à deux substituants, identiques ou différents, fluoro, chloro, cyano, alkyle en C₁-C₄, difluorométhyle, trifluorométhyle, alcoxy en C₁-C₄, difluorométhoxy et/ou trifluorométhoxy,
R² représente un atome d'hydrogène, de fluor ou de chlore,
R³ représente un groupe cyano ou un groupe de formule -C (=O) -R⁸, -C (=O) -O-R⁸, -C (=O) -NH₂ ou -C (=O) -NH-R⁸, où R⁸ représente un groupe alkyle en C₁-C₆, alcényle en C₃-C₆ ou cycloalkyle en C₃-C₆, les groupes alkyle en C₁-C₆ et cycloalkyle en C₃-C₆ pouvant pour leur part porter jusqu'à deux substituants, identiques ou différents, hydroxy, alcoxy en C₁-C₄, hydroxycarbonyle, alcoxy(C₁-C₄)carbonyle, amino, mono- et/ou di-[alkyl(C₁-C₄)]amino et dans les groupes alkyle en C₁-C₆ et cycloalkyle en C₃-C₆ chaque fois un groupe CH₂ pouvant, tant qu'il en résulte un composé chimiquement stable, être échangé contre un atome d'oxygène,
R⁴ représente le groupe méthyle ou éthyle
ou R³ et R⁴ sont liés l'un à l'autre et forment ensemble un groupe condensé de formule 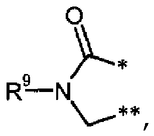 dans laquelle * désigne le point de liaison avec la position 5, indiquée dans la formule (I), du cycle dihydropyrimidine et ** désigne le point de liaison avec la position 6, indiquée dans la formule (I), du cycle dihydropyrimidine et R⁹ représente un atome d'hydrogène, un groupe alkyle en C₁-C₆ ou cycloalkyle en C₃-C₆, le groupe alkyle en C₁-C₆ pouvant être substitué par hydroxy, alcoxy en C₁-C₄, aminocarbonyle, acyl(C₁-C₄)amino ou cycloalkyle en C₃-C₆,
R⁵ représente un atome d'hydrogène ou un groupe alkyle en C₁-C₆, qui peut être jusqu'à trois fois substitué par le fluor, ou représente un groupe phényle, pyridyle ou pyrimidinyle, les groupes phényle, pyridyle et pyrimidinyle pouvant pour leur part porter jusqu'à deux substituants, identiques ou différents, fluoro, chloro, cyano, alkyle en C₁-C₄, trifluorométhyle, alcoxy en C₁-C₄ et/ou trifluorométhoxy,
ou R⁵ représente un groupe de formule -C(=O)-O-R¹⁰, -L¹-C(=O) -O-R¹¹, -L²-C (=O) -NR¹²R¹³, -L²-SO₂-NR¹²R¹³, -L²-C(=O) -NR¹⁴-NR¹²R¹³ ou -L²-SO₂-R¹⁵, où L¹ représente un groupe alcane(C₁-C₆)diyle, L² représente une liaison ou un groupe alcane (C₁-C₆) diyle, R¹⁰ représente un groupe alkyle en C₁-C₆, R¹¹ représente un atome d'hydrogène ou un groupe alkyle en C₁-C₆, R¹² et R¹³ sont identiques ou différents et représentent, indépendamment l'un de l'autre, un atome d'hydrogène, un groupe alkyle en C₁-C₆, cycloalkyle en C₃-C₆ ou hétérocyclyle à 4-6 chaînons, les groupes alkyle en C₁-C₆, cycloalkyle en C₃-C₆ et hétérocyclyle à 4-6 chaînons pouvant pour leur part porter jusqu'à deux substituants, identiques ou différents, fluoro, hydroxy, alcoxy en C₁-C₄, oxo, amino, mono- ou di-[alkyl(C₁-C₄)] amino, hydroxycarbonyle, alcoxy(C₁-C₄)carbonyle et/ou aminocarbonyle et dans le groupe alkyle en C₁-C₆ un groupe CH₂ pouvant, tant qu'il en résulte un composé chimiquement stable, être échangé contre un atome d'oxygène, ou R¹² et R¹³ forment ensemble, avec l'atome d'azote auquel ils sont fixés, un hétérocycle à 4-6 chaînons, qui peut contenir un autre hétéroatome formant le cycle, choisi dans la série constituée par N, O, S, SO ou SO₂ et porter jusqu'à deux substituants, identiques ou différents, alkyle en C₁-C₄, hydroxy, alcoxy en C₁-C₄, oxo, amino, mono- et/ou di-[alkyl (C₁-C₄) ] amino, le groupe alkyle en C₁-C₄ pouvant pour sa part être substitué par hydroxy ou alcoxy en C₁-C₄, R¹⁴ représente un atome d'hydrogène ou un groupe alkyle en C₁-C₄ et R¹⁵ représente un groupe alkyle en C₁-C₆, cycloalkyle en C₃-C₆, phényle ou hétéroaryle à 5 ou 6 chaînons, le groupe alkyle en C₁-C₆ pouvant être substitué par fluoro, chloro, hydroxy, alcoxy en C₁-C₄, mono- ou di-[alkyl(C₁-C₄)]amino et les groupes phényle et hétéroaryle à 5 ou 6 chaînons pouvant pour leur part porter jusqu'à deux substituants, identiques ou différents, fluoro, chloro, cyano, alkyle en C₁-C₄, trifluorométhyle, alcoxy en C₁-C₄ et/ou trifluorométhyle,
et R⁶ représente un atome d'hydrogène, de fluor ou de chlore,
ainsi que ses sels, produits de solvatation et produits de solvatation des sels, pharmaceutiquement acceptables.

3. Composé de formule (I) selon la revendication 1 ou 2, dans lequel
A et E représentent l'un et l'autre CH,
Z représente 0,
n représente le nombre 0 ou 2,
R¹ représente un groupe alkyle en C₁-C₄, qui peut être substitué par hydroxy, alcoxy en C₁-C₄, hydroxycarbonyle, aminocarbonyle, cycloalkyle en C₃-C₆, phényle ou hétéroaryle à 5 chaînons ou jusqu'à trois fois par le fluor, ou représente un groupe cycloalkyle en C₃-C₆, phényle ou hétéroaryle à 5 chaînons, les groupes phényle et hétéroaryle nommés pouvant porter jusqu'à deux substituants, identiques ou différents, fluoro, chloro, cyano, méthyle, trifluorométhyle, méthoxy et/ou trifluorométhoxy.
R² représente un atome d'hydrogène,
R³ représente un groupe cyano, acétyle ou (2-hydroxyéthoxy)carbonyle,
R⁴ représente le groupe méthyle
ou R³ et R⁴ sont liés l'un à l'autre et forment ensemble un groupe condensé de formule 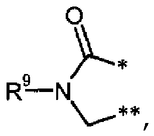 dans laquelle * désigne le point de liaison avec la position 5, indiquée dans la formule (I), du cycle dihydropyrimidine et ** désigne le point de liaison avec la position 6, indiquée dans la formule (I), du cycle dihydropyrimidine et R⁹ représente un atome d'hydrogène, un groupe alkyle en C₁-C₄ ou cycloalkyle en C₃-C₆, le groupe alkyle en C₁-C₄ pouvant être substitué par hydroxy ou alcoxy en C₁-C₄,
R⁵ représente un atome d'hydrogène ou un groupe alkyle en C₁-C₄, qui peut être substitué par cyano ou di[alkyl(C₁-C₄)]amino, ou représente un groupe de formule -L²-C (=O) -NR¹²R,¹³ -L²-C (=O)H-NR¹²R¹³ ou -L²-SO₂-R¹⁵, où L² représente une liaison, -CH₂-, -CH₂CH₂- ou -CH (CH₃)-, R¹² représente un atome d'hydrogène ou un groupe alkyle en C₁-C₄, qui peut être substitué par hydroxy ou alcoxy en C₁-C₄, R¹³ représente un atome d'hydrogène, un groupe alkyle en C₁-C₆ ou cycloalkyle en C₃-C₆, le groupe alkyle en C₁-C₆ pouvant porter jusqu'à deux substituants, identiques ou différents, hydroxy, alcoxy en C₁-C₄, hydroxycarbonyle, alcoxy(C₁-C₄)carbonyle et/ou aminocarbonyle et dans le groupe alkyle en C₁-C₆ un groupe CH₂ pouvant, tant qu'il en résulte un composé chimiquement stable, être échangé contre un atome d'oxygène, ou R¹² et R¹³ forment ensemble, avec l'atome d'azote auquel ils sont fixés, un hétérocycle à 5-6 chaînons, qui peut contenir un autre hétéroatome formant le cycle, choisi dans la série constituée par N, 0 ou S et être substitué par alkyle en C₁-C₄, hydroxy, alcoxy en C₁-C₄, oxo, hydroxycarbonyle, aminocarbonyle, hétérocyclyle à 4-6 chaînons ou hétéroaryle à 5 ou 6 chaînons, le groupe alkyle en C₁-C₄ pouvant pour sa part être substitué par hydroxy, alcoxy en C₁-C₄ ou hydroxycarbonyle, et R¹⁵ représente un groupe alkyle en C₁-C₄, cycloalkyle en C₃-C₆ ou phényle, le groupe alkyle en C₁-C₄ pouvant être substitué par cycloalkyle en C₃-C₆ et le groupe phényle pouvant porter jusqu'à deux substituants, identiques ou différents, fluoro, chloro, cyano, méthyle, trifluorométhyle, méthoxy et/ou trifluorométhoxy,
et R⁶ représente un atome d'hydrogène ou de fluor,
ainsi que ses sels, produits de solvatation et produits de solvatation des sels, pharmaceutiquement acceptables.

4. Composé de formule (I) selon une ou plusieurs des revendications 1 à 3, dans lequel
A et E représentent l'un et l'autre CH,
Z représente 0,
n représente le nombre 0 ou 2,
R¹ représente un groupe alkyle en C₁-C₄, qui peut être substitué par hydroxy, alcoxy en C₁-C₄, hydroxycarbonyle, aminocarbonyle, cycloalkyle en C₃-C₆ ou phényle, ou représente un groupe cycloalkyle en C₃-C₆ ou phényle, les groupes phényle nommés pouvant porter jusqu'à deux substituants, identiques ou différents, fluoro, chloro, cyano, méthyle, trifluorométhyle, méthoxy et/ou trifluorométhoxy.
R² représente un atome d'hydrogène,
R³ représente un groupe cyano ou acétyle,
R⁴ représente le groupe méthyle
ou R³ et R⁴ sont liés l'un à l'autre et forment ensemble un groupe condensé de formule 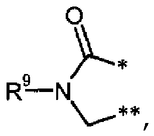 dans laquelle * désigne le point de liaison avec la position 5, indiquée dans la formule (I), du cycle dihydropyrimidine et ** désigne le point de liaison avec la position 6, indiquée dans la formule (I), du cycle dihydropyrimidine et R⁹ représente un atome d'hydrogène, un groupe alkyle en C₁-C₄ ou cycloalkyle en C₃-C₆, le groupe alkyle en C₁-C₄ pouvant être substitué par hydroxy ou alcoxy en C₁-C₄,
R⁵ représente un atome d'hydrogène, un groupe alkyle en C₁-C₄ ou un groupe de formule -L²-C(=O)-NR¹²R¹³, -L²-C(=O)-NH-NR¹²R¹³ ou -L²-SO₂-R¹⁵, où L² représente une liaison, -CH₂-, -CH₂CH₂- ou -CH(CH³)-, R¹² représente un atome d'hydrogène ou un groupe alkyle en C₁-C₄, qui peut être substitué par hydroxy ou alcoxy en C₁-C₄, R¹³ représente un atome d'hydrogène, un groupe alkyle en C₁-C₆ ou cycloalkyle en C₃-C₆, le groupe alkyle en C₁-C₆ pouvant porter jusqu'à deux substituants, identiques ou différents, hydroxy, alcoxy en C₁-C₄, hydroxycarbonyle, alcoxy(C₁-C₄)carbonyle et/ou aminocarbonyle et dans le groupe alkyle en C₁-C₆ un groupe CH₂ pouvant, tant qu'il en résulte un composé chimiquement stable, être échangé contre un atome d'oxygène, ou R¹² et R¹³ forment ensemble, avec l'atome d'azote auquel ils sont fixés, un hétérocycle à 5-6 chaînons, qui peut contenir un autre hétéroatome formant le cycle, choisi dans la série constituée par N, 0 ou S et être substitué par alkyle en C₁-C₄, hydroxy, alcoxy en C₁-C₄ ou oxo, le groupe alkyle en C₁-C₄ pouvant pour sa part être substitué par hydroxy ou alcoxy en C₁-C₄, et R¹⁵ représente un groupe alkyle en C₁-C₄, cycloalkyle en C₃-C₆ ou phényle, le groupe phényle pouvant porter jusqu'à deux substituants, identiques ou différents, fluoro, chloro, cyano, méthyle, trifluorométhyle, méthoxy et/ou trifluorométhoxy,
et R⁶ représente un atome d'hydrogène ou de fluor,
ainsi que ses sels, produits de solvatation et produits de solvatation des sels, pharmaceutiquement acceptables.

5. Composé de formule (I) selon une ou plusieurs des revendications 1 à 4, dans lequel
A et E représentent l'un et l'autre CH,
Z représente 0,
n représente le nombre 2,
R¹ représente un groupe alkyle en C₁-C₄, qui peut être substitué par hydroxy, alcoxy en C₁-C₄, cyclopropyle, cyclobutyle ou phényle, ou jusqu'à trois fois par le fluor,
R² représente un atome d'hydrogène,
R³ représente un groupe cyano ou (2-hydroxyéthoxy)-carbonyle,
R⁴ représente le groupe méthyle,
R⁵ représente un atome d'hydrogène, un groupe alkyle en C₁-C₄ ou un groupe de formule -L²-C(=O)-NH-R¹³ ou -SO₂-R¹⁵, où L² représente une liaison ou -CH₂-, R¹³ représente un atome d'hydrogène ou un groupe alkyle en C₁-C₄ qui peut être substitué par hydroxy ou alcoxy en C₁-C₄, ou représente un groupe cycloalkyle en C₃-C₆, et R¹⁵ représente un groupe alkyle en C₁-C₄ ou cycloalkyle en C₃-C₆,
et R⁶ représente un atome d'hydrogène,
ainsi que ses sels, produits de solvatation et produits de solvatation des sels, pharmaceutiquement acceptables.

6. Composé de formule (I) selon une ou plusieurs des revendications 1 à 5, dans lequel
A et E représentent l'un et l'autre CH,
Z représente 0,
n représente le nombre 2,
R¹ représente un groupe alkyle en C₁-C₄, qui peut être substitué par hydroxy ou alcoxy en C₁-C₄,
R² représente un atome d'hydrogène,
R³ représente le groupe cyano,
R⁴ représente le groupe méthyle,
R⁵ représente un atome d'hydrogène, un groupe alkyle en C₁-C₄ ou un groupe de formule -CH₂-C((=O)-NH-R¹³ ou -SO₂-R¹⁵, où R¹³ représente un atome d'hydrogène ou un groupe alkyle en C₁-C₄ qui peut être substitué par hydroxy ou alcoxy en C₁-C₄, et R¹⁵ représente un groupe alkyle en C₁-C₄ ou cycloalkyle en C₃-C₆,
et R⁶ représente un atome d'hydrogène,
ainsi que ses sels, produits de solvatation et produits de solvatation des sels, pharmaceutiquement acceptables.

7. Composé selon la formule (I) ayant la configuration représentée dans la formule (I-ent) en la position 4 du cycle dihydropyrimidine selon une ou plusieurs des revendications 1 à 6, 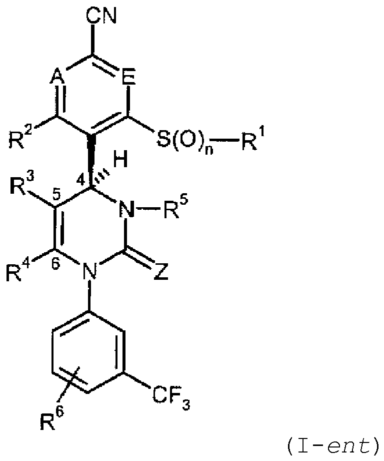 dans laquelle
A et E représentent l'un et l'autre CH,
Z représente 0,
n représente le nombre 2,
R¹ représente un groupe alkyle en C₁-C₄, qui peut être substitué par hydroxy ou alcoxy en C₁-C₄,
R² représente un atome d'hydrogène,
R³ représente le groupe cyano,
R⁴ représente le groupe méthyle,
R⁵ représente un atome d'hydrogène, un groupe alkyle en C₁-C₄ ou un groupe de formule -CH₂-C((=O)-NH-R¹³ ou -SO₂-R¹⁵, où
R¹³ représente un atome d'hydrogène ou un groupe alkyle en C₁-C₄ qui peut être substitué par hydroxy ou alcoxy en C₁-C₄, et
R¹⁵ représente un groupe alkyle en C₁-C₄ ou cycloalkyle en C₃-C₆, et
R⁶ représente un atome d'hydrogène,
ainsi que ses sels, produits de solvatation et produits de solvatation des sels, pharmaceutiquement acceptables.

8. Composé de formule (I) selon une ou plusieurs des revendications 1 à 7, choisi dans le groupe suivait :
4-{(4*S*)-5-acétyl-6-méthyl-2-oxo-1-[3-(trifluorométhyl)-phényl]-1,2,3,4-tétrahydropyrimidin-4-yl}-3-(méthylsulfonyl)benzonitrile
(4*S*)-4-[4-cyano-2-(méthylsulfonyl)phényl]-6-méthyl-2-oxo-1-[3-(trifluorométhyl)phényl]-1,2,3,4-tétrahydropyrimidine-5-carbonitrile
(6*S*)-5-cyano-6-[4-cyano-2-(méthylsulfonyl)phényl]-*N*-(2-hydroxyéthyl)-4-méthyl-2-oxo-3-[3-(trifluorométhyl)-phényl]-3,6-dihydropyrimidine-1(2*H*)-carboxamide
(6*S*)-5-cyano-6-[4-cyano-2-(méthylsulfonyl)phényl]-*N',N'*-bis(2-hydroxyéthyl)-4-méthyl-2-oxo-3-[3-(trifluorométhyl)phényl]-3,6-dihydropyrimidine-1(2*H*)-carbohydrazide
(6*S*)-5-cyano-6-[4-cyano-2-(méthylsulfonyl)phényl]-*N*-[2-(2-hydroxyéthoxy)éthyl]-4-méthyl-2-oxo-3-[3-(trifluorométhyl)phényl]-3,6-dihydropyrimidin-1(2*H*)-carboxamide (4*S*)-4-[4-cyano-2-(méthylsulfonyl)phényl]-3-{[(3S)-3-hydroxypyrrolidin-1-yl]carbonyl}-6-méthyl-2-oxo-1-[3-(trifluorométhyl)phényl]-1,2,3,4-tétrahydropyrimidin-5-carbonitrile
(4*S*)-4-[4-cyano-2-{méthylsulfonyl)phényl]-3-{[4-(2-hydroxyéthyl)pipérazin-1-yl]-carbonyl}-6-méthyl-2-oxo-1-[3-(trifluorométhyl)phényl]-1,2,3,4-tétrahydropyrimidine-5-carbonitrile
2-[(6*S*)-5-cyano-6-[4-cyano-2-(méthylsulfonyl)phényl]-4-méthyl-2-oxo-3-[3-(trifluorométhyl)phényl]-3,6-dihydropyrimidin-1(2*H*)-yl]acétamide
(4*S*)-4-[4-cyano-2-(méthylsulfonyl)phényl]-6-méthyl-3-(méthylsulfonyl)-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-6-méthyl-3-(cyclopropylsulfonyl)-2-oxo-1-[3-(trifluorométhyl)-phényl]-1,2,3,4-tétrahydropyrimidine-5-carbonitrile
(4S)-4-[4-cyano-2-(méthylsulfonyl)phényl]-3,6{diméthyl-2-oxo-1-[3-(trifluorométhyl)phényl]-1,2,3,4-tétrahydropyrimidine-5-carbonitrile
4-{(4*S*)-6-méthyl-3-(méthylsulfonyl)-2,5-dioxo-1-[3-(trifluorométhyl)phényl]-2,3,4,5,6,7-hexahydro-1*H-*pyrrolo[3,4-d]pyrimidin-4-yl}-3-(méthylsulfonyl)-benzonitrile
(4*S*)-4-[4-cyano-2-(méthylsulfanyl)phényl]-6-méthyl-2-oxo-1-[3-(trifluorométhyl)phényl]-1,2,3,4-tétrahydropyrimidine-5-carbonitrile
(4*S*)-4-{4-cyano-2-[(S)-méthylsulfinyl]phényl}-6-méthyl-2-oxo-1-[3-(trifluorométhyl)-phényl]-1,2,3,4-tétrahydropyrimidine-5-carbonitrile
(*rac*)-1-(2-{4-[4-cyano-2-(méthylsulfonyl)phényl]-2,5-dioxo-1-[3-(trifluorométhyl)phényl]-1,2,3,4,5,7-hexahydro-6*H*-pyrrolo[3,4-d]pyrimidin-6-yl}éthyl)urée
(4S)-4-[4-cyano-2-(méthylsulfonyl)phényl]-3-[(3-hydroxyazétidin-1-yl)carbonyl]-6-méthyl-2-oxo-1-[3-(trifluorométhyl)phényl]-1,2,3,4-tétrahydropyrimidine-5-carbonitrile
(4*S*)-3-[(3*R*)-3-aminopipéridin-1-yl]carbonyl-4-[4-cyano-2-(méthylsulfonyl)phényl]-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-6-méthyl-2-oxo-3-{[4-(pyridin-2-yl)pipérazin-1-yl]carbonyl}-1-[3-(trifluorométhyl)phényl]-1,2,3,4-tétrahydropyrimidine-5-carbonitrile
(6*S*)-5-cyano-6-[4-cyano-2-(méthylsulfonyl)phényl]-*N,N-*bis(2-hydroxypropyl)-4-méthyl-2-oxo-3-[3-(trifluorométhyl)phényl]-3,6-dihydropyrimidine-1(2*H*)-carboxamide
(6*S*)-5-cyano-6-[4-cyano-2-(méthylsulfonyl)phényl]-*N*-(1-hydroxy-2-méthylpropan-2-yl)-4-méthyl-2-oxo-3-[3-(trifluorométhyl)phényl]-3,6-dihydropyrimidin-1(2*H*)-carboxamide
(4*S*)-4-[4-cyano-2-(méthylsulfonyl)phényl]-3-[(4-fluorophényl)sulfonyl]-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl)phényl-3-{[1-(difluorométhyl)-5-méthyl-1H-pyrazol-4-yl]sulfonyl}-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-3-[(2-cyanophényl)sulfonyl]-6-méthyl-2-oxo-1-[3-(trifluorométhyl)phényl]-1,2,3,4-tétrahydropyrimidine-5-carbonitrile
(4*S*)-3-(cyanométhyl)-4-[4-cyano-2-(méthylsulfonyl)-phényl]-6-méthyl-2-oxo-1-[3-(trifluorométhyl)phényl]-1,2,3,4-tétrahydropyrimidine-5-carbonitrile
5-cyano-6-[4-cyano-2-(méthylsulfonyl)phényl]-4-méthyl-2-oxo-3-[3-(trifluorométhyl)phényl]-3,6-dihydropyrimidine-1(2*H*)-carboxylate de benzyle
(4*S*)-4-[4-cyano-2-(éthylsulfonyl)phényl]-3,6-diméthyl-2-oxo-1-[3-(trifluorométhyl)phényl]-1,2,3,4-tétrahydropyrimidine-5-carbonitrile
(*rac*)-4-[4-cyano-2-((2-hydroxyéthyl)sulfonyl)phényl]-6-méthyl-2-oxo-1-[3-(trifluorométhyl)phényl]-1,2,3,4-tétrahydropyrimidine-5-carbonitrile
(4*S*)-4-{4-cyano-2-[phénylsulfonyl]phényl}-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-6-méthyl-3-(méthylsulfonyl)-2-oxo-1-[3-(trifluorométhyl)phényl]-1,2,3,4-tétrahydropyrimidine-5-carboxylate de 2-hydroxyéthyle
ainsi que ses sels, produits de solvatation et produits de solvatation des sels, pharmaceutiquement acceptables.

9. Composé de formule (I) selon une ou plusieurs des revendications 1 à 7, choisi dans le groupe suivait :
(4*S*)-4-[4-cyano-2-(méthylsulfonyl)phényl]-6-méthyl-2-oxo-1-[3-(trifluorométhyl)phényl]-1,2,3,4-tétrahydropyrimidine-5-carboxamide
(4*S*)-4-[4-cyano-2-(méthylsulfonyl)phényl]-6-méthyl-2-oxo-1-[3-(trifluorométhyl)phényl]-1,2,3,4-tétrahydropyrimidine-5-carbonitrile
(6*S*)-5-cyano-6-[4-cyano-2-(méthylsulfonyl)phényl]-*N*-(2-hydroxyéthyl)-4-méthyl-2-oxo-3-[3-(trifluorométhyl)-phényl]-3,6-dihydropyrimidin-1(2*H*)-carboxamide
(6*S*)-5-cyano-6-[4-cyano-2-(méthylsulfonyl)phényl]-*N',N'*-bis(2-hydroxyéthyl)-4-méthyl-2-oxo-3-[3-(trifluorométhyl)phényl]-3,6-dihydropyrimidin-1(2*H*)-carbohydrazide
2-[(6*S*)-5-cyano-6-[4-cyano-2-(méthylsulfonyl)phényl]-4-méthyl-2-oxo-3-[3-(trifluorométhyl)phényl]-3,6-dihydropyrimidin-1(2*H*)-yl]acétamide
(4*S*)-4-[4-cyano-2-(méthylsulfonyl)phényl]-6-méthyl-2-oxo-3-[2-oxo-2-(3-oxopipérazin-1-yl)éthyl]-1-[3-(trifluorométhyl)phényl]-1,2,3,4-tétrahydropyrimidin-5-carbonitrile
(4*S*)-4-[4-cyano-2-(méthylsulfonyl)phényl]-6-méthyl-3-(méthylsulfonyl)-2-oxo-1-[3-(trifluorométhyl)phényl]-1,2,3,4-tétrahydropyrimidine-5-carbonitrile
(4S)-4-[4-cyano-2-(méthylsulfonyl)phényl]-6-méthyl-3-{[2-(trifluorométhoxy)phényl]sulfonyl}-2-oxo-1-[3-(trifluorométhyl)phényl]-1,2,3,4-tétrahydropyrimidine-5-carbonitrile
(4S)-4-[4-cyano-2-(méthylsulfonyl)phényl]-6-méthyl-3-(éthylsulfonyl)-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-6-méthyl-3-(cyclopropylsulfonyl)-2-oxo-1-[3-(trifluorométhyl)-phényl]-1,2,3,4-tétrahydropyrimidine-5-carbonitrile
(4*S*)-4-[4-cyano-2-(méthylsulfonyl)phényl]-3,6-diméthyl-2-oxo-1-[3-(trifluorométhyl)phényl]-1,2,3,4-tétrahydropyrimidine-5-carbonitrile
4-{(4*S*)-6-méthyl-3-(méthylsulfonyl)-2,5-dioxo-1-[3-(trifluorométhyl)phényl]-2,3,4,5,6,7-hexahydro-1*H-*pyrrolo[3,4-d]pyrimidin-4-y1}-3-(méthylsulfonyl)-benzonitrile
(*rac*)-4-[4-cyano-2-(méthylsulfonyl)phényl]-*N*-(2-hydroxyéthyl)-6-méthyl-2,5-dioxo-1-[3-(trifluorométhyl)phényl]-1,2,4,5,6,7-hexahydro-3*H*-pyrrolo[3,4-d]-pyrimidine-3-carboxamide
(4*S*)-4-{4-cyano-2-[(S)-méthylsulfinyl]phényl}-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-3-[(4-fluorophényl)sulfonyl]-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl) phényl]-3-[(1,2-diméthyl-1*H*-imidazol-4-yl)sulfonyl]-6-méthyl-2-oxo-1-[3-(trifluorométhyl)phényl]-1,2,3,4-tétrahydropyrimidine-5-carbonitrile
(4*S*)-3-(cyanométhyl)-4-[4-cyano-2-{méthylsulfonyl)-phényl]-6-méthyl-2-oxo-1-[3-(trifluorométhyl)phényl]-1,2,3,4-tétrahydropyrimidine-5-carbonitrile
(4*S*)-4-[4-cyano-2-(méthylsulfonyl) phényl]-3-(4-cyanophényl)-6-méthyl-2-oxo-1-[3-(trifluorométhyl)-phényl]-1,2,3,4-tétrahydropyrimidine-5-carbonitrile
(4*S*)-4-[4-cyano-2-(méthylsulfonyl)phényl]-6-méthyl-2-oxo-1,3-bis[3-(trifluorométhyl)phényl]-1,2,3,4-tétrahydropyrimidine-5-carbonitrile
4-(4*S*)-3,6-diméthyl-2,5-dioxo-1-[3-(trifluorométhyl)-phényl]-2,3,4,5,6,7-hexahydro-1*H*-pyrrolo[3,4-d]-pyrimidin-4-yl-3-{méthylsulfonyl)benzonitrile
(4*S*)-4-[4-cyano-2-(éthylsulfonyl)phényl]-6-méthyl-2-oxo-1-[3-(trifluorométhyl)phényl]-1,2,3,4-tétrahydropyrimidine-5-carbonitrile
(4*S*)-4-[4-cyano-2-(éthylsulfonyl)phényl]-3,6-diméthyl-2-oxo-1-[3-(trifluorométhyl)phényl]-1,2,3,4-tétrahydropyrimidine-5-carbonitrile
(4*S*)-4-[4-cyano-2-(éthylsulfonyl)phényl]-6-méthyl-3-(méthylsulfonyl)-2-oxo-1-[3-(trifluorométhyl)phényl]-1,2,3,4-tétrahydropyrimidine-5-carbonitrile
(4*S*)-4-[4-cyano-2-(méthylsulfonyl)phényl]-6-méthyl-3-(méthylsulfonyl)-2-oxo-1-[3-(trifluorométhyl)phényl]-1,2,3,4-tétrahydropyrimidine-5-carboxylate de 2-hydroxyéthyle
ainsi que ses sels, produits de solvatation et produits de solvatation des sels, pharmaceutiquement acceptables.

10. Composé de formule (I) selon une ou plusieurs des revendications 1 à 7, ayant la structure suivants :
(4*S*)-4-[4-cyano-2-(méthylsulfonyl)phényl]-3,6-diméthyl-2-oxo-1-[3-(trifluorométhyl)phényl]-1,2,3,4-tétrahydropyrimidine-5-carbonitrile
ainsi que ses sels, produits de solvatation et produits de solvatation des sels, pharmaceutiquement acceptables.

11. Composé de formule (I) selon une ou plusieurs des revendications 1 à 7, ayant la structure suivante :
(4*S*)-4-[4-cyano-2-(méthylsulfonyl)phényl]-6-méthyl-3-(méthylsulfonyl)-2-oxo-1-[3-(trifluorométhyl)phényl]-1,2,3,4-tétrahydropyrimidine-5-carbonitrile
ainsi que ses sels, produits de solvatation et produits de solvatation des sels, pharmaceutiquement acceptables.

12. Composé de formule (I) selon une ou plusieurs des revendications 1 à 7, ayant la structure suivante :
(4*S*)-4-[4-cyano-2-(méthylsulfonyl)phényl]-6-méthyl-2-oxo-1-[3-(trifluorométhyl)phényl]-1,2,3,4-tétrahydropyrimidine-5-carbonitrile
ainsi que ses sels, produits de solvatation et produits de solvatation des sels, pharmaceutiquement acceptables.

13. Procédé pour la préparation des composés de formule (I), tel que défini dans les revendications 1 à 12, **caractérisé en ce qu'**on fait réagir un composé de formule (II) 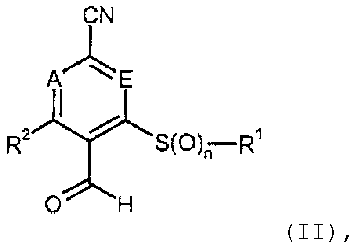 dans laquelle A, E, n, R¹ et R² ont les significations indiquées dans les revendications 1 à 12,
en présence d'un acide ou d'un anhydride d'acide, dans une réaction à 3 composants en un seul récipient ou séquentiellement, avec un composé de formule (III) 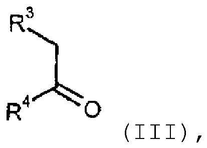 dans laquelle R³ et R⁴ ont les significations indiquées dans les revendications 1 à 8,
et un composé de formule (IV) 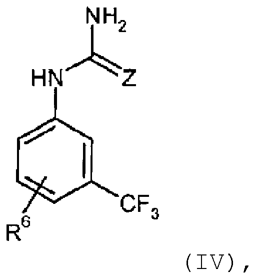 dans laquelle Z et R⁶ ont les significations indiquées dans les revendications 1 à 12, pour obtenir un composé de formule (I-A) 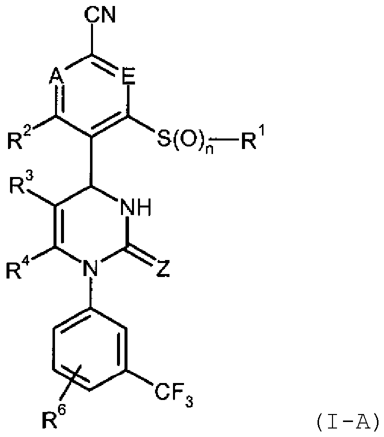 dans laquelle A, E, Z, n, R¹, R², R³, R⁴ et R⁶ ont chacun les significations indiquées plus haut,
et on fait réagir ce dernier, dans le cas où R⁵ dans la formule (I) ne représente pas un atome d'hydrogène, en présence d'une base, avec un composé de formule (V)
R^{5A}-X (V)
dans laquelle
R^{5A} a la signification de R⁵ indiquée dans les revendications 1 à 12, mais ne représente pas un atome d'hydrogène,
et X représente un groupe partant, comme par exemple un atome d'halogène, le groupe mésylate, tosylate ou triflate,
pour obtenir un composé de formule (I-B) 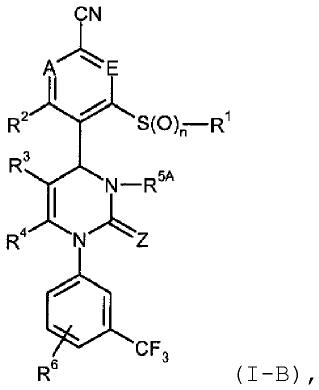 dans laquelle A, E, Z, n, R¹, R², R³, R⁴ R^{5A} et R⁶ ont chacun les significations indiquées plus haut,
et éventuellement, selon des méthodes connues de l'homme de métier, on sépare en leurs énantiomères et/ou diastéréoisomères et/ou convertit en leurs produits de solvatation, sels et/ou produits de solvatation des sels, à l'aide (i) des solvants correspondants et/ou (ii) bases correspondantes, les composés de formule (I-A) ou (I-B) ainsi obtenus.

14. Composé, tel que défini dans une ou plusieurs des revendications 1 à 12, pour le traitement et/ou la prévention de maladies.

15. Composé, tel que défini dans une ou plusieurs des revendications 1 à 12, pour utilisation dans un procédé visant au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience d'antitrypsine-alpha-1 (AATD) et de la fibrose kystique (CF).

16. Composé, tel que défini dans une ou plusieurs des revendications 1 à 12, pour utilisation dans un procédé visant au traitement et/ou à la prévention de la bronchiectasie.

17. Utilisation d'un composé, tel que défini dans une ou plusieurs des revendications 1 à 12, pour la fabrication d'un médicament destiné au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience d'antitrypsine-alpha-1 (AATD) et de la fibrose kystique (CF).

18. Médicament contenant un composé tel que défini dans une ou plusieurs des revendications 1 à 12, en association avec un ou plusieurs adjuvants inertes, non toxiques, pharmaceutiquement appropriés.

19. Médicament contenant un composé tel que défini dans une ou plusieurs des revendications 1 à 12, en association avec une ou plusieurs autres substances actives choisies dans le groupe constitué par des inhibiteurs de kinase, des inhibiteurs de métalloprotéases matricielles, des stimulants et des activateurs de la guanylate cyclase soluble, des analogues de prostacycline, des antagonistes des récepteurs de l'endothéline, des inhibiteurs de phosphodiestérase, des agonistes des récepteurs bêta-adrénergiques, des anticholinergiques et des glucocorticoïdes.

20. Médicament selon la revendication 18 ou 19, destiné au traitement et/ou à la prévention de l'hypertonie artérielle pulmonaire (PAH), et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience en antitrypsine-alpha-1 (AATD) et de la fibrose kystique (CF).

21. Médicament selon la revendication 18 ou 19, destiné au traitement et/ou à la prévention de la bronchiectasie.

22. Utilisation d'un médicament, tel que défini dans une ou plusieurs des revendications 18 à 20, pour la fabrication d'un médicament destiné au traitement et/ou à la prévention de l'hypertonie artérielle pulmonaire (PAH) et d'autres formes de l'hypertonie pulmonaire (PH), de pneumopathies chroniques obstructives (COPD), de la lésion pulmonaire aiguë (ALI), du syndrome de détresse respiratoire aigu (ARDS), de l'emphysème pulmonaire, de la déficience en antitrypsine-alpha-1 (AATD) et de la fibrose kystique (CF).
